# EUROPEAN PATENT APPLICATION

(11) **EP 1 970 375 A1**
(43) Date of publication of application: **17.09.2008**
(21) Application number: 06833566.0
(22) Date of filing: 29.11.2006
(51) Int. Cl.: C07D 401/14, A61K 31/496, A61K 31/501, A61K 31/506, A61K 31/5377, A61K 31/541, A61K 31/55, A61P 1/04, C07D 403/12, C07D 403/14, C07D 405/14, C07D 409/14, C07D 413/14, C07D 417/12, C07D 491/113

(54) **ARYLMETHYLENE UREA DERIVATIVE AND USE THEREOF**

(30) Priority: 29.11.2005 JP 2005344180; 01.02.2006 JP 2006024952; 17.04.2006 JP 2006112955
(71) Applicant: TORAY INDUSTRIES, INC., Tokyo 103-8666 (JP)
(72) Inventor: OHNO, Michihiro, Yokohama-shi, Kanagawa, 245-0061 (JP); INOUE, Hideki, Kamakura-shi, Kanagawa, 248-0036 (JP); HAYASHI, Shinnosuke, Ota-ku, Tokyo, 143-0027 (JP); KAINO, Mie, Kanagawa, 251-0052 (JP); HARA, Sunao, Kamakura-shi, Kanagawa, 248-0031 (JP); YOSHIKAWA, Satoru, Yokohama-shi, Kanagawa, 221-0834 (JP)
(74) Representative: Kador & Partner
(86) International application number: PCT/JP2006/323762
(87) International publication number: WO 2007/063868

(57) **Abstract**

This invention relates to a pharmaceutical comprising as an effective ingredient an arylmethylene urea exemplified by the following formula: 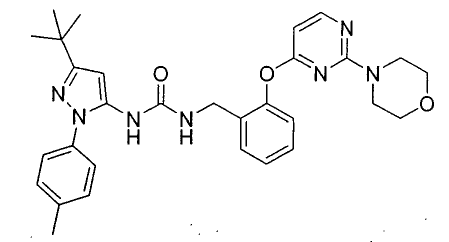 or a pharmaceutically acceptable salt thereof. The arylmethylene urea and the pharmaceutically acceptable salts thereof are useful for therapy or prophylaxis of inflammatory bowel disease and overactive bladder.

## Description

### Technical Field

The present invention relates to an arylmethylene urea derivative and medical use thereof, especially a therapeutic or prophylactic agent.

### Background Art

Inflammatory bowel disease is a generic term of intractable bowel diseases whose etiologies are still unknown, and includes mainly ulcerative colitis and Crohn's disease. Ulcerative colitis accompanies mainly erosion and ulcer due to injury of mucosa of colon. Its lesion is observed mainly in submucosa. On the other hand, Crohn's disease is sudden chronic enteritis with unknown etiology that may take place in any region of the entire gastrointestinal tract. This disease shows inflammation with deep ulcer and perforation, and is histologically characterized by noncaseating granuloma. It may occur in the entire gastrointestinal tract, from oral cavity to anus. Causes of the both diseases may include immunological abnormalities, genetic abnormalities, environmental factors, disorders of blood vessels and lymph vessels of gastrointestinal tract and the like, and multiple factors are intricately intertwined to cause their pathology, with their fundamental causes being not yet known.

Clinical symptoms represented by ulcerative colitis are mucous and bloody stool, bloody stool, diarrhea, abdominal pain, anorexia, weight loss due to impaired absorption, general malaise, anemia, tachycardia and the like. Clinical symptoms represented by Crohn's disease are diarrhea, abdominal pain, fever, general malaise, melena, weight loss, anemia, ileus symptoms, nausea, peritonitis and the like.

Pharmacotherapy of ulcerative colitis generally employs salazosulfapyridine and 5-aminosalicylic acid. For moderate to severe patients, adrenocortical steroids mainly prednisolone, and immunosuppressive agents such as azathioprine are employed. In addition to pharmacotherapy, apheresis or surgical therapy is applicable. Therapy of Crohn's disease is carried out by the combination of nutritional therapy, pharmacotherapy and surgical treatment. In the pharmacotherapy of Crohn's disease, 5-aminosalicylic acid preparation, adrenocortical steroid and immunosuppressive agents are employed. However, these drugs and pharmacotherapies are not sufficient in terms of potency and side effects, and additional drugs for improvement of therapeutic effects are demanded.

In recent years, involvement of inflammatory cytokines to development of inflammatory bowel diseases is drawing attention. Therefore, researches on drugs that inhibit production of inflammatory cytokines (TNFα, IL-1, IL-6, IL-8, IL-12, IL-18 and the like) or neutralize cytokines are actively carried out. Among inflammatory cytokines, therapies by biologicals targeting tumor necrosis factor α (TNFα) have been already practically applied, and anti-TNFα antibody therapy and soluble TNFα receptor therapy for Crohn's disease have been shown to be effective for amelioration of lesions and decreasing the amount of steroids in severe patients. However, use of these biologicals requires careful observation since they show side effects such as infection. In addition to TNFα, clinical tests on biologicals targeting IL-6, IL-2 and IL-12 are being attempted, but their effects and safety remain to be proved.

Thus, known therapeutic drugs for inflammatory bowel diseases are not sufficient as a pharmaceutical, and development of an orally administrable, excellent drug for improvement in effects of therapy or prophylaxis is demanded, given that mechanisms of onset of inflammatory bowel diseases are not sufficiently proven at present.

On the other hand, along with the increase of elderly population in recent years, patients suffering from overactive bladder are rapidly increasing, which grows the pharmacotherapy needs. Overactive bladder is a pathological condition defined in International Continence Society in 2002 as "meaning a condition of having urinary urgency, which usually accompanies urinary frequency and nocturia, regardless of existence of urge urinary incontinence". The causes are various, and are roughly classified into neurogenic overactive bladder and non-neurogenic overactive bladder.

Neurogenic overactive bladder is caused in cases where a disorder exists in innervation of the lower urinary tract, whose causes include cerebrovascular disease, brain tumor, brain injury, encephalitis, brain tumor, normal pressure hydrocephalus, dementia, Parkinson's disease, striato-nigral degeneration, progressive supranuclear palsy, olivo-ponto-cerebellar atrophy, Shy-Drager syndrome, spinal cord injury, vascular disease of spinal cord and brain, spinal cord tumor, myelitis, cervical cord compression disorder, syringomyelia, multiple sclerosis, spina bifida, myelomeningocele, Tetherrd cord syndrome, myelopathy and the like.

Non-neurogenic overactive bladder is overactive bladder which does not accompany clinically evident neuropathy, whose causes include obstructive disease of the lower urinary tract, aging, disorder of pelvic floor muscles and the like, but is mostly idiopathy whose cause is not identifiable.

Existing therapeutic drugs for overactive bladder are exclusively anticholinergic drugs, whose effects are based on reduction of detrusor contraction power by inhibiting activation of muscarine 3 (M3) receptors which exists in detrusor. However, by inhibiting muscarine receptors in other organs at the same time, side effects in multiple organs, such as dry mouth, constipation, dizziness, tachycardia and the like, accompany therewith. In addition, these drugs also inhibit contraction of detrusor itself on micturition, which causes side effects such as the increase in residual urine volume. Thus, known therapeutic drugs for overactive bladder are not sufficient as pharmaceuticals, and development of a more satisfactory new drug is demanded.

As a therapeutic drug for inflammatory bowel disease, the compounds of the following formula:

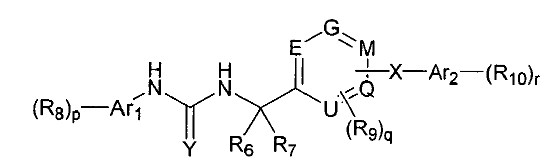

[wherein Ar₁ and Ar₂ each represents an aromatic hydrocarbon (6 to 14 carbon atoms) or an aromatic heterocycle; R₆ to R₁₀ each represents hydrogen atom, alkyl, alkenyl, alkylenecycloalkyl, alkoxy, alkoxyalkyl, saturated heterocycle, CH₂-halogen, CH(halogen)₂, C(halogen)₃, NO₂ (CH₂)ₙCN or the like; E, G, M, Q and U each represents C or N; p and r each represents an integer of 0 to 5; q represents an integer of 0 to 4; X represents a bond, carbon chain or the like; and Y represents O, S, NH, N-alkyl or the like]
and salts thereof have been disclosed in Patent Literature 1.

A part of the present invention is included literally in the scope of Patent Literature 1. However, in Patent Literature 1, an example or embodiment wherein the position substituted by X is ortho position is not described, and the section describing preferred modes of the invention merely describes that substitution at meta or para position is preferred, and is totally silent about the substitution at ortho position. Further, pyridine is the only structure of Ar₂ described in the embodiments and examples, and other structures are not concretely described.

Further, although not a therapeutic drug for inflammatory bowel disease or not a therapeutic drug for overactive bladder, as analogous compounds, Patent Literature 2 discloses the compounds of the following formula as glucokinase inhibitors:

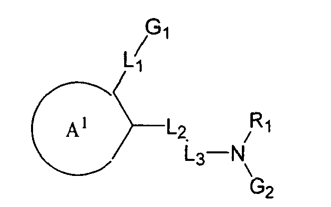

[wherein A¹ represents arylene, fused cycloalkylarylene, fused heterocyclylarylene, fused cycloalkylheteroarylene or fused heterocyclylheteroarylene; L₁ represents -D-C₁₋₆-alkylene-E-, -D-C₂₋₆-alkenylene-E-, -D-C₂₋₆-alkynylene-E-, -D-cycloalkylene-E-, -D-heterocyclene-E-, -O-, -S-, -S(O)-, -S(O)₂ -, -C(O)-, -NH-, -N(alkyl)-, -C(=N-OH)- or -C(=N-O-alkyl)-; D and E each represents -O- or -S-; G₁ represents C₁₋₆ alkyl, C₃₋₁₀ cycloalkyl, C₃₋₁₀ cycloalkyl-C₁₋₆ alkylene-, C₂₋₆alkenyl, C₂₋₆ alkynyl, C₃₋₁₀ cycloalkylaryl, heteroaryl, heterocyclyl, fused cycloalkylheteroaryl, fused heterocyclylaryl, fused arylheterocyclyl or fused cycloalkylaryl; L₂ represents C₁₋₆ alkylene, C₂₋₆ alkenylene, C₂₋₆ alkynylene-NR²⁰-, - C₁₋₆-alkylene-NR²⁰ -, -C₂₋₆-alkenylene-NR²⁰ - or -C₂₋₆-alkyleneNR²⁰ -; L₃ represents -C(O)-, -C(O)-C(O)-, -C(O)CH₂ C(O) or -S(O)₂ -; R²⁰ represents a hydrogen atom, C₁₋₆ alkyl, C₂₋₆ alkenyl, C₂₋₆ alkynyl, cycloalkyl-alkylene, aryl-alkylene or heteroarylalkylene; R₁ represents C₁₋₆ alkyl, C₂₋₆ alkenyl, C₂₋₆ alkynyl, cycloalkylalkylene, arylalkylene, heteroarylalkylene or the like; and G₂ represents heteroaryl, fused heterocyclylheteroaryl, fused cycloalkylheteroaryl or the like].
A part of the present invention is included literally in the scope of Patent Literature 2. However, no urea derivatives having a heterocyclic substituent are concretely described.

Patent Literature 1: WO 04/037789
Patent Literature 2: WO 04/002481

### Disclosure of the Invention

### Problems Which the Invention Tries to Solve

In view of the fact that a pharmaceutical useful for the therapy or prophylaxis of inflammatory bowel disease and overactive bladder is strongly demanded, it is an object of the present invention to provide a small molecule useful as a pharmaceutical for the therapy or prophylaxis of inflammatory bowel disease and overactive bladder.

### Means for Solving the Problems

The present inventors intensively investigated to discover that the arylmethylene urea derivatives of the Formula (I) and pharmaceutically acceptable salts thereof are small molecules useful as a pharmaceutical for the therapy or prophylaxis of inflammatory bowel disease and overactive bladder, thereby completing the present invention.

That is, the present invention provides an arylmethylene urea derivative of the Formula (I) or a pharmaceutically acceptable salt thereof:

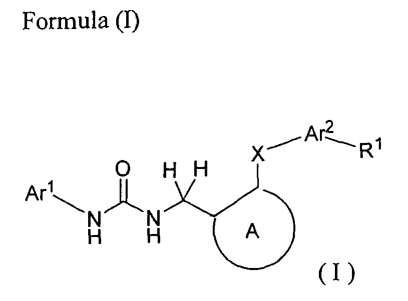

[wherein
A represents the Formula (II), (III), (IV) or (V):

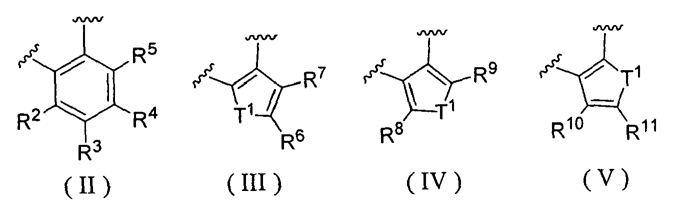

(wherein
R² to R¹¹ each independently represents hydrogen, cyano, hydroxy, amino, mercapto, nitro, fluoro, chloro, bromo, iodo, phenyl, benzyl, carboxy, carbamoyl, thiocarbamoyl, formyl, C₁-C₈ alkyl (which may be linear, branched or partially or entirely cyclized, and which is optionally substituted with a fluorine(s), chlorine(s), bromine(s) and/or iodine(s). The term "C₁-C₈ alkyl" means, in the present Description and Claims, C₁-C₈ alkyl which may be linear, branched or partially or entirely cyclized, and which is optionally substituted with a fluorine(s), chlorine(s), bromine(s) and/or iodine(s) unless otherwise specified), C₁-C₈ hydroxyalkyl (which may be linear, branched or partially cyclized. The term "C₁-C₈ hydroxyalkyl" means, in the present Description and Claims, C₁-C₈ hydroxyalkyl which may be linear, branched or partially cyclized unless otherwise specified), or C₁-C₈ alkoxy (which may be linear, branched or partially cyclized. The term "C₁-C₈ alkoxy" means, in the present Description and Claims, C₁-C₈ alkoxy which may be linear, branched or partially cyclized unless otherwise specified);
T¹ represents -O-, -S- or -NR¹²-
(wherein R¹² represents hydrogen, C₁-C₈ alkyl, C₁-C₈ hydroxyalkyl, C₁-C₈ alkoxy or C₁-C₈ alkylsulfanyl (which may be linear, branched or partially cyclized. The term "C₁-C₈ alkylsulfanyl" means, in the present Description and Claims, C₁-C₈ alkylsulfanyl which may be linear, branched or partially cyclized unless otherwise specified);
X represents -O-, -S-, -SO-, -SO₂- , -NR¹³ - or -CH₂ -
(wherein R¹³ represents hydrogen, C₁-C₈ alkyl, C₁-C₈ hydroxyalkyl or C₁-C₈ alkoxy);
Ar¹ represents pyrazole, thiophene, furan, pyrrole, imidazole, oxazole, thiazole, oxadiazole or thiadiazole
(wherein each of these optionally has one or more substituents independently selected from the group consisting of the following substituents:
cyano, hydroxy, amino, mercapto, nitro, fluoro, chloro, bromo, iodo, phenyl, benzyl, carboxy, carbamoyl, thiocarbamoyl, formyl, C₁-C₈ alkyl, C₂-C₈ alkenyl (which may be linear, branched or partially cyclized. The term "C₂-C₈ alkenyl" means, in the present Description and Claims, C₂-C₈ alkenyl which may be linear, branched or partially cyclized unless otherwise specified), C₂-C₈ alkynyl (which may be linear, branched or partially cyclized. The term "C₂-C₈ alkynyl" means, in the present Description and Claims, C₂-C₈ alkynyl which may be linear, branched or partially cyclized unless otherwise specified), C₁-C₈ hydroxyalkyl, C₁-C₈ alkoxy, C₁-C₁₆ alkylamino (which means an amino group substituted with one or two independently selected C₁-C₈ alkyls, which alkyl may be linear, branched or partially or entirely cyclized. The term "C₁-C₁₆ alkylamino" means, in the present Description and Claims, an amino group substituted with one or two independently selected C₁-C₈ alkyls, which alkyl may be linear, branched or partially or entirely cyclized unless otherwise specified), C₁-C₈ alkylsulfanyl and 5- to 10-membered aromatic heterocycle (which means a 5- to 10-membered monocyclic or bicyclic heterocyclic aryl having 1 to 5 hetero atoms of 1 to 3 types selected from nitrogen atom, sulfur atom and oxygen atom in addition to carbon atoms. The term "5- to 10-membered aromatic heterocycle" means, in the present Description and Claims, a 5- to 10-membered monocyclic or bicyclic heterocyclic aryl having 1 to 5 hetero atoms of 1 to 3 types selected from nitrogen atom, sulfur atom and oxygen atom in addition to carbon atoms unless otherwise specified),
(wherein each of these substituents optionally further has one or more substituents independently selected from the following substituents:
cyano, hydroxy, amino, mercapto, nitro, fluoro, chloro, bromo, iodo, phenyl, benzyl, carboxy, benzyloxy, carbamoyl, thiocarbamoyl, formyl, C₁-C₈ alkyl, C₂-C₈ alkenyl, C₂-C₈ alkynyl, C₁-C₈ hydroxyalkyl, C₁-C₈ alkoxy, C₁-C₈ alkoxycarbonyl, C₁-C₈ hydroxyalkyloxy (which may be linear, branched or partially cyclized. The term "C₁-C₈ hydroxyalkyloxy" means, in the present Description and Claims, a C₁-C₈ hydroxyalkyloxy which may be linear, branched or partially cyclized unless otherwise specified), C₁-C₁₆ alkylamino, C₁-C₈ alkylsulfanyl, C₁-C₈ alkylsulfonyl (which may be linear, branched or partially cyclized. The term "C₁-C₈ alkylsulfonyl" means, in the present Description and Claims, a C₁-C₈ alkylsulfonyl which may be linear, branched or partially cyclized unless otherwise specified), trifluoromethanesulfonyl, sulfonamide alkylated with C₁-C₈ alkyl and 5- to 10-membered aromatic heterocycle);
   Ar² represents the Formula (VI) or Formula (VII):

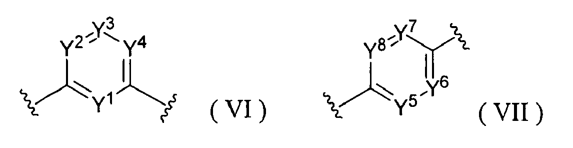

[wherein Y¹ to Y⁸ each independently represents N or CR¹⁴ with the proviso that at least two of Y¹ to Y⁴ are N and that at least two of Y⁵ to Y⁸ are N
(wherein R¹⁴ is independently selected from the following:
hydrogen, cyano, hydroxy, amino, mercapto, nitro, fluoro, chloro, bromo, iodo, phenyl, benzyl, carboxy, carbamoyl, thiocarbamoyl, formyl, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₈ alkylsulfanyl and 5- to 10-membered aromatic heterocycle
(wherein each of these substituents optionally further has one or more substituents independently selected from the following:
cyano, hydroxy, amino, mercapto, nitro, fluoro, chloro, bromo, iodo, phenyl, benzyl, carboxy, carbamoyl, thiocarbamoyl, formyl, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₈ alkylsulfanyl and 5- to 10-membered aromatic heterocycle)]

R¹ represents
hydrogen, cyano, hydroxy, amino, mercapto, nitro, fluoro, chloro, bromo, iodo, phenyl, benzyl, carboxy, carbamoyl, thiocarbamoyl, formyl, formylamino, aminosulfonyl, phenoxy, indan, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₁₆ hydroxyalkylamino (amino group substituted with one or two hydroxyalkyls selected independently), C₁-C₈ alkylsulfanyl, C₁-C₈ alkylsulfinyl (which may be linear, branched or partially cyclized. The term "C₁-C₈ alkylsulfinyl" means, in the present Description and Claims, a C₁-C₈ alkylsulfinyl which may be linear, branched or partially cyclized unless otherwise specified), C₁-C₈ alkylsulfonyl, C₆-C₁₄ arylsulfanyl, C₆-C₁₄ arylsulfinyl, C₆-C₁₄ arylsulfonyl, amide alkylated with C₁-C₈ alkyl, sulfonamide alkylated with C₁-C₈ alkyl, carbamate alkylated with C₁-C₈ alkyl, urea alkylated with C₁-C₈ alkyl, carboxylated C₁-C₈ alkyl, C₁-C₈ alkoxycarbonyl, alkylcarbonyl containing C₁-C₈ alkyl, 5- to 10-membered aromatic heterocycle, 5- to 10-membered monocyclic heterocycle (which means a monocyclic heterocyclic aryl having 1 to 5 hetero atoms of 1 to 3 types selected from nitrogen atom, sulfur atom and oxygen atom in addition to carbon atoms, or the monocyclic heterocyclic aryl which is partially or entirely saturated and which optionally has a carbonyl group(s) in a part(s) of its ring. The term "5- to 10-membered monocyclic heterocycle" means, in the present Description and Claims, a monocyclic heterocyclic aryl having 1 to 5 hetero atoms of 1 to 3 types selected from nitrogen atom, sulfur atom and oxygen atom, or the monocyclic heterocyclic aryl which is partially or entirely saturated and which optionally has a carbonyl group(s) in a part(s) of its ring unless otherwise specified), or 5- to 10-membered bicyclic heterocycle (which means a bicyclic heterocyclic aryl having 1 to 5 hetero atoms of 1 to 3 types selected from nitrogen atom, sulfur atom and oxygen atom in addition to carbon atoms, or the bicyclic heterocyclic aryl which is partially or entirely saturated. The term "5- to 10-membered bicyclic heterocycle" means, in the present Description and Claims, a bicyclic heterocyclic aryl having 1 to 5 hetero atoms of 1 to 3 types selected from nitrogen atom, sulfur atom and oxygen atom in addition to carbon atoms, or the bicyclic heterocyclic aryl which is partially or entirely saturated unless otherwise specified),
(wherein each of these substituents optionally has one or more substituents independently selected from the following:
cyano, hydroxy, amino, mercapto, nitro, fluoro, chloro, bromo, iodo, phenyl, benzyl, carboxy, carbamoyl, thiocarbamoyl, formyl, formylamino, aminosulfonyl, phenoxy, indan, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₁₆ hydroxyalkylamino, C₁-C₈ alkylsulfanyl, C₁-C₈ alkylsulfinyl, C₁-C₈ alkylsulfonyl, C₆-C₁₄ arylsulfanyl, C₆-C₁₄ arylsulfinyl, C₆-C₁₄ arylsulfonyl, amide alkylated with C₁-C₈ alkyl, sulfonamide alkylated with C₁-C₈ alkyl, carbamate alkylated with C₁-C₈ alkyl, urea alkylated with C₁-C₈ alkyl, carboxylated C₁-C₈ alkyl, C₁-C₈ alkoxycarbonyl, C₁-C₈ alkylcarbonyl, 5- to 10-membered aromatic heterocycle, 5- to 10-membered monocyclic heterocycle, 5- to 10-membered bicyclic heterocycle, C₁-C₈ alkylene (which may be linear, branched or partially cyclized. The term "C₁-C₈ alkylene" means, in the present Description and Claims, a C₁-C₈ alkylene which may be linear, branched or partially cyclized unless otherwise specified), C₂-C₈ alkenylene (which may be linear, branched or partially cyclized, the term "C₁-C₈ alkenylene" meaning, in the present Description and Claims, a C₁-C₈ alkenylene which may be linear, branched or partially cyclized unless otherwise specified) and C₁-C₈ alkylenedioxy (which may be linear, branched or partially cyclized. The term "C₁-C₈ alkylenedioxy" means, in the present Description and Claims, a C₁-C₈ alkylenedioxy which may be linear, branched or partially cyclized unless otherwise specified)
(wherein each of these substituents optionally further has one or more substituents independently selected from the following:
cyano, hydroxy, amino, mercapto, nitro, fluoro, chloro, bromo, iodo, phenyl, benzyl, carboxy, carbamoyl, thiocarbamoyl, formyl, formylamino, aminosulfonyl, indan, C₁-C₈ alkyl, C₁-C₈ hydroxyalkyl, C₁-C₈ alkoxy, C₁-C₁₆ alkylamino, C₁-C₁₆ hydroxyalkylamino, amide alkylated with C₁-C₈ alkyl, carbamate alkylated with C₁-C₈ alkyl, carboxylated C₁-C₈ alkyl, C₁-C₈ alkoxycarbonyl, C₁-C₈ alkylcarbonyl, 5- to 10-membered monocyclic heterocycle, 5- to 10-membered bicyclic heterocycle, 5- to 10-membered aromatic heterocycle and C₁-C₈ alkylenedioxy)].
The present invention also provides a pharmaceutical comprising the above-described arylmethylene urea derivative or the pharmaceutically acceptable salt thereof according to the present invention. The present invention further provides a therapeutic or prophylactic agent for inflammatory bowel disease or overactive bladder, comprising the above-described arylmethylene urea derivative or the pharmaceutically acceptable salt thereof according to the present invention.

### Effects of the Invention

The arylmethylene urea derivatives disclosed by the present invention have higher therapeutic or prophylactic effect for inflammatory bowel disease and overactive bladder than the conventional small molecules.

### Brief Description of the Drawings

Fig. 1 shows the effect of the compound (Example 2) of the present invention, which is shown in terms of the ratio of change in the number of rhythmic bladder contractions (an index of the rhythmic bladder contraction reaction) (intravenous administration).
Fig. 2 shows the effect of the compound (Example 9) of the present invention, which is shown in terms of the ratio of change in the number of rhythmic bladder contractions (an index of the rhythmic bladder contraction reaction) (intravenous administration).
Fig. 3 shows the effects of the compound (Example 2) of the present invention on the frequency of urination (A) and detrusor contraction pressure (B) in cyclophosphamide-induced cystitis rats (intravenous administration).
Fig. 4 shows the effects of the compound (Example 81) of the present invention on the frequency of urination (A) and detrusor contraction pressure (B) in cyclophosphamide-induced cystitis rats (intravenous administration).

### Best Mode for Carrying out the Invention

In the present Description and Claims, in chemical formulae representing a substituent group, the wave line which contacts the tip of a single line representing a single bond and which is perpendicular to the single line means that the substituent group and the other structure in the formula, the formula including the substituent group, are bound through the single bond which the wavy line contacts. For example, among the two wave lines existing in Formula (II) listed as "A" in Formula (I), the single bond contacting the left wavy line is the single bond binding the carbon atom bound to the urea structure in Formula (I), and the single bond contacting the right wavy line is the single bond binding to X. Similarly, among the two wavy lines existing in Formula (VI) listed as "Ar²" in Formula (I), the single bond contacting the left wavy line is the single bond binding to X, and the single bond contacting the right wavy line is the single bond binding to R¹.

Unless otherwise specified, the present invention includes all isomers. For example, alkyl, alkoxy and alkylene include linear and branched ones, respectively. Further, all of the isomers related to double bonds, rings, fused rings (E-isomers, Z-isomers, cis-isomers and trans-isomers), isomers due to existence of an asymmetric carbon (R-isomers, S-isomers, α-isomers, β-isomers, enantiomers and diastereomers), optical isomers having an optical rotation (D-isomers, L-isomers, d-isomers and 1-isomers), polarity isomers (high polarity isomers and low polarity isomers) in chromatographic separation, equilibrium compounds, mixtures thereof at an optional ratio and racemic mixtures are all included in the present invention.

In the present invention, C₁-C₈ alkyl includes those which are linear or branched, and those which are partially or entirely cyclized. A part thereof may be substituted by a fluorine(s), chlorine(s), bromine(s) and/or iodine(s). Examples thereof include methyl, ethyl, propyl, isopropyl, n-butyl, sec-butyl, isobutyl, t-butyl, pentyl, t-pentyl, neopentyl, hexyl, heptyl, octyl, cyclopropyl, cyclobutyl, cyclopentyl, cyclohexyl, cycloheptyl, cyclooctyl, cyclopropylmethyl, cyclopropylethyl, cyclohexylmethyl, cyclohexylethyl, fluoromethyl, difluoromethyl, trifluoromethyl, trifluoroethyl, trichloromethyl, trichloroethyl, and isomers thereof and the like.

C₂-C₈ alkenyl includes those which are linear or branched, and those which are partially or entirely cyclized. Examples thereof include ethenyl, propenyl, butenyl, butadienyl, pentenyl, pentadienyl, hexenyl, hexadienyl, heptenyl, heptadienyl, octenyl, octadienyl, cyclopentenyl, cyclohexenyl, cycloheptenyl, cyclooctenyl, cyclopentadienyl, cyclohexadienyl, cycloheptadienyl, cyclooctadienyl and the like.

C₂-C₈ alkynyl includes those which are linear or branched, and those which are partially or entirely cyclized. Examples thereof include ethynyl, propynyl, butynyl, butadiynyl, pentynyl, pentadiynyl, hexynyl, hexadiynyl, heptynyl, heptadiynyl, octynyl, octadiynyl, and isomers thereof and the like.

C₁-C₈ alkylene includes those which are linear, branched or partially cyclized. Examples thereof include methylene, ethylene, propylene, dimethylethylene, tetramethylethylene, and isomers thereof and the like.

C₂-C₈ alkenylene includes those which are linear, branched or partially cyclized. Examples thereof include ethenylene, propenylene, butenylene, and isomers thereof and the like.

C₁-C₈ alkylenedioxy includes those which are linear, branched or partially cyclized. Examples thereof include methylenedioxy, ethylenedioxy, propylenedioxy, dimethylethylenedioxy, tetramethylethylenedioxy, cyclopentan-1,1-dioxy, cyclohexan-1,1-dioxy, and isomers thereof and the like.

C₁-C₈ hydroxyalkyl includes those which are linear, branched or partially cyclized. Examples thereof include hydroxymethyl, hydroxyethyl, hydroxypropyl, hydroxybutyl, hydroxypentyl, hydroxyhexyl, hydroxyheptyl, hydroxyoctyl, hydroxycyclopropyl, hydroxycyclobutyl, hydroxycyclopentyl, hydroxycyclohexyl, hydroxycycloheptyl, hydroxycyclooctyl, hydroxycyclopropylmethyl, hydroxycyclopropylethyl, hydroxycyclohexylmethyl, hydroxycyclohexylethyl, and isomers thereof and the like.

C₁-C₈ alkoxy includes those which are linear or branched, and those which are partially cyclized. Examples thereof include methoxy, ethoxy, propoxy, butoxy, pentyloxy, hexyloxy, heptyloxy, octyloxy, cyclopropoxy, cyclobutoxy, cyclopentyloxy, cyclohexyloxy, cycloheptyloxy, cyclooctyloxy, cyclopropylmethoxy, cyclopropylethoxy, cyclohexylmethoxy, and isomers thereof, and the like.

C₁-C₁₆ alkylamino means an amino group substituted by one or two independently selected C₁-C₈ alkyl (having the same meanings as described above), wherein alkyl includes those which are linear or branched, and those which are partially or entirely cyclized. Examples thereof include methylamino, ethylamino, propylamino, isopropylamino, n-butylamino, sec-butylamino, isobutylamino, t-butylamino, pentylamino, hexylamino, heptylamino, octylamino, cyclopropylamino, cyclobutylamino, cyclopentylamino, cyclohexylamino, cycloheptylamino, cyclooctylamino, (cyclopropylmethyl)amino, (cyclopropylethyl)amino, (cyclohexylmethyl)amino, (cyclohexylethyl)amino, dimethylamino, diethylamino, dipropylamino, dibutylamino, methyl-ethyl-amino, methyl-propyl-amino, ethylpropyl-amino, cyclopropyl-methyl-amino, cyclopropyl-ethyl-amino, cyclohexylmethyl-amino, cyclohexyl-ethyl-amino, aziridine, trifluoroethylamino, methyl-t-butyl-amino, isopropyl-t-butyl-amino, ethyl-isopropyl-amino, and isomers thereof and the like.

C₁-C₁₆ hydroxyalkylamino means an amino group substituted by one or two independently selected C₁-C₈ hydroxyalkyl (having the same meanings as described above), wherein hydroxyalkyl includes those which are linear or branched, and those which are partially or entirely cyclized. Examples thereof include hydroxymethylamino, hydroxyethylamino, hydroxypropylamino, hydroxybutylamino, hydroxypentylamino, hydroxyhexylamino, hydroxyheptylamino, hydroxyoctylamino, hydroxycyclopropylamino, hydroxycyclobutylamino, hydroxycyclopentylamino, hydroxycyclohexylamino, hydroxycycloheptylamino, hydroxycyclooctylamino, (hydroxycyclopropylmethyl)amino, (hydroxycyclopropylethyl)amino, (hydroxycyclohexylmethyl)amino, (hydroxycyclohexylethyl)amino, di(hydroxymethyl)amino, di(hydroxyethyl)amino, di(hydroxypropyl)amino, di(hydroxybutyl)amino, and isomers thereof and the like.

C₁-C₈ alkylsulfanyl includes those which are linear or branched, and those which are partially cyclized. Examples thereof include methylsulfanyl, ethylsulfanyl, propylsulfanyl, butylsulfanyl, pentylsulfanyl, hexylsulfanyl, heptylsulfanyl, octylsulfanyl, cyclopropylsulfanyl, cyclobutylsulfanyl, cyclopentylsulfanyl, cyclohexylsulfanyl, cycloheptylsulfanyl, cyclooctylsulfanyl, cyclopropylmethylsulfanyl, cyclopropylethylsulfanyl, cyclohexylmethylsulfanyl, cyclohexylethylsulfanyl, and isomers thereof and the like.

C₁-C₈ alkylsulfinyl includes those which are linear or branched, and those which are partially cyclized. Examples thereof include methylsulfinyl, ethylsulfinyl, propylsulfinyl, butylsulfinyl, pentylsulfinyl, hexylsulfinyl, heptylsulfinyl, octylsulfinyl, cyclopropylsulfinyl, cyclobutylsulfinyl, cyclopentylsulfinyl, cyclohexylsulfinyl, cycloheptylsulfinyl, cyclooctylsulfinyl, cyclopropylmethylsulfinyl, cyclopropylethylsulfinyl, cyclohexylmethylsulfinyl, cyclohexylethylsulfinyl, and isomers thereof and the like.

C₁-C₈ alkylsulfonyl includes those which are linear or branched, and those which are partially cyclized. Examples thereof include methylsulfonyl, ethylsulfonyl, propylsulfonyl, butylsulfonyl, pentylsulfonyl, hexylsulfonyl, heptylsulfonyl, octylsulfonyl, cyclopropylsulfonyl, cyclobutylsulfonyl, cyclopentylsulfonyl, cyclohexylsulfonyl, cycloheptylsulfonyl, cyclooctylsulfonyl, cyclopropylmethylsulfonyl, cyclopropylethylsulfonyl, cyclohexylmethylsulfonyl, cyclohexylethylsulfonyl, and isomers thereof and the like.

Examples of C₆-C₁₄ arylsulfanyl include benzenesulfanyl, toluenesulfanyl, pyridinesulfanyl, naphthalenesulfanyl and the like.

Examples of C₆-C₁₄ arylsulfinyl include benzenesulfinyl, toluenesulfinyl, pyridinesulfinyl, naphthalenesulfinyl and the like.

Examples of C₆-C₁₄ arylsulfonyl include benzenesulfonyl, toluenesulfonyl, pyridinesulfonyl, naphthalenesulfonyl and the like.

Amide alkylated with C₁-C₈ alkyl means amide substituted by C₁-C₈ alkyl (having the same meanings as described above), which represents (C₁-C₈ alkyl)-CONH-, (C₁-C₈ alkyl)-NHCO-, (C₁-C₈ alkyl)-CON(C₁-C₈ alkyl)-, or (C₁-C₈ alkyl)-N(C₁-C₈ alkyl)-CO-. Examples thereof include acetamido, propionamido, n-butylamido, pivaloylamido, methylcarbamoyl, ethylcarbamoyl, propylcarbamoyl, isopropylcarbamoyl, butylcarbamoyl, N-methylacetamide, dimethylcarbamoyl, diethylcarbamoyl, and isomers thereof and the like.

Sulfonamide alkylated with C₁-C₈ alkyl means sulfonamide substituted by C₁-C₈ alkyl (having the same meanings as described above), which represents (C₁-C₈ alkyl)-SO₂NH-, or (C₁-C₈ alkyl)-NHSO₂-. Examples thereof include methylsulfonamido, ethylsulfonamido, propylsulfonamido, butylsulfonamido, pentylsulfonamido, hexylsulfonamido, heptylsulfonamido, octylsulfonamido, cyclopropylsulfonamido, cyclobutylsulfonamido, cyclopentylsulfonamido, cyclohexylsulfonamido, cycloheptylsulfonamido, cyclooctylsulfonamido, cyclopropylmethylsulfonamido, cyclopropylethylsulfonamido, cyclohexylmethylsulfonamido, cyclohexylethylsulfonamido, methylaminosulfonyl, ethylaminosulfonyl, propylaminosulfonyl, butylaminosulfonyl, pentylaminosulfonyl, hexylaminosulfonyl, heptylaminosulfonyl, octylaminosulfonyl, cyclopropylaminosulfonyl, cyclobutylaminosulfonyl, cyclopentylaminosulfonyl, cyclohexylaminosulfonyl, cycloheptylaminosulfonyl, cyclooctylaminosulfonyl, cyclopropylmethylaminosulfonyl, cyclopropylethylaminosulfonyl, cyclohexylmethylaminosulfonyl, cyclohexylethylaminosulfonyl, and isomers thereof and the like.

Carbamate alkylated with C₁-C₈ alkyl means urethan substituted by C₁-C₈ alkyl (having the same meanings as described above), which represents (C₁-C₈ alkyl)-OCONH-, or (C₁-C₈ alkyl)-NHCOO-. Examples thereof include methyl carbamate, ethyl carbamate, propyl carbamate, isopropyl carbamate, n-butyl carbamate, sec-butyl carbamate, isobutyl carbamate, t-butyl carbamate, methylcarbamoyloxy, ethylcarbamoyloxy, propylcarbamoyloxy, isopropylcarbamoyloxy, n-butylcarbamoyloxy, sec-butylcarbamoyloxy, isobutylcarbamoyloxy, t-butylcarbamoyloxy, and isomers thereof and the like.

Urea alkylated with C₁-C₈ alkyl means urea substituted by C₁-C₈ alkyl (having the same meanings as described above), which represents (C₁-C₈ alkyl)-NHCONH-, or -N(C₁-C₈ alkyl)CONH₂. Examples thereof include methylurea, ethylurea, propylurea, isopropylurea, n-butylurea, sec-butylurea, isobutylurea, t-butylurea, and isomers thereof and the like.

Calboxylated C₁-C₈ alkyl means C₁-C₈ alkyl (having the same meanings as described above) substituted by carboxyl, which represents -(C₁-C₈ alkyl)-COOH. Examples thereof include carboxymethyl, carboxyethyl, carboxypropyl, carboxybutyl, and isomers thereof and the like.

Carbamoyl means -CONH₂.

Thiocarbamoyl means -CSNH₂.

C₁-C₈ alkoxycarbonyl means carbonyl substituted by C₁-C₈ alkoxy (having the same meanings as described above), which represents (C₁-C₈ alkoxy)-CO-. Examples thereof include methoxycarbonyl, ethoxycarbonyl, propoxycarbonyl, butoxycarbonyl, pentyloxycarbonyl, hexyloxycarbonyl, heptyloxycarbonyl, octyloxycarbonyl, cyclopropoxycarbonyl, cyclobutoxycarbonyl, cyclopentyloxycarbonyl, cyclohexyloxycarbonyl, cycloheptyloxycarbonyl, cyclooctyloxycarbonyl, cyclopropylmethoxycarbonyl, cyclopropylethoxycarbonyl, cyclohexylmethoxycarbonyl, and isomers thereof and the like.

C₁-C₈ alkylcarbonyl means carbonyl substituted by C₁-C₈ alkyl (having the same meanings as described above), which represents -CO-(C₁-C₈ alkyl). Examples thereof include methylcarbonyl(acetyl), ethylcarbonyl, propylcarbonyl, isopropylcarbonyl, n-butylcarbonyl, sec-butylcarbonyl, t-butylcarbonyl(pivaloyl), pentylcarbonyl, hexylcarbonyl, heptylcarbonyl, octylcarbonyl, cyclopropylcarbonyl, cyclobutylcarbonyl, cyclopentylcarbonyl, cyclohexylcarbonyl, cycloheptylcarbonyl, cyclooctylcarbonyl, cyclopropylmethylcarbonyl, cyclopropylethylcarbonyl, cyclohexylmethylcarbonyl, cyclohexylethylcarbonyl, trifluoromethylcarbonyl, and isomers thereof and the like.

The 5- to 10-membered aromatic heterocycle means 5- to 10-membered monocyclic or bicyclic heterocyclic aryl comprising 1 to 5 heteroatoms of 1 to 3 types selected from nitrogen atom, sulfur atom and oxygen atom in addition to carbon atoms. Examples thereof include pyrrole, imidazole, triazole, tetrazole, pyrazole, pyridine, pyrazine, pyrimidine, pyridazine, triazine, furan, pyran, thiophene, thiopyran, thiepine, oxazole, isooxazole, thiazole, isothiazole, furazan, oxadiazole, indole, isoindole, indolizine, benzofuran, isobenzofuran, benzothiophene, isobenzothiophene, indazole, quinoline, isoquinoline, quinolizine, purine, phthalazine, quinoxaline, quinazoline, cinnoline, benzooxazole, benzothiazole, benzoimidazole and the like.

The 5- to 6-membered aromatic heterocycle means 5- to 6-membered monocyclic aryl comprising 1 to 4 heteroatoms of 1 to 3 types selected from nitrogen atom, sulfur atom and oxygen atom in addition to carbon atoms. Examples thereof include pyrrole, imidazole, triazole, tetrazole, pyrazole, pyridine, pyrazine, pyrimidine, pyridazine, triazine, furan, pyran, thiophene, oxazole, isooxazole, thiazole, isothiazole and the like.

The 5- to 10-membered monocyclic heterocycle means 5- to 10-membered monocyclic heterocyclic aryl comprising 1 to 5 heteroatoms of 1 to 3 types selected from nitrogen atom, sulfur atom and oxygen atom in addition to carbon atoms, which aryl may optionally be partially or entirely saturated. Carbonyl may be included in a part of the ring. Examples thereof include pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isooxazole, thiazole, isothiazole, furazan, oxadiazole, oxazine, oxadiazine, oxazepine, oxadiazepine, thiadiazole, thiazine, thiadiazine, thiazepine, thiadiazepine, pyrroline, dihydropyrrole, pyrrolidine, imidazoline, imidazolidine, triazine, triazoline, triazolidine, tetrazoline, tetrazolidine, pyrazoline, pyrazolidine, dihydropyridine, tetrahydropyridine, piperidine, piperidine-4-one, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, piperidine-2-one, pyrrolidine-2-one, azepane-2-one, azocane-2-one, dihydrofuran, tetrahydrofuran, dihydropyran, tetrahydropyran, dihydrooxepine, tetrahydrooxepine, perhydrooxepine, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisooxazole, tetrahydroisooxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazin, tetrahydrooxazin, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, oxathiolane, dioxolane, dioxane and the like.

The 5- to 10-membered bicyclic heterocycle means 5- to 10-membered bicyclic heterocyclic aryl comprising 1 to 5 heteroatom(s) of 1 to 3 kind(s) selected from nitrogen atom(s), sulfur atom(s) and oxygen atom(s) in addition to carbon atoms, which aryl may optionally be partially or entirely saturated. Examples thereof include indole, isoindole, indolizine, benzofuran, isobenzofuran, benzothiophene, isobenzothiophene, indazole, quinoline, isoquinoline, quinolizine, purine, phthalazine, quinoxaline, quinazoline, cinnoline, benzooxazole, benzothiazole, benzoimidazole, chromene, benzofurazan, benzothiadiazole, benzotriazole, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzooxathiane, dihydrobenzooxazin, dihydrobenzothiazine, pyrazinomorpholine, dihydrobenzooxazole, perhydrobenzooxazole, dihydrobenzothiazole, perhydrobenzothiazole, dihydrobenzoimidazole, perhydrobenzoimidazole, dioxaindane, benzodioxan, benzodioxole, benzodioxine, benzo[1,4]dioxine, dihydrobenzo[1,4]dioxine, chroman, chromene and the like.

C₁-C₈ hydroxyalkyloxy includes those which are linear or branched, and those which are partially cyclized. Examples thereof include hydroxymethyloxy, hydroxyethyloxy, hydroxypropyloxy, hydroxybutyloxy, hydroxypentyloxy, hydroxyhexyloxy, hydroxyheptyloxy, hydroxyoctyloxy, hydroxycyclopropyloxy, hydroxycyclobutyloxy, hydroxycyclopentyloxy, hydroxycyclohexyloxy, hydroxycycloheptyloxy, hydroxycyclooctyloxy, hydroxycyclopropylmethyloxy, hydroxycyclopropylethyloxy, hydroxycyclohexylmethyloxy, hydroxycyclohexylethyloxy, and isomers thereof and the like.

The present invention includes all the pharmaceutically acceptable salts. The arylmethylene urea derivatives represented by the Formula (I) according to the present invention are converted to corresponding salts by known methods. The salts are preferably atoxic and water-soluble. Appropriate salts include salts of alkali metal (potassium, sodium and the like), salts of alkaline-earth metal (calcium, magnesium and the like), ammonium salt, and salts of pharmaceutically acceptable organic amines (tetramethylammonium, triethylamine, methylamine, dimethylamine, diethylamine, t-butylamine, cyclopentylamine, benzylamine, phenethylamine, piperidine, monoethanolamine, diethanolamine, tris(hydroxymethyl)amine, lysine, arginine, N-methyl-D-glucamine and the like).

The arylmethylene urea derivatives represented by the Formula (I) according to the present invention are converted to the corresponding acid addition salts by known methods. The acid addition salts are preferably atoxic and water-soluble. Appropriate acid addition salts include inorganic acid salts such as hydrochloric acid salt, hydrobromic acid salt, sulfuric acid salt, phosphoric acid salt and nitric acid salt; and organic acid salts such as acetic acid salt, trifluoroacetic acid salt, lactic acid salt, tartaric acid salt, oxalic acid salt, fumaric acid salt, maleic acid salt, citric acid salt, benzoic acid salt, methanesulfonic acid salt, ethanesulfonic acid salt, benzenesulfonic acid salt, toluenesulfonic acid salt, isethionic acid salt, glucuronic acid salt and gluconic acid salt.

The compounds represented by the Formula (I) or the pharmaceutically acceptable salts thereof according to the present invention may also be converted to solvates by known methods. The solvates are preferably atoxic and water-soluble. Appropriate solvates include, for example, solvates of water and alcoholic solvents (ethanol and the like, for example).

Among the arylmethylene urea derivatives represented by the Formula (I) according to the present invention, preferred modes are as described below.

"A" represents the Formulae (II)-(V), and the Formula (II) or the Formula (III) is preferred, and the Formula (II) is especially preferred.

R²-R¹¹ are each hydrogen, cyano, hydroxy, amino, mercapto, nitro, fluoro, chloro, bromo, iodo, phenyl, benzyl, carboxy, carbamoyl, thiocarbamoyl, formyl, C₁-C₈ alkyl, C₁-C₈ hydroxyalkyl or C₁-C₈ alkoxy, and preferably hydrogen, cyano, hydroxy, amino, mercapto, fluoro, chloro, bromo, iodo, phenyl, C₁-C₈ alkyl, C₁-C₃ hydroxyalkyl or C₁-C₈ alkoxy, more preferably hydrogen, fluoro, chloro, bromo, iodo or C₁-C₈ alkyl, and most preferably hydrogen, fluoro, chloro, trifluoromethyl or methyl.

T¹ is -O-, -S- or -NR¹²- (R¹² is the same as described above), and preferably - O- or -S-, more preferably -S-.

X is -O-, -S-, -SO-, -SO₂-, -NR¹³- (R¹³ is the same as described above) or - CH₂-, and preferably -O-, -S-, -NR¹³- (R¹³ represents hydrogen or C₁-C₈ alkyl) or - CH₂-, more preferably -O- or -CH₂-, most preferably -O-.

Ar¹ is pyrazole, thiophene, furan, pyrrole, imidazole, oxazole, thiazole, oxadiazole or thiadiazole, and preferably pyrazole, thiophene, oxazole, thiazole, oxadiazole or thiadiazole, and more preferably pyrazole represented by the Formula (XII):

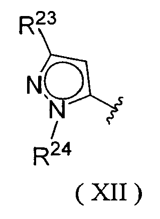

[wherein R²³ is
t-butyl, t-pentyl, neopentyl, cyclopropyl, cyclopentyl, furyl or 2-methylfuryl; and
R²⁴ represents
phenyl or pyridyl (these optionally have one or more substituents independently selected from the following: hydroxy, amino, nitro, fluoro, chloro, bromo, iodo, carboxy, benzyloxy, C₁-C₈ alkyl, C₁-C₈ alkoxy, C₁-C₈ alkoxycarbonyl, C₁-C₈ hydroxyalkyloxy, C₁-C₁₆ alkylamino, C₁-C₈ alkylsulfanyl, C₁-C₈ alkylsulfonyl, trifluoromethanesulfonyl and sulfonamide alkylated with C₁-C₈ alkyl)].

The substituent(s) of Ar¹ is(are) cyano, hydroxy, amino, mercapto, nitro, fluoro, chloro, bromo, iodo, benzyl, carboxy, carbamoyl, thiocarbamoyl, formyl, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₈ alkylsulfanyl, phenyl or a 5- to 10-membered aromatic heterocycle (these substituents may further have one or more substituents independently selected from the following: cyano, hydroxy, amino, mercapto, nitro, fluoro, chloro, bromo, iodo, phenyl, benzyl, carboxy, benzyloxy, carbamoyl, thiocarbamoyl, formyl, C₁-C₈ alkyl, C₂-C₈ alkenyl, C₂-C₈ alkynyl, C₁-C₈ hydroxyalkyl, C₁-C₈ alkoxy, C₁-C₈ alkoxycarbonyl, C₁-C₈ hydroxyalkyloxy, C₁-C₁₆ alkylamino, C₁-C₈ alkylsulfanyl, C₁-C₈ alkylsulfonyl, trifluoromethanesulfonyl, sulfonamide alkylated withC₁-C₈ alkyl, or a 5- to 10-membered aromatic heterocycle), and preferably phenyl, C₁-C₈ alkyl, C₁-C₈ hydroxyalkyl, pyridyl or furyl (phenyl, pyridyl or furyl may further have one or more substituents independently selected from the following: hydroxy, amino, nitro, fluoro, chloro, bromo, iodo, carboxy, benzyloxy, C₁-C₈ alkyl, C₁-C₈ alkoxy, C₁-C₈ alkoxycarbonyl, C₁-C₈ hydroxyalkyloxy, C₁-C₁₆ alkylamino, C₁-C₈ alkylsulfanyl, C₁-C₈ alkylsulfonyl, trifluoromethanesulfonyl, and sulfonamide alkylated withC₁-C₈ alkyl), more preferably methyl, ethyl, propyl, isopropyl, n-butyl, isobutyl, t-butyl, sec-butyl, n-pentyl, t-pentyl, neopentyl, cyclopropyl, cyclobutyl, cyclopentyl, cyclohexyl, cyclopropylmethyl, cyclobutylmethyl, cyclopentylmethyl, cyclohexylmethyl, phenyl, pyridyl, furyl, 2-methylfuryl, 3-methylfuryl, 2-methylphenyl, 3-methylphenyl, 4-methylphenyl, 4-ethylphenyl, 3-hydroxyphenyl, 3-methoxyphenyl, 4-methoxyphenyl, 4-methyl-3-hydroxyphenyl, 4-methyl-2-hydroxyphenyl, 4-methyl-3-aminophenyl, 4-methyl-3-methoxyphenyl, 3-methoxyphenyl, 3-benzyloxyphenyl, 3-aminophenyl, 2-methylpyridyl, 3-methylpyridyl or 4-methylpyridyl.

The number of the substituents is preferably 1 to 3, more preferably 2.

R²³ is t-butyl, t-pentyl, neopentyl, cyclopropyl, cyclopentyl, furyl or 2-methylfuryl, and preferably t-butyl, t-pentyl, neopentyl, cyclopropyl, cyclopentyl, cyclohexyl or furyl, more preferably t-butyl, t-pentyl or cyclopentyl, most preferably t-butyl.

R²⁴ is phenyl or pyridyl (these substituents may further have one or more substituents independently selected from the following: hydroxy, amino, nitro, fluoro, chloro, bromo, iodo, carboxy, benzyloxy, C₁-C₈ alkyl, C₁-C₈ alkoxy, C₁-C₈ alkoxycarbonyl, C₁-C₈ hydroxyalkyloxy, C₁-C₁₆ alkylamino, C₁-C₈ alkylsulfanyl, C₁-C₈ alkylsulfonyl, trifluoromethanesulfonyl and sulfonamide alkylated with C₁-C₈ alkyl), and preferably phenyl, pyridyl, 2-methylphenyl, 3-methylphenyl, 4-methylphenyl, 2-methylpyridyl, 3-methylpyridyl, 4-methyl-3-hydroxyphenyl or 4-methylpyridyl, more preferably phenyl, 4-methylphenyl, 4-methyl-3-hydroxyphenyl or 4-methylpyridyl, and most preferably 4-methylphenyl or 4-methyl-3-hydroxypheny.

Ar² is the Formula (VI) or the Formula (VII), and preferably the Formula (VIII), (IX), (X) or (XI):

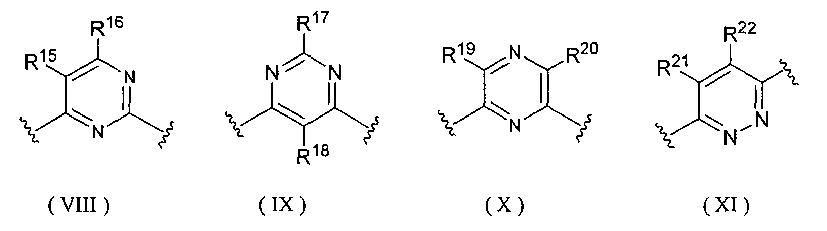

[wherein R¹⁵-R²² each independently represents hydrogen or C₁-C₈ alkyl], and more preferably the Formula (VIII) or (IX).

R¹⁵-R²² are each hydrogen or C₁-C₈ alkyl, and preferably hydrogen or methyl.

R¹ is hydrogen, cyano, hydroxy, amino, mercapto, nitro, fluoro, chloro, bromo, iodo, phenyl, benzyl, carboxy, carbamoyl, thiocarbamoyl, formyl, formylamino, aminosulfonyl, phenoxy, indan, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₁₆ hydroxyalkylamino, C₁-C₈ alkylsulfanyl, C₁-C₈ alkylsulfinyl, C₁-C₈ alkylsulfonyl, C₆-C₁₄ arylsulfanyl, C₆-C₁₄ aryl sulfinyl, C₆-C₁₄ aryl sulfonyl, amide alkylated with C₁-C₈ alkyl, sulfonamide alkylated with C₁-C₈ alkyl, carbamate alkylated with C₁-C₈ alkyl, urea alkylated with C₁-C₈ alkyl, carboxylated C₁-C₈ alkyl, C₁-C₈ alkoxycarbonyl, C₁-C₈ alkylcarbonyl, 5-to 10-membered aromatic heterocycle, 5- to 10-membered monocyclic heterocycle or 5- to 10-membered bicyclic heterocycle

(these substituents may comprise one or more of the substituents, independently selected from the following: cyano, hydroxy, amino, mercapto, nitro, fluoro, chloro, bromo, iodo, phenyl, benzyl, carboxy, carbamoyl, thiocarbamoyl, formyl, formylamino, aminosulfonyl, phenoxy, indan, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₁₆ hydroxyalkylamino, C₁-C₈ alkylsulfanyl, C₁-C₈ alkylsulfinyl, C₁-C₈ alkylsulfonyl, C₆-C₁₄ arylsulfanyl, C₆-C₁₄ arylsulfinyl, C₆-C₁₄ arylsulfonyl, amide alkylated with C₁-C₈ alkyl, sulfonamide alkylated with C₁-C₈ alkyl, carbamate alkylated with C₁-C₈ alkyl, urea alkylated with C₁-C₈ alkyl, carboxylated C₁-C₈ alkyl, C₁-C₈ alkoxycarbonyl, C₁-C₈ alkylcarbonyl, 5- to 10-membered aromatic heterocycle, 5- to 10-membered monocyclic heterocycle, 5- to 10-membered bicyclic heterocycle, C₁-C₈ alkylene, C₂-C₈ alkenylene and C₁-C₈ alkylenedioxy (these substituents may further comprise one or more of the substituents, independently selected from the following: cyano, hydroxy, amino, mercapto, nitro, fluoro, chloro, bromo, iodo, phenyl, benzyl, carboxy, carbamoyl, thiocarbamoyl, formyl, formylamino, aminosulfonyl, indan, C₁-C₈ alkyl, C₁-C₈ hydroxyalkyl, C₁-C₈ alkoxy, C₁-C₁₆ alkylamino, C₁-C₁₆ hydroxyalkylamino, amide alkylated with C₁-C₈ alkyl, carbamate alkylated with C₁-C₈ alkyl, carboxylated C₁-C₈ alkyl, C₁-C₈ alkoxycarbonyl, C₁-C₈ alkylcarbonyl, 5- to 10-membered monocyclic heterocycle, 5-to 10-membered bicyclic heterocycle, 5- to 10-membered aromatic heterocycle, and C₁-C₈ alkylenedioxy)),
and preferably, hydrogen, amino, mercapto, fluoro, chloro, bromo, iodo, C₁-C₁₆ alkylamino, C₁-C₁₆ hydroxyalkylamino, C₁-C₈ alkylsulfanyl, 5- to 10-membered aromatic heterocycle, 5- to 10-membered monocyclic heterocycle or 5- to 10-membered bicyclic heterocycle

(these substituents may comprise one or more of the substituents, independently selected from the following: cyano, hydroxy, amino, mercapto, fluoro, chloro, bromo, iodo, phenyl, benzyl, carboxy, carbamoyl, C₁-C₈ alkyl, C₁-C₈ hydroxyalkyl, C₁-C₈ alkoxy, C₁-C₁₆ alkylamino, C₁-C₁₆ hydroxyalkylamino, amide alkylated with C₁-C₈ alkyl, carboxylated C₁-C₈ alkyl, C₁-C₈ alkoxycarbonyl, C₁-C₈ alkylcarbonyl, 5- to 10-membered aromatic heterocycle, 5- to 10-membered monocyclic heterocycle, 5- to 10-membered bicyclic heterocycle and C₁-C₈ alkylenedioxy (these substituents may further comprise one or more of the substituents, independently selected from the following: cyano, hydroxy, amino, mercapto, fluoro, chloro, bromo, iodo, phenyl, benzyl, carboxy, carbamoyl, C₁-C₈ alkyl, C₁-C₈ hydroxyalkyl, C₁-C₈ alkoxy, C₁-C₁₆ alkylamino, C₁-C₁₆ hydroxyalkylamino, amide alkylated with C₁-C₈ alkyl, carboxylated C₁-C₈ alkyl, C₁-C₈ alkoxycarbonyl, C₁-C₈ alkylcarbonyl, 5- to 10-membered monocyclic heterocycle, 5- to 10-membered bicyclic heterocycle, 5- to 10-membered aromatic heterocycle and C₁-C₈ alkylenedioxy)), more preferably, chloro, methylsulfanyl, morpholino, 3-carbamoylpiperidino, 4-carbamoylpiperidino, 4-acetylpiperazino, 3-(dimethylamino)propylamino, 3-(diethylamino)propylamino, 3-(2-oxo-pyrrolidin-1-yl)propylamino, 4-methylpiperazino, 4-(2-methoxyethyl)piperazino, or 2-(1-methylpyrrolidin-2-yl)ethylamino, and most preferably, 3-(2-oxo-pyrrolidin-1-yl)propylamino, morpholino, 4-acetylpiperazino, 4-methylpiperazino or 4-(2-methoxyethyl)piperazino.

The substituents which R¹ optionally has are cyano, hydroxy, amino, mercapto, nitro, fluoro, chloro, bromo, iodo, phenyl, benzyl, carboxy, carbamoyl, thiocarbamoyl, formyl, formylamino, aminosulfonyl, phenoxy, indan, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₁₆ hydroxyalkylamino, C₁-C₈ alkylsulfanyl, C₁-C₈ alkylsulfinyl, C₁-C₈ alkylsulfonyl, C₆-C₁₄ arylsulfanyl, C₆-C₁₄ arylsulfinyl, C₆-C₁₄ arylsulfonyl, amide alkylated with C₁-C₈ alkyl, sulfonamide alkylated with C₁-C₈ alkyl, carbamate alkylated with C₁-C₈ alkyl, urea alkylated with C₁-C₈ alkyl, carboxylated C₁-C₈ alkyl, C₁-C₈ alkoxycarbonyl, C₁-C₈ alkylcarbonyl, 5- to 10-membered aromatic heterocycle, 5- to 10-membered monocyclic heterocycle, 5- to 10-membered bicyclic heterocycle, C₁-C₈ alkylene, C₂-C₈ alkenylene and C₁-C₈ alkylenedioxy (these substituents may further comprise one or more of the substituents, independently selected from the following: cyano, hydroxy, amino, mercapto, fluoro, chloro, bromo, iodo, phenyl, benzyl, carboxy, carbamoyl, thiocarbamoyl, formyl, formylamino, aminosulfonyl, indan, C₁-C₈ alkyl, C₁-C₈ hydroxyalkyl, C₁-C₈ alkoxy, C₁-C₁₆ alkylamino, C₁-C₁₆ hydroxyalkylamino, amide alkylated with C₁-C₈ alkyl, carbamate alkylated with C₁-C₈ alkyl, carboxylated C₁-C₈ alkyl, C₁-C₈ alkoxycarbonyl, C₁-C₈ alkylcarbonyl, 5- to 10-membered monocyclic heterocycle, 5- to 10-membered bicyclic heterocycle, 5- to 10-membered aromatic heterocycle and C₁-C₈ alkylenedioxy).

Among these, preferred are cyano, hydroxy, amino, mercapto, fluoro, chloro, bromo, iodo, phenyl, benzyl, carboxy, carbamoyl, C₁-C₈ alkyl, C₁-C₈ hydroxyalkyl, C₁-C₈ alkoxy, C₁-C₁₆ alkylamino, C₁-C₁₆ hydroxyalkylamino, amide alkylated with C₁-C₈ alkyl, carboxylated C₁-C₈ alkyl, C₁-C₈ alkoxycarbonyl, C₁-C₈ alkylcarbonyl, 5-to 10-membered aromatic heterocycle, 5- to 10-membered monocyclic heterocycle, 5- to 10-membered bicyclic heterocycle and C₁-C₈ alkylenedioxy (these substituents may further comprise one or more of the substituents, independently selected from the following: cyano, hydroxy, amino, mercapto, fluoro, chloro, bromo, iodo, phenyl, benzyl, carboxy, carbamoyl, C₁-C₈ alkyl, C₁-C₈ hydroxyalkyl, C₁-C₈ alkoxy, C₁-C₁₆ alkylamino, C₁-C₁₆ hydroxyalkylamino, amide alkylated with C₁-C₈ alkyl, carboxylated C₁-C₈ alkyl, C₁-C₈ alkoxycarbonyl, C₁-C₈ alkylcarbonyl, 5- to 10-membered monocyclic heterocycle, 5- to 10-membered bicyclic heterocycle, 5- to 10-membered aromatic heterocycle and C₁-C₈ alkylenedioxy).

More preferred are cyano, hydroxy, amino, mercapto, nitro, fluoro, chloro, bromo, iodo, phenyl, benzyl, carboxy, carbamoyl, thiocarbamoyl, C₁-C₈ alkyl, C₁-C₈ alkylamino, C₁-C₅ hydroxyalkyl, C₁-C₅ alkoxy, C₁-C₅ alkylated amide, carbamate alkylated with C₁-C₈ alkyl, C₁-C₅ alkylcarbonyl and 5- to 10-membered monocyclic heterocycle (these substituents may further comprise one, two or three of the substituents, independently selected from the following: cyano, hydroxy, amino, mercapto, nitro, fluoro, chloro, bromo, iodo, phenyl, benzyl, carboxy, carbamoyl, thiocarbamoyl, C₁-C₅ alkyl, C₁-C₅ hydroxyalkyl and C₁-C₅ alkoxy).

Although R¹ may have one or more substituents, the number of the substituents is preferably 1 to 5, more preferably 1 to 3.

Among the preferred compounds mentioned above, the compounds,
wherein A is the Formula (II) or (III), are especially preferred, and those wherein R²-R⁷ are each hydrogen, fluoro, chloro, bromo or methyl, and T¹ in the Formula (III) is S, are especially preferred. Among these, the compounds in which Ar¹ is represented by the Formula (XII) are preferred, and those in which R²³ in the Formula (XII) is t-butyl and in which R²⁴ is 4-methylphenyl or 4-methyl-3-hydroxyphenyl are especially preferred. Further, among these, the compounds wherein X is -O- are preferred.

As the arylmethylene urea derivatives used for the treatment or prophylaxis of overactive bladder, the compounds wherein A in the Formula (I) is represented either by the Formula (II) or (III) (wherein T¹ and R²-R⁷ have the same meanings as defined above) are preferred, and those wherein T¹ is -S-, and R²-R⁷ are each independently hydrogen, fluoro, chloro, bromo or methyl, are more preferred. Also, the compounds wherein Ar¹ is the Formula (XII) (wherein R²³ and R²⁴ have the same meanings as defined above) are preferred, and those wherein R²³ is t-butyl and R²⁴ is 4-methylphenyl or 4-methyl-3-hydroxyphenyl are particularly preferred, those wherein X is -O- are preferred, and those wherein Ar² is preferably represented by the Formula (VIII) or (IX) (wherein R¹⁵-R¹⁸ have the same meanings as defined above) are preferred. Further, R¹ preferably is chloro, methylsulfanyl, morpholino, 3-carbamoylpiperidino, 4-carbamoylpiperidino, 4-acetylpiperazino, 3-(dimethylamino)propylamino, 3-(diethylamino)propylamino, 3-(2-oxo-pyrrolidin-1-yl)propylamino, 4-methylpiperazino, 4-(2-methoxyethyl)piperazino or 2-(1-methylpyrrolidin-2-yl)ethylamino, and among these, morpholino is especially preferred.

Preferred arylmethylene urea derivatives according to the present invention include compounds represented by the Formulae (Ia) to (Idd) listed below. In present Description, a single line to which end nothing is bound (for example, the single line at the 4th position of phenyl group, or three single lines bound to the carbon atom bound to the pyrazole ring in the Formula (Ia) below) indicates a methyl group.

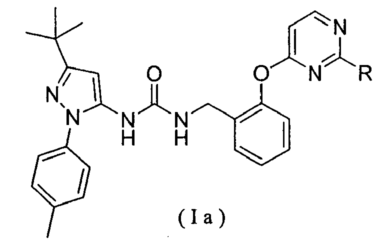

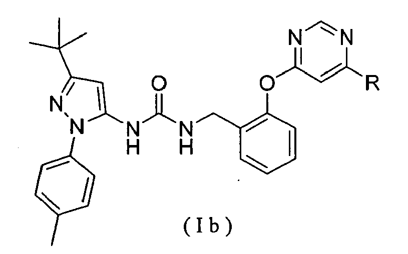

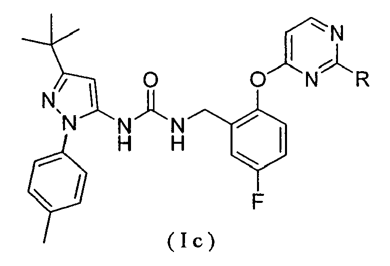

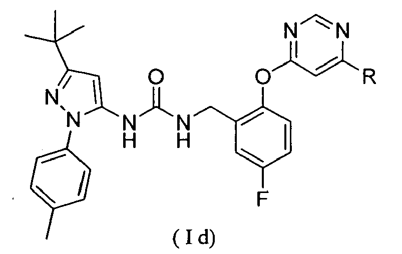

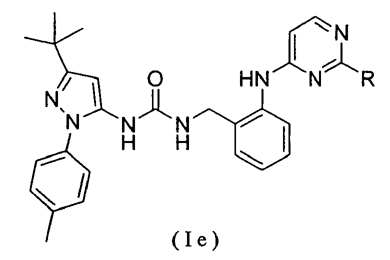

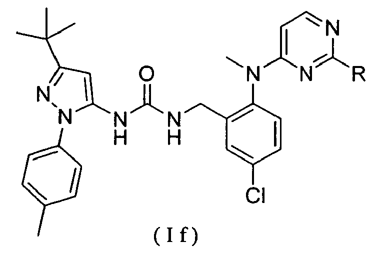

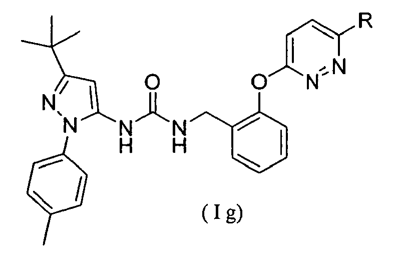

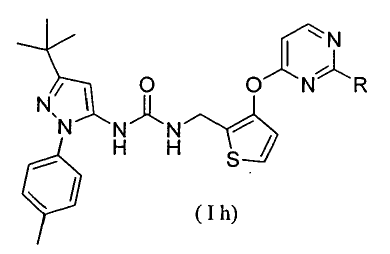

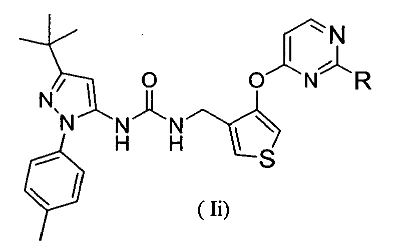

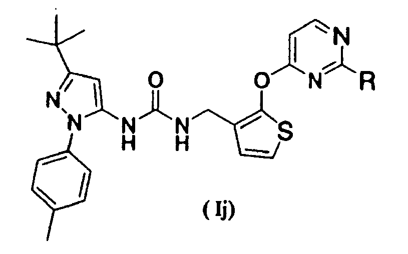

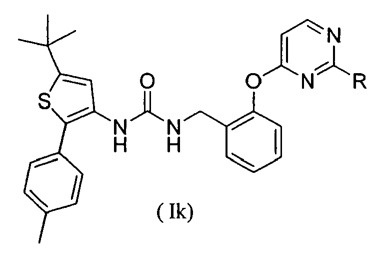

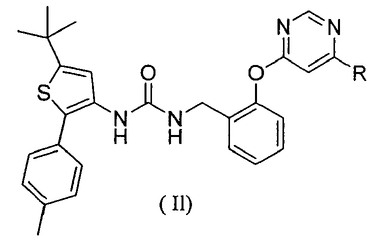

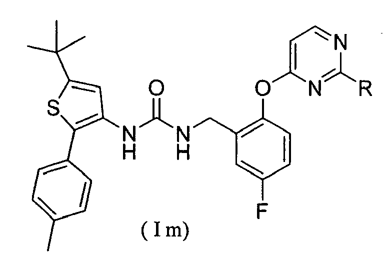

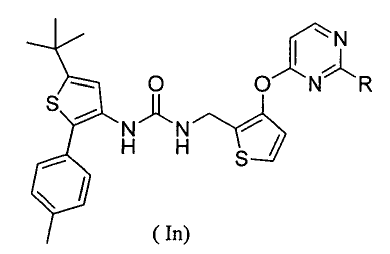

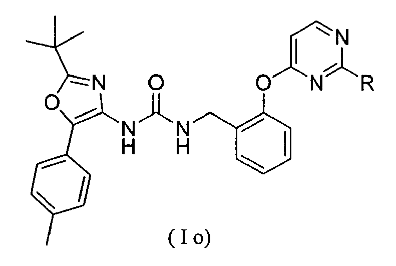

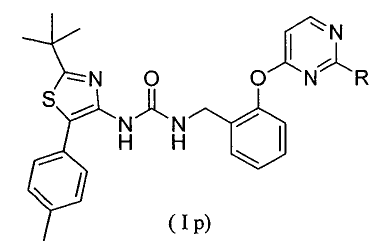

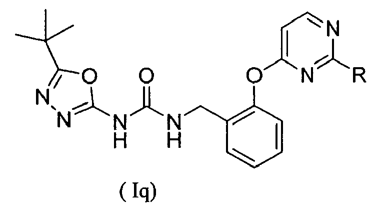

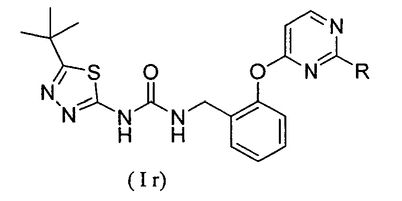

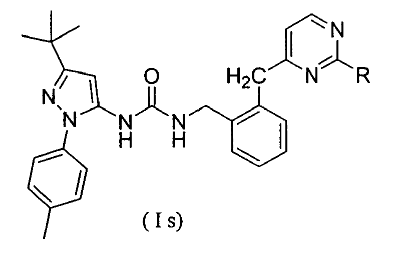

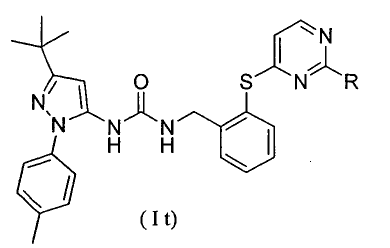

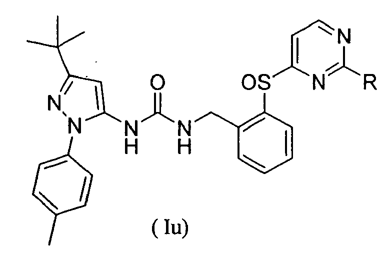

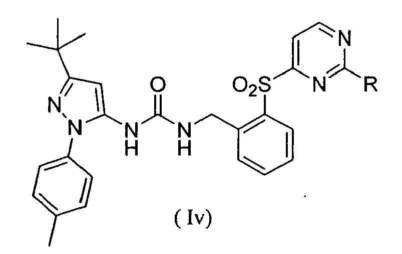

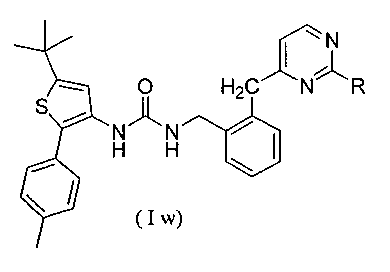

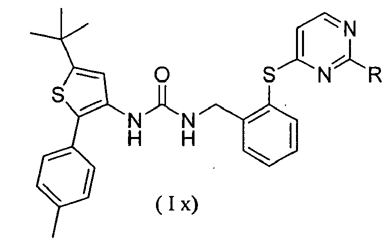

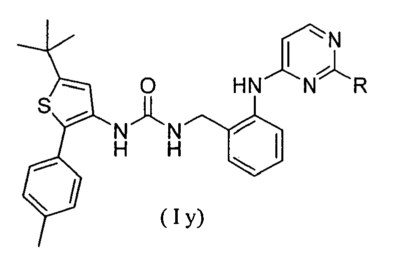

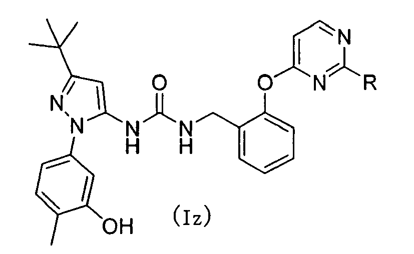

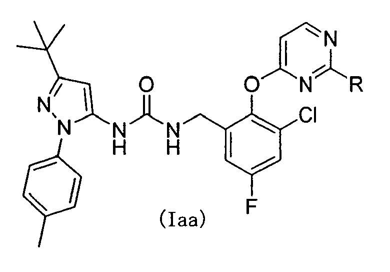

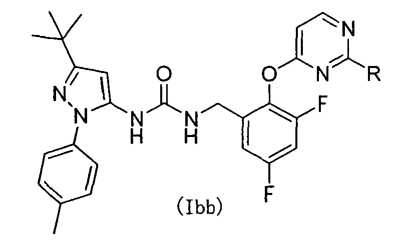

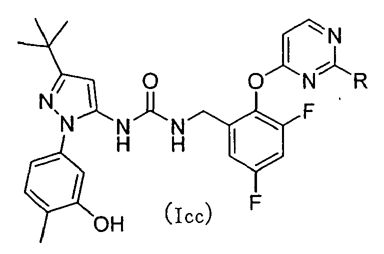

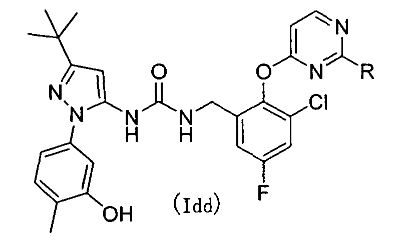

Specific examples of substituent R in the above Formula (Ia) to (Idd) are shown below. The definition of the wave line contacting the end of a single bond in a perpendicular direction in the chemical formulae below has the same meaning as described above (i.e. a single bond contacting a wave line represents the single bond connecting a carbon atom in the pyrimidine ring with R). It should also be noted that the wave line connecting an asymmetric carbon atom with an atomic group indicates a single bond, and this single bond indicates that the atomic group can take any configuration which can occur on the asymmetric carbon atom as the centre.

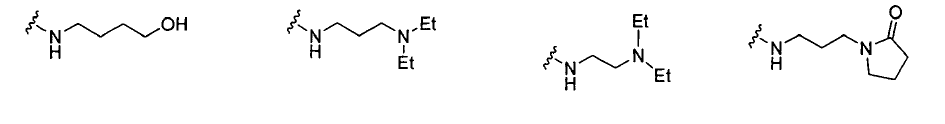

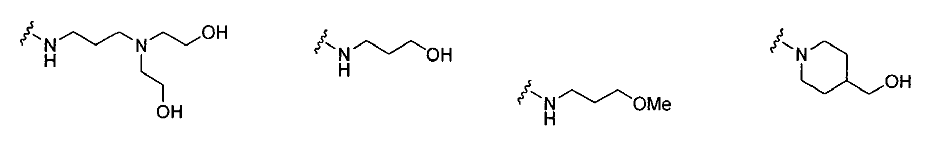

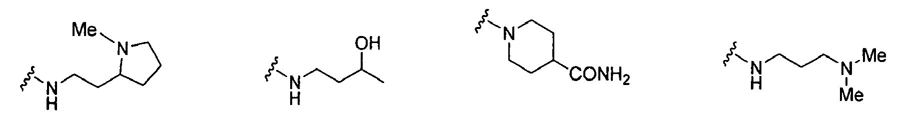

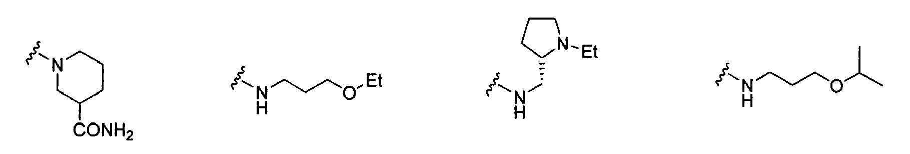

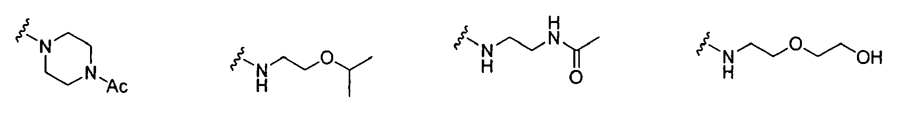

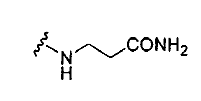

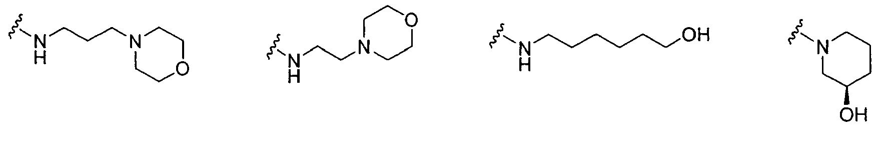

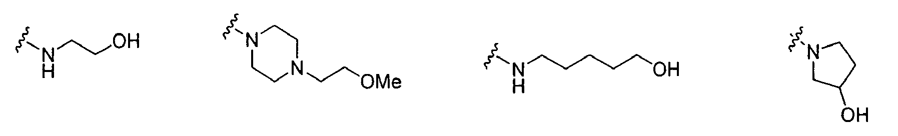

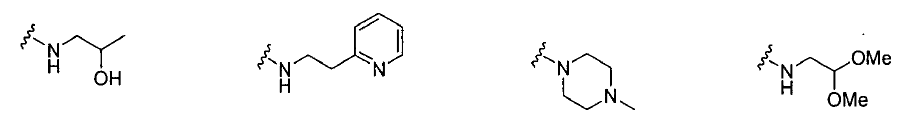

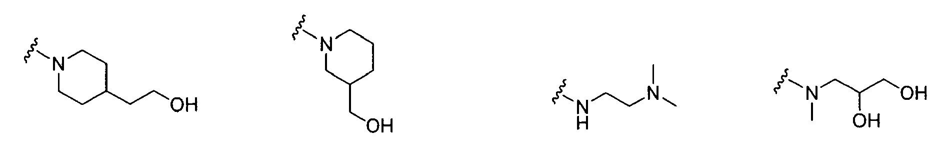

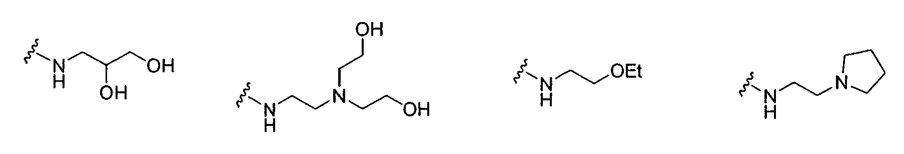

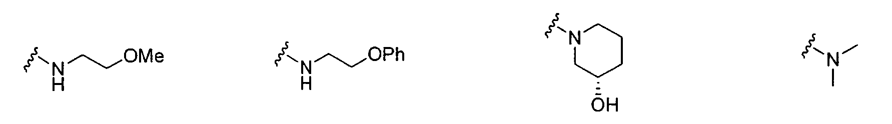

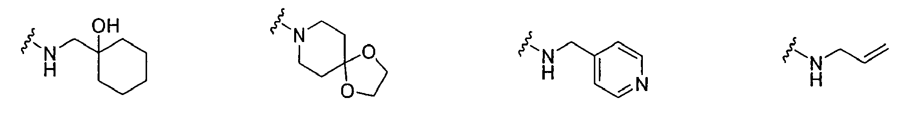

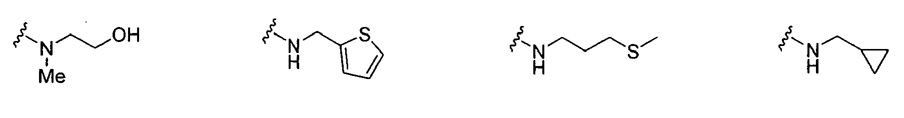

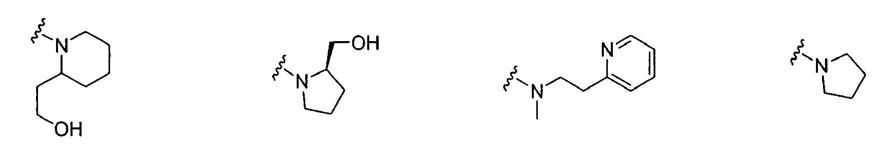

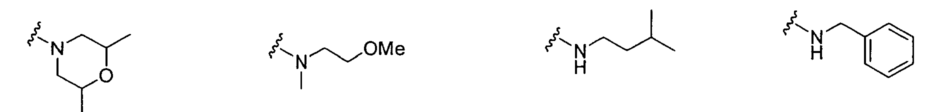

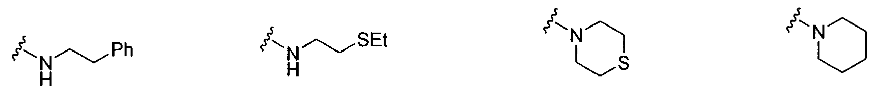

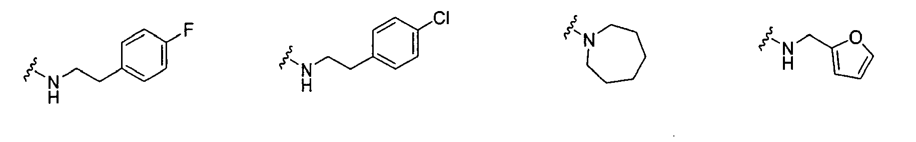

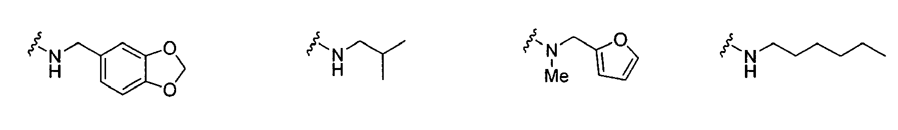

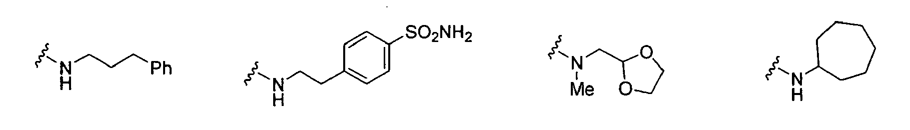

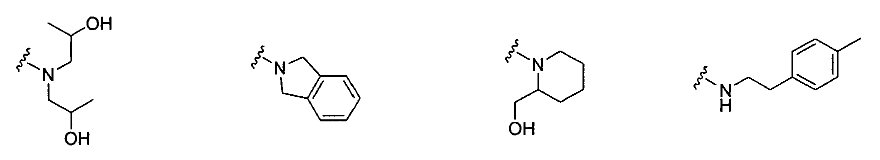

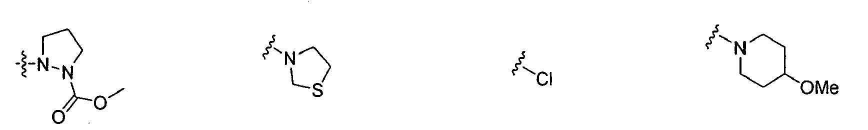

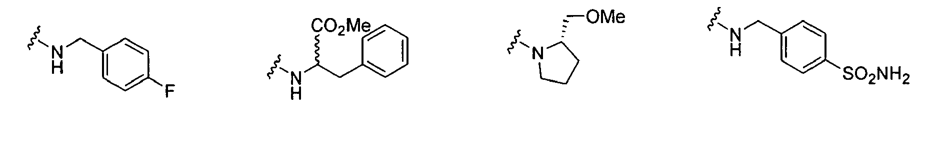

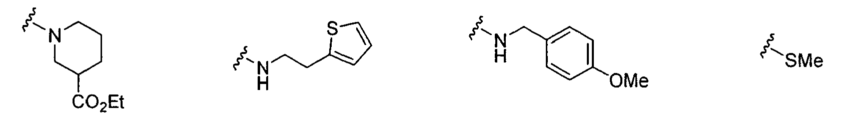

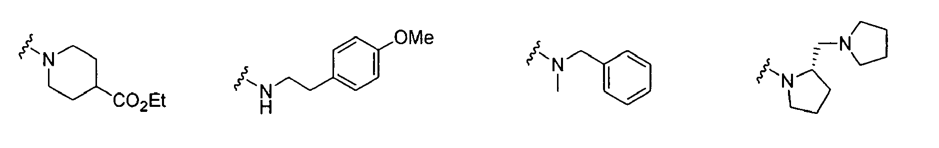

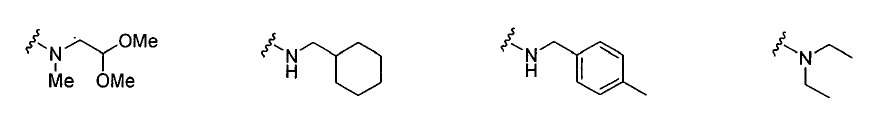

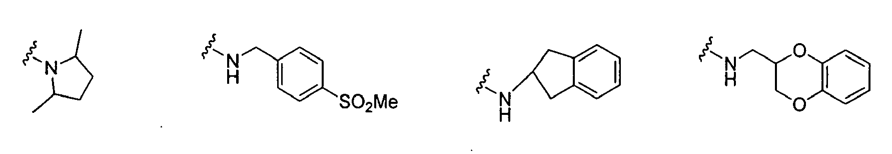

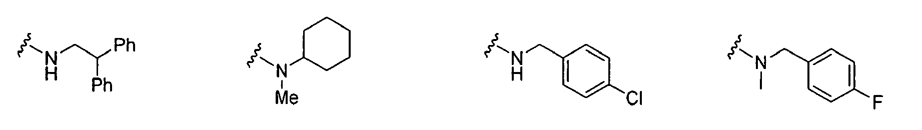

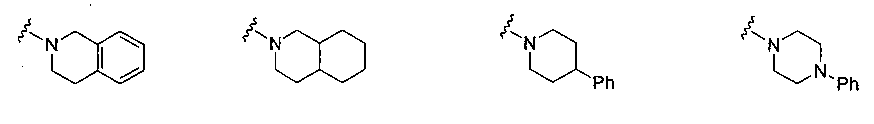

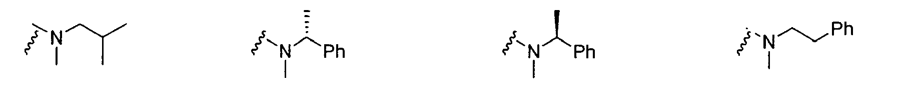

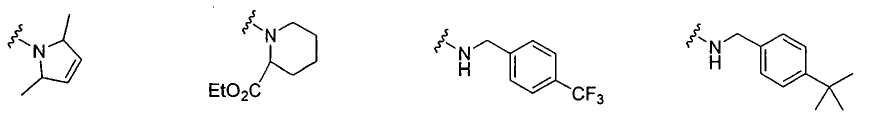

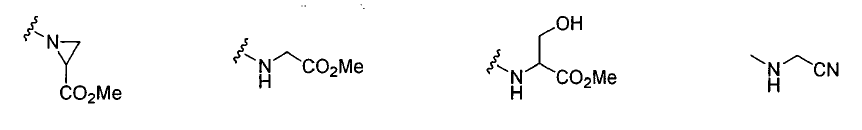

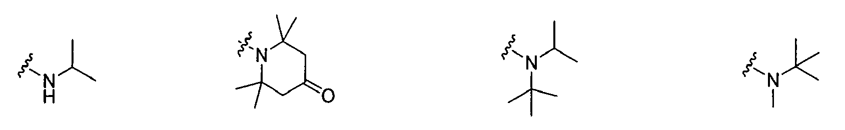

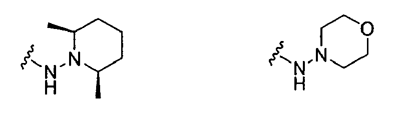

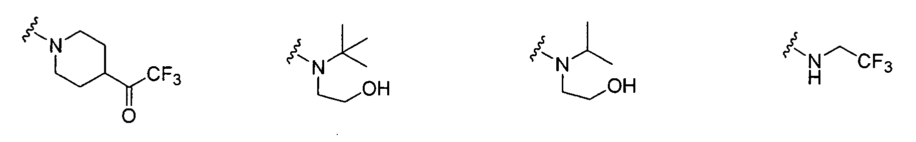

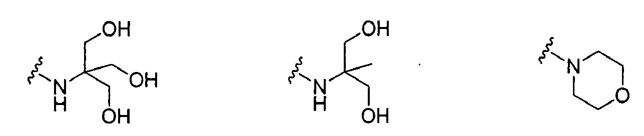

Arylmethylene urea derivatives of the Formula (I) according to the invention or the pharmaceutically acceptable salts thereof can be synthesized through the following steps:

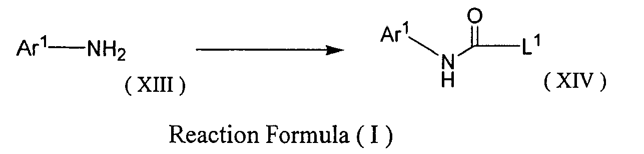

[wherein L¹ represents Cl, phenoxy, p-nitrophenoxy, o-nitrophenoxy or 2,2,2-trichloroethoxy, and the other symbols have the same meanings as described above].

The reaction represented by the above reaction formula is performed by known methods. Compound (XIV) can be synthesized by condensing Compound (XIII) and chloroformate reagent in an appropriate solvent in the presence of a base.

Examples of the chloroformate reagent include phosgene, diphosgene, triphosgene, phenyl chloroformate, p-nitrophenyl chloroformate, o-nitrophenyl chloroformate, 2,2,2-trichloroethyl chloroformate and the like. Preferably, triphosgene, p-nitrophenyl chloroformate, o-nitrophenyl chloroformate, or 2,2,2-trichloroethyl chloroformate is used. Examples of the base include organic amine bases such as triethylamine, diisopropylethylamine and pyridine; and inorganic bases such as sodium hydroxide, potassium hydroxide, sodium carbonate, potassium carbonate and sodium hydrogen carbonate. Preferably, triethylamine, diisopropylethylamine, sodium hydroxide or potassium carbonate is used. Examples of the solvent include protic solvents such as water, methanol and ethanol; dipolar aprotic solvents such as DMF, DMSO and 1-methyl-2-pyrrolidinone; ester solvents such as ethyl acetate; ether solvents such as THF, DME and dioxane; and halogenated solvents such as dichloroethane, chloroform and 2-dichloroethane; and mixture thereof. Preferably, DMF, THF or a mixed solvent of water and ethyl acetate is used. The reaction temperature may be in the range between -40°C and 140°C, and preferably, in the range between 0°C and 80°C. Although the reaction time may be selected based on the other conditions such as reaction temperature, satisfactory results are usually obtained by employing a time from about 1 minute to about 30 hours.

In the reaction formula above, the compound of the Formula (XIII) employed as the starting material is known in the art, or can easily be produced according to known methods (e.g. a method described in WO 99/23091).

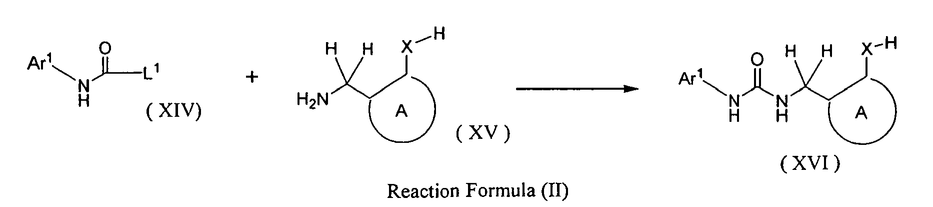

[wherein the symbols have the same meanings as described above].

The reaction represented by the above reaction formula is performed by known methods. Compound (XVI) can be synthesized by condensing Compounds (XIV) and (XV) in an appropriate solvent in the presence of a base.

Examples of the base include organic amine bases such as triethylamine, diisopropylethylamine and pyridine, and inorganic bases such as sodium hydroxide, potassium hydroxide, sodium carbonate, potassium carbonate and sodium hydrogen carbonate. Preferably, triethylamine or diisopropylethylamine is used. Examples of the solvent include dipolar aprotic solvents such as DMF, DMSO and 1-methyl-2-pyrrolidinone; ether solvents such as THF, DME and dioxane; and halogenated solvents such as dichloroethane, chloroform and 2-dichloroethane. Preferably, DMF or THF is used. The reaction temperature may be in the range between -40°C and 140°C, and preferably, in the range between 10°C and 80°C. Although the reaction time may be selected based on the other conditions such as reaction temperature, satisfactory results are usually obtained by employing a time from about 1 minute to about 30 hours.

Alternatively, an isocyanate which can easily be generated by heating Compound (XIV) in an appropriate solvent in the presence of a base may be used after isolation in place of Compound (XIV). Examples of the base include organic amine bases such as triethylamine, diisopropylethylamine and pyridine; and inorganic bases such as sodium hydroxide, potassium hydroxide, sodium carbonate, potassium carbonate and sodium hydrogen carbonate. Preferably, triethylamine, diisopropylethylamine, sodium carbonate or potassium carbonate is used. Examples of the solvent include dipolar aprotic solvents such as DMF, DMSO and 1-methyl-2-pyrrolidinone; ether solvents such as THF, DME and dioxane, and halogenated solvents such as dichloroethane, chloroform and 2-dichloroethane. Preferably, DMF or THF is used. The reaction temperature may be in the range between -40°C and 140°C, and preferably, in the range between 10°C and 80°C. Although the reaction time may be selected based on the other conditions such as reaction temperature, satisfactory results are usually obtained by employing a time from about 1 minute to about 30 hours.

In the reaction formula above, the compound of the Formula (XV) employed as the starting material is known in the art, or can easily be produced by the methods described in reference examples below or by known methods (e.g. a method described in WO 04/037789).

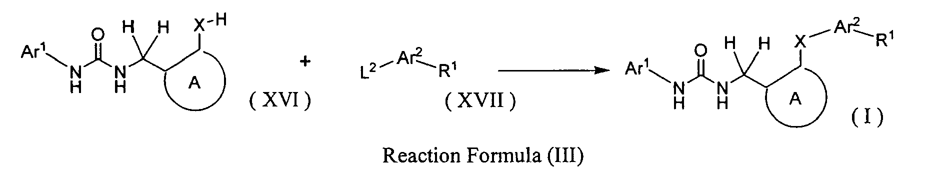

[wherein L² represents Cl, Br, p-toluenesulfonyloxy, methanesulfonyloxy, or trifluoromethanesulfonyloxy, and the other symbols have the same meanings as described above].

The reaction represented by the above reaction formula is performed by known methods. Compound (I) can be synthesized by condensing Compounds (XVI) and (XVII) in an appropriate solvent in the presence of a base.

Examples of the base include organic amine bases such as triethylamine, diisopropylethylamine and pyridine, and inorganic bases such as sodium hydroxide, potassium hydroxide, sodium carbonate, potassium carbonate and sodium hydrogen carbonate. Preferably, sodium hydroxide, potassium hydroxide, sodium carbonate, potassium carbonate, triethylamine or diisopropylethylamine is used. Examples of the solvent include dipolar aprotic solvents such as acetone, DMF, DMSO and 1-methyl-2-pyrrolidinone; ether solvents such as THF, DME and dioxane; halogenated solvents such as dichloroethane, chloroform and 2-dichloroethane; alcohol solvents such as methanol and ethanol; and water. Preferably, acetone, methanol, ethanol, DMF, THF or water is used. The reaction temperature may be in the range between -40°C and 140°C, and preferably, in the range between 10°C and 80°C. Although the reaction time may be selected based on the other conditions such as reaction temperature, satisfactory results are usually obtained by employing a time from about 1 minute to about 30 hours.

In the reaction formula above, the compound of the Formula (XVII) employed as the starting material is commercially available or known in the art, or can easily be produced according to known methods (e.g. a method described in "A New Course of Experimental Chemistry" (published by Maruzen)).

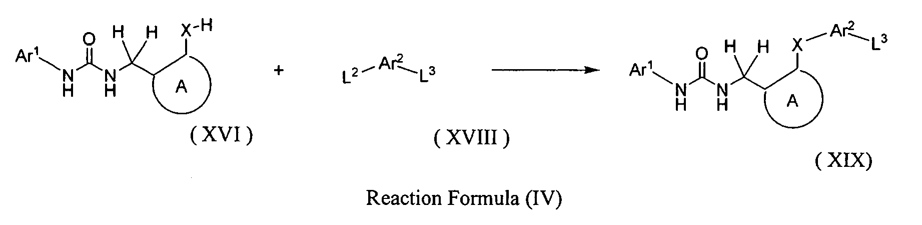

[wherein L³ represents Cl, Br, p-toluenesulfonyloxy, methanesulfonyloxy, or trifluoromethanesulfonyloxy, and the other symbols have the same meanings as described above].

The reaction represented by the above reaction formula is performed by known methods. Compound (XIX) can be synthesized by condensing Compounds (XVI) and (XVIII) in an appropriate solvent in the presence of a base.

Examples of the base include organic amine bases such as triethylamine, diisopropylethylamine and pyridine; and inorganic bases such as sodium hydroxide, potassium hydroxide, sodium carbonate, potassium carbonate and sodium hydrogen carbonate. Preferably, sodium hydroxide, potassium hydroxide, sodium carbonate, potassium carbonate, triethylamine or diisopropylethylamine is used. Examples of the solvent include dipolar aprotic solvents such as acetone, DMF, DMSO and 1-methyl-2-pyrrolidinone; ether solvents such as THF, DME and dioxane; halogenated solvents such as dichloroethane, chloroform and 2-dichloroethane; alcohol solvents such as methanol and ethanol; and water. Preferably, acetone, methanol, ethanol, DMF, THF or water is used. The reaction temperature may be in the range between -40°C and 140°C, and preferably, in the range between 10°C and 80°C. Although the reaction time may be selected based on the other conditions such as reaction temperature, satisfactory results are usually obtained by employing a time from about 1 minute to about 30 hours.

In the reaction formula above, the compound of the Formula (XVIII) employed as the starting material is commercially available or known in the art, or can easily be produced according to known methods (e.g. a method described in "A new course of experimental chemistry" (published from Maruzen)).

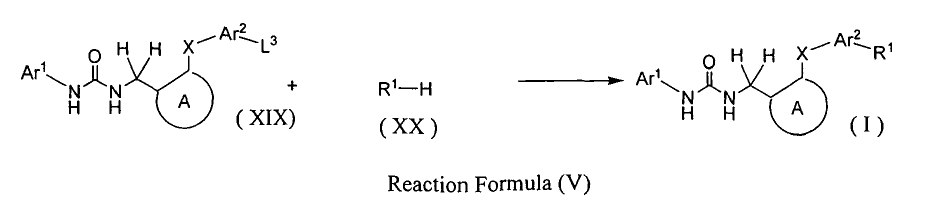

[wherein R¹ -H represents a proton-donor group such as primary and secondary amines, alcohols, and mercapto groups, and the other symbols have the same meanings as described above].

The reaction represented by the above reaction formula is performed by known methods. Compound (I) can be synthesized by condensing Compounds (XIX) and (XX) in an appropriate solvent in the presence of a base.

Examples of the base include organic amine bases such as triethylamine, diisopropylethylamine and pyridine; and inorganic bases such as sodium hydroxide, potassium hydroxide, sodium carbonate, potassium carbonate and sodium hydrogen carbonate. Preferably, sodium carbonate, potassium carbonate, triethylamine or diisopropylethylamine is used. Examples of the solvent include dipolar aprotic solvents such as DMF, DMSO and 1-methyl-2-pyrrolidinone; ether solvents such as THF, DME and dioxane; halogenated solvents such as dichloroethane, chloroform and 2-dichloroethane; and alcohol solvents such as methanol and ethanol. Preferably, methanol, ethanol, DMF or THF is used. The reaction temperature may be in the range between -40°C and 140°C, and preferably, in the range between 10°C and 80°C. Although the reaction time may be selected based on the other conditions such as reaction temperature, satisfactory results are usually obtained by employing a time from about 1 minute to about 30 hours.

Compound (I) can be synthesized by condensing Compounds (XIX) and (XX) in an appropriate solvent in the presence of a base and a metal catalyst. Examples of the base include organic amine bases such as triethylamine, diisopropylethylamine and pyridine; and inorganic bases such as sodium hydroxide, potassium hydroxide, sodium carbonate, potassium carbonate and sodium hydrogen carbonate. Preferably, sodium carbonate, potassium carbonate, triethylamine or diisopropylethylamine is used. Examples of the metal catalyst include Cu, CuI, CuCl, Pd, Pd(OAc)₂, and Pd(PPh₃)₄, and preferably, Cu or CuI is used. Examples of the solvent include dipolar aprotic solvents such as DMF, DMSO and 1-methyl-2-pyrrolidinone; ether solvents such as THF, DME and dioxane; and aromatic solvents such as pyridine. Preferably, pyridine or DMF is used. The reaction temperature may be in the range between -40°C and 140°C, and preferably, in the range between 10°C and 100°C. Although the reaction time may be selected based on the other conditions such as reaction temperature, satisfactory results are usually obtained by employing a time from about 1 minute to about 30 hours. In the reaction formula above, the compound of the Formula (XX) employed as the reactant is commercially available or known in the art.

Conversion of the arylmethylene urea derivatives of the Formula (I):

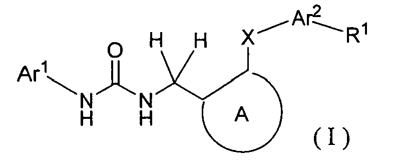

(wherein the symbols have the same meanings as described above)

into a pharmaceutically acceptable salt thereof can be performed by ordinary methods wherein the arylmethylene urea derivative of the Formula (I) is treated with an acid (e.g. inorganic acid such as hydrochloric acid, nitric acid or sulfuric acid; organic acid such as acetic acid or maleic acid; organic sulfonic acid such as p-toluenesulfonic acid, or acidic amino acid such as aspartic acid) or a base (inorganic base such as sodium hydroxide, organic base such as triethylamine, basic amino acid such as lysine or the like) in an appropriate solvent. Examples of the solvent include dipolar aprotic solvents such as DMF, DMSO and 1-methyl-2-pyrrolidinone; ether solvents such as THF, DME and dioxane; halogenated solvents such as dichloroethane, chloroform and 2-dichloroethane; and alcohol solvents such as methanol and ethanol. Preferably, methanol, ethanol or THF is used. The reaction temperature may be in the range between -40°C and 140°C, and preferably, in the range between 10°C and 80°C. Although the reaction time may be selected based on the other conditions such as reaction temperature, satisfactory results are usually obtained by employing a time from about 1 minute to about 30 hours.

In each reaction disclosed herein, the reaction product can be purified by ordinary purification methods, for example, distilation under a normal or reduced pressure, high-performance liquid chromatography, thin layer chromatography or column chromatography, which employ silica gels, as well as washing, recrystallization, reprecipitation and the like. The purification may be performed after each reaction, or after several reactions. Other starting materials and each reagent according to the invention are as such commercially available or known in the art.

The arylmethylene urea derivatives or the pharmaceutically acceptable salts thereof according to the invention are useful as a pharmaceutical for therapy or prophylaxis of inflammatory bowel diseases (e.g. ulcerative colitis and Crohn's disease) and overactive bladder.

The excellent ameliorating effects of the invention on inflammatory bowel diseases can be evaluated by using suitable animal models. The suitable animal models of inflammatory bowel diseases include, but not limited to, for example, dextran sulfate sodium (DSS)-induced mouse models (see e.g. Laboratory Investig., 69, 238-249 (1993)), CD45RB^{Hi} cell-transferred SCID mouse models (see e.g. Immunity, 1, 553-562 (1994)), IL-10 knockout mice (see e.g. Cell, 75, 203-205 (1993)) as well as TNBS-induced models and spontaneous models (see e.g. J. Gastroenterol, 37, 409-17(2002)). The effects on such models which have developed colitis can be evaluated by monitoring the weight loss, diarrhea, bloody stool, hypertrophy of colon, leukocyte infiltration and the changes of inflammatory marker proteins (CRP, serum amyloid A) in blood as indices.

The effect of the arylmethylene urea derivative of the invention can be evaluated by employing the effect on the production of substances involved in the development of inflammatory bowel diseases, for example, inflammatory cytokines and inflammatory mediators, as an index. Estimation of such an inhibitory effect can be performed by, for example, quantifying the amount of cytokines or mediators produced in the culture supernatant of cells that were given appropriate stimulation (endotoxin, ionomycin or the like, for example). As for the cells employed for the evaluation, it is preferable to employ peripheral blood, various types of isolated blood cells (monocytes, neutrophils, platelet and the like), human monocyte cell strain THP-1 and mouse monocyte cell strain RAW264.7 or the like, but the types of the cells are not limited to these. The inflammatory cytokines and mediators to be quantified include, for example, IL-1β, IL-6, IL-8, IL-10, IL-18, TNF-α, TNF-β, TXB2, LTB4 and the like. As for the method for quantifying such cytokines and mediators, any of enzyme-linked immunoassay (ELISA), homogeneous timeresolved fluorescence (HTRF) and radioimmunoassay (RIA) may be employed. It can be confirmed that the arylmethylene urea derivatives of the present invention is required for inhibiting the production of inflammatory cytokines and inflammatory mediators by these measurements.

The excellent ameliorating effects of the invention on overactive bladder can be evaluated by using suitable animal models. The suitable animal models of frequent urination include cystometries of rat models for rhythmic bladder contraction (see e.g. Eur J Pharmacol (2000) 395, 241-246) and cyclophosphamide-induced cystitis rat (see e.g. J Pharmacol Sci (2004) 95, 458-465), whose frequencies of bladder contraction or micturition can be used as indices for evaluation of the effects. However, the method of evaluation is not limited to these.

The arylmethylene urea derivatives of the invention can be used as a pharmaceutical useful for the treatment or prophylaxis of inflammatory bowel diseases and overactive bladder in mammals (e.g. mice, rats, hamsters, rabbits, dogs, monkeys, humans and the like). When the pharmaceutical is clinically applied, the pharmaceutical may be the compound or the salt thereof itself, or the pharmaceutical may comprise a pharmaceutically acceptable carrier or diluent, examples of which include binders (such as syrup, gelatin, gum arabic; sorbitol, polyvinyl chloride and tragacanth), excipients (such as sugar, lactose, cornstarch, calcium phosphate, sorbitol and glycine), lubricants (such as magnesium stearate, polyethylene glycol, talc and silica) and the like. Examples of the form of administration include drugs for oral administration such as tablet, capsule, granule, powder and syrups as well as drugs for parenteral administration such as inhalants, injections, suppositories and solutions. The form of administration may also be topical administrations, examples of which include ointments, creams and cataplasms. Such formulations can be prepared by methods generally known in the art. The pharmaceutical of the invention preferably contains the arylmethylene urea derivative or the pharmaceutically acceptable salt thereof according to the invention in an amount of 0.001-99% by weight, more preferably, 0.01-70 % by weight. The dosage may be appropriately determined based on symptoms, age, weight, gender, method of administration and the like. For an adult human, the dose in terms of the active ingredient may be 0.01 mg - 5 g/day for injections, and 0.1 mg - 10 g/day for oral administration, and the pharmaceutical can be administered in one time or dividedly in several times.

The arylmethylene urea derivatives of the invention can be admixed with other drug(s) in an appropriate ratio or in combination with other drug(s) for complementation or enhancement of the therapeutic or prophylactic effect on inflammatory bowel diseases, or for the reduction of dosage. Examples of the pharmaceuticals which can be used in combination with the arylmethylene urea derivatives of the invention include aminosalicylic acid preparations (such as salazopyrin and mesalazine) and derivatives thereof, prostaglandin synthetase inhibitors, steroids (such as prednisolone, methylprednisolone, hydrocortisone, betamethasone and budesonide), immunosuppressive drugs (such as mercaptopurine, methotrexate, azathioprine, cyclosporine and tacrolimus), protease inhibitors (such as ulinastatin), leukotriene production inhibitors, leukotriene receptor antagonists, TNF-α antagonists, IL-6 antagonists, adhesion molecule inhibitors, 5-lipoxygenase inhibitors, elastase inhibitors, metalloprotease inhibitors, PDE inhibitors, active oxygen removers, active oxygen-production inhibitors, mucosal protective agents, mucosal repairing agents, adrenocorticotropic hormones, antibiotics (such as metronidazole) and the like.

The arylmethylene urea derivatives of the invention can be admixed with other drug(s) in an appropriate ratio or in combination with other drug(s) for complementation or enhancement of the therapeutic or prophylactic effect on overactive bladder, or for the reduction of dosage. Examples of the pharmaceuticals which may be used in combination with the arylmethylene urea derivatives of the present invention include other prophylactic or therapeutic drugs for dysuria.

Other prophylactic or therapeutic drugs for dysuria include, for example, anticholinergic drugs such as propantheline, oxybutynin, propiverine, tolterodine, temiverine, trospium, darifenacin, solifenacin and KRP-197; smooth muscle relaxants such as flavoxate; potassium channel opener such as NS-8, ZD-0947, KW-7158, ABT-598 and WAY-151616; calcium channel antagonists such as nifedipine and flunarizine; skeletal muscle relaxants such as baclofen, diazepam and lanperisone; antidepressants such as imipramine, desipramine, fluoxetine, fluvoxamine, milnacipran, paroxetine and duloxetine; vasopressin agonists such as desmopressin; tachykinin antagonists such as TAK-637, SR-48968 and talnetant; β-agonists such as clenbuterol and KUC-7483; vanilloid agonists such as capsaicin and resiniferatoxin; PGE antagonists such as ONO-8711 and ONO-8992; COX inhibitors such as flurbiprofen; α1 agonists such as R-450; and α1 antagonists such as doxazosin, indramin, terazosin, urapidil, alfuzosin, prazosin, naftopidil, tamsulosin, selodosin, fiduxosin and KMD-3213.

### [Examples]

The present invention will now be described in detail by way of reference examples and examples thereof. However, these are merely illustrative examples, and not limiting the scope of the invention.

"Room temperature" used in the following reference examples and examples normally indicates from about 10°C to about 35°C. Solvents set forth in parentheses in NMR data indicate solvents used for the measurements.

Other abbreviations used herein indicate the following meanings:
s: singlet
d: doublet
t: triplet
q: quartet
dd: double doublets
br: broad
J: coupling constant
Hz: hertz
CDCl₃ : deuteriochloroform
CD₃ OD: deuteriomethanol
¹H-NMR: proton nuclear magnetic resonance
MS: mass spectrometry
ESI: electron spray ionization method
THF: tetrahydrofuran
DMF: dimethylformamide
DMSO: dimethyl sulfoxide

Reference Example 1

### 3-t-butyl-1-p-tolyl-1H-pyrazol-5-amine hydrochloric acid salt

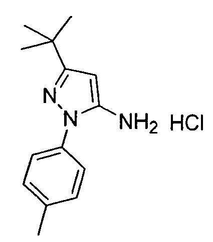

To methanol (350 mL), 4,4-dimethyl-3-oxopropanenitrile (85.0 g) and p-tolhydrazine hydrochloric acid salt (76.0 g) were added, and the resulting mixture was gently refluxed for 15 hours. Methanol was evaporated and diethyl ether was added to the residue to crystallize it. The crystals were collected by filtration through a glass filter, and washed with a small amount of diethyl ether to obtain the desired product (108.3 g, yield: 85%).
¹H-NMR (CDCl₃):δ 7.42 (d, 2H, J = 8.2 Hz), 7.24 (d, 2H, J = 8.2 Hz), 5.51 (s, 1H), 3.69 (br, 2H), 2.38 (s, 3H), 1.30 (s, 9H)
MS (ESI):230 (M+H⁺)

Reference Example 2

### 3-(furan-2-yl)-1-p-tolyl-1H-pyrazol-5-amine hydrochloric acid salt

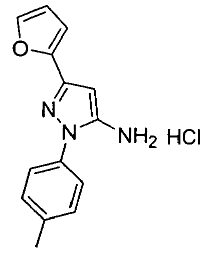

To ethanol (50 mL), 3-(furan-2-yl)-3-oxopropanenitrile (4.10 g) and p-tolhydrazine hydrochloric acid salt (5.30 g) were added, and the resulting mixture was gently refluxed for 4 hours. Ethanol was evaporated and the residue was recrystallized from ethyl acetate to obtain the desired product (7.70 g, yield: 92%). ¹H-NMR (CDCl₃):δ 7.44 (dd, 1H, J = 0.8, 1.7 Hz), 7.42 (d, 2H, J = 8.3 Hz), 7.27 (d, 2H, J = 8.3 Hz), 7.30 (br, 1H), 6.45 (dd, 1H, J = 1. 7, 3.4 Hz), 5.93 (s, 1H), 2.38 (s, 3H)
MS (ESI):240 (M+H⁺)

Reference Example 3

### N-(4-chloro-2-cyanophenyl)formamide

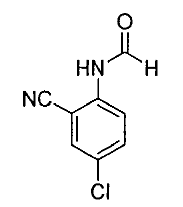

A mixture of acetic anhydride (14 mL) and formic acid (6.0 mL) was stirred at 60°C for 3 hours, and the resulting mixture was added to a solution prepared from 2-amino-5-chlorobenzonitrile (1.1 g) and ethyl formate (5.6 mL). The resulting mixture was stirred at room temperature for 1 hour. The precipitated solids were filtered and washed with hexane to obtain the desired product (1.1 g, yield: 91%). ¹H-NMR (CDCl₃):δ 8.50 (s, 1H), 8.47 (d, 1H, J = 9.5 Hz), 7.59-7.57 (m, 2H)
MS (ESI):179, 181 (M-H)

Reference Example 4

### 2-(aminomethyl)-4-chloro-N-methylbenzenamine

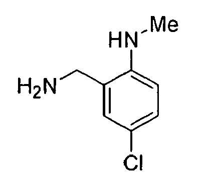

N-(4-chloro-2-cyanophenyl) formamide (59 mg) was weighed and added to a 50 mL two-necked flask in which the atmosphere had been replaced with argon, and then dry THF (2.5 mL) was added into the flask to make a solution. To this solution, a solution (1.2 mL) of borane-THF complex in THF was added and the resulting mixture was stirred at room temperature for 45 minutes. The mixture was quenched by addition of distilled water, and aqueous 1M sodium hydroxide solution (4 mL) was added, after which the organic layer was evaporated under reduced pressure. The obtained residue was extracted with dichloromethane (7 mL) three times. The organic layers were combined and dried over anhydrous sodium sulfate. The obtained residue was purified by a 0.5mm TLC plate (ethyl acetate/methanol = 15/1) to obtain the desired product (26 mg, yield: 46%).
¹H-NMR (CDCl₃):δ 7.14 (dd, 1H, J = 8.7, 2.0 Hz), 7.00 (d, 1H, J = 2.0 Hz), 6.54 (d, 1H, J = 8.7 Hz), 3.84 (s, 2H), 2.83 (s, 3H)

Reference Example 5

### 2,2,2-trichloroethyl 3-t-butyl-1-p-tolyl-1H-pyrazol-5-yl carbamate

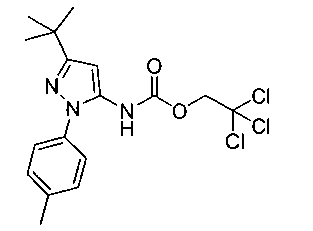

In ethyl acetate (500 mL), 3-t-butyl-1-p-tolyl-1H-pyrazol-5-amine hydrochloric acid salt (75.0 g) was dissolved and the resulting mixture was cooled to 0°C. To the mixture, an aqueous sodium hydroxide (30 g) solution (250 mL) was added and 2,2,2-trichloroethyl chloroformate (85.0 g) was also added thereto dropwise at 0°C for 1 hour. The mixture was allowed to react at room temperature for 90 minutes and the organic layer was separated. The aqueous layer was extracted with ethyl acetate (100 mL) twice. The organic layers were combined, washed with saturated brine and dried over anhydrous sodium sulfate. The residue obtained by concentration was washed with n-hexane to obtain the desired product. (97.8 g, yield: 86%).
¹H-NMR (CDCl₃):δ 7.34 (d, 2H, J = 8.3 Hz), 7.29 (d, 2H, J = 8.3 Hz), 6.80 (br, 1H), 6.42 (br, 1H), 4.81 (br, 2H), 2.41 (s, 3H), 1.34 (s, 9H)
MS (ESI):404, 406, 408 (M+H⁺)

Reference Example 6

### 2,2,2-trichloroethyl 3-(furan-2-yl)-1-p-tolyl-1H-pyrazol-5-yl carbamate

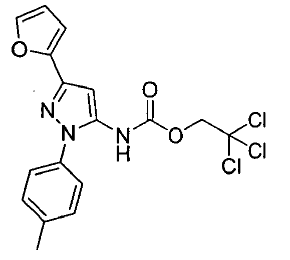

In ethyl acetate (30 mL), 3-(furan-2-yl)-1-p-tolyl-1H-pyrazol-5-amine hydrochloric acid salt (1.67 g) was dissolved and the resulting mixture was cooled to 0°C. To the mixture, aqueous 1N sodium hydroxide solution (20 mL) was added and 2,2,2-trichloroethyl chloroformate (2.0 mL) was added thereto dropwise at 0°C. The mixture was allowed to react at room temperature for 60 minutes and the organic layer was separated. The aqueous layer was extracted with ethyl acetate. The organic layers were combined, washed with water and saturated brine, and dried over anhydrous sodium sulfate. The residue obtained by concentration was purified by silica gel column chromatography (ethyl acetate/n-hexane = 1/9 - 3/7) to obtain the desired product (2.48 g, yield: 99%).
¹H-NMR (CDCl₃):δ 7.47 (d, 1H, J = 1.7 Hz), 7.41-7.32 (m, 4H), 6.91 (br, 1H), 6.80 (br, 1H), 6.73 (d, 1H, J = 3.2 Hz), 6.47 (dd, 1H, J = 1.7, 3.2 Hz), 4.83 (s, 2H), 2.43 (s, 3H)
MS (ESI):414 (M+H⁺)

Reference Example 7

### o-hydroxybenzylamine

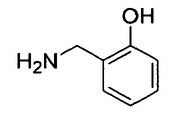

To a solution of o-cyanophenol (3.00 g) in THF (10 mL), 1M borane-THF complex solution in THF (25.0 mL) was added at -78°C, and the resulting mixture was stirred at 0°C for 1 hour. Triethanolamine (3.40 g) and water (30 mL) were added to the mixture, which was then stirred sufficiently and extracted with ethyl acetate. The extract was washed with saturated sodium hydrogen carbonate solution (15 mL) and with saturated brine (15 mL), dried over anhydrous sodium sulfate, and evaporated under reduced pressure to obtain the desired product (2.77 g, yield: 90%).
¹H-NMR (CD₃ OD):δ 7.08 (m, 2H), 6.74 (m, 2H), 3.84 (s, 2H)
MS (ESI):124 (M+H⁺)

Reference Example 8

### 2-(aminomethyl)-4-fluorophenol

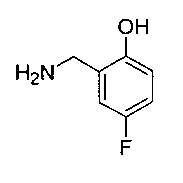

By following the same procedure as described in Reference Example 7, the desired product (263 mg, yield: 56%) was obtained from 5-fluoro-2-hydroxybenzonitrile (457 mg).
¹H-NMR (CDCl₃):δ 6.88-6.68 (m, 3H), 4.09 (s, 2H)
MS (ESI):142 (M+H⁺)

Reference Example 9

### 1-(2-hydroxybenzyl)-3-(3-t-butyl-1-p-tolyl-1H-pyrazol-5-yl)urea

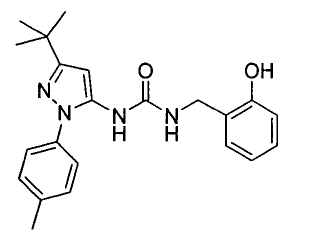

To a solution of o-hydroxybenzylamine (369 mg) in DMSO (15 mL), 2,2,2-trichloroethyl 3-t-butyl-1-p-tolyl-1H-pyrazol-5-yl carbamate (1213 mg) and diisopropylethylamine (0.65 mL) were added and the resulting mixture was stirred at 60°C for 24 hours. Saturated ammonium chloride was added to the reaction mixture, which was then extracted with ethyl acetate. The extract was dried over anhydrous sodium sulfate, and evaporated under reduced pressure. The obtained residue was purified by silica gel column chromatography (ethyl acetate/n-hexane = 1/3) to obtain the desired product (808 mg, yield: 71%).
¹H-NMR (CDCl₃):δ 9.12 (br, 1H), 7.24-6.79 (m, 8H), 6.20 (s, 2H), 5.61 (t, 1H, J = 6.3 Hz), 4.30 (d, 2H, J = 6.3 Hz), 2.33 (s, 3H), 1.33 (s, 9H)
MS (ESI):379 (M+H⁺)

Reference Example 10

### 1-(2-nitrobenzyl)-3-(3-t-butyl-1-p-tolyl- 1H-pyrazol-5-yl)urea

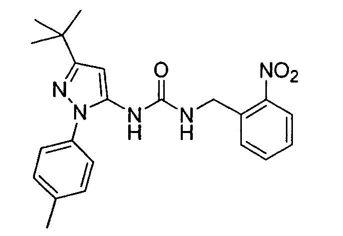

By following the same procedure as described in Reference Example 9, the desired product (1.2 g, yield: 95%) was obtained from 2,2,2-trichloroethyl 3-t-butyl-1-p-tolyl-1H-pyrazol-5-yl carbamate (1.3 g) and 2-nitrobenzylamine hydrochloric acid salt (651 mg).
¹H-NMR (CDCl₃):δ 8.04-7.43 (m, 4H), 7.28 (d, 2H, J = 8.4 Hz), 7.17 (d, 2H, J = 8.4 Hz), 6.21 (s, 1H), 6.20 (s, 1H), 5.84 (t, 1H, J = 6.4 Hz), 4.60 (d, 2H, J = 6.4 Hz), 2.34 (s, 3H), 1.33 (s, 9H)
MS (ESI):408 (M+H⁺)

Reference Example 11

### 1-(2-aminobenzyl)-3-(3-t-butyl-1-p-tolyl-1H-pyrazol-5-yl)urea

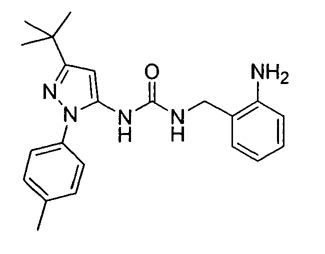

Palladium carbon (133 mg), methanol (27 mL), and THF (13 mL) were added to 1-(2-nitrobenzyl)-3-(3-t-butyl-1-p-tolyl-1H-pyrazol-5-yl)urea (1.1 g), and the resulting mixture was stirred overnight under hydrogen atmosphere at 1 atm. Dichloromethane was added to the reaction mixture, which was then filtered through Celite, and the organic solvent was evaporated under reduced pressure. The obtained residue was filtered and washed with hexane to obtain the desired product (956 mg, yield: 93%).
¹H-NMR (CD₃ OD):δ 7.31-6.60 (m, 8H), 6.30 (s, 1H), 4.23 (s, 2H), 2.39 (s, 3H), 1.31 (s, 9H)
MS (ESI):378 (M+H⁺)

Reference Example 12

### 1-(5-chloro-2-(methylamino) benzyl)-3-(3-t-butyl-1-p-tolyl-1H-pyrazol-5-yl)urea

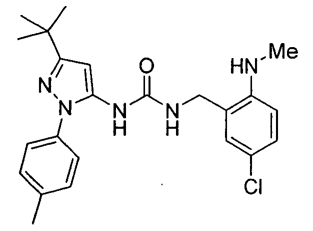

By following the same procedure as described in Reference Example 9, the desired product (45 mg, yield: 71 %) was obtained from 2,2,2-trichloroethyl 3-t-butyl-1-p-tolyl-1H-pyrazol-5-yl carbamate (79 mg) and 2-(aminomethyl)-4-chloro-N-methylbenzenamine (26 mg).
¹H-NMR (CD₃ OD):δ 7.30-7.25 (m, 4H), 7.09 (dd, 1H, J = 8.6, 2.4 Hz), 6.99 (d, 1H, J = 2.4 Hz), 6.54 (d, 1H, J = 8.6 Hz), 6.29 (s, 1H), 4.16 (s, 2H), 2.75 (s, 3H), 2.38 (s, 3H), 1.31 (s, 9H)
MS (ESI):424, 426 (M-H)

Reference Example 13

### 1-(5-fluoro-2-hydroxybenzyl)-3-(3-t-butyl-1-p-tolyl-1H-pyrazol-5-yl)urea

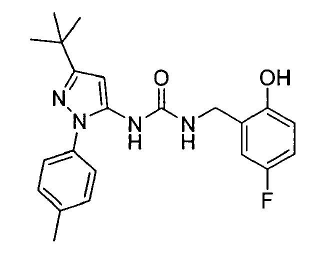

By following the same procedure as described in Reference Example 9, the desired product (520 mg, yield: 85%) was obtained from 2,2,2-trichloroethyl 3-t-butyl-1-p-tolyl-1H-pyrazol-5-yl carbamate (628 mg) and 2-(aminomethyl)-4-fluorophenol (263 mg).
¹H-NMR (CDCl₃):δ 9.01 (br, 1H), 7.19-6.68 (m, 7H), 6.38 (br, 1H), 6.20 (s, 1H), 5.74 (br, 1H), 4.20 (d, 2H, J = 6.6 Hz), 2.31 (s, 3H), 1.31 (s, 9H)
MS (ESI):397 (M+H⁺)

Reference Example 14

### Methyl 3-(2-chloropyrimidin-4-yloxy)thiophen-2-caboxylate

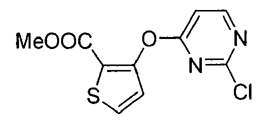

To a solution of 2,4-dichloropyrimidine (408 mg) and methyl 3-hydroxythiophen-2-caboxylate (475 mg) in DMF (1 mL), anhydrous potassium carbonate (600 mg) was added and the resulting mixture was stirred at room temperature for 20 hours. The reaction mixture was poured into 5% citric acid solution and the resulting mixture was extracted with ethyl acetate. The extract was dried over anhydrous sodium sulfate, and evaporated under reduced pressure. The obtained residue was purified by recrystallization from ethyl acetate/n-hexane to obtain the desired product (462 mg, yield: 62%).
¹H-NMR (CDCl₃):δ 8.48 (d, 1H, J = 5.6 Hz), 7.54 (d, 1H, J = 5.6 Hz), 6.99 (d, 1H, J = 5.6 Hz), 6.95 (d, 1H, J = 5.6 Hz), 3.77 (s, 3H)
MS (ESI):271, 273 (M+H⁺)

Reference Example 15

### Methyl 3-(2-morpholinopyrimidin-4-yloxy)thiophen-2-caboxylate

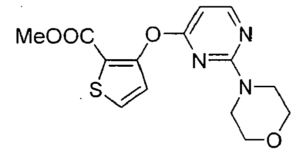

To a solution of methyl 3-(2-chloropyrimidin-4-yloxy)thiophen-2-carboxylate (323 mg) in ethanol (1.2 mL), morpholine (0.21 mL) was added and the resulting mixture was stirred at room temperature for 2 hours. The solvent was evaporated under reduced pressure, and then the obtained residue was purified by silica gel column chromatography (ethyl acetate/n-hexane = 1/2) to obtain the desired product (250 mg, yield: 65%).
¹H-NMR (CDCl₃):δ 8.22 (d, 1H, J = 5.4 Hz), 7.48 (d, 1H, J = 5.4 Hz), 6.96 (d, 1H, J = 5.4 Hz), 6.22 (d, 1H, J = 5.4 Hz), 3.78 (s, 3H), 3.69-3.62 (m, 8H)
MS (ESI):320, 322 (M+H⁺)

Reference Example 16

### 3-(2-morpholinopyrimidin-4-yloxy)thiophen-2-caboxylic acid

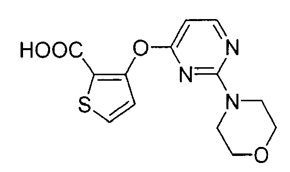

To a solution of methyl 3-(2-morpholinopyrimidin-4-yloxy)thiophen-2-carboxylate (240 mg) in THF (1.2 mL), 1N sodium hydroxide solution (1.8 mL) was added and the resulting mixture was stirred at room temperature for 4 hours. To the reaction mixture, 1N hydrochloric acid (2 mL) was added, and the mixture was then extracted with chloroform/methanol (10/1) mixed solution. The extract was dried over anhydrous sodium sulfate, and evaporated under reduced pressure to obtain the desired product (250 mg, yield: 100%).
¹H-NMR (CDCl₃):δ 8.23 (d, 1H, J = 5.4 Hz), 7.54 (d, 1H, J = 5.4 Hz), 6.97 (d, 1H, J = 5.4 Hz), 6.21 (d, 1H, J = 5.4 Hz), 3.77-3.62 (m, 8H)
MS (ESI):306, 308 (M+H⁺)

Reference Example 17

### 3-(2-morpholinopyrimidin-4-yloxy)thiophen-2-carboxylic acid amide

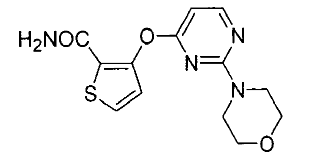

To a solution of 3-(2-morpholinopyrimidin-4-yloxy)thiophen-2-caboxylic acid (235 mg) in DMF (3 mL), (benzotriazol-1-yloxy)tripyrrolidinophosphonium hexafluorophosphate (597 mg), 1-hydroxybenzotriazole (155 mg), ammonium chloride (82 mg) and diisopropylethylamine (0.55 mL) were added and the resulting mixture was stirred at room temperature for 1 hour. Saturated ammonium chloride was added to the reaction mixture, which was then extracted with dichloromethane. The extract was dried over anhydrous sodium sulfate, and evaporated under reduced pressure. The obtained residue was purified by silica gel column chromatography (ethyl acetate/n-hexane = 2/1) to obtain the desired product (165 mg, yield: 70%).
¹H-NMR (CDCl₃):δ 8.27 (d, 1H, J = 5.4 Hz), 7.47 (d, 1H, J = 5.4 Hz), 6.99 (d, 1H, J = 5.4 Hz), 6.71 (br, 1H), 6.20 (d, 1H, J = 5.4 Hz), 5.82 (br, 1H), 3.70 (br, 8H)
MS (ESI):305, 307 (M+H⁺)

Reference Example 18

### 3-t-pentyl-1-p-tolyl-1H-pyrazol-5-amine hydrochloric acid salt

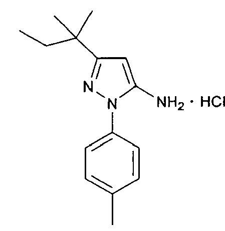

In THF (6 mL), 2,2-dimethylbutanoic acid (2.20 mL) was dissolved, and methanol (4 mL) was added thereto. The resulting mixture was cooled to 0°C, and trimethylsilyldiazomethane (13.2 mL) was added thereto. The resulting mixture was then stirred at room temperature for 3 days. The resulting reaction solution was evaporated under reduced pressure to obtain methyl 2,2-dimethylbutanoate (1.79 g, yield: 78%).
¹H-NMR (CDCl₃):δ 3.66 (s, 3H), 1.56 (q, 2H, J = 7.5 Hz), 1.16 (s, 6H), 0.83 (t, 3H, J = 7.5Hz)

Sodium hydride (1.04 g) was weighed and added to a 50 mL two-necked flask
wherein the atmosphere had been replaced with argon, and was then washed with hexane (10 mL) three times and dried. To this solution, dry THF (15 mL) was added and the resulting mixture was heated to 75°C. A solution separately prepared from methyl 2,2-dimethylbutanoate (2.34 g), dry acetonitrile (1.25 mL) and dry THF (10 mL) was added to the above mixture, which was then stirred overnight at 75°C. Ethyl acetate (20 mL) and aqueous 1M hydrochloric acid solution (20 mL) were added to the reaction solution to extract the solution. The obtained organic layer was washed with distilled water (25 mL) and then with saturated brine (25 mL), dried over anhydrous sodium sulfate, and evaporated under reduced pressure. The obtained residue and p-tolylhydrazine hydrochloric acid salt (1.45 g) were dissolved in methanol (20 mL) and the resulting mixture was refluxed for 3 nights, after which the reaction solution was evaporated under reduced pressure. The obtained residue was filtered and washed with ether to obtain the desired product (2.38 g, yield: 47%). ¹H-NMR (DMSO-d₆):δ 7.47-7.39 (m, 4H), 5.60 (s, 1H), 2.39 (s, 3H), 1.60 (q, 2H, J = 7.5 Hz), 1.23 (s, 6H), 0.77 (t, 3H, J = 7.5 Hz)
MS (ESI):244 (M+H⁺)

Reference Example 19

### 2,2,2-trichloroethyl 3-t-pentyl-1-p-tolyl-1H-pyrazol-5-yl carbamate

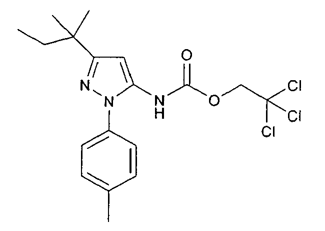

To ethyl acetate (20 mL), 3-t-pentyl-1-p-tolyl-1H-pyrazol-5-amine hydrochloric acid salt (2.38 g) was added and the resulting mixture was cooled to 0°C. A solution separately prepared from sodium hydroxide (851 mg) and distilled water (10 mL) was then added thereto. The resulting mixture was stirred at 0°C for 30 minutes and 2,2,2-trichloroethyl chloroformate (1.64 mL) was added to the mixture, which was then stirred overnight at room temperature. The reaction solution was extracted, and the obtained aqueous layer was extracted with ethyl acetate (20 mL) twice. The organic layers were combined, washed with distilled water (30 mL) twice, dried over anhydrous sodium sulfate, and evaporated under reduced pressure. The obtained residue was filtered and washed with hexane to obtain the desired product (1.78 g, yield: 50%).
¹H-NMR (CDCl₃):δ 7.35-7.26 (m, 4H), 6.39 (s, 1H), 4.81 (s, 2H), 2.41 (s, 3H), 1.66 (q, 2H, J = 7.5 Hz), 1.30 (s, 6H), 0.82 (t, 3H, J = 7.5 Hz)

Reference Example 20

### 1-(2-hydroxybenzyl)-3-(3-t-pentyl-1-p-tolyl-1H-pyrazol-5-yl)urea

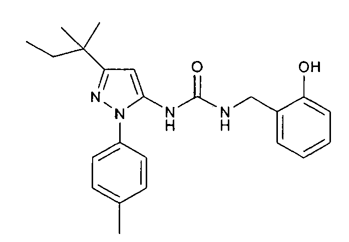

In acetonitrile (10 mL), 2,2,2-trichloroethyl 3-t-pentyl-1-p-tolyl-1H-pyrazol-5-yl carbamate (612 mg) and 2-hydroxybenzylamine (150 mg) were dissolved, after which diisopropylethylamine (382 µL) was added thereto. The resulting mixture was stirred at 60°C for 13 hours. The reaction solution was evaporated under reduced pressure, and the obtained residue was purified by silica gel column chromatography (ethyl acetate/n-hexane =1/3 → 1/1) to obtain the desired product (455 mg, yield: 95%).
¹H-NMR (CDCl₃):δ 9.11 (s, 1H), 7.24-6.79 (m, 8H), 6.21 (s, 1H), 6.16 (s, 1H), 5.63 (t, 1H, J = 6.4 Hz), 4.29 (d, 2H, J = 6.4 Hz), 2.32 (s, 3H), 1.64 (q, 2H, J = 7.5 Hz), 1.27 (s, 6H), 0.82 (t, 3H, J = 7.5 Hz)
MS (ESI):393 (M+H⁺)

Reference Example 21

### 3-cyclopentyl-1-p-tolyl-1H-pyrazol-5-amine hydrochloric acid salt

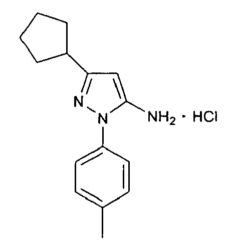

To methanol (35 mL) which had been cooled to -10°C, thionyl chloride (3.23 mL) was added and the resulting mixture was stirred for 30 min. Cyclopentanecarboxylic acid (3.00 mL) was added to the mixture, which was then stirred at -10°C for 1 hour and at room temperature for 12 hours. The reaction solution was evaporated under reduced pressure, and the obtained residue was neutralized with saturated aqueous sodium hydrogen carbonate solution. The neutralized solution was extracted with dichloromethane (5 mL) three times. The organic layers were combined, dried over anhydrous sodium sulfate, and evaporated under reduced pressure to obtain methyl cyclopentanoate (2.55 g, yield: 72%).
¹H-NMR (CDCl₃):δ 3.67 (s, 3H), 2.77-2.69 (m, 1H), 1.93-1.53 (m, 8H)

Sodium hydride (562 mg) was weighed and added to a 100 mL three-necked flask in which the atmosphere had been replaced with argon, and the resulting mixture was washed with hexane (5 mL) three times and dried. To this solution, dry THF (7 mL) was added and the resulting mixture was heated to 75°C, after which a solution separately prepared from methyl cyclopentanoate (1.10 g), dry acetonitrile (586 µL) and dry THF (5 mL) was added. The resulting mixture was stirred at 75°C for 13 hours. Ethyl acetate (10 mL) and aqueous 1M hydrochloric acid solution (10 mL) were added to the reaction solution, which was then extracted. The obtained aqueous layer was extracted with ethyl acetate (10 mL) three times. The organic layers were combined, dried over anhydrous sodium sulfate, and evaporated under reduced pressure. The obtained residue and p-tolylhydrazine hydrochloric acid salt (524 mg) were dissolved in methanol (8 mL) and the resulting mixture was refluxed for 12.5 hours, after which the reaction solution was evaporated under reduced pressure. The obtained residue was filtered and washed with ether to obtain the desired product (825 mg, yield: 35%).
¹H-NMR (DMSO-d₆):δ 7.46-7.37 (m, 4H), 5.59 (s, 1H), 3.00-2.96 (m, 1H), 2.38 (s, 3H), 1.99-1.60 (m, 8H)
MS (ESI):242 (M+H⁺)

Reference Example 22

### 2,2,2-trichloroethyl 3-cyclopentyl-1-p-tolyl-1H-pyrazol-5-yl carbamate

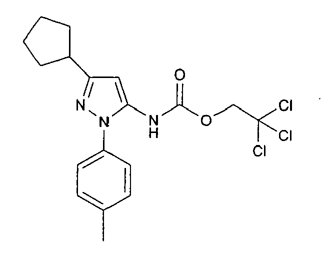

To ethyl acetate (7 mL), 3-cyclopentyl-1-p-tolyl-1H-pyrazol-5-amine hydrochloric acid salt (825 mg) was added and the resulting mixture was cooled to 0°C. A solution separately prepared from sodium hydroxide (297 mg) and distilled water (7 mL) was then added thereto. The mixture was stirred at 0°C for 30 minutes and 2,2,2-trichloroethyl chloroformate (572 µL) was added thereto, followed by stirring at room temperature for another 13 hours. The reaction solution was extracted, and the obtained aqueous layer was extracted with ethyl acetate (7 mL) twice. Then the organic layers were combined, washed with distilled water (10 mL) twice, dried over anhydrous sodium sulfate, and evaporated under reduced pressure. The obtained residue was purified by silica gel column chromatography (ethyl acetate/n-hexane =1/50 → 2/9) to obtain the desired product (1.29 g, quantitative). ¹H-NMR (CDCl₃):δ 7.35-7.29 (m, 4H), 6.82 (br, 1H), 6.38 (s, 1H), 4.81 (s, 2H), 3.14-3.06 (m, 1H), 2.41 (s, 3H), 2.09-1.65 (m, 8H)

Reference Example 23

### 1-(2-hydroxybenzyl)-3-(3-cyclopentyl-1-p-tolyl-1H-pyrazol-5-yl)urea

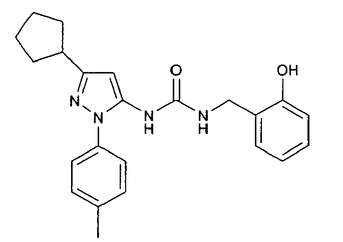

In acetonitrile (3 mL), 2,2,2-trichloroethyl 3-cyclopentyl-1-p-tolyl-1H-pyrazol-5-yl carbamate (130 mg) and 2-hydroxybenzylamine (50.0 mg) were dissolved, after which diisopropylethylamine (81.6 µL) was added thereto. The resulting mixture was stirred at 60°C for 14 hours. The reaction solution was evaporated under reduced pressure, and the obtained residue was purified by silica gel column chromatography (ethyl acetate/n-hexane =2/3 → 3/2) to obtain the desired product (102 mg, yield: 84%).
¹H-NMR (CDCl₃):δ 9.09 (br, 1H), 7.24-6.79 (m, 8H), 6.22 (s, 1H), 6.15 (s, 1H), 5.62 (t, 1H, J = 6.4 Hz), 4.29 (d, 2H, J = 6.4 Hz), 3.11-3.02 (m, 1H), 2.34 (s, 3H), 2.10-1.64 (m, 8H)
MS (ESI):391 (M+H⁺)

Reference Example 24

### 3-cyclopropyl-1-p-tolyl-1H-pyrazol-5-amine hydrochloric acid salt

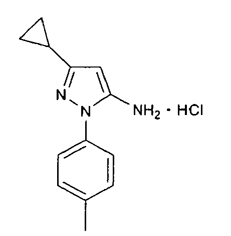

Sodium hydride (775 mg) was weighed and added to a 100 mL three-necked flask, in which the atmosphere had been replaced with argon, and then washed with hexane (6 mL) three times and dried. To this solution, dry THF (8 mL) was added and the resulting mixture was heated to 75°C. A solution separately prepared from methyl cyclopropanoate (1.11 g), dry acetonitrile (757 µL) and dry THF (5 mL) was added and the resulting mixture was stirred at 75°C for 17.5 hours. Ethyl acetate (10 mL) and aqueous 1M hydrochloric acid solution (10 mL) were added to the reaction solution, which was then extracted. The obtained aqueous layer was extracted with ethyl acetate (10 mL) three times. The organic layers were combined, dried over anhydrous sodium sulfate, and evaporated under reduced pressure. The obtained residue and p-tolylhydrazine hydrochloric acid salt (1.22 g) were dissolved in methanol (16 mL) and the resulting mixture was refluxed for 15.5 hours, and then the reaction solution was evaporated under reduced pressure. The obtained residue was filtered with ether, and the filtrate was evaporated under reduced pressure to obtain the desired product (1.30 g, yield: 47%).
¹H-NMR (DMSO-d₆):δ 7.45-7.37 (m, 4H), 5.48 (s, 1H), 2.38 (s, 3H), 1.94-1.88 (m, 1H), 1.06-1.02 (m, 2H), 0.87-0.83 (m, 2H)

Reference Example 25

### 2,2,2-trichloroethyl 3-cyclopropyl-1-p-tolyl-1H-pyrazol-5-yl carbamate

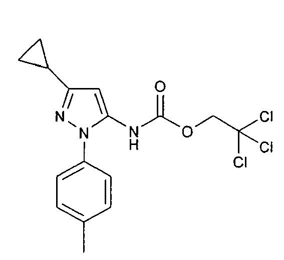

To ethyl acetate (12 mL), 3-cyclopropyl-1-p-tolyl-1H-pyrazol-5-amine hydrochloric acid salt (1.30 g) was added and the resulting mixture was cooled to 0°C. A solution separately prepared from sodium hydroxide (525 mg) and distilled water (12 mL) was then added thereto. The mixture was stirred at 0°C for 30 minutes and 2,2,2-trichloroethyl chloroformate (1.01 mL) was added thereto, followed by stirring at room temperature for 18 hours. The reaction solution was extracted, and the obtained aqueous layer was extracted with ethyl acetate (12 mL) twice. The organic layers were combined, washed with distilled water (15 mL) twice, dried over anhydrous sodium sulfate, and evaporated under reduced pressure. The obtained residue was purified by silica gel column chromatography (ethyl acetate/n-hexane =1/3 → 1/2 → ethyl acetate), and solids were allowed to precipitate in dichloromethane, hexane and ether solvent system, followed by filtration and washing with hexane of the obtained precipitates to obtain the desired product (1.46 g, yield: 71 %).
¹H-NMR (CDCl₃):δ 7.34-7.28 (m, 4H), 6.84 (br, 1H), 6.19 (s, 1H), 4.80 (s, 2H), 2.41 (s, 3H), 1.99-1.93 (m, 1H), 0.97-0.78 (m, 4H)

Reference Example 26

### 1-(2-hydroxybenzyl)-3-(3-cyclopropyl-1-p-tolyl-1H-pyrazol-5-yl)urea

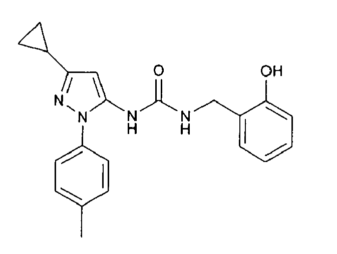

In acetonitrile (3 mL), 2,2,2-trichloroethyl 3-cyclopentyl-1-p-tolyl-1H-pyrazol-5-yl carbamate (100 mg) and 2-hydroxybenzylamine (41.2 mg) were dissolved, after which diisopropylethylamine (67.2 µL) was added thereto. The resulting mixture was stirred at 60°C for 13 hours. Ether and hexane were added to the reaction solution, and the precipitated solids were filtered and washed with hexane to obtain the desired product (86.8 mg, yield: 93%).
¹H-NMR (CDCl₃ + CD₃ OD):δ 7.26-6.79 (m, 8H), 6.07 (s, 1H), 4.27 (s, 1H), 2.36 (s, 3H), 1.95-1.89 (m, 1H), 0.96-0.74 (m, 4H)

Reference Example 27

### 3-neopentyl-1-p-tolyl-1H-pyrazol-5-amine hydrochloric acid salt

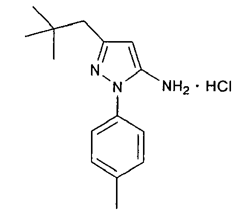

Sodium hydride (629 mg) was weighed and added to a 100 mL three-necked flask, in which the atmosphere had been replaced with argon, and then washed with hexane (6 mL) three times and dried. To this solution, dry THF (8 mL) was added, and the resulting mixture was heated to 75°C. A solution separately prepared from ethyl-3,3-dimethylbutanoate (1.30 g), dry acetonitrile (615 µL) and dry THF (5 mL) was added thereto, and the resulting mixture was stirred at 75°C for 21 hours. Ethyl acetate (10 mL) and aqueous 1M hydrochloric acid solution (10 mL) were added to the reaction solution to extract the solution. The obtained aqueous layer was extracted with ethyl acetate (12 mL) three times, and the organic layers were combined, dried over anhydrous sodium sulfate, and evaporated under reduced pressure. The obtained residue and p-tolylhydrazine hydrochloric acid salt (1.23 g) were dissolved in methanol (16 mL) and the resulting mixture was refluxed for 88.5 hours, after which the reaction solution was evaporated under reduced pressure. The obtained residue was filtered with hexane, and the filtrate was evaporated under reduced pressure, followed by filtration and washing of the obtained residue with ether to obtain the desired product (807 mg, yield: 32%).
¹H-NMR (DMSO-d₆):δ 7.47-7.40 (m, 4H), 5.61 (s, 1H), 2.46 (s, 2H), 2.39 (s, 3H), 0.95 (s, 9H)
MS (ESI):244 (M+H⁺)

Reference Example 28

### 2,2,2-trichloroethyl 3-neopentyl-1-p-tolyl-1H-pyrazol-5-yl carbamate

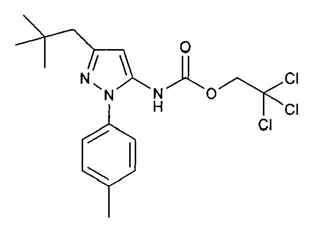

To ethyl acetate (7 mL), 3-neopentyl-1-p-tolyl-1H-pyrazol-5-amine hydrochloric acid salt (807 mg) was added and the resulting mixture was cooled to 0°C. A solution separately prepared from sodium hydroxide (288 mg) and distilled water (7 mL) was then added thereto. The mixture was stirred at 0°C for 30 minutes and 2,2,2-trichloroethyl chloroformate (556 µL) was added thereto, followed by sturring the resulting mixture at room temperature for 20.5 hours. The reaction solution was extracted, and the obtained aqueous layer was extracted with ethyl acetate (7 mL) twice. The organic layers were combined, washed with distilled water (10 mL) twice, dried over anhydrous sodium sulfate, and evaporated under reduced pressure. The obtained residue was purified by silica gel column chromatography (ethyl acetate/n-hexane =1/3 → 1/2), and solids were allowed to precipitate in hexane and ether solvent system, followed by filtration and washing of the obtained precipitates with hexane to obtain the desired product (683 mg, yield: 57%).
¹H-NMR (CDCl₃):δ 7.35-7.29 (m, 4H), 6.82 (br, 1H), 6.35 (s, 1H), 4.81 (s, 2H), 2.53 (s, 2H), 2.42 (s, 3H), 0.99 (s, 9H)

Reference Example 29

### 1-(2-hydroxybenzyl)-3-(3-neopentyl-1-p-tolyl-1H-pyrazol-5-yl)urea

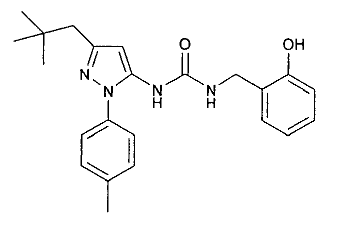

In acetonitrile (3 mL), 2,2,2-trichloroethyl 3-neopentyl-1-p-tolyl-1H-pyrazol-5-yl carbamate (131 mg) and 2-hydroxybenzylamine (50.0 mg) were dissolved, after which diisopropylethylamine (81.6 µL) was added thereto. The resulting mixture was stirred at 60°C for 14 hours. The reaction solution was evaporated under reduced pressure, and the obtained residue was purified by silica gel column chromatography (ethyl acetate/n-hexane =2/3 → 3/2) to obtain the desired product (127 mg, quantitative).
¹H-NMR (CDCl₃):δ 9.06 (br, 1H), 7.23-6.79 (m, 8H), 6.24 (s, 1H), 6.13 (s, 1H), 5.63 (t, 1H, J = 6.4 Hz), 4.29 (d, 2H, J = 6.4 Hz), 2.51 (s, 2H), 2.34 (s, 3H), 0.97 (s, 9H)
MS (ESI):393 (M+H⁺)

Reference Example 30

### 3-(2-methylfuran-3-yl)-1-p-tolyl-1H-pyrazol-5-amine hydrochloric acid salt

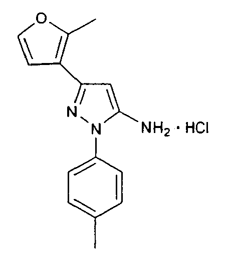

In methanol (8 mL), 3-(2-methyl-3-furyl)-3-oxopropanenitrile (656 mg) and p-tolylhydrazine hydrochloric acid salt (663 mg) were dissolved, and the resulting mixture was refluxed for 22 hours. The reaction solution was evaporated under reduced pressure. The obtained residue was filtered and washed with ether to obtain the desired product (1.16 g, yield: 96%).
¹H-NMR (DMSO-d₆):δ 7.57 (s, 1H), 7.53-7.35 (m, 4H), 6.72 (s, 1H), 5.79 (s, 1H), 2.49 (s, 3H), 2.38 (s, 3H)
MS (ESI):254 (M+H⁺)

Reference Example 31

### 2,2,2-trichloroethyl 3-(2-methylfuran-3-yl)-1-p-tolyl-1H-pyrazol-5-yl carbamate

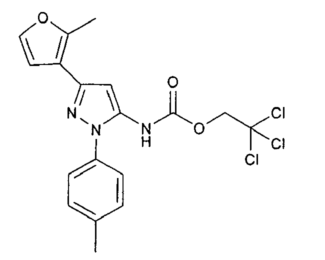

To ethyl acetate (14 mL), 3-(2-methylfuran-3-yl)-1-p-tolyl-1H-pyrazol-5-amine hydrochloric acid salt (1.16 g) was added and the resulting mixture was cooled to 0°C. A solution separately prepared from sodium hydroxide (400 mg) and distilled water (14 mL) was then added thereto. The mixture was stirred at 0°C for 30 minutes and 2,2,2-trichloroethyl chloroformate (772 µL) was added thereto, followed by stirring at room temperature for 15 hours. The reaction solution was extracted, and the obtained aqueous layer was extracted with ethyl acetate (14 mL) twice. The organic layers were combined, washed with distilled water (15 mL) twice, dried over anhydrous sodium sulfate, and evaporated under reduced pressure. The obtained residue was filtered and washed with hexane to obtain the desired product (1.65 g, yield: 96%).
¹H-NMR (CD₃ OD):δ 7.41-7.30 (m, 4H), 7.36 (d, 1H, J = 2.0 Hz), 6.67 (d, 1H, J = 2.0 Hz), 6.52 (s, 1H), 4.82 (s, 2H), 2.50 (s, 3H), 2.40 (s, 3H)

Reference Example 32

### 1-(2-hydroxybenzyl)-3-(3-(2-methylfuran-3-yl)-1-p-tolyl-1H-pyrazol-5-yl)urea

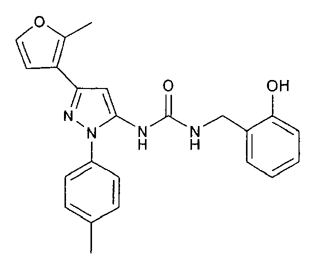

In acetonitrile (3 mL), 2,2,2-trichloroethyl 3-(2-methylfuran-3-yl)-1-p-tolyl-1H-pyrazol-5-yl carbamate (134 mg) and 2-hydroxybenzylamine (50.0 mg) were dissolved, after which diisopropylethylamine (81.6 µL) was added thereto. The resulting mixture was stirred at 60°C for 14 hours. The reaction solution was evaporated under reduced pressure, and the obtained residue was purified by silica gel column chromatography (ethyl acetate/n-hexane =2/3 → 3/2) to obtain the desired product (117 mg, yield: 93%).
¹H-NMR (CDCl₃):δ 9.04 (br, 1H), 7.31 (d, 1H, J = 1.7 Hz), 7.29-6.79 (m, 8H), 6.64 (d, 1H, J = 1.7 Hz), 6.43 (s, 1H), 6.25 (s, 1H), 5.61 (t, 1H, J = 6.5 Hz), 4.30 (d, 2H, J = 6.5 Hz), 2.55 (s, 3H), 2.36 (s, 3H)
MS (ESI):403 (M+H⁺)

Reference Example 33

### 3-cyclohexyl-1-p-tolyl-1H-pyrazol-5-amine hydrochloric acid salt

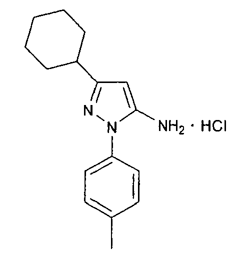

Sodium hydride (540 mg) was weighed and added to a 100 mL three-necked flask, in which the atmosphere had been replaced with argon, and then washed with hexane (5 mL) three times and dried. To this solution, dry THF (6 mL) was added and the resulting mixture was heated to 75°C. A solution separately prepared from ethyl cyclohexanecarboxylate (1.10 g), dry acetonitrile (528 µL) and dry THF (5 mL) was added thereto and then dry THF (30 mL) was added thereto, followed by stirring at 75°C for 20 hours. Ethyl acetate (15 mL) and aqueous 1M hydrochloric acid solution (15 mL) were added to the reaction solution and the organic layer was evaporated under reduced pressure. The obtained aqueous layer was extracted with ethyl acetate (15 mL) three times, and the organic layers were combined, dried over anhydrous sodium sulfate, and evaporated under reduced pressure. The obtained residue and p-tolylhydrazine hydrochloric acid salt (557 mg) were dissolved in methanol (10 mL) and the resulting mixture was refluxed for 15 hours, after which the reaction solution was evaporated under reduced pressure. The obtained residue was filtered and washed with ether to obtain the desired product (998 mg, yield: 44%).
¹H-NMR (DMSO-d₆):δ 7.48-7.40 (m, 4H), 5.67 (s, 1H), 2.68-2.61 (m, 1H), 2.40 (s, 3H), 1.93-1.17 (m, 10H)
MS (ESI):256 (M+H⁺)

Reference Example 34

### 2,2,2-trichloroethyl 3-cyclohexyl-1-p-tolyl-1H-pyrazol-5-yl carbamate

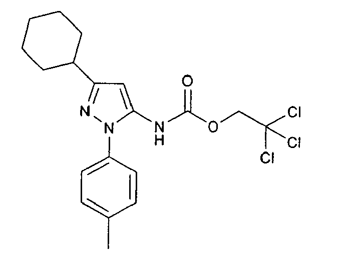

To ethyl acetate (10 mL), 3-cyclohexyl-1-p-tolyl-1H-pyrazol-5-amine hydrochloric acid salt (998 mg) was added and the resulting mixture was cooled to 0°C. A solution separately prepared from sodium hydroxide (342 mg) and distilled water (10 mL) was then added thereto. The mixture was stirred at 0°C for 30 minutes and 2,2,2-trichloroethyl chloroformate (659 µL) was added thereto, followed by stirring at room temperature for 18.5 hours. The reaction solution was extracted, and the obtained aqueous layer was extracted with ethyl acetate (10 mL) twice. The organic layers were combined, washed with distilled water (10 mL) twice, dried over anhydrous sodium sulfate, and evaporated under reduced pressure. From the obtained residue, solids were allowed to precipitate in hexane and ether solvent system, and the obtained solids were filtered and washed with hexane to obtain the desired product (1.31 g, yield: 89%).
¹H-NMR (CDCl₃):δ 7.35-7.28 (m, 4H), 6.83 (br, 1H), 6.37 (s, 1H), 4.81 (s, 2H), 2.70-2.63 (m, 1H), 2.41 (s, 3H), 2.03-1.24 (m, 10H)

Reference Example 35

### 1-(2-hydroxybenzyl)-3-(3-cyclohexyl-1-p-tolyl-1H-pyrazol-5-yl)urea

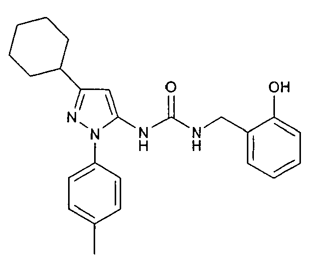

In acetonitrile (3 mL), 2,2,2-trichloroethyl 3-cyclohexyl-1-p-tolyl-1H-pyrazol 5-yl carbamate (100 mg) and 2-hydroxybenzylamine (37.2 mg) were dissolved. Diisopropylethylamine (60.7 µL) was then added thereto and the resulting mixture was stirred at 60°C for 15.5 hours. The reaction solution was evaporated under reduced pressure, and the obtained residue was purified by silica gel column chromatography (ethyl acetate/n-hexane =1/2 → 1/1) to obtain the desired product (85.2 mg, yield: 91%).
¹H-NMR (CDCl₃):δ 9.13 (br, 1H), 7.27-6.79 (m, 8H), 6.14 (s, 1H), 6.10 (s, 1H), 5.5 (t, 1H, J = 6.4 Hz), 4.31 (d, 2H, J = 6.4 Hz), 2.68-2.61 (m, 1H), 2.35 (s, 3H), 2.04-1.19 (m, 10H)
MS (ESI):405 (M+H⁺)

Reference Example 36

### 1-(3-t-butyl-1-phenyl-1H-pyrazol-5-yl)-3-(2,2,2-trichloroethyl)urea

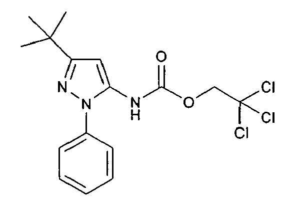

To ethyl acetate (10 mL), 3-t-butyl-1-phenyl-1H-pyrazol-5-amine hydrochloric acid salt (1.04 g) was added and the resulting mixture was cooled to 0°C. A solution separately prepared from sodium hydroxide (413 mg) and distilled water (10 mL) was then added thereto. The mixture was stirred at 0°C for 30 minutes and 2,2,2-trichloroethyl chloroformate (796 µL) was added thereto, followed by stirring at room temperature for 20 hours. The reaction solution was extracted, and the obtained aqueous layer was extracted with ethyl acetate (10 mL) twice. The organic layers were combined, washed with distilled water (10 mL) twice, dried over anhydrous sodium sulfate, and evaporated under reduced pressure. The obtained residue was filtered and washed with hexane to obtain the desired product (1.36 g, yield: 84%).
¹H-NMR (CDCl₃):δ 7.53-7.39 (m, 4H), 6.81 (br, 1H), 6.44 (s, 1H), 4.82 (s, 2H), 1.35 (s, 9H)

Reference Example 37

### 1-(2-hydroxybenzyl)-3-(3-t-butyl-1-phenyl-1H-pyrazol-5-yl)urea

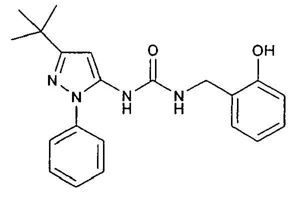

In acetonitrile (3 mL), 1-(3-t-butyl-1-phenyl-1H-pyrazol-5-yl)-3-(2,2,2-trichloroethyl)urea (122 mg) and 2-hydroxybenzylamine (50.0 mg) were dissolved, after which diisopropylethylamine (81.6 µL) was added thereto. The resulting mixture was stirred at 60°C for 14 hours. The reaction solution was evaporated under reduced pressure, and the obtained residue was purified by silica gel column chromatography (ethyl acetate/n-hexane =2/3 → 3/2) to obtain the desired product (116 mg, quantitative).
¹H-NMR (CDCl₃):δ 9.07 (br, 1H), 7.36-6.79(m, 9H), 6.26 (s, 1H), 6.22 (s, 1H), 5.64 (t, 1H, J = 6.5 Hz), 4.30 (d, 2H, J = 6.5 Hz), 1.33 (s, 9H)
MS (ESI):365 (M+H⁺)

Reference Example 38

### 1-(2-hydroxybenzyl)-3-(3-(furan-2-yl)-1-p-tolyl-1H-pyrazol-5-yl)urea

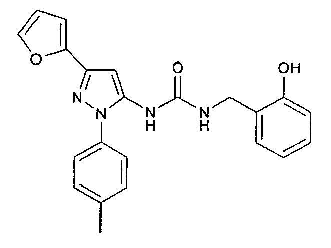

In acetonitrile (3 mL), 2,2,2-trichloroethyl 3-(furan-2-yl)-1-p-tolyl-1H-pyrazol-5-yl carbamate (100 mg) and 2-hydroxybenzylamine (38.6 mg) were dissolved, after which diisopropylethylamine (63.0 µL) was added thereto. The resulting mixture was stirred at 60°C for 13 hours. The reaction solution was evaporated under reduced pressure, and solids were allowed to precipitate by addition of ethyl acetate, ether and hexane. The obtained solids were then filtered and washed with hexane to obtain the desired product (87.5 mg, yield: 93%).
¹H-NMR (CDCl₃):δ 9.00 (br, 1H), 7.46 (d, 1H, J = 1.7 Hz), 7.25-6.79 (m, 8H), 6.73 (d, 1H, J = 3.3 Hz), 6.60 (s, 1H), 6.48 (dd, 1H, J = 1.7, 3.3 Hz), 6.31 (s, 1H), 5.70 (t, 1H, J = 6.6 Hz), 4.32 (d, 2H, J = 6.6 Hz), 2.36 (s, 3H), 2.04-1.19 (m, 10H)

Reference Example 39

### 2-(aminomethyl)-4-methylphenol

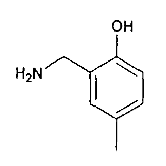

Sodium hydride (1.08 g) was weighed and added to a 50 mL two-necked flask in which the atmosphere had been replaced with argon. Dry DMF (7.0 mL) was then added thereto and the resulting mixture was cooled to 0°C. To this solution, 2-hydroxyethylmethylsulfone (997 µL) was added, after which a solution separately prepared from 2-fluoro-5-methylbenzonitrile (1.11 g) and dry DMF (3.0 mL) was added. The resulting mixture was then stirred at room temperature for 15.5 hours. The reaction solution was cooled to 0°C, and aqueous 1M hydrochloric acid solution (12 mL) was added thereto. The resulting solution was extracted with ethyl acetate (15 mL) three times. The organic layers were combined, dried over anhydrous sodium sulfate and evaporated under reduced pressure. The obtained residue was purified by silica gel column chromatography (ethyl acetate/n-hexane =1/4 → 2/3), dissolved in dry THF (15 mL) and cooled to 0°C. Borane-THF complex solution in THF (23.9 mL) was added to the solution, which was then stirred at 0°C for 30 minutes and at room temperature for another 15 hours. An excess amount of distilled water was added to the reaction solution, and triethanolamine (4.28 g) was added thereto, followed by evaporation of the organic solvent under reduced pressure. The precipitated solids were filtered and washed with distilled water, ethyl acetate, and ether to obtain the desired product (891 mg, yield: 79%).
¹H-NMR (CD₃ OD):δ 6.98-6.69 (m, 3H), 3.91 (s, 2H), 2.22 (s, 3H)

Reference Example 40

### 1-(2-hydroxy-5-methylbenzyl)-3-(3-t-butyl-1-p-tolyl-1H-pyrazol-5-yl)urea

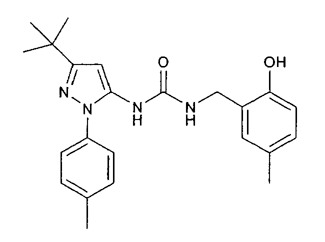

In acetonitrile (3.5 mL), 2,2,2-trichloroethyl 3-t-butyl-1-p-tolyl-1H-pyrazol-5-yl carbamate (100 mg) and 2-(aminomethyl)-4-methylphenol (51.4 mg) were dissolved, after which diisopropylethylamine (127 µL) was added thereto. The resulting mixture was stirred at 60°C for 16 hours. The reaction solution was evaporated under reduced pressure, and the obtained residue was purified by silica gel column chromatography (ethyl acetate/n-hexane =3/17 → 2/3) to obtain the desired product (102 mg, yield: 69%).
¹H-NMR (CDCl₃):δ 8.88 (br, 1H), 7.24-6.82 (m, 7H), 6.19 (s, 1H), 6.11 (s, 1H), 5.55 (t, 1H, J = 6.5 Hz), 4.26 (d, 2H, J = 6.5 Hz), 2.34 (s, 3H), 2.24 (s, 3H), 1.33 (s, 9H)
MS (ESI):393 (M+H⁺)

Reference Example 41

### 2-(aminomethyl)-4-(trifluoromethyl)phenol

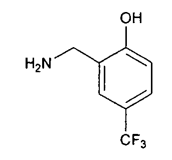

Sodium hydride (789 mg) was weighed and added to a 50 mL two-necked flask in which the atmosphere had been replaced with argon, and then dry DMF (7.0 mL) was added thereto. The resulting mixture was cooled to 0°C. To this solution, 2-hydroxyethylmethylsulfone (732 µL) was added, after which a solution separately prepared from 2-fluoro-5-(trifluoromethyl)benzonitrile (1.14 g) and dry DMF (3.0 mL) was added thereto. The resulting mixture was then stirred at room temperature for 15.5 hours. The reaction solution was cooled to 0°C, and aqueous 1M hydrochloric acid solution (12 mL) was added thereto. The solution was extracted with ethyl acetate (15 mL) three times. The organic layers were combined, dried over anhydrous sodium sulfate, and evaporated under reduced pressure. The obtained residue was purified by silica gel column chromatography (ethyl acetate/n-hexane = 3/7 → 9/11), dissolved in dry THF (15 mL), and cooled to 0°C. Borane-THF complex solution in THF (17.3 mL) was added to the solution, which was then stirred at 0°C for 30 minutes and at room temperature for another 15 hours. An excess amount of distilled water was added to the reaction solution, and triethanolamine (3.10 g) was added thereto, followed by evaporation of the organic solvent under reduced pressure. The obtained aqueous solution was extracted with ethyl acetate (20 mL) three times. The organic layers were combined, dried over anhydrous sodium sulfate, and evaporated under reduced pressure. The obtained residue was filtered and washed with ethyl acetate and ether to obtain the desired product (752 mg, yield: 65%).
¹H-NMR (CD₃ OD):δ 7.39-6.77 (m, 3H), 3.95 (s, 2H)

Reference Example 42

### 1-(2-hydroxy-5-(trifluoromethyl)benzyl)-3-(3-t-butyl-1-p-tolyl-1H-pyrazol-5-yl)urea

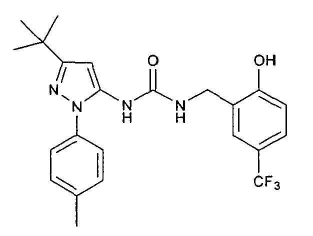

In acetonitrile (3.5 mL), 2,2,2-trichloroethyl 3-t-butyl-1-p-tolyl-1H-pyrazol-5-yl carbamate (168 mg) and 2-(aminomethyl)-4-(trifluoromethyl)phenol (61.0 mg) were dissolved. Diisopropylethylamine (108 µL) was then added thereto and the resulting mixture was stirred at 60°C for 16 hours. The reaction solution was evaporated under reduced pressure, and the obtained residue was purified by silica gel column chromatography (ethyl acetate/n-hexane =1/9 → 3/7) to obtain the desired product (125 mg, yield: 88%).
¹H-NMR (CDCl₃):δ 9.90 (br, 1H), 7.48-6.97 (m, 7H), 6.22 (s, 1H), 6.21 (s, 1H), 5.59 (t, 1H, J = 6.4 Hz), 4.30 (d, 2H, J = 6.4 Hz), 2.32 (s, 3H), 1.34 (s, 9H)
MS (ESI):447 (M+H⁺)

Reference Example 43

### N-(5-chloro-2-hydroxybenzyl)-2-chloroacetamide

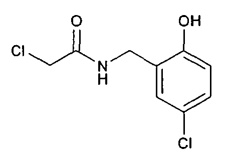

In a mixed solvent of acetic acid (2 mL) and sulfuric acid (2 mL), 4-chlorophenol (539 mg) and N-hydroxymethyl-2-chloroacetamide (518 mg) were dissolved, and the resulting mixture was stirred at room temperature for 17 hours. The reaction solution was added to an excess amount of ice water, and neutralized with aqueous 1N sodium hydroxide solution followed by extraction with ethyl acetate (50 mL) three times. The organic layers were combined, dried over anhydrous sodium sulfate, and evaporated under reduced pressure. The obtained residue was filtered and washed with methanol and ether, and the filtrate was evaporated under reduced pressure, followed by purification by silica gel column chromatography (ethyl acetate/n-hexane =1/3 → 1/1) to obtain the desired product (538 mg, yield: 55%).
¹H-NMR (CD₃ OD):δ 7.15-6.74 (m, 3H), 4.36 (s, 2H), 4.10 (s, 2H)
MS (ESI):234 (M+H⁺)

Reference Example 44

### 2-(aminomethyl)-4-chlorophenol

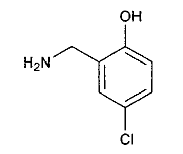

In a mixed solvent of THF (1.5 mL) and aqueous 1M hydrochloric acid solution (4.0 mL), N-(5-chloro-2-hydroxybenzyl)-2-chloroacetamide (535 mg) was dissolved, and the resulting mixture was refluxed for 37 hours. The reaction solution was neutralized with aqueous 1N sodium hydroxide solution and extracted with ethyl acetate (8 mL) nine times. The organic layers were combined, dried over anhydrous sodium sulfate, and evaporated under reduced pressure. The obtained residue was filtered and washed with ethyl acetate and ether to obtain the desired product (513 mg, quantitative).
¹H-NMR (CD₃ OD):δ 7.31-6.86 (m, 3H), 4.06 (s, 2H)
MS (ESI):158 (M+H⁺)

Reference Example 45

### 1-(2-hydroxy-5-chlorobenzyl)-3-(3-t-butyl-1-p-tolyl-1H-pyrazol-5-yl)urea

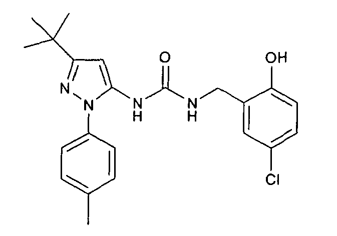

In acetonitrile (6.5 mL), 2,2,2-trichloroethyl 3-t-butyl-1-p-tolyl-1H-pyrazol-5-yl carbamate (334 mg) and 2-(aminomethyl)-4-chlorophenol (100 mg) were dissolved. Diisopropylethylamine (216 µL) was then added thereto and the resulting mixture was stirred at 60°C for 18 hours. The reaction solution was evaporated under reduced pressure, and the obtained residue was purified by silica gel column chromatography (ethyl acetate/n-hexane =3/17 → 7/13) to obtain the desired product (174 mg, yield: 66%).
¹H-NMR (CDCl₃):δ 9.33 (br, 1H), 7.22-6.84 (m, 7H), 6.22 (s, 1H), 6.20 (s, 1H), 5.59 (t, 1H, J = 6.5 Hz), 4.21 (d, 2H, J = 6.5 Hz), 2.32 (s, 3H), 1.33 (s, 9H)
MS (ESI):413 (M+H⁺)

Reference Example 46

### N-(3-chloro-5-fluoro-2-hydroxybenzyl)-2-chloroacetamide

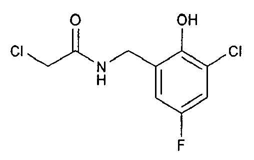

In a mixed solvent of acetic acid (2 mL) and sulfuric acid (2 mL), 2-chloro-4-fluorophenol (632 mg) and N-hydroxymethyl-2-chloroacetamide (533 mg) were dissolved, and the resulting mixture was stirred at room temperature for 18 hours. The reaction solution was added to an excess amount of ice water, and neutralized with aqueous 2N sodium hydroxide solution followed by extraction with ethyl acetate (30 mL) three times. The organic layers were combined, dried over anhydrous sodium sulfate, and evaporated under reduced pressure. The obtained residue was purified by silica gel column chromatography (ethyl acetate/n-hexane =1/4 → 2/3) to obtain the desired product (843 mg, yield: 78%).
¹H-NMR (CDCl₃):δ 7.72 (s, 1H), 7.34 (br, 1H), 7.09-6.86 (m, 2H), 4.45 (d, 2H, J = 6.6 Hz), 4.11 (s, 2H)
MS (ESI):252 (M+H⁺)

Reference Example 47

### 2-(aminomethyl)-6-chloro-4-fluorophenol

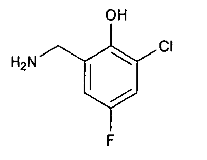

In a mixed solvent of THF (1.0 mL) and aqueous 1M hydrochloric acid solution (6.0 mL), N-(3-chloro-5-fluoro-2-hydroxybenzyl)-2-chloroacetamide (843 mg) was dissolved, and the resulting mixture was refluxed for 64 hours. The reaction solution was neutralized with aqueous 1N sodium hydroxide solution and extracted with ethyl acetate (12 mL) nine times. The organic layers were combined, dried over anhydrous sodium sulfate, and evaporated under reduced pressure. The obtained residue was filtered and washed with ethyl acetate and ether to obtain the desired product (419 mg, yield: 71%).
¹H-NMR (CD₃ OD):δ 7.04-6.81 (m, 2H), 3.99 (s, 2H)
MS (ESI):176 (M+H⁺)

Reference Example 48

### 1-(3-chloro-5-fluoro-2-hydroxybenzyl)-3-(3-t-butyl-1-p-tolyl-1H-pyrazol-5-yl)urea

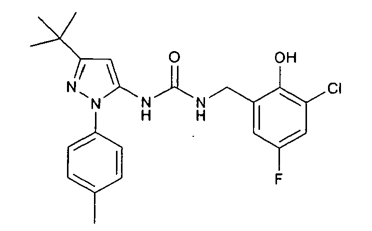

In acetonitrile (7 mL), 2,2,2-trichloroethyl 3-t-butyl-1-p-tolyl-1H-pyrazol-5-yl carbamate (366 mg) and 2-(aminomethyl)-6-chloro-4-fluorophenol (122 mg) were dissolved. Diisopropylethylamine (230 µL) was then added thereto and the resulting mixture was stirred at 60°C for 9 hours. The reaction solution was evaporated under reduced pressure, and the obtained residue was purified by silica gel column chromatography (ethyl acetate/n-hexane =3/17 → 2/3) to obtain the desired product (301 mg, quantitative).
¹H-NMR (CDCl₃):δ 8.75 (br, 1H), 7.24-6.66 (m, 6H), 6.30 (s, 1H), 6.21 (s, 1H), 5.62 (t, 1H, J = 6.2 Hz), 4.26 (d, 2H, J = 6.2 Hz), 2.33 (s, 3H), 1.33 (s, 9H)
MS (ESI):431 (M+H⁺)

Reference Example 49

### p-chlorophenylhydrazine hydrochloric acid salt

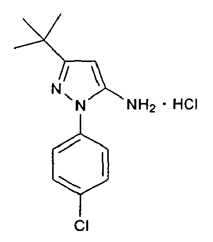

Pivaloylacetonitrile (979 mg) and p-chlorophenylhydrazine hydrochloric acid salt (1.00 g) were dissolved in methanol (5 mL) and the resulting mixture was refluxed for 16 hours, followed by evaporation of the reaction solution under reduced pressure. The obtained residue was filtered and washed with ether to obtain the desired product (1.58 g, yield: 99%).
¹H-NMR (DMSO-d₆):δ 7.63 (br, 4H), 5.60 (s, 1H), 1.27 (s, 9H)
MS (ESI):250 (M+H⁺)

Reference Example 50

### 2,2,2-trichloroethyl 3-t-butyl-1-(4-chlorophenyl)-1H-pyrazol-5-yl carbamate

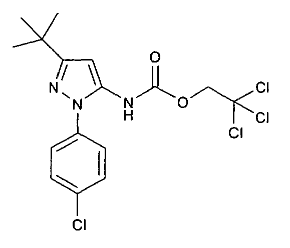

To ethyl acetate (10 mL), 3-t-butyl-1-(4-chlorophenyl)-1H-pyrazol-5-amine hydrochloric acid salt (1.58 g) was added and the resulting mixture was cooled to 0°C. A solution separately prepared from sodium hydroxide (552 mg) and distilled water (5 mL) was then added thereto. The mixture was stirred at 0°C for 30 minutes, and 2,2,2-trichloroethyl chloroformate (1.06 mL) was added thereto, followed by stirring at room temperature for another 18 hours. The reaction solution was extracted, and the obtained aqueous layer was extracted with ethyl acetate (10 mL) twice. The organic layers were combined, dried over anhydrous sodium sulfate, and evaporated under reduced pressure. The obtained residue was filtered and washed with hexane to obtain the desired product (2.01 g, yield: 86%). ¹H-NMR (CDCl₃):δ 7.47-7.42 (m, 4H), 6.73 (br, 1H), 6.40 (s, 1H), 4.81 (s, 2H), 1.33 (s, 9H)

Reference Example 51

### 1-(2-hydroxybenzyl)-3-(3-t-butyl-1-(4-chlorophenyl)-1H-pyrazol-5-yl)urea

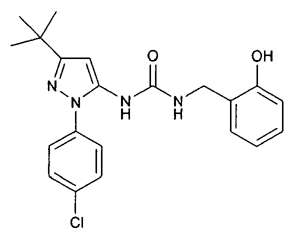

In acetonitrile (6 mL), 2,2,2-trichloroethyl 3-t-butyl-1-(4-chlorophenyl)-1H-pyrazol-5-yl carbamate (324 mg) and 2-hydroxybenzylamine (122 mg) were dissolved. Diisopropylethylamine (199 µL) was added thereto and the resulting mixture was stirred at 60°C for 8 hours. The reaction solution was evaporated under reduced pressure, and the obtained residue was purified by silica gel column chromatography (ethyl acetate/n-hexane =1/4 → 7/13) to obtain the desired product (310 mg, quantitative).
¹H-NMR (CDCl₃):δ 8.90 (br, 1H), 7.34-6.79 (m, 8H), 6.44 (s, 1H), 6.21 (s, 1H), 5.56 (t, 1H, J = 6.5 Hz), 4.28 (d, 2H, J = 6.5 Hz), 1.32 (s, 9H)

Reference Example 52

### 3-t-butyl-1-(pyridin-3-yl)-1H-pyrazol-5-amine

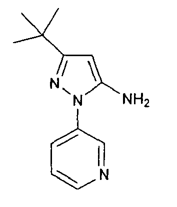

To hydrochloric acid (25 mL), 3-aminopyridine (4.00 g) was added and the resulting mixture was cooled to -5°C. A solution separately prepared from sodium nitrite (3.30 g) and distilled water (8 mL) was then added thereto, and the resulting mixture was stirred at 0°C for 1 hour. The mixture was cooled to -5°C, and a solution separately prepared from stannous chloride dihydrate (24.0 g) and hydrochloric acid (12 mL) was added thereto, followed by stirring at 0°C for 6 hours. The reaction solution was filtered, and the obtained solids were mixed with ice. The pH of the resulting mixture was adjusted to 10 or higher with aqueous 50% KOH solution, and the mixture was filtered with ethyl acetate. The filtrate was extracted, and the obtained aqueous layer was extracted with ethyl acetate (60 mL) six times. The organic layers were combined, dried over anhydrous sodium sulfate, and evaporated under reduced pressure. The obtained residue and pivaloyl acetonitrile (2.61 g) were dissolved in methanol (12 mL) and the resulting mixture was refluxed for 20 hours, followed by evaporation of the resulting reaction solution under reduced pressure. The obtained residue was purified by silica gel column chromatography (ethyl acetate/n-hexane =3/2 → ethyl acetate) to obtain the desired product (2.51 g, yield: 27%).
¹H-NMR (CDCl₃):δ 8.91 (d, 1H, J = 2.6 Hz), 8.54 (dd, 1H, J = 1.6, 4.8 Hz), 7.96 (ddd, 1H, J = 1.6, 2.6, 8.2 Hz), 7.40 (dd, 1H, J = 4.8, 8.2 Hz), 5.82 (s, 1H), 3.72 (br, 2H), 1.31 (s, 9H)

Reference Example 53

### 2,2,2-trichloroethyl 3-t-butyl-1-(pyridin-3-yl)-1H-pyrazol-5-yl carbamate

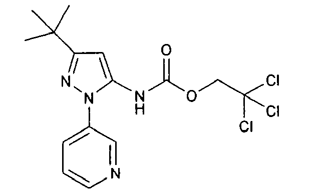

To ethyl acetate (20 mL), 3-t-butyl-1-(pyridin-3-yl)-1H-pyrazol-5-amine (2.51 g) was added and a solution separately prepared from sodium hydroxide (696 mg) and distilled water (10 mL) was added thereto. To the resulting mixture, 2,2,2-trichloroethyl chloroformate (2.24 mL) was then added and the mixture was stirred at room temperature for 16 hours. A solution prepared from sodium hydroxide (696 mg) and distilled water (5 mL) was added thereto, and then 2,2,2-trichloroethyl chloroformate (2.24 mL) was added to the resulting solution, followed by stirring at room temperature for 68 hours. A solution prepared from sodium hydroxide (1.16 g) and distilled water (10 mL) was further added thereto and then 2,2,2-trichloroethyl chloroformate (3.83 mL) was added, followed by stirring at room temperature for 5 hours. The reaction solution was extracted, and the obtained aqueous layer was extracted with ethyl acetate (20 mL) twice. The organic layers were combined, dried over anhydrous sodium sulfate, and evaporated under reduced pressure. The obtained residue was purified by silica gel column chromatography (ethyl acetate/n-hexane = 1/1) to obtain the desired product (3.15 g, yield: 69%).
¹H-NMR (CDCl₃):δ 8.76 (d, 1H, J = 2.3 Hz), 8.51 (dd, 1H, J = 1.4, 4.8 Hz), 7.88 (ddd, 1H, J = 1.4, 2.3, 8.2 Hz), 7.40 (dd, 1H, J = 4.8, 8.2 Hz), 6.44 (s, 1H), 4.80 (s, 2H), 1.35 (s, 9H)

Reference Example 54

### 1-(2-hydroxybenzyl)-3-(3-t-butyl-1-(pyridin-3-yl)-1 H-pyrazol-5-yl)urea

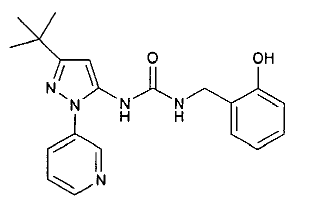

In acetonitrile (6 mL), 2,2,2-trichloroethyl 3-t-butyl-1-(pyridin-3-yl)-1H-pyrazol-5-yl carbamate (354 mg) and 2-hydroxybenzylamine (145 mg) were dissolved, after which diisopropylethylamine (236 µL) was added thereto. The resulting mixture was stirred at 60°C for 16 hours. The precipitated solids were filtered and washed with cooled acetonitrile to obtain the desired product (262 mg, yield: 79%).
¹H-NMR (CDCl₃):δ 9.14 (br, 1H), 8.56-7.22 (m, 4H), 7.73 (br, 1H), 7.18-6.75 (m, 4H), 6.38 (s, 1H), 6.08 (t, 1H, J = 6.2 Hz), 4.29 (d, 2H, J = 6.2 Hz), 1.33 (s, 9H)

Reference Example 55

### 1-(4-methoxy-3-methylphenyl)-1,2-dicarbobutoxy-hydrazine

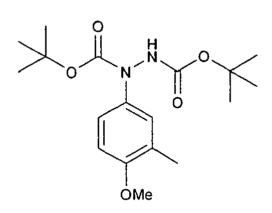

To a 500 mL three-necked flask in which the atmosphere had been replaced with argon, 4-bromo-2-methylanisole (5.14 g) was weighed, and then dry THF (75 mL) was added thereto. The resulting mixture was cooled to -78°C, after which 2.66 M n-butyllithium solution in hexane (11.5 mL) was added thereto, followed by stirring at -78°C for 1 hour. To this solution, a solution separately prepared from dit-butyl azodicarboxylate (7.06 g) and dehydrate THF (20 mL) was added, and the resulting mixture was stirred at -78°C for 1 hour and then at room temperature for 2 hours. Distilled water (15 mL) was added to the reaction solution, which was then evaporated under reduced pressure. Saturated brine (150 mL) was added to the obtained residue, which was then extracted with ether (150 mL) three times. The organic layers were combined, dried over anhydrous sodium sulfate, and evaporated under reduced pressure. Solids were allowed to precipitate by addition of ether and hexane, followed by filtration and washing of the obtained precipitates with hexane to obtain the desired product (5.39 g, yield: 60%).
¹H-NMR (CDCl₃):δ 7.18-6.74 (m, 3H), 3.81 (s, 3H), 2.19 (s, 3H), 1.49 (s, 18H)

Reference Example 56

### 1-(4-methoxy-3-methylphenyl) hydrazine hydrochloric acid salt

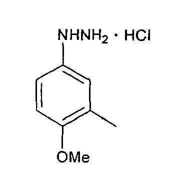

Di-t-butyl 1-(4-methoxy-3-methylphenyl) hydrazin-1,2-dicarboxylate (5.39 g) was dissolved in 1,4-dioxane (6 mL), and then 4M hydrochloric acid in dioxane was added thereto. The resulting mixture was stirred at room temperature for 27 hours. The reaction solution was evaporated under reduced pressure, and ether (30 mL) was added to the obtained residue, which was then stirred at 0°C for 30 minutes. The resulting reaction product was filtered and washed with ether to obtain the desired product (2.74 g, yield: 95%).
¹ H-NMR (DMSO-d₆):δ 6.89-6.82 (m, 3H), 3.73 (s, 3H), 2.12 (s, 3H)

Reference Example 57

### 3-t-butyl-1-(4-methoxy-3-methylphenyl)-1H-pyrazol-5-amine hydrochloric acid salt

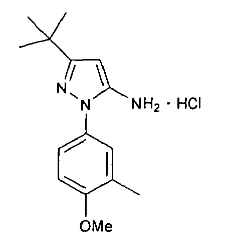

Pivaloylacetonitrile (2.55 g) and 1-(4-methoxy-3-methylphenyl) hydrazine hydrochloric acid salt (2.74 g) were dissolved in methanol (15 mL), and then the resulting mixture was refluxed for 62 hours. The reaction solution was evaporated under reduced pressure. The obtained residue was filtered and washed with ether to obtain the desired product (4.11 g, yield: 96%).
¹H-NMR (DMSO-d₆):δ 7.41-7.14 (m, 4H), 5.64 (s, 1H), 3.87 (s, 3H), 2.22 (s, 3H), 1.30 (s, 9H)

Reference Example 58

### 2,2,2-trichloroethyl 3-t-butyl-1-(4-methoxy-3-methylphenyl)-1H-pyrazol-5-yl carbamate

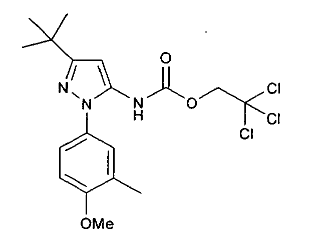

To ethyl acetate (20 mL), 3-t-butyl-1-(4-methoxy-3-methylphenyl)-1H-pyrazol-5-amine hydrochloric acid salt (4.11 g) was added and the resulting mixture was cooled to 0°C. A solution separately prepared from sodium hydroxide (1.39 g) and distilled water (10 mL) was added thereto. The resulting mixture was stirred at 0°C for 30 minutes, and 2,2,2-trichloroethyl chloroformate (2.68 mL) was added thereto, followed by stirring at room temperature for another 13 hours. The reaction solution was extracted, and the obtained aqueous layer was extracted with ethyl acetate (20 mL) twice. The organic layers were combined, dried over anhydrous sodium sulfate, and evaporated under reduced pressure. The obtained residue was filtered and washed with hexane to obtain the desired product (3.97 g, yield: 66%). ¹ H-NMR (CDCl₃):δ 7.23-6.89 (m, 3H), 6.81 (br, 1H), 6.40 (s, 1H), 4.81 (s, 2H), 3.88 (s, 3H), 2.25 (s, 3H), 1.34 (s, 9H)

Reference Example 59 1-(2-hydroxybenzyl)-3-(3-t-butyl-1-(4-methoxy-3-methylphenyl)-1H-pyrazol-5-yl)urea

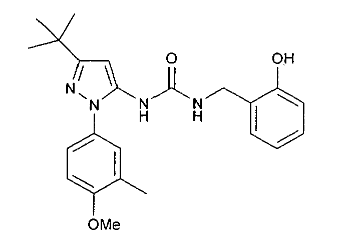

In acetonitrile (15 mL), 2,2,2-trichloroethyl 3-t-butyl-1-(4-methoxy-3-methylphenyl)-1H-pyrazol-5-yl carbamate (913 mg) and 2-hydroxybenzylamine (336 mg) were dissolved, and then diisopropylethylamine (549 µL) was added thereto. The resulting mixture was stirred at 60 °C for 11 hours. The precipitated solids were filtered, and washed with ether to obtain the desired product (845 mg, yield: 99%).
¹ H-NMR (CDCl₃+DMSO-d₆):δ 9.48 (br, 1H), 7.84 (s, 1H), 7.21-6.76 (m, 8H), 6.35 (s, 1H), 4.29 (d, 2H, J = 6.4 Hz), 3.84 (s, 3H), 2.20 (s, 3H), 1.31 (s, 9H)

Reference Example 60

### 3-methoxy-4-methylaniline

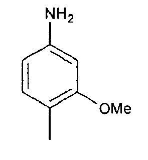

To methanol (8 mL) and THF (4 mL), 2-methyl-5-nitroanisole (2.00 g) and palladium carbon (650 mg) were added, and the resulting mixture was stirred at room temperature for 21 hours under hydrogen atmosphere at 1 atm. Reaction solution was filtered through Celite, and the filtrate was evaporated under reduced pressure to obtain the desired product (1.67 g, quantitative).
¹H-NMR (CDCl₃):δ 6.90-6.20 (m, 3H), 3.77 (s, 3H), 3.55 (br, 2H), 2.10 (s, 3H)

Reference Example 61

### 3-t-butyl-1-(3-methoxy-4-methylphenyl)-1H-pyrazol-5-amine hydrochloric acid salt

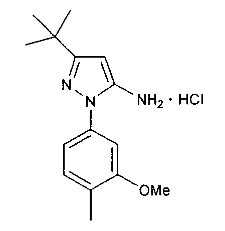

To hydrochloric acid (7.5 mL), 3-methoxy-4-methylaniline (1.67 g) was added and the resulting mixture was cooled to -5 °C. A solution separately prepared from sodium nitrite (924 mg) and distilled water (2.5 mL) was added thereto and the resulting mixture was stirred for at 0°C 1 hour. The mixture was cooled to -5°C, and a solution separately prepared from stannous chloride dihydrate (6.87 g) and hydrochloric acid (3.6 mL) was added thereto, followed by stirring at 0°C for 6 hours. The reaction solution was filtered, and the obtained solids were mixed with ice, after which the pH of the resulting mixture was adjusted to 10 or higher with aqueous 50% KOH solution. The mixture was then filtered with ethyl acetate. The filtrate was extracted, and the obtained aqueous solution was extracted with ethyl acetate (30 mL) three times. The organic layers were combined, dried over anhydrous sodium sulfate, and evaporated under reduced pressure. The obtained residue and pivaloyl acetonitrile (1.44 g) were dissolved in methanol (10 mL) and the resulting mixture was refluxed for 12 hours, followed by evaporation of the reaction solution under reduced pressure. The obtained residue was purified by silica gel column chromatography (ethyl acetate/n-hexane =3/17 → 2/3), dissolved in dichloromethane and then salified using hydrochloric acid. The organic solvent was evaporated under reduced pressure, and the obtained residue was filtered and washed with ether to obtain the desired product (1.39 g, yield: 39%).
¹H-NMR (DMSO-d₆):δ 7.38-7.04 (m, 3H), 5.66 (s, 1H), 3.87 (s, 3H), 2.22 (s, 3H), 1.31 (s, 9H)

Reference Example 62

### 2,2,2-trichloroethyl 3-t-butyl-1-(3-methoxy-4-methylphenyl)-1H-pyrazol-5-yl carbamate

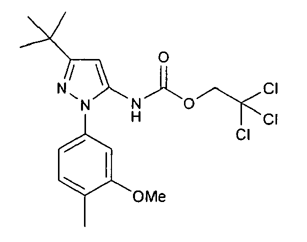

To ethyl acetate (7 mL), 3-t-butyl-1-(3-methoxy-4-methylphenyl)-1H-pyrazol-5-amine hydrochloric acid salt (1.38 g) was added and the resulting mixture was cooled to 0°C. A solution separately prepared from sodium hydroxide (467 mg) and distilled water (3.5 mL) was added thereto. The resulting mixture was stirred at 0°C for 30 minutes, and 2,2,2-trichloroethyl chloroformate (899 µL) was added thereto, followed by stirring at room temperature for another 11 hours. The reaction solution was extracted, and the obtained aqueous layer was extracted with ethyl acetate (7 mL) twice. The organic layers were combined, dried over anhydrous sodium sulfate, and evaporated under reduced pressure. The obtained residue was filtered and washed with hexane to obtain the desired product (1.53 g, yield: 75%).
¹H-NMR (CDCl₃):δ 7.23-6.92 (m, 3H), 6.90 (s, 1H), 6.43 (s, 1H), 4.81 (s, 2H), 3.86 (s, 3H), 2.25 (s, 3H), 1.35 (s, 9H)

Reference Example 63

### 1-(2-hydroxybenzyl)-3-(3 -t-butyl-1-(3-methoxy-4-methylphenyl)-1H-pyrazol-5-yl)urea

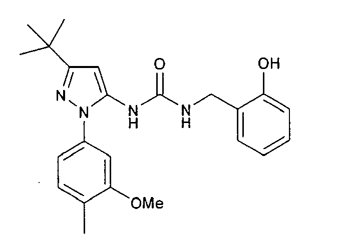

In acetonitrile (15 mL), 2,2,2-trichloroethyl 3-t-butyl-1-(3-methoxy-4-methylphenyl)-1H-pyrazol-5-yl carbamate (910 mg) and 2-hydroxybenzylamine (335 mg) were dissolved, and then diisopropylethylamine (547 µL) was added thereto. The resulting mixture was stirred at 60 °C for 8 hours. The precipitated solids were filtered, and washed with ether to obtain the desired product (850 mg, yield: 99%). ¹ H-NMR (CDCl₃+DMSO-d₆):δ 9.42 (br, 1H), 8.03 (s, 1H), 7.16-6.76 (m, 8H), 6.38 (s, 1H), 4.30 (d, 1H, J = 6.1 Hz), 3.78 (s, 3H), 2.21 (s, 3H), 1.32 (s, 9H)

Reference Example 64

### 3-(benzyloxy)-4-methylaniline

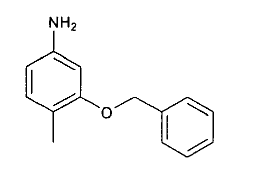

In DMF (15 mL), 5-nitro-2-methylphenol (2.07 g) was dissolved and potassium carbonate (2.80 g) was added thereto. The resulting mixture was cooled to 0°C, after which benzyl bromide (2.09 mL) was added thereto. The resulting mixture was stirred at room temperature for 16 hours. The reaction solution was filtered through Celite, and the filtrate was evaporated under reduced pressure. Distilled water (15 mL) was added to the obtained residue, which was then extracted with dichloromethane (20 mL) three times. The organic layers were combined, dried over anhydrous sodium sulfate, and evaporated under reduced pressure. The obtained residue was purified by silica gel column chromatography (ethyl acetate/n-hexane=1/3). The purified product was dissolved in ethanol (25 mL), THF (15 mL) and distilled water (5 mL), and then ammonium chloride (3.61 g) and iron powder (3.77 g) were added thereto, followed by refluxing the resulting mixture for 16 hours. The reaction solution was filtered through Celite, and the filtrate was evaporated under reduced pressure. Saturated aqueous sodium hydrogen carbonate solution (30 mL) was added to the obtained residue, which was then extracted with dichloromethane (30 mL) three times. The organic layers were combined, dried over anhydrous sodium sulfate, and evaporated under reduced pressure to obtain the desired product (2.85 g, yield: 99%).
¹H-NMR (CDCl₃):δ 7.44-7.24 (m, 5H), 6.92 (d, 1H, J = 7.9 Hz), 6.28 (d, 1H, J = 2.0 Hz), 6.23 (dd, 1H, J = 2.0, 7.9 Hz), 5.02 (s, 2H), 3.53 (br, 2H), 2.17 (s, 3H)
MS (ESI):214 (M+H⁺)

Reference Example 65

### 1-(3-(benzyloxy)-4-methylphenyl)-3-t-butyl-1H-pyrazol-5-amine

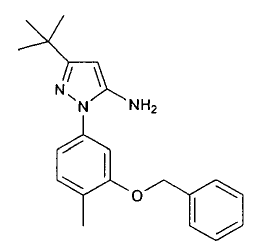

To hydrochloric acid (8 mL), 3-(benzyloxy)-4-methylaniline (2.85 g) was added and the resulting mixture was cooled to -5 °C. A solution separately prepared from sodium nitrite (1.01 g) and distilled water (3 mL) was added thereto and the resulting mixture was stirred at 0°C for 1 hour. The mixture was cooled to -5°C, and a solution separately prepared from stannous chloride dihydrate (7.54 g) and hydrochloric acid (4 mL) was added thereto. The resulting mixture was stirred at 0°C for 5 hours. The reaction solution was filtered, and the obtained solids were mixed with ice, after which the pH of the resulting mixture was adjusted to 10 or higher with aqueous 50% KOH solution. The mixture was then filtered with ethyl acetate. The filtrate was extracted, and the obtained aqueous solution was extracted with ethyl acetate (40 mL) four times. The organic layers were combined, dried over anhydrous sodium sulfate, and evaporated under reduced pressure. The obtained residue and pivaloyl acetonitrile (1.38 g) were dissolved in methanol (10 mL) and the resulting mixture was refluxed for 16 hours, after which the reaction solution was evaporated under reduced pressure. The obtained residue was purified by silica gel column chromatography (ethyl acetate/n-hexane =1/4 → 1/3) to obtain the desired product (959 mg, yield: 21%).
¹H-NMR (CDCl₃):δ 7.46-7.01 (m, 8H), 5.49 (s, 1H), 5.11 (s, 2H), 3.65 (br, 2H), 2.29 (s, 3H), 1.31 (s, 9H)
MS (ESI):336 (M+H⁺)

Reference Example 66

### 2,2,2-trichloroethyl 1-(3-(benzyloxy)-4-methylphenyl)-3-t-butyl-1H-pyrazol-5-yl carbamate

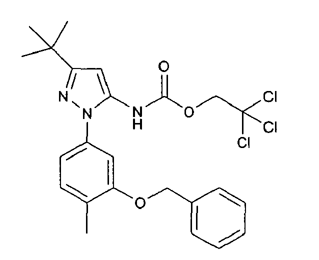

To ethyl acetate (8 mL), 1-(3-(benzyloxy)-4-methylphenyl)-3-t-butyl-1H-pyrazol-5-amine (959 mg) was added, after which a solution separately prepared from sodium hydroxide (172 mg) and distilled water (4 mL) was added thereto.
The mixture was cooled to 0°C and then 2,2,2-trichloroethyl chloroformate (551 µL) was added thereto, followed by stirring at room temperature for 17 hours. The reaction solution was extracted, and the obtained aqueous layer was extracted with ethyl acetate (8 mL) twice. The organic layers were combined, dried over anhydrous sodium sulfate, and evaporated under reduced pressure. The obtained residue was filtered and washed with hexane to obtain the desired product (1.06 g, yield: 73%).
¹ H-NMR (CDCl₃):δ 7.46-6.92 (m, 8H), 6.87 (br, 1H), 6.43 (br, 1H), 5.10 (s, 2H), 4.81 (s, 2H), 2.31 (s, 3H), 1.35 (s, 9H)

Reference Example 67

### 1-(2-hydroxybenzyl)-3-(1-(3-(benzyloxy)-4-methylphenyl)-3-t-butyl-1H-pyrazol-5-yl)urea

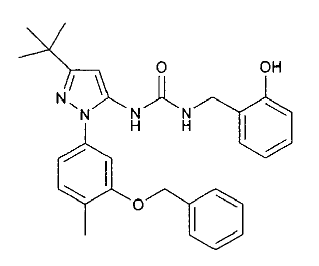

In acetonitrile (7 mL), 2,2,2-trichloroethyl 1-(3-(benzyloxy)-4-methylphenyl)-3-t-butyl-1H-pyrazol-5-yl carbamate (499 mg) and 2-hydroxybenzylamine (156 mg) were dissolved, and then diisopropylethylamine (255 µL) was added thereto. The resulting mixture was stirred at 60°C for 12 hours. DMSO (0.5 mL) was added thereto and the resulting mixture was stirred at 60°C for 3 hours. The reaction solution was evaporated under reduced pressure. The obtained residue was purified by silica gel column chromatography (ethyl acetate/n-hexane = 1/3 → 1/1) to obtain the desired product (438 mg, yield: 93%).
¹H-NMR (CDCl₃):δ 9.14 (br, 1H), 7.42-6.77 (m, 12H), 6.22 (s, 1H), 6.20 (s, 1H), 5.50 (t, 1H, J = 6.5. Hz), 5.00 (s, 2H), 4.28 (d, 2H, J = 6.5 Hz), 2.25 (s, 3H), 1.33 (s, 9H)

Reference Example 68

### 2-(4-methylphenyl)-2-oxoethyl 2,2-dimethylpropanoate

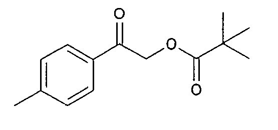

In DMF (15 mL), 2-bromo-4'-methylacetophenone (3.27 g) and pivalic acid (2.04 g) were dissolved, after which potassium carbonate (3.18 g) was added thereto. The resulting mixture was stirred at 80 °C for 14 hours. The reaction solution was filtered, and the filtrate was evaporated under reduced pressure, after which distilled water (20 mL) was added thereto. The mixture was then extracted with dichloromethane (20 mL) three times, and the organic layers were combined, dried over anhydrous sodium sulfate and evaporated under reduced pressure. The obtained residue was purified by silica gel column chromatography (chloroform/n-hexane = 7/13 → chloroform) to obtain the desired product (3.20 g, yield: 89%). ¹H-NMR (CDCl₃):δ 7.82-7.26 (m, 4H), 5.30 (s, 2H), 2.42 (s, 3H), 1.31 (s, 9H)

Reference Example 69

### 2-t-butyl-4-p-tolyloxazole

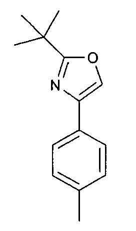

To acetic acid (2 mL), 2-(4-methylphenyl)-2-oxoethyl 2,2-dimethylpropanoate (930 mg) and ammonium acetate (3.06 g) were added and the resulting mixture was stirred at 90°C for 16 hours. Saturated aqueous sodium carbonate solution (10 mL) was added to the reaction solution, which was then extracted with ether (10 mL) three times. The organic layers were combined, dried over anhydrous sodium sulfate and evaporated under reduced pressure. The obtained residue was purified by silica gel column chromatography (n-hexane → chloroform/n-hexane = 3/7) to obtain the desired product (231 mg, yield: 27%).
¹H-NMR (CDCl₃):δ 7.75 (s, 1H), 7.62-7.18 (m, 4H), 2.36 (s, 3H), 1.42 (s, 9H)

Reference Example 70

### 2-t-butyl-4-p-tolyloxazol-5-carboxylic acid

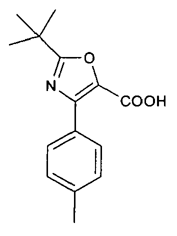

In a 100 mL eggplant type flask, 2-t-butyl-4-p-tolyloxazol (231 mg) was weighed, and dry THF (3 mL) was added thereto. The resulting mixture was cooled to -78°C, after which 2.66M n-butyllithium solution in hexane (484 µL) was added thereto. The resulting mixture was stirred at -78°C for 1 hour. Dry ice was added to the mixture and the resulting mixture was stirred at -78°C for 1.5 hours. An excess amount of distilled water was added to the reaction solution, which was then evaporated under reduced pressure. The obtained aqueous layer was extracted with dichloromethane (10 mL) three times. The obtained aqueous layer was extracted with dichloromethane (10 mL) three times, and the pH thereof was made acidic with aqueous 1M hydrochloric acid solution. The organic layers were combined, dried over anhydrous sodium sulfate and evaporated under reduced pressure to obtain the desired product (246 mg, yield: 88%).
¹H-NMR (CDCl₃):δ 7.94-7.23 (m, 4H), 2.40 (s, 3H), 1.48 (s, 9H)
MS (ESI):260 (M+H⁺)

Reference Example 71

### 1-(2-hydroxybenzyl)-3-(2-t-butyl-4-p-tolyloxazol-5-yl)urea

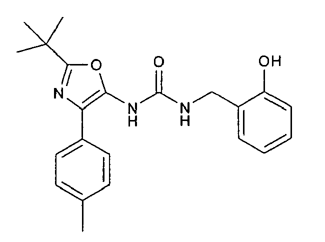

In toluene (2.5 mL), 2-t-butyl-4-p-tolyloxazol-5-carboxylic acid (241 mg) and diphenylphosphorylazide (240 µL) were dissolved, and triethylamine (194 µL) was added thereto. The resulting mixture was stirred at room temperature for 1 hour and then at 80 °C for 2 hours. To this mixture, 2-hydroxybenzylamine (149 mg) and acetonitrile (3.0 mL) were added and the resulting mixture was stirred at 60 °C for 15 hours. The reaction solution was evaporated under reduced pressure, and the resulted residue was purified by silica gel column chromatography (ethyl acetate/n-hexane = 1/9 → 3/7) to obtain the desired product (73.8 mg, yield: 21%).
¹ H-NMR (CDCl₃):δ 8.99 (br, 1H), 7.62-6.73 (m, 8H), 6.30 (s, 1H), 5.42 (t, 1H, J = 6.5 Hz), 4.25 (d, 2H, J = 6.5 Hz), 2.32 (s, 3H), 1.39 (s, 9H)
MS (ESI):380 (M+H⁺)

Reference Example 72

### 2,2,2-trichloroethyl 5-t-butyl-1,3,4-thiadiazol-2-yl carbamate

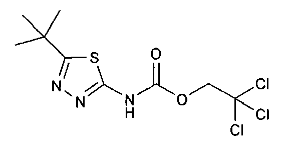

In THF (25 mL), 2-amino-5-t-butyl-1,3,4-thiadiazole (1.00 g), triethylamine (1.33 mL) and 2,2,2-trichloroethyl chloroformate (1.23 mL) were dissolved, followed by stirring at room temperature for 16 hours. The reaction solution was evaporated under reduced pressure, and distilled water (15 mL) was added to the obtained residue, which was then extracted with ethyl acetate (15 mL) three times. The organic layers were combined, dried over anhydrous sodium sulfate, and evaporated under reduced pressure. The obtained residue was filtered and washed with hexane, and the obtained solids were purified by silica gel column chromatography (ethyl acetate/n-hexane = 1/4 → 9/11) to obtain the desired product (1.31 g, yield: 62%). ¹H-NMR (CDCl₃):δ 4.92 (s, 2H), 1.46 (s, 9H)

Reference Example 73

### 1-(2-hydroxybenzyl)-3-(5-t-butyl-1,3,4-thiadiazol-2-yl)urea

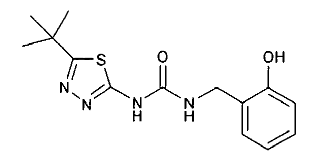

In acetonitrile (12 mL), 2,2,2-trichloroethyl 5-t-butyl-1,3,4-thiadiazol-2-yl carbamate (312 mg) and 2-hydroxybenzylamine (150 mg) were dissolved, and then diisopropylethylamine (245 µL) was added thereto. The resulting mixture was stirred at 60 °C for 44 hours. The reaction solution was evaporated under reduced pressure, and the obtained residue was purified by silica gel column chromatography (ethyl acetate/n-hexane = 1/1 → 4/1) to obtain the desired product (208 mg, yield: 73%).
¹H-NMR (CDCl₃):δ 7.23-6.83 (m, 4H), 4.44 (d, 2H, J = 6.4 Hz), 1.47 (s, 9H)
MS (ESI):307 (M+H⁺)

Reference Example 74

### 2,2,2-trichloroethyl 5-(furan-2-yl)-1,3,4-oxaoxadiazol-2-yl carbamate

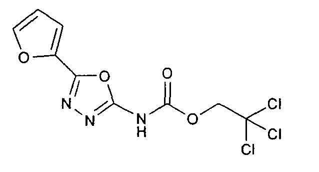

In THF (25 mL), 2-amino-5-(2-furyl)-1,3,4-oxadiazole (981 mg), triethylamine (1.36 mL) and 2,2,2-trichloroethyl chloroformate (1.16 mL) were dissolved, followed by stirring at room temperature for 20 hours. The reaction solution was evaporated under reduced pressure, and distilled water (15 mL) was added to the obtained residue, which was then extracted with ethyl acetate (15 mL) three times. The organic layers were combined, dried over anhydrous sodium sulfate, and evaporated under reduced pressure. The obtained residue was purified by silica gel column chromatography (ethyl acetate/n-hexane = 1/4 → 11/9) to obtain the desired product (666 mg, yield: 31 %).
¹H-NMR (CDCl₃):δ 7.64 (d, 1H, J = 1.7 Hz), 7.16 (d, 1H, J = 3.4 Hz), 6.60 (dd, 1H, J = 1.7, 3.4 Hz), 4.90 (s, 2H)

Reference Example 75

### 1-(2-hydroxybenzyl)-3-(5-(furan-2-yl)-1,3,4-oxadiazol-2-yl)urea

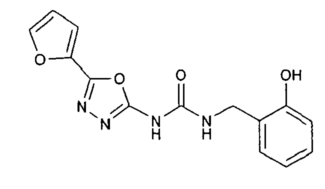

In acetonitrile (12 mL), 2,2,2-trichloroethyl 5-(furan-2-yl)-1,3,4-oxadiazol-2-yl carbamate (320 mg) and 2-hydroxybenzylamine (157 mg) were dissolved, and then diisopropylethylamine (256 µL) were added thereto. The resulting mixture was stirred at 60 °C for 16 hours. The precipitated solids were filtered, and washed with ether and hexane to obtain the desired product (259 mg, yield: 88%).
¹H-NMR (CD₃ OD):δ 7.77 (d, 1H, J = 1.5 Hz), 7.22-6.76 (m, 5H), 6.67 (dd, 1H, J = 1.5, 3.5 Hz), 4.45 (s, 2H)
MS (ESI):301 (M+H⁺)

Reference Example 76

### 2,2,2-trichloroethyl-4-t-butylthiazol-2-yl carbamate

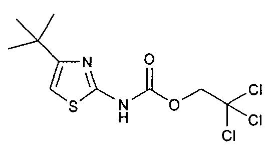

In THF (25 mL), 2-amino-4-t-butylthiazole (1.02 g), triethylamine (1.18 mL) and 2,2,2-trichloroethyl chloroformate (899 µL) were dissolved, followed by stirring at room temperature for 17 hours. The reaction solution was evaporated under reduced pressure, and distilled water (15 mL) was added to the obtained residue, which was then extracted with ethyl acetate (15 mL) three times. The organic layers were combined, dried over anhydrous sodium sulfate, and evaporated under reduced pressure. The obtained residue was purified by silica gel column chromatography (ethyl acetate/n-hexane = 1/19 → 1/3) to obtain the desired product (1.16 g, yield: 53%).
¹H-NMR (CDCl₃): δ 6.55 (s, 1H), 4.87 (s, 2H), 1.29 (s, 9H)

Reference Example 77

### 1-(2-hydroxybenzyl)-3-(4-t-butylthiazol-2-yl)urea

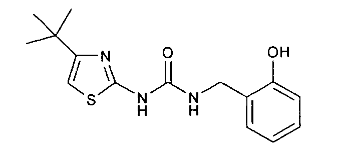

In acetonitrile (12 mL), 2,2,2-trichloroethyl 4-t-butylthiazol-2-yl carbamate (380 mg) and 2-hydroxybenzylamine (183 mg) were dissolved, and then diisopropylethylamine (299 µL) was added thereto. The resulting mixture was stirred at 60 °C for 44 hours. The reaction solution was evaporated under reduced pressure, and the obtained residue was purified by silica gel column chromatography (ethyl acetate/n-hexane = 1/4 → 2/3) to obtain the desired product (327 mg, yield: 94%).
¹H-NMR (CDCl₃): δ 7.27-6.84 (m, 5H), 6.37 (s, 1H), 6.36 (s, 1H), 4.47 (d, 2H, J = 6.6 Hz), 1.29 (s, 9H)
MS (ESI):306 (M+H⁺)

Reference Example 78

### 2,2,2-trichloroethyl 4-methyloxazol-2-yl carbamate

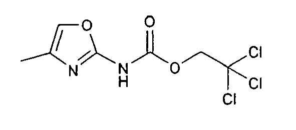

To ethyl acetate (8 mL), 4-methyl-1,3-oxazol-2-amine (522 mg) was added, and then a solution separately prepared from sodium hydroxide (320 mg) and distilled water (8 mL) was added thereto. To the mixture, 2,2,2-trichloroethyl chloroformate (953 µL) was added, followed by stirring at room temperature for 12 hours. The reaction solution was extracted, and the obtained aqueous layer was extracted with ethyl acetate (10 mL) twice. The organic layers were combined, dried over anhydrous sodium sulfate, and evaporated under reduced pressure. The obtained residue was purified by silica gel column chromatography (ethyl acetate/n-hexane = 7/3 → 11/9) to obtain the desired product (501 mg, yield: 34%).
¹H-NMR (CDCl₃):δ 7.18 (d, 1H, J = 1.2 Hz), 4.87 (s, 2H), 2.19 (d, 3H, J = 1.2 Hz)

Reference Example 79

### 1-(2-hydroxybenzyl)-3-(4-methyloxazol-2-yl)urea

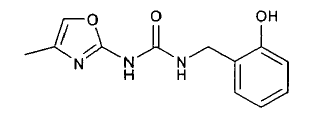

In acetonitrile (7.0 mL), 2,2,2-trichloroethyl 4-methyloxazol-2-yl carbamate (252 mg) and 2-hydroxybenzylamine (148 mg) were dissolved, and then diisopropylethylamine (241 µL) was added thereto. The resulting mixture was stirred at 60°C for 17 hours. The reaction solution was evaporated under reduced pressure, and the obtained residue was purified by silica gel column chromatography (ethyl acetate/n-hexane = 3/7 → 1/1) to obtain the desired product (142 mg, yield: 62%).
¹H-NMR (CDCl₃): δ 9.21-9.16 (m, 2H), 8.54 (br, 1H), 7.24-6.83 (m, 5H), 4.47 (d, 2H, J = 6.4 Hz), 2.10 (d, 3H, J = 1.2 Hz)
MS (ESI):248 (M+H⁺)

Reference Example 80

### 5-t-butyl-2-(6-methylpyridin-3-yl)-2H-pyrazol-3-ylamine

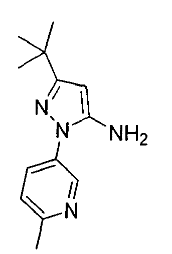

An aqueous sodium nitrite (770 mg) solution (5 mL) was added to 6-methylpyridin-3-ylamine (1.13 g) in 6N hydrochloric acid (10 mL), and the resulting mixture was stirred at 0°C for 1 hour. Then a solution of SnCl₂-2H₂O (5.90 g) in 6N hydrochloric acid (10 mL) was added thereto and the resulting mixture was stirred at 0 °C for 4 hours. Aqueous 5N NaOH solution (25 mL) was added thereto to make the pH thereof above 9, and the resulting mixture was extracted with ethyl acetate. The organic layers were combined, washed with water and saturated brine, and dried over anhydrous sodium sulfate. The solvent was evaporated, and 4,4-dimethyl-3-oxopentanitrile (1.35 g) and ethanol (15 mL) were added to the obtained residue, which was then stirred at 60 °C for 15 hours. Ethanol was evaporated, and the residue was poured into saturated aqueous sodium bicarbonate solution, and then extracted with ethyl acetate. The organic layers were combined, washed with water and saturated brine, and dried over anhydrous sodium sulfate. The solvent was evaporated, and the obtained residue was purified by silica gel column chromatography (ethyl acetate/n-hexane = 3/2 → 3/1) to obtain the desired product (566 mg, yield: 23.5%).
¹H-NMR (CDCl₃):δ 8.74 (d, 1H, J=2.5 Hz), 7.81 (dd, 1H, J=2.5, 8.1 Hz), 7.25 (d, 1H, J=8.1 Hz), 5.56 (s, 1H), 3.68 (s, 2H), 2.59 (s, 3H), 1.31(s, 9H)
MS (ESI):231 (M+H⁺)

Reference Example 81

### (5-t-butyl-2-(6-methylpyridin-3-yl)-2H-pyrazol-3-yl) carbamic acid 2,2,2-trichloroethyl ester

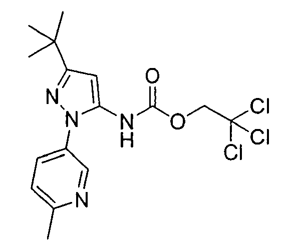

In ethyl acetate (15 mL), 5-t-butyl-2-(6-methylpyridin-3-yl)-2H-pyrazol-3-ylamine (566 mg) was dissolved and the resulting mixture was cooled to 0°C. To the mixture, aqueous 5N sodium hydroxide solution (1 mL) was added, and 2,2,2-trichloroethyl chloroformate (1.0 g) was added dropwise thereto. The mixture was stirred at 0°C for 2.5 hours and then at room temperature for 28 hours, after which the organic layer was separated. The aqueous layer was extracted with ethyl acetate (100 mL) twice. The organic layers were combined, washed with water and saturated brine, and dried over anhydrous sodium sulfate. The residue obtained by concentration was purified by silica gel column chromatography (ethyl acetate/n-hexane = 1/1 - 1/0) to obtain the desired product (606 mg, yield: 60.7%).
¹H-NMR (CDCl₃):δ 8.62 (d, 1H, J=2.5 Hz), 7.75 (dd, 1H, J=2.5, 8.1 Hz), 7.27 (d, 1H, J=8.1 Hz), 7.17 (s, 1H), 6.43 (s, 1H), 4.79 (s, 2H), 2.58 (s, 3H), 1.34 (s, 9H)
MS (ESI):405, 407, 409 (M+H⁺), 403, 405, 407 (M-H⁺)

Reference Example 82

### 1-(2-hydroxybenzyl)-3-(3-t-butyl-1-(6-methylpyridin-3-yl)-1H-pyrazol-5-yl)urea

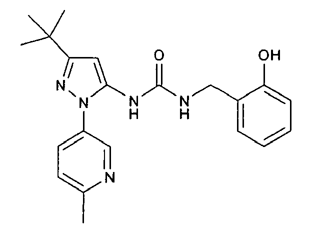

In acetonitrile (3 mL), 2,2,2-trichloroethyl 3-t-butyl-1-(6-methylpyridin-3-yl)-1H-pyrazol-5-yl carbamate (105 mg) and 2-hydroxybenzylamine (41.4 mg) were dissolved, and diisopropylethylamine (67.6 µL) was added thereto. The resulting mixture was stirred at 60 °C for 13 hours. The reaction solution was evaporated under reduced pressure, and the obtained residue was purified by silica gel column chromatography (ethyl acetate/n-hexane = 1/1 → 4/1) to obtain the desired product (78.5 mg, yield: 80%).
¹H-NMR (CDCl₃):δ 9.09 (br, 1H), 8.37-6.74 (m, 7H), 7.92 (br, 1H), 6.41 (s, 1H), 6.07 (t, 1H, J = 6.4 Hz), 4.29 (d, 2H, J = 6.4 Hz), 2.28 (s, 3H), 1.33 (s, 9H)

Reference Example 83

### 2-chloro-N-(2-hydroxy-3-methyl-5-fluorobenzyl) acetamide

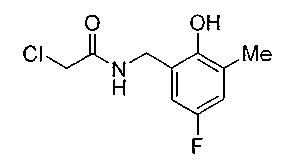

In acetic acid (4.0 mL), -methyl-4-fluorophenol (5.00 g) and 2-chloro-N-hydroxymethyl acetamide (6.02 g) were dissolved, and concentrated sulfuric acid (4.0 mL) was added to the resulting mixture, which was then stirred at room temperature for 16 hours. The reaction solution was poured into ice water and extracted with ethyl acetate. The extract was washed with water and saturated brine, dried over anhydrous sodium sulfate, and evaporated under reduced pressure to obtain the desired product (8.80 g, yield: 90%).
¹H-NMR (CDCl₃):δ 8.57 (s, 1H), 7.38 (bs, 1H), 6.84 (dd, 1H, J=2.9, 8.8 Hz), 6.70 (dd, 1H, J=2.9, 8.3 Hz), 4.37 (d, 2H, J = 6.6 Hz), 4.11 (s, 2H), 2.25 (s, 3H).
MS (ESI):232, 234 (M+H⁺), 230, 232 (M-H⁺).

Reference Example 84

### 2-chloro-N-(2-hydroxy-3,5-difluorobenzyl) acetamide

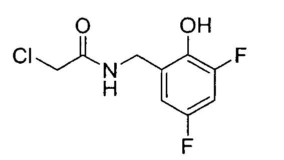

In acetic acid (4.0 mL), 2,4-difluorophenol (5.00 g) and 2-chloro-N-hydroxymethyl acetamide (6.04 g) were dissolved, and concentrated sulfuric acid (4.0 mL) was added to the resulting mixture, which was then stirred at room temperature for 17 hours. The reaction solution was poured into ice water and extracted with ethyl acetate. The extract was washed with water and saturated brine, dried over anhydrous sodium sulfate, and evaporated under reduced pressure. The obtained residue was purified by silica gel column chromatography (ethyl acetate/n-hexane = 1/1) to obtain the desired product (4.38 g, yield: 48%).
¹H-NMR (CDCl₃):δ 8.10 (bs, 1H), 7.41 (bs, 1H), 6.83 (m, 1H), 6.72 (m, 1H), 4.43 (d, 2H, J = 6.3 Hz), 4.12 (s, 2H).
MS (ESI):236, 238 (M+H⁺), 234, 236 (M-H⁺).

Reference Example 85

### 2-hydroxy-3-methyl-5-fluorobenzylamine

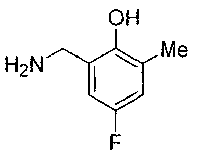

In the mixture of ethanol (10 mL) and aqueous 1N hydrochloric acid solution (50 mL), 2-chloro-N-(2-hydroxy-3-methyl-5-fluorobenzyl) acetamide (8.80 g) was dissolved, and the resulting mixture was stirred at 80 °C for 4 days. The reaction mixture was cooled to room temperature, neutralized with sodium bicarbonate, and extracted with ethyl acetate. The extract was washed with water and saturated brine, then dried over anhydrous sodium sulfate, and evaporated under reduced pressure. The obtained residue was recrystallized from ethyl acetate to obtain the desired product (4.32 g, yield: 73%).
¹H-NMR (DMSO-d₆):δ 6.87 (dd, 1H, J=3.1, 9.2 Hz), 6.81 (dd, 1H, J=3.1, 9.2 Hz), 3.91 (s, 2H), 2.13 (s, 3H). MS (ESI):156 (M+H⁺), 154 (M-H⁺).

Reference Example 86

### 2-hydroxy-3,5-difluorobenzylamine

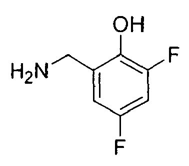

In the mixture of ethanol (30 mL) and aqueous 1N hydrochloric acid solution (30 mL), 2-chloro-N-(2-hydroxy-3,5-difluorobenzyl) acetamide (4.30 g) was dissolved, and the resulting mixture was stirred for 4 days at 80 °C. The reaction mixture was cooled to room temperature, neutralized with sodium bicarbonate, and extracted with ethyl acetate. The extract was washed with water and saturated brine, then dried over anhydrous sodium sulfate, and evaporated under reduced pressure. The obtained residue was recrystallized from ethyl acetate to obtain the desired product (2.67 g, yield: 92%).
¹H-NMR (DMSO-d₆):δ 6.99 (m, 1H), 6.79 (m, 1H), 3.88 (s, 2H).
MS (ESI):160 (M+H⁺), 158 (M-H⁺).

Reference Example 87

### 1-(2-hydroxy-3-methyl-5-fluorobenzyl)-3-(3-t-butyl-1-p-tolyl-1H-pyrazol-5-yl)urea

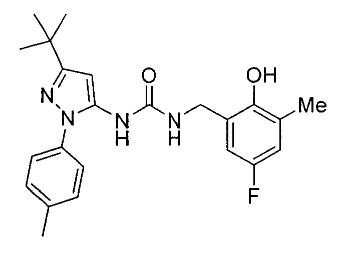

To a solution of 2-hydroxy-3-methyl-5-fluorobenzylamine (1.18 g) in DMSO (2.0 mL), 2,2,2-trichloroethyl 3-t-butyl-1-p-tolyl-1H-pyrazol-5-yl carbamate (2.52 g) and diisopropylethylamine (1.50 mL) were added, and the resulting mixture was stirred at 50 °C for 6 hours. Aqueous 5% citric acid solution was poured into the reaction mixture, which was then extracted with ethyl acetate. The extract was washed with water and saturated brine, then dried over anhydrous sodium sulfate, and evaporated under reduced pressure. The obtained residue was purified by silica gel column chromatography (ethyl acetate/n-hexane = 1/9 - 1/1) to obtain the desired product (2.31 g, yield: 90%).
¹H-NMR (CDCl₃):δ 9.05 (br, 1H), 7.23 (d, 2H, J=8.4 Hz), 7.14 (d, 2H, J=8.4 Hz), 6.81 (dd, 1H, J=3.0, 8.8 Hz), 6.55 (dd, 1H, J=3.0, 7.6 Hz), 6.23 (s, 1H), 6.20 (s, 1H), 5.58 (t, 1H, J = 6.4 Hz), 4.22 (d, 2H, J = 6.4 Hz), 2.33 (s, 3H), 2.25 (s, 3H), 1.33 (s, 9H)
MS (ESI):411 (M+H⁺), 409 (M-H⁺).

Reference Example 88

### 1-(2-hydroxy-3,5-difluorobenzyl)-3-(3-t-butyl-1-p-tolyl-1H-pyrazol-5-yl)urea

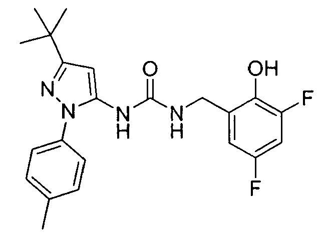

To a solution of 2-hydroxy-3,5-difluorobenzylamine (1.11 g) in DMSO (2.0 mL), 2,2,2-trichloroethyl 3-t-butyl-1-p-tolyl-1H-pyrazol-5-yl carbamate (2.90 g) and diisopropylethylamine (1.50 mL) were added, and the resulting mixture was stirred at 55 °C for 9 hours. Aqueous 5% citric acid solution was poured into the reaction mixture, which was then extracted with ethyl acetate. The extract was washed with water and saturated brine, then dried over anhydrous sodium sulfate, and evaporated under reduced pressure. The obtained residue was purified by silica gel column chromatography (ethyl acetate/n-hexane = 3/7 - 1/1) to obtain the desired product (1.73 g, yield: 60%).
¹ H-NMR (CDCl₃):δ 8.93 (br, 1H), 7.59 (d, 2H, J=8.0 Hz), 7.17 (d, 2H, J=8.0 Hz), 6.80 (m, 1H), 6.55 (m, 1H), 6.21 (s, 1H), 6.18 (bs, 1H), 5.54 (t, 1H, J = 6.6 Hz), 4.26 (d, 2H, J = 6.6 Hz), 2.34 (s, 3H), 1.34 (s, 9H)
MS (ESI):415 (M+H⁺), 413 (M-H⁺).

Reference Example 89

### 2-((tributylstanyl) methyl) benzonitrile

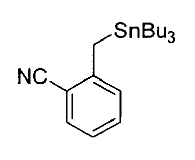

To a solution of 2-cyanobenzyl bromide (1 g) in toluene (10 mL), bistributyltin (2.58 mL) and tetrakis(triphenylphosphine)palladium (177 mg) were added, and the resulting mixture was stirred at 120 °C for 2 hours. The reaction solution was filtered, and the obtained filtrate was evaporated under reduced pressure. The obtained residue was purified by silica gel column chromatography (ethyl acetate/n-hexane = 1/20) to obtain the desired product (1.08 g, yield: 52%).
¹H-NMR (CDCl₃):δ 7.48 (d, 1H, J = 6.6 Hz), 7.33-7.37 (m, 1H), 7.08 (d, 1H, J = 8.0 Hz), 7.03 (t, 1H, J = 7.6), 2.55 (s, 2H), 1.65-1.70 (m, 2H), 1.20-1.58 (m, 16H), 0.80-1.02 (m, 9H)
MS (ESI):408 (M+H⁺)

Reference Example 90

### 2-((2-chloropyrimidin-4-yl)methyl)benzonitrile

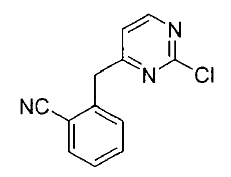

To a solution of 2-((tributylstanyl)methyl)benzonitrile (1.08 g) in DMF (2 mL), 2,4-dichloropyrimidine (416 mg) and diphenylphosphine palladium dichloride (177 mg) were added, and the resulting mixture was stirred at 120 °C for 2 hours. Saturated aqueous sodium bicarbonate solution was added to the reaction solution, which was then extracted with ethyl acetate. The organic layer was washed with aqueous 1N hydrochloric acid solution and saturated brine, and dried over magnesium sulfate. The organic layer was filtered, and the obtained filtrate was evaporated under reduced pressure. The obtained residue was purified by silica gel column chromatography (ethyl acetate/n-hexane = 1/2) to obtain the desired product (254 mg, yield: 42%).
¹ H-NMR (CDCl₃):δ 8.55 (d, 1H, J = 5.1 Hz), 7.69 (d, 1H, J = 7.8 Hz), 7.60 (t, 1H, J = 7.6 Hz), 7.49 (d, 1H, J = 7.8 Hz), 7.42 (t, 1H, J = 7.8 Hz), 7.21 (d, 1H, J = 4.9 Hz), 4.34 (s, 2H)
MS (ESI):230 (M+H⁺)

Reference Example 91

### (2-((2-chloropyrimidin-4-yl)methyl)phenyl)methanamine

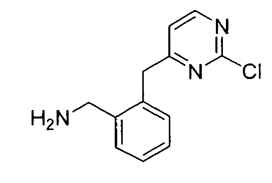

To a solution of 2-((2-chloropyrimidin-4-yl)methyl)benzonitrile (252 mg) in methanol (5 mL), cobalt chloride (II) hexahydrate (629 mg) and sodium borohydride (166 mg) were added, and the resulting mixture was stirred at room temperature for 30 minutes. Saturated aqueous sodium bicarbonate solution was added to the reaction solution, which was then extracted with ethyl acetate. The organic layer was washed with saturated brine, and dried over magnesium sulfate. The organic layer was filtered, and the obtained filtrate was evaporated under reduced pressure to obtain a residue (106 mg, yield: 41 %).
MS (ESI):234 (M+H⁺)

Reference Example 92

### 1-(2-hydroxybenzyl)-3-(3-t-butyl-1-(4-isopropylphenyl)-1H-pyrazol-5-yl)urea

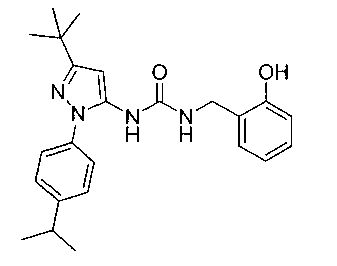

To a solution of o-hydroxybenzylamine (185 mg) in acetonitrile (6 mL), 2,2,2-trichloroethyl 3-t-butyl-1-(4-isopropylphenyl)-1H-pyrazol-5-yl carbamate (650 mg) and diisopropylethylamine (0.40 mL) were added, and the resulting mixture was stirred at 60 °C for two hours. The reaction mixture was evaporated under reduced pressure, and then the obtained residue was purified by silica gel column chromatography (ethyl acetate/n-hexane = 0/10 → 5/5) to obtain the desired product (603 mg, yield: 99%).
¹H-NMR (CDCl₃):δ 9.17 (br, 1H), 7.27-6.79 (m, 8H), 6.19 (s, 1H), 6.14 (s, 1H), 5.61 (t, 1H, J = 6.3 Hz), 4.33 (d, 2H, J = 6.3 Hz), 2.91 (dq, 1H, J = 6.8 Hz), 1.33 (s, 9H), 1.23 (d, 6H, J = 6.8 Hz).
MS (ESI):407 (M+H⁺).

Reference Example 93

### 1-(2-hydroxybenzyl)-3-(3-t-butyl-1-(4-methoxyphenyl)-1H-pyrazol-5-yl)urea

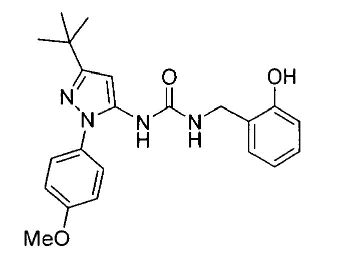

To a solution of o-hydroxybenzylamine (271 mg) in acetonitrile (9 mL), 2,2,2-trichloroethyl 3-t-butyl-1-(4-methoxyphenyl)-1H-pyrazol-S-yl carbamate (926 mg) and diisopropylethylamine (0.58 mL) were added, and the resulting mixture was stirred at 60 °C for 6 hours. The reaction mixture was evaporated under reduced pressure, and the obtained residue was purified by silica gel column chromatography (ethyl acetate/n-hexane = 0/10 → 6/4) to obtain the desired product (698 mg, yield: 81%).
¹H-NMR (CDCl₃):δ 9.14 (br, 1H), 7.27-6.79 (m, 8H), 6.18 (s, 1H), 6.05 (s, 1H), 5.55 (t, 1H, J = 6.3 Hz), 4.32 (d, 2H, J = 6.3 Hz), 3.80 (s, 3H), 1.33 (s, 9H).
MS (ESI):395 (M+H⁺).

Reference Example 94

### Methyl 3-(5-(3-(2-hydroxybenzyl)ureido)-3-t-butyl-1H-pyrazol-1-yl) benzoate

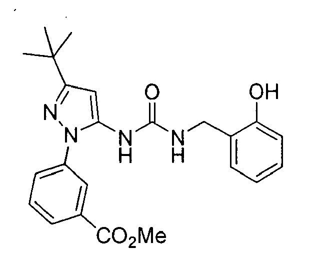

To a solution of o-hydroxybenzylamine (139 mg) in acetonitrile (4.5 mL), methyl 3-(3-t-butyl-5-((2,2,2-trichloroethoxy)carbonyl)-1H-pyrazol-1-yl) benzoate (507 mg) and diisopropylethylamine (0.30 mL) were added, and the resulting mixture was stirred at 60 °C overnight. The reaction mixture was evaporated under reduced pressure, and then the obtained residue was purified by silica gel column chromatography (ethyl acetate/n-hexane = 0/10 → 5/5) to obtain the desired product (383 mg, yield: 80%).
¹H-NMR (CDCl₃):δ 9.01 (br, 1H), 8.06-6.77 (m, 8H), 6.37 (s, 1H), 6.26 (s, 1H), 5.63 (t, 1H, J = 6.3 Hz), 4.29 (d, 2H, J = 6.3 Hz), 3.90 (s, 3H), 1.34 (s, 9H).
MS (ESI):423 (M+H⁺).

Reference Example 95

### 1-(2-hydroxybenzyl)-3-(3-t-butyl-1-(3-methoxyphenyl)-1H-pyrazol-5-yl)urea

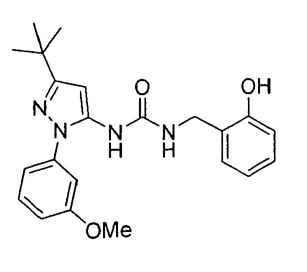

To a solution of o-hydroxybenzylamine (383 mg) in acetonitrile (12 mL), 2,2,2-trichloroethyl 3-t-butyl-1-(3-methoxyphenyl)-1H-pyrazol-5-yl carbamate (1310 mg) and diisopropylethylamine (0.82 mL) were added, and the resulting mixture was stirred at 60 °C for 6 hours. The reaction mixture was evaporated under reduced pressure, and then the obtained residue was purified by silica gel column chromatography (ethyl acetate/n-hexane = 0/10 → 5/5) to obtain the desired product (786 mg, yield: 64%).
¹H-NMR (CDCl₃):δ 9.13 (br, 1H), 7.29-6.79 (m, 8H), 6.23 (s, 1H), 6.20 (s, 1H), 5.59 (t, 1H, J = 6.6 Hz), 4.32 (d, 2H, J = 6.6 Hz), 3.73 (s, 3H), 1.34 (s, 9H).
MS (ESI):395 (M+H⁺).

Reference Example 96

### 1-(2-hydroxybenzyl)-3-(3-t-butyl-1-(3-(2-hydroxyethoxy)phenyl)-1H-pyrazol-5-yl)urea

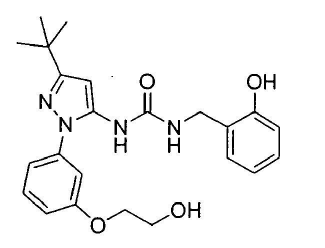

To a solution of o-hydroxybenzylamine (75 mg) in acetonitrile (2.5 mL), 2,2,2-trichloroethyl 3-t-butyl-1-(3-(2-hydroxyethoxy)phenyl)-1 H-pyrazol-5-yl carbamate (275 mg) and diisopropylethylamine (0.16 mL) were added, and the resulting mixture was stirred at 60 °C for 6 hours. The reaction mixture was evaporated under reduced pressure, and then the obtained residue was purified by silica gel column chromatography (ethyl acetate/n-hexane = 3/7 → 10/0) to obtain the desired product (203 mg, yield: 78%).
¹H-NMR (CDCl₃):δ 9.12 (br, 1H), 7.22-6.74 (m, 8H), 6.59 (s, 1H), 6.25 (s, 1H), 5.98 (t, 1H, J = 6.3 Hz), 4.27 (d, 2H, J = 6.3 Hz), 3.94-3.85 (m, 4H), 2.68 (br, 1H), 1.32 (s, 9H).
MS (ESI):425 (M+H⁺).

Reference Example 97

### 1-(2-hydroxybenzyl)-3-(3-t-butyl-1-(4-ethylphenyl)-1H-pyrazol-5-yl)urea

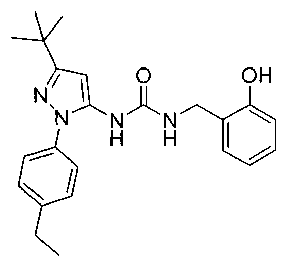

To a solution of o-hydroxybenzylamine (161 mg) in acetonitrile (5 mL), 2,2,2-trichloroethyl 3-t-butyl-1-(4-ethylphenyl)-1H-pyrazol-5-yl carbamate (548 mg) and diisopropylethylamine (0.35 mL) were added, and the resulting mixture was stirred at 60°C overnight. The reaction mixture was evaporated under reduced pressure, and then the obtained residue was purified by silica gel column chromatography (ethyl acetate/n-hexane = 2/8 → 5/5) to obtain the desired product (395 mg, yield: 77%).
¹H-NMR (CDCl₃):δ 9.15 (br, 1H), 7.26-6.79 (m, 8H), 6.20 (s, 1H), 6.13 (s, 1H), 5.58 (t, 1H, J = 6.6 Hz), 4.32 (d, 2H, J = 6.6 Hz), 2.64 (q, 2H, J = 7.6 Hz), 1.33 (s, 9H), 1.22 (t, 3H, J = 7.6 Hz).
MS (ESI):393 (M+H⁺).

Reference Example 98

### 1-(2-hydroxybenzyl)-3-(3-t-butyl-1-(3-(dimethylamino)phenyl)-1H-pyrazol-5-yl)urea

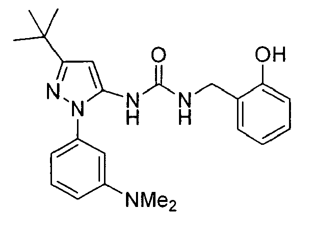

To a solution of o-hydroxybenzylamine (246 mg) in acetonitrile (8 mL), 2,2,2-trichloroethyl 3-t-butyl-1-(3-(dimethylamino)phenyl)-1H-pyrazol-5-yl carbamate (868 mg) and diisopropylethylamine (0.52 mL) were added, and the resulting mixture was stirred at 60°C overnight. The resulting mixture was evaporated under reduced pressure, and then the obtained residue was purified by silica gel column chromatography (ethyl acetate/n-hexane = 0/10 → 5/5) to obtain the desired product (682 mg, yield: 84%).
¹H-NMR (CDCl₃):δ 9.25 (br, 1H), 7.23-6.60 (m, 8H), 6.24 (s, 1H), 6.22 (s, 1H), 5.63 (t, 1H, J = 6.6 Hz), 4.32 (d, 2H, J = 6.6 Hz), 2.88 (s, 6H), 1.34 (s, 9H)
MS (ESI):408 (M+H⁺)

Reference Example 99

### 1-(2-hydroxybenzyl)-3-(3-t-butyl-1-(3-(methylthio)phenyl)-1H-pyrazol-5-yl)urea

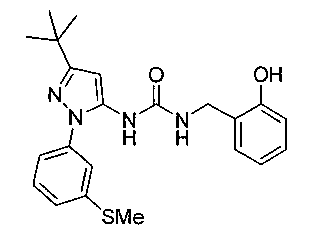

To a solution of o-hydroxybenzylamine (308 mg) in acetonitrile (10 mL), 2,2,2-trichloroethyl 3-t-butyl-1-(3-(methylthio)phenyl)-1H-pyrazol-5-yl carbamate (1092 mg) and diisopropylethylamine (0.65 mL) were added, and the resulting mixture was stirred at 60°C overnight. The resulting mixture was evaporated under reduced pressure, and then the obtained residue was purified by silica gel column chromatography (ethyl acetate/n-hexane = 0/10 → 3/7) to obtain the desired product (881 mg, yield: 86%).
¹H-NMR (CDCl₃):δ 9.08 (br, 1H), 7.28-6.79 (m, 8H), 6.25 (s, 1H), 6.23 (s, 1H), 5.61 (t, 1H, J = 6.3 Hz), 4.31 (d, 2H, J = 6.3 Hz), 2.41 (s, 3H), 1.34 (s, 9H)
MS (ESI):411 (M+H⁺)

Reference Example 100

### 1-(2-hydroxybenzyl)-3-(3-t-butyl-1-(3-(methylsulfonyl)phenyl)-1H-pyrazol-5-yl)urea

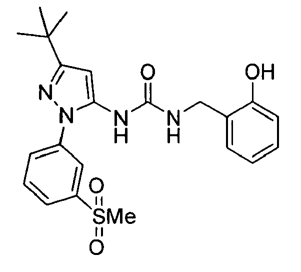

A solution of 1-(2-hydroxybenzyl)-3-(3-t-butyl-1-(3-(methylthio)phenyl)-1H-pyrazol-5-yl)urea (165 mg) in dichloromethane (3 mL) was cooled to 0°C, and 3-chloroperbenzoic acid (173 mg) was added thereto. The mixture was allowed to warm to room temperature, stirred for 1 hour, and then cooled again to 0°C, and an aqueous sodium sulfite solution was added thereto. After stirring the resulting mixture at room temperature for 10 minutes, the reaction mixture was evaporated under reduced pressure. Ethyl acetate was added to the obtained residue, which was then washed with saturated aqueous sodium hydrogen carbonate solution. The organic layer was dried over anhydrous sodium sulfate, and evaporated under reduced pressure. The obtained residue was purified by silica gel column chromatography (ethyl acetate/n-hexane = 3/7 → 10/0) to obtain the desired product (123 mg, yield: 69%).
¹H-NMR (CDCl₃):δ 8.84 (br, 1H), 7.96-6.75 (m, 9H), 6.31 (s, 1H), 5.94 (t, 1H, J = 6.1 Hz), 4.23 (d, 2H, J = 6.1 Hz), 2.93 (s, 3H), 1.32 (s, 9H)
MS (ESI):443 (M+H⁺)

Reference Example 101

### 1-(2-hydroxybenzyl)-3-(3-t-butyl-1-(3-(trifluoromethylsulfonyl) phenyl)-1H-pyrazol-5-yl)urea

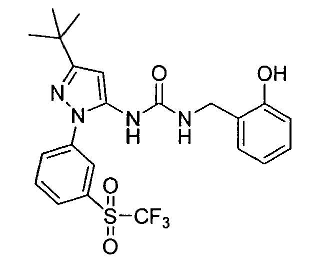

To a solution of o-hydroxybenzylamine (471 mg) in acetonitrile (15 mL), 2,2,2-trichloroethyl 3-t-butyl-1-(3-(trifluoromethylsulfonyl)phenyl)-1H-pyrazol-5-yl carbamate (2000 mg) and diisopropylethylamine (1 mL) were added, and the resulting mixture was stirred at 60°C for 2 hours. The resulting mixture was evaporated under reduced pressure, and then the obtained residue was purified by silica gel column chromatography (ethyl acetate/n-hexane = 3/7 → 7/3) to obtain the desired product (1330 mg, yield: 70%).
¹H-NMR (CDCl₃):δ 8.66 (br, 1H), 8.18-6.78 (m, 8H), 6.73 (s, 1H), 6.28 (s, 1H), 5.62 (t, 1H, J = 6.3 Hz), 4.26 (d, 2H, J = 6.3 Hz), 1.34 (s, 9H)
MS (ESI):497 (M+H⁺)

Reference Example 102

### 2,2,2-trichloroethyl 3-t-butyl-1-(4-isopropylphenyl)-1H-pyrazol-5-yl carbamate

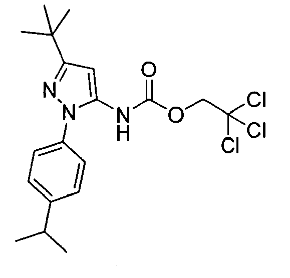

A solution of 3-t-butyl-1-(4-isopropylphenyl)-1H-pyrazol-5-amine hydrochloric acid salt (930 mg) in ethyl acetate (5 mL) was cooled to 0°C, and aqueous 1.8M sodium hydroxide solution (2.5 mL) was added thereto. After stirring the resulting mixture at 0°C for 30 minutes, 2,2,2-trichloroethyl chloroformate (0.58 mL) was added to the mixture, which was then stirred at room temperature for 2 hours. The reaction mixture was extracted with ethyl acetate, and the obtained organic layer was washed with saturated brine, dried over anhydrous sodium sulfate, and evaporated under reduced pressure. The obtained residue was purified by silica gel column chromatography (ethyl acetate/n-hexane = 0/10 → 2/8) to obtain the desired product (1.24 g, yield: 96%).
¹H-NMR (CDCl₃):δ7.38-7.33 (m, 4H), 6.90 (s, 1H), 6.41 (s, 1H), 4.81 (s, 2H), 2.96 (dq, 1H, J = 6.8,6.8 Hz), 1.34 (s, 9H), 1.27 (d, 6H, J = 6.8 Hz)
MS (ESI):432 (M+H⁺)

Reference Example 103

### 2,2,2-trichloroethyl 3-t-butyl-1-(4-methoxyphenyl)-1H-pyrazol-5-yl carbamate

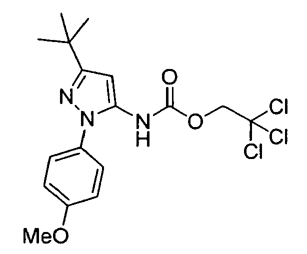

A solution of 3-t-butyl-1-(4-methoxyphenyl)-1H-pyrazol-5-amine hydrochloric acid salt (5.43 g) in ethyl acetate (34 mL) was cooled to 0°C, and aqueous 1.8M sodium hydroxide solution (17 mL) was added thereto. After stirring the resulting mixture at 0°C for 30 minutes, 2,2,2-trichloroethyl chloroformate (3.70 mL) was added to the mixture, which was then stirred at room temperature for 2 hours. The reaction mixture was extracted with ethyl acetate, and the obtained organic layer was washed with saturated brine, dried over anhydrous sodium sulfate, and evaporated under reduced pressure. Hexane was added to the obtained residue, and the precipitated solids were filtered and washed to obtain the desired product (7.33 g, yield: 90%).
¹H-NMR (CDCl₃):δ7.38-6.99 (m, 4H), 6.73 (s, 1H), 6.41 (s, 1H), 4.81 (s, 2H), 3.86 (s, 3H), 1.34 (s, 9H)
MS (ESI):420 (M+H⁺)

Reference Example 104

### Methyl 3-(3-t-butyl-5-((2,2,2-trichloroethoxy) carbonyl)-1H-pyrazol-1-yl) benzoate

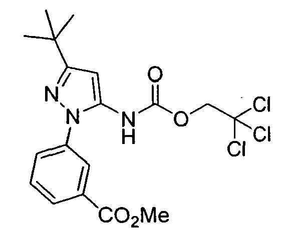

A solution of methyl 3-(5-amino-3-t-butyl-1H-pyrazol-1-yl) benzoate hydrochloric acid salt (891 mg) in ethyl acetate (5 mL) was cooled to 0°C, and aqueous 1.8M sodium hydroxide solution (2.5 mL) was added thereto. After stirring the resulting mixture at 0°C for 30 minutes, 2,2,2-trichloroethyl chloroformate (0.55 mL) was added to the mixture, which was then stirred at room temperature for 2 hours. The reaction mixture was extracted with ethyl acetate, and the obtained organic layer was washed with saturated brine, dried over anhydrous sodium sulfate, and evaporated under reduced pressure. The obtained residue was purified by silica gel column chromatography (ethyl acetate/n-hexane = 0/10 → 3/7) to obtain the desired product (1282 mg, yield: 98%).
¹H-NMR (CDCl₃):δ8.15-7.56 (m, 4H), 6.78 (s, 1H), 6.42 (s, 1H), 4.80 (s, 2H), 3.94 (s, 3H), 1.35 (s, 9H)
MS (ESI):448 (M+H⁺)

Reference Example 105

### 2,2,2-trichloroethyl 3-t-butyl-1-(3-methoxyphenyl)-1H-pyrazol-5-yl carbamate

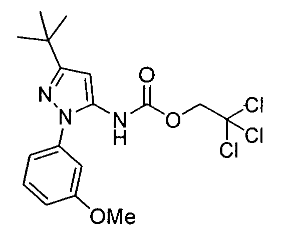

A solution of 3-t-butyl-1-(3-methoxyphenyl)-1H-pyrazol-5-amine hydrochloric acid salt (1.12 g) in ethyl acetate (6 mL) was cooled to 0°C, and aqueous 2M sodium hydroxide solution (3 mL) was added thereto. After stirring the resulting mixture at 0°C for 30 minutes, 2,2,2-trichloroethyl chloroformate (0.77 mL) was added to the mixture, which was then stirred at room temperature for 2 hours. The reaction mixture was extracted with ethyl acetate, and the obtained organic layer was washed with saturated brine, dried over anhydrous sodium sulfate, and evaporated under reduced pressure. The obtained residue was purified by silica gel column chromatography (ethyl acetate/n-hexane = 0/10 → 2/8) to obtain the desired product (1.35 g, yield: 80%).
¹H-NMR (CDCl₃):δ7.42-6.93 (m, 4H), 6.89 (s, 1H), 6.44 (s, 1H), 4.82 (s, 2H), 3.85 (s, 3H), 1.35 (s, 9H)
MS (ESI):420 (M+H⁺)

Reference Example 106

### 2,2,2-trichloroethyl 3-t-butyl-1-(3-(2-hydroxyethoxy)phenyl)-1H-pyrazol-5-yl carbamate

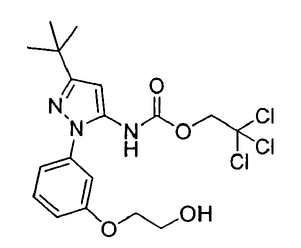

A solution of 2-(3-(5-amino-3-t-butyl-1H-pyrazol-1-yl) phenoxy) ethanol hydrochloric acid salt (1.12 g) in ethyl acetate (3 mL) was cooled to 0°C, and aqueous 1.8M sodium hydroxide solution (1.5 mL) was added thereto. After stirring the resulting mixture at 0°C for 30 minutes, 2,2,2-trichloroethyl chloroformate (0.34 mL) was added to the mixture, which was then stirred at room temperature for 2 hours. The reaction mixture was extracted with ethyl acetate, and the obtained organic layer was washed with saturated brine, dried over anhydrous sodium sulfate, and evaporated under reduced pressure. The obtained residue was purified by silica gel column chromatography (ethyl acetate/n-hexane = 0/10 → 10/0) to obtain the desired product (732 mg, yield: 92%).
¹H-NMR (CDCl₃):δ7.42-6.95 (m, 4H), 6.89 (s, 1H), 6.44 (s, 1H), 4.82 (s, 2H), 4.15-3.98 (m, 4H), 1.35 (s, 9H)
MS (ESI):450 (M+H⁺)

Reference Example 107

### 2,2,2-trichloroethyl 3-t-butyl-1-(4-ethylphenyl)-1H-pyrazol-5-yl carbamate

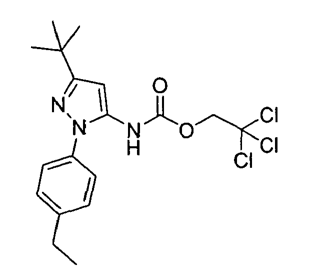

A solution of 3-t-butyl-1-(4-ethylphenyl)-1H-pyrazol-5-amine hydrochloric acid salt (1.04 g) in ethyl acetate (5.4 mL) was cooled to 0°C, and aqueous 2M sodium hydroxide solution (2.7 mL) was added thereto. After stirring the resulting mixture at 0°C for 30 minutes, 2,2,2-trichloroethyl chloroformate (0.69 mL) was added to the mixture, which was then stirred at room temperature for 2 hours. The reaction mixture was extracted with ethyl acetate, and the obtained organic layer was washed with saturated brine, dried over anhydrous sodium sulfate, and evaporated under reduced pressure. The obtained residue was purified by silica gel column chromatography (ethyl acetate/n-hexane = 0/10 → 2/8) to obtain the desired product (1.55 g, yield: 99%).
¹H-NMR (CDCl₃):δ7.38-7.3 (m, 4H), 6.83 (s, 1H), 6.42 (s, 1H), 4.81 (s, 2H), 2.70 (q, 2H, J = 7.6 Hz), 1.34 (s, 9H), 1.26 (t, 3H, J = 7.6 Hz)
MS (ESI):418 (M+H⁺)

Reference Example 108

### 2,2,2-trichloroethyl 3-t-butyl-1-(3-(dimethylamino)phenyl)-1H-pyrazol-5-yl carbamate

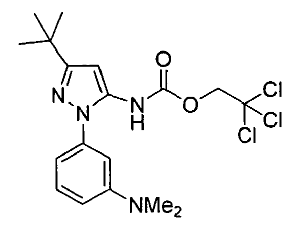

A solution of 3-t-butyl-1-(3-(dimethylamino)phenyl)-1H-pyrazol-5-amine hydrochloric acid salt (1.15 g) in ethyl acetate (7 mL) was cooled to 0°C, and aqueous 2M sodium hydroxide solution (3.5 mL) was added thereto. After stirring the resulting mixture at 0°C for 30 minutes, 2,2,2-trichloroethyl chloroformate (0.86 mL) was added to the mixture, which was then stirred at room temperature for 6 hours. The reaction mixture was extracted with ethyl acetate, and the obtained organic layer was washed with saturated brine, dried over anhydrous sodium sulfate, and evaporated under reduced pressure. The obtained residue was purified by silica gel column chromatography (ethyl acetate/n-hexane = 0/10 → 3/7) to obtain the desired product (876 mg, yield: 45%).
¹H-NMR (CDCl₃):δ 7.35-6.73 (m, 5H), 6.45 (s, 1H), 4.82 (s, 2H), 2.99 (s, 6H), 1.35 (s, 9H)
MS (ESI):433 (M+H⁺)

Reference Example 109

### 2,2,2-trichloroethyl 3-t-butyl-1-(3-(methylthio)phenyl)-1H-pyrazol-5-yl carbamate

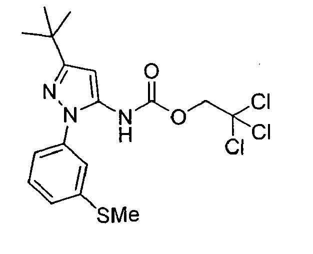

A solution of 3-t-butyl-1-(3-(methylthio)phenyl)-1H-pyrazol-5-amine hydrochloric acid salt (1.25 g) in ethyl acetate (6.4 mL) was cooled to 0°C, and aqueous 2M sodium hydroxide solution (3.2 mL) was added thereto. After stirring the resulting mixture at 0°C for 30 minutes, 2,2,2-trichloroethyl chloroformate (0.80 mL) was added to the mixture, which was then stirred at room temperature overnight. The reaction mixture was extracted with ethyl acetate, and the obtained organic layer was washed with saturated brine, dried over anhydrous sodium sulfate, and evaporated under reduced pressure. Hexane was added to the obtained residue, and the precipitated solids were filtered and washed to obtain the desired product (1.16 g, yield: 63%).
¹H-NMR (CDCl₃):δ7.41-7.20 (m, 4H), 6.81 (s, 1H), 6.43 (s, 1H), 4.82 (s, 2H), 2.51 (s, 3H), 1.35 (s, 9H)
MS (ESI):436 (M+H⁺)

Reference Example 110

### 2,2,2-trichloroethyl 3-t-butyl-1-(3-(trifluoromethylsulfonyl)phenyl)-1H-pyrazol-5-yl carbamate

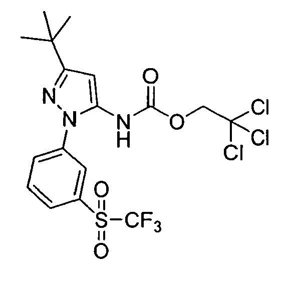

A solution of 3-t-butyl-1-(3-(trifluoromethylsulfonyl)phenyl)-1H-pyrazol-5-amine hydrochloric acid salt (2.13 g) in ethyl acetate (9 mL) was cooled to 0°C, and aqueous 2M sodium hydroxide solution (4.5 mL) was added thereto. After stirring the resulting mixture at 0°C for 30 minutes, 2,2,2-trichloroethyl chloroformate (1.15 mL) was added to the mixture, which was then stirred at room temperature for 2 hours. The reaction mixture was extracted with ethyl acetate, and the obtained organic layer was washed with saturated brine, dried over anhydrous sodium sulfate, and evaporated under reduced pressure. The obtained residue was purified by silica gel column chromatography (ethyl acetate/n-hexane = 0/10 → 2/8) to obtain the desired product (3.10 g, yield: 98%).
¹H-NMR (CDCl₃):δ8.20-7.77 (m, 4H), 6.78 (s, 1H), 6.40 (s, 1H), 4.77 (s, 2H), 1.35 (s, 9H)
MS (ESI):522 (M+H⁺)

Reference Example 111

### 3-t-butyl-1-(4-isopropylphenyl)-1H-pyrazol-5-amine hydrochloric acid salt

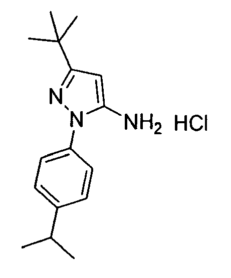

To methanol (1.5 mL), 4,4-dimethyl-3-oxopentanenitrile (376 mg) and 4-isopropylphenylhydrazine hydrochloric acid salt (560 mg) were added, and the resulting mixture was refluxed overnight. Methanol was evaporated, and diethyl ether was added to the residue to allow for crystallization. The crystals were collected by filtration and washed to obtain the desired product (952 mg, yield: 99%).
¹H-NMR (CDCl₃):δ 7.49 (d, 2H, J = 8.1 Hz), 7.40 (d, 2H, J = 8.1 Hz), 5.69 (s, 1H), 4.75 (br, 2H), 2.96 (dq, 1H, J = 7.1, 7.1 Hz), 1.47 (s, 9H), 1.21 (d, 6H, J = 7.1 Hz)
MS (ESI):258 (M+H⁺)

Reference Example 112

### 3-t-butyl-1-(4-methoxyphenyl)-1H-pyrazol-5-amine hydrochloric acid salt

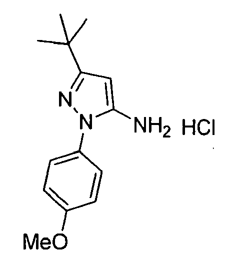

To methanol (10 mL), 4,4-dimethyl-3-oxopentanenitrile (2.50 g) and 4-methoxyphenylhydrazine hydrochloric acid salt (3.49 g) were added, and the resulting mixture was refluxed overnight. Methanol was evaporated, and diethyl ether was added to the residue to allow for crystallization. The crystals were collected by filtration and washed to obtain the desired product (5.43 g, yield: 96%).
¹H-NMR (CDCl₃):δ 7.43 (d, 2H, J = 9.0 Hz), 7.00 (d, 2H, J = 9.0 Hz), 5.76 (s, 1H), 5.30 (br, 2H), 3.83 (s, 3H), 1.42 (s, 9H)
MS (ESI):246 (M+H⁺)

Reference Example 113

### Methyl 3-(5-amino-3-t-butyl-1H-pyrazol-1-yl) benzoate hydrochloric acid salt

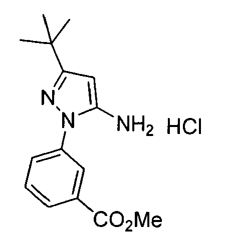

To methanol (1.5 mL), 4,4-dimethyl-3-oxopentanenitrile (401 mg) and ethyl 3-hydrazinylbenzoate hydrochloric acid salt (725 mg) were added, and the resulting mixture was refluxed overnight. Methanol was evaporated, and diethyl ether was added to the residue to allow for crystallization. The crystals were collected by filtration and washed to obtain the desired product (893 mg, yield: 86%).
¹H-NMR (CD₃ OD):δ 8.28-7.77 (m, 4H), 3.96 (s, 3H), 1.38 (s, 9H)
MS (ESI):274 (M+H⁺)

Reference Example 114

### 3-t-butyl-1-(3-methoxyphenyl)-1H-pyrazol-5-amine hydrochloric acid salt

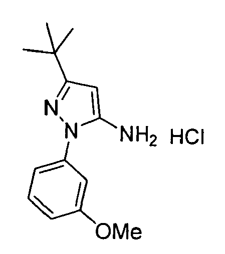

To methanol (2.5 mL), 4,4-dimethyl-3-oxopentanenitrile (628 mg) and 3-methoxyphenylhydrazine hydrochloric acid salt (876 mg) were added, and the resulting mixture was refluxed overnight. Methanol was evaporated, and diethyl ether was added to the residue to allow for crystallization. The crystals were collected by filtration and washed to obtain the desired product (1.13 g, yield: 80%). ¹H-NMR (CD₃ OD):δ 7.58-7.11 (m, 4H), 3.88 (s, 3H), 1.37 (s, 9H)
MS (ESI):246 (M+H⁺)

Reference Example 115

### 2-(3-(5-amino-3-t-butyl-1H-pyrazol-1-yl)phenoxy)ethanol hydrochloric acid salt

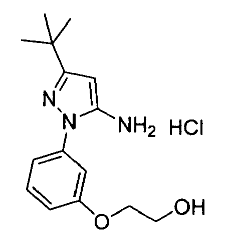

To methanol (1 mL), 4,4-dimethyl-3-oxopentanenitrile (229 mg) and 2-(3-hydrazinylphenoxy) ethanol hydrochloric acid salt (374 mg) were added, and the resulting mixture was refluxed overnight. Methanol was evaporated, and diethyl ether was added to the residue to allow for crystallization. The crystals were collected by filtration and washed to obtain the desired product (570 mg, yield: 99%).
¹H-NMR (CD₃ OD):δ 7.58-7.11 (m, 4H), 4.13 (t, 2H, J = 4.6 Hz), 3.89 (t, 2H, J = 4.6 Hz), 1.37 (s, 9H)
MS (ESI):276 (M+H⁺)

Reference Example 116

### 3-t-butyl-1-(4-ethylphenyl)-1H-pyrazol-5-amine hydrochloric acid salt

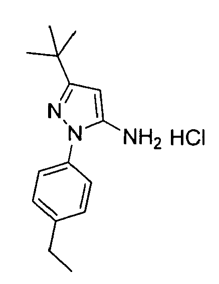

To methanol (2 mL), 4,4-dimethyl-3-oxopentanenitrile (451 mg) and 1-(4-ethylphenyl) hydrazine hydrochloric acid salt (622 mg) were added, and the resulting mixture was refluxed overnight. Methanol was evaporated, and diethyl ether was added to the residue to allow for crystallization. The crystals were collected by filtration and washed to obtain the desired product (1.04 g, yield: 99%).
¹H-NMR (CD₃ OD):δ 7.52-7.45 (m, 4H), 2.77 (q, 2H, J = 7.6 Hz), 1.37 (s, 9H), 1.28 (t, 3H, J = 7.6 Hz)
MS (ESI):244 (M+H⁺)

Reference Example 117

### 3-t-butyl-1-(3-(dimethylamino)phenyl)-1H-pyrazol-5-amine hydrochloric acid salt

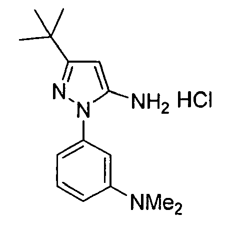

A solution of N,N-dimethylamino-1,3-phenylenediamine dihydrochloric acid salt (2.50 g) in hydrochloric acid (7.5 mL) was cooled to 0°C, and a solution separately prepared from sodium nitrite (907 mg) and distilled water (2.5 mL) was added thereto, followed by stirring at 0°C for 1 hour. A solution prepared from stannous chloride dihydrate (5.69 g) and hydrochloric acid (3.6 mL) was added to the above solution, and the resulting mixture was stirred at 0°C for 5 hours. The reaction solution was filtered, and the obtained solids were mixed with ice, after which the pH thereof was adjusted to 10 or higher with aqueous 50% KOH solution. Ethyl acetate was added thereto and the resulting mixture was filtered. The obtained organic layer and aqueous layer were separated, and the aqueous layer was extracted with ethyl acetate. The obtained organic layers were combined, dried over anhydrous sodium sulfate, and evaporated under reduced pressure. The obtained 1-(3-dimethylaminophenyl)hydrazine hydrochloric acid salt (1.52 g) and 4,4-dimethyl-3-oxopentanenitrile (1.26 g) were added to methanol (5 mL), and the resulting mixture was refluxed overnight. Methanol was evaporated, and the obtained residue was purified by silica gel column chromatography (ethyl acetate/n-hexane = 0/10 → 5/5) to obtain the desired product (1.15 g, yield: 37%).
¹ H-NMR (CDCl₃ ):δ 7.29-6.65 (m, 4H), 5.50 (s, 1H), 3.76 (br, 2H), 2.98 (s, 6H), 1.32 (s, 9H)
MS (ESI):259 (M+H⁺)

Reference Example 118

### 3-t-butyl-1-(3-(methylthio)phenyl)-1H-pyrazol-5-amine hydrochloric acid salt

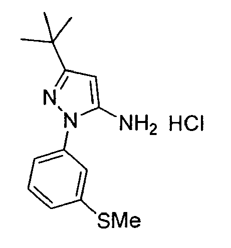

To methanol (4.5 mL), 4,4-dimethyl-3-oxopentanenitrile (1.10 g) and 1-(3-(methylthio)phenyl)hydrazine hydrochloric acid salt (1.69 g) were added, and the resulting mixture was refluxed overnight. Methanol was evaporated, and diethyl ether was added to the residue to allow for crystallization. The crystals were collected by filtration and washed to obtain the desired product (1.55 g, yield: 59%).
¹H-NMR (CD₃ OD):δ 7.58-7.29 (m, 4H), 2.55 (s, 3H), 1.37 (s, 9H)
MS (ESI):262 (M+H⁺)

Reference Example 119

### 3 -t-butyl- 1 -(3 -(trifluoromethylsulfonyl)phenyl)- 1 H-pyrazol-5-amine hydrochloric acid salt

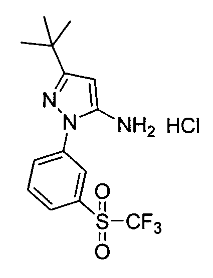

A solution of 3-(trifluoromethylsulfonyl)aniline (2.25 g) in hydrochloric acid (6.3 mL) was cooled to 0°C, and a solution separately prepared from sodium nitrite (759 mg) and distilled water (2 mL) was added thereto, followed by stirring at 0°C for 1 hour. A solution prepared from stannous chloride dihydrate (4.74 g) and hydrochloric acid (3 mL) were added to the above solution, and the resulting mixture was stirred at 0°C for 5 hours. The reaction solution was filtered, and the obtained solids were mixed with ice, after which the pH was adjusted to 10 or higher with aqueous 50% KOH solution. Ethyl acetate was added thereto and the resulting mixture was filtered. The obtained organic layer and aqueous layer were separated, and the aqueous layer was extracted with ethyl acetate. The obtained organic layers were combined, dried over anhydrous sodium sulfate, and evaporated under reduced pressure. The obtained 1-(3-(trifluoromethylsulfonyl)phenyl)hydrazine hydrochloric acid salt (1.44 g) and 4,4-dimethyl-3-oxopentanenitrile (751 mg) were added to methanol (3 mL), and the resulting mixture was refluxed overnight. Methanol was evaporated, and the obtained residue was purified by silica gel column chromatography (ethyl acetate/n-hexane = 0/10 → 3/7) to obtain the desired product (2.13 g, yield: 60%).
¹H-NMR (CDCl₃ ):δ 8.39-7.52 (m, 4H), 5.63 (s, 1H), 3.72 (br, 2H), 1.31 (s, 9H)
MS (ESI):348 (M+H⁺)

Reference Example 120

### Ethyl 3-hydrazinylbenzoate hydrochloric acid salt

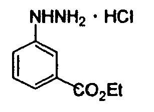

Into an eggplant type flask, ethyl 3-iodobenzoate (1.10 g), 1-carbobutoxyhydrazine (635 mg), copper (I) iodide (38 mg), 1,10-phenanthroline (144 mg), cesium carbonate (1.83 g) and DMF (4 mL) were added sequentially, and then the atmosphere inside the flask was replaced with argon, after which the resulting mixture was stirred at 80 °C for 20 hours. The resulting reaction solution was filtered and washed with ethyl acetate. The filtrate was evaporated under reduced pressure, and the obtained residue was purified by silica gel column chromatography (ethyl acetate/n-hexane = 0/10 to 3/7). Hydrogen chloride solution in methanol (6.5 mL) was added to the obtained compound, and the resulting mixture was stirred at room temperature overnight. The reaction solution was evaporated under reduced pressure, and ether (3 mL) was added to the obtained residue, which was then stirred at 0 °C for 30 minutes. The resulting mixture was filtered thereafter, and washed with ether to obtain the desired product (803 mg, yield: 80%).
¹H-NMR (CD₃ OD):δ 7.69-7.18 (m, 4H), 4.36 (q, 2H, J = 7.1 Hz), 1.38 (t, 3H, J = 7.1 Hz)

Reference Example 121

### 1-(3-methoxyphenyl)hydrazine hydrochloric acid salt

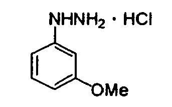

Into an eggplant type flask, 3-iodoanisole (1.17 g), 1-carbobutoxyhydrazine (793 mg), copper (I) iodide (48 mg), 1,10-phenanthroline (180 mg), cesium carbonate (1.96 g), and DMF (5 mL) were added sequentially, and then the atmosphere inside the flask was replaced with argon, after which the resulting mixture was stirred at 80 °C for 21 hours. The resulting reaction solution was filtered and washed with ethyl acetate. The filtrate was evaporated under reduced pressure, and the obtained residue was purified by silica gel column chromatography (ethyl acetate/n-hexane = 0/10 to 3/7). Hydrogen chloride solution in methanol (10 mL) was added to the obtained compound, and the resulting mixture was stirred at room temperature overnight. The reaction solution was evaporated under reduced pressure, and ether (5 mL) was added to the obtained residue, which was then stirred at 0 °C for 30 minutes. The resulting mixture was filtered thereafter, and washed with ether to obtain the desired product (901 mg, yield: 99%).
¹H-NMR (CD₃ OD):δ 7.25-6.52 (m, 4H), 3.78 (s, 3H)

Reference Example 122

### 2-(3-hydrazinylphenoxy)ethanol hydrochloric acid salt

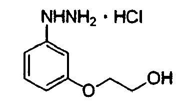

To a solution of 3-iodophenol (1.10 g) in DMF (5 mL), sodium hydroxide (240 mg) and (2-bromoethoxy)-t-butyldimethylsilane (1.1 mL) were added, and the resulting mixture was stirred at 40 °C overnight. Saturated brine was added to the reaction solution, which was then extracted with ethyl acetate, and the organic layers were combined, dried over anhydrous sodium sulfate, and evaporated under reduced pressure. The obtained residue was purified by silica gel column chromatography (ethyl acetate/n-hexane = 0/10 → 2/8) to obtain t-butyl(2-(3-iodophenoxy)ethoxy)dimethylsilane (0.95 g, yield: 50%). This compound was added to an eggplant type flask, and 1-carbobutoxyhydrazine (397 mg), copper (I) iodide (24 mg), 1,10-phenanthroline (90 mg), cesium carbonate (1.14 g) and DMF (2.5 mL) were further added thereto sequentially. Thereafter, the atmosphere inside the flask was replaced with argon, and the mixture was stirred at 80 °C for 21 hours. The resulting reaction solution was filtered and washed with ethyl acetate. The filtrate was evaporated under reduced pressure, and the obtained residue was purified by silica gel column chromatography (ethyl acetate/n-hexane = 0/10 → 3/7). Hydrogen chloride solution in methanol (4.5 mL) was added to the obtained compound, and the resulting mixture was stirred at room temperature overnight. The reaction solution was evaporated under reduced pressure, and ether (2 mL) was added to the obtained residue, which was then stirred at 0 °C for 30 minutes. The resulting mixture was filtered thereafter, and washed with ether to obtain the desired product (378 mg, yield: 99%).
¹H-NMR (CD₃ OD):δ 7.25-6.52 (m, 4H), 4.03 (t, 2H, J = 4.6 Hz), 3.86 (t, 2H, J = 4.6 Hz)

Reference Example 123

### 1-(4-ethylphenyl)hydrazine hydrochloric acid salt

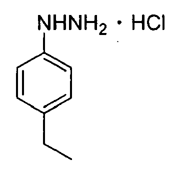

A solution of 1-bromo-4-ethylbenzene (1.85 g) in dry THF (25 mL) was cooled to -78 °C, after which 2.71M n-butyllithium solution in hexane (4.5 mL) was added thereto, and the resulting mixture was stirred at -78°C for 1 hour. A solution of di-t-butyl azodicarboxylate (2.30 g) in dry THF (10 mL) was added to the above mixed solution, which was then stirred at -78 °C for 1 hour and at room temperature for another 2 hours. Distilled water (3 mL) was added to the reaction solution, which was evaporated under reduced pressure thereafter. Saturated brine was added to the obtained residue, which was then extracted with ethyl acetate, and the organic layers were combined, dried over anhydrous sodium sulfate, and evaporated under reduced pressure. The obtained residue was purified by silica gel column chromatography (ethyl acetate/n-hexane = 0/10 → 3/7) to obtain 1-(4-ethylphenyl)-1,2-dicarbobutoxy-hydrazine (1.98 g, yield: 59%). Hydrogen chloride solution in methanol (12 mL) was added to the compound, and the resulting mixture was stirred at room temperature overnight. The reaction solution was evaporated under reduced pressure, and ether (6 mL) was added to the obtained residue, which was then stirred at 0 °C for 30 minutes. The resulting mixture was filtered thereafter, and washed with ether to obtain the desired product (663 mg, yield: 65%).
¹H-NMR (CD₃ OD):δ 7.19-6.89 (m, 4H), 2.59 (q, 2H, J = 7.6 Hz), 1.19 (t, 3H, J = 7.6 Hz)

Reference Example 124

### 1-(3-(methylthio)phenyl)hydrazine hydrochloric acid salt

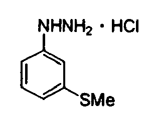

A solution of 3-bromothioanisole (3.05 g) in dry THF (35 mL) solution was cooled, after which 2.71 M n-butyllithium solution in hexane (6.6 mL) was added thereto, and the resulting mixture was stirred at -78 °C for 1 hour. A solution of dit-butyl azodicarboxylate (3.46 g) in dry THF (15 mL) was added to the above mixed solution, which was then stirred at -78 °C for 1 hour and at room temperature for another 3 hours. Distilled water (5 mL) was added to the reaction solution, which was evaporated under reduced pressure thereafter. Saturated brine was added to the obtained residue, which was then extracted with ethyl acetate, and the organic layers were combined, dried over anhydrous sodium sulfate, and evaporated under reduced pressure. The obtained residue was purified by silica gel column chromatography (ethyl acetate/n-hexane = 0/10 → 3/7) to obtain 1-(3-(methylthio)phenyl)-1,2-dicarbobutoxy-hydrazine (3.33 g, yield: 63%). Hydrogen chloride solution in methanol (12 mL) was added to the above 1-(3-(methylthio)phenyl)-1,2-dicarbobutoxy-hydrazine (2.25 g), and the resulting mixture was stirred at room temperature overnight. The reaction solution was evaporated under reduced pressure, and ether (6 mL) was added to the obtained residue, which was then stirred at 0 °C for 30 minutes. The resulting mixture was filtered thereafter, and washed with ether to obtain the desired product (1.69 g, yield: 99%).
¹ H-NMR (CD₃ OD):δ 7.26-6.71 (m, 4H), 2.47 (s, 3H)

Reference Example 125

### 1-(5-fluoro-2-hydroxybenzyl)-3-(3-t-pentyl-1-p-tolyl-1H-pyrazol-5-yl)urea

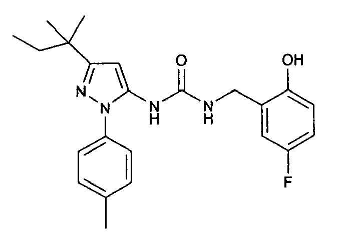

To acetonitrile (10 mL), 2,2,2-trichloroethyl 3-t-pentyl-1-p-tolyl-1H-pyrazol-5-yl carbamate (623 mg) and 2-(aminomethyl)-4-fluorophenol (175 mg) were dissolved. Then diisopropylethylamine (389 µL) was added thereto and the resulting mixture was stirred at 60 °C for 13 hours. The reaction solution was evaporated under reduced pressure, and the obtained residue was purified by silica gel column chromatography (ethyl acetate/n-hexane = 1/3 → 1/1) to obtain the desired product (490 mg, yield: 96%).
¹H-NMR (CDCl₃ ):δ 9.03 (s, 1H), 7.23-6.69 (m, 7H), 6.21 (s, 1H), 6.17 (s, 1H), 5.57 (t, 1H, J = 6.4 Hz), 4.23 (d, 2H, J = 6.4 Hz), 2.33 (s, 3H), 1.65 (q, 2H, J = 7.4 Hz), 1.28 (s, 6H), 0.82 (t, 3H, J = 7.4 Hz)
MS (ESI):411 (M+H⁺)

Example 1

### 1-(2-(2-chloropyrimidin-4-yloxy)benzyl)-3-(3-t-butyl-1-p-tolyl-1 H-pyrazol-5-yl)urea

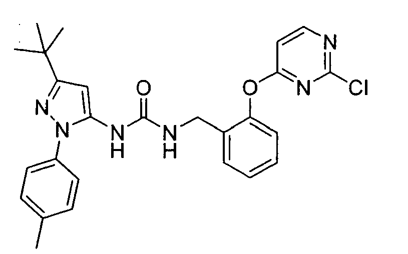

A solution of 2,4-dichloropyrimidine (1.13 g) in acetone (15 mL) was cooled to 0°C, and a solution of 1-(2-hydroxybenzyl)-3-(3-t-butyl-1-p-tolyl-1H-pyrazol-5-yl)urea (2.88 g) and sodium hydroxide (330 mg) in water (15 mL) was added thereto. This reaction mixture was allowed to warm to room temprature, and the mixture was stirred for 24 hours. Saturated ammonium chloride was added to the reaction mixture and the resulting mixture was extracted with ethyl acetate. The extract was dried over anhydrous sodium sulfate, and then evaporated under reduced pressure. The obtained residue was purified by silica gel column chromatography (ethyl acetate/n-hexane = 1/3) to obtain the desired product (2.32 g, yield: 62%).
¹H-NMR (CDCl₃ ):δ 8.40 (d, 1 H, J = 5.6 Hz), 7.35-7.03 (m, 8H), 6.77 (d, 1H, J = 5.6 Hz), 6.20 (s, 1H), 6.07 (s, 1H), 5.26 (t, 1H, J = 5.9 Hz), 4.28 (d, 2H, J = 5.9 Hz), 2.34 (s, 3H), 1.30 (s, 9H)
MS (ESI):491 (M+H⁺)

Example 2

### 1-(2-(2-morpholinopyrimidin-4-yloxy)benzyl)-3-(3-t-butyl-1-p-tolyl- H-pyrazol-5-yl)urea

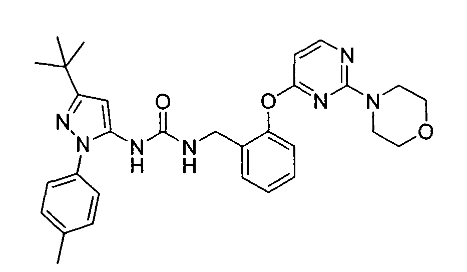

To a solution of 1-(2-(2-chloropyrimidin-4-yloxy)benzyl)-3-(3-t-butyl-1-p-tolyl-1H-pyrazol-5-yl)urea (20 mg) in ethanol (0.1 mL), morpholine (0.005 mL) and sodium carbonate (13 mg) were added, and the resulting mixture was stirred at 60°C for 1 hour. The reaction liquid was filtered, and washed with a small amount of ethanol. The obtained filtrate was evaporated under reduced pressure, and the residue was purified by silica gel column chromatography (ethyl acetate/n-hexane = 2/1) to obtain the desired product (13 mg, yield: 59%).
¹H-NMR (CDCl₃):δ 8.08 (d, 1H, J = 5.6 Hz), 7.25-6.98 (m, 8H), 6.12 (s, 1H), 5.96 (s, 1H), 5.89 (d, 1H, J = 5.6 Hz), 5.19 (t, 1H, J = 5.9 Hz), 4.26 (d, 2H, J = 5.9 Hz), 3.59-3.53 (m, 8H), 2.29 (s, 3H), 1.25 (s, 9H)
MS (ESI):542 (M+H⁺)

Example 3

### 1-(2-(2-chloropyrimidin-4-ylamino)benzyl)-3-(3-t-butyl-1-p-tolyl-1H-pyrazol-5-yl)urea

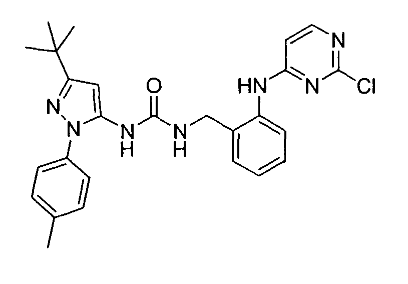

To a solution of 1-(2-aminobenzyl)-3-(3-t-butyl-1-p-tolyl-1H-pyrazol-5-yl)urea (450 mg) in DMF (1 mL), 2,4-dichloropyrimidine (79 mg) and N,N-diisopropylethylamine (0.09 mL) were added, and the resulting mixture was stirred at 60 °C for 4 hours. The reaction mixture was cooled to room temperature, and saturated ammonium chloride was added thereto, after which the mixture was extracted with ethyl acetate. The extract was dried over anhydrous sodium sulfate, and evaporated under reduced pressure. The obtained residue was purified by silica gel column chromatography (ethyl acetate/n-hexane = 2/1) to obtain the desired product (330 mg, yield: 57%).
¹H-NMR (CDCl₃):δ 8.06 (d 1H J = 5.9 Hz) 7.40-7.08 (m 8H) 6.59 (d 1H J = 5.9 Hz) 6.19 (s 1H) 6.07 (s 1H) 5.42 (t 1H J = 6.3 Hz) 4.35 (d 2H J = 6.3 Hz) 2.33 (s 3H) 1.34 (s 9H)
MS (ESI):490 (M+H⁺)

Example 4

### 1-(2-(6-chloropyrimidin-4-yloxy)benzyl)-3-(3-t-butyl-1-p-tolyl-1H-pyrazol-5-yl)urea

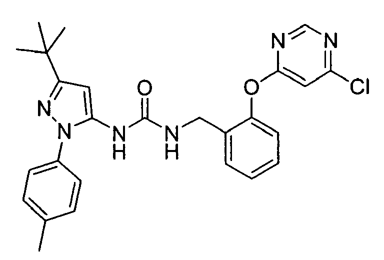

A solution of 4,6-dichloropyrimidine (79 mg) in acetone (1 mL) was cooled to 0°C, and a solution of 1-(2-hydroxybenzyl)-3-(3-t-butyl-1-p-tolyl-1H-pyrazol-5-yl)urea (190 mg) and sodium hydroxide (22 mg) in water (1 mL) was added thereto. This reaction mixture was allowed to warm to room temperature, and then stirred overnight. Saturated ammonium chloride was added to the reaction mixture, which was then extracted with ethyl acetate. The extract was dried over anhydrous sodium sulfate, and then evaporated under reduced pressure. The obtained residue was purified by silica gel column chromatography (ethyl acetate/n-hexane = 1/5) to obtain the desired product (158 mg, yield: 64%).
¹H-NMR (CDCl₃ ):δ 8.43 (s 1H) 7.38-7.03 (m 8H) 6.93 (s 1H) 6.17 (s 1H) 6.05 (s 1H) 5.21 (t 1H J = 5.9 Hz) 4.30 (d 2H J = 5.9 Hz) 2.36 (s 3H) 1.33 (s 9H)
MS (ESI):491 (M+H⁺)

Example 5

### 1-(2-(6-morpholinopyrimidin-4-yloxy)benzyl)-3-(3-t-butyl-1-p-tolyl-1H-pyrazol-5-yl)urea

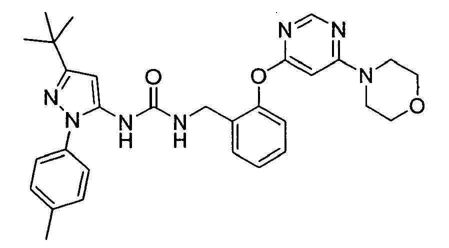

To a solution of 1-(2-(6-chloropyrimidin-4-yloxy)benzyl)-3-(3-t-butyl-1-p-tolyl-1H-pyrazol-5-yl)urea (31 mg) in ethanol (0.1 mL), morpholine (0.01 mL) and sodium carbonate (20 mg) were added, and the resulting mixture was stirred at room temperature for 6 hours. The reaction solution was filtered, and then washed with a small amount of ethanol. The obtained filtrate was evaporated under reduced pressure, and the residue was purified by silica gel column chromatography (ethyl acetate/n-hexane = 2/1) to obtain the desired product (31 mg, yield: 91 %). Colorless crystals, mp.128.5 °C
¹H-NMR (CDCl₃ ):δ 8.05 (s 1H) 7.32-6.99 (m 8H) 6.20 (s 2H) 5.89 (s 1H) 5.50 (t 1H J = 5.6 Hz) 4.29 (d 2H J = 5.6 Hz) 3.75-3.54 (m 8H) 2.35 (s 3H) 1.32 (s 9H)
MS (ESI):542 (M+H⁺)
Elementary analysis:
Calcd. (C₃₀H₃₅N₇O₃+1.5H₂O)
C:63.36, H:6.74, N:17.24
Found: C:63.49, H:6.39, N:16.94

Example 6

### 1-(2-(2-(3-(2-oxo-pyrrolidin-1-yl)propylamino)pyrimidin-4-yloxy)benzyl)-3-(3-t-butyl-1-p-tolyl-1H-pyrazol-5-yl)urea

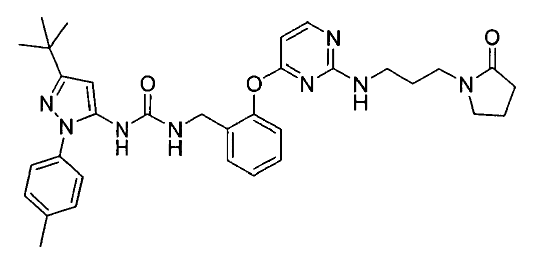

To a solution of 1-(2-(2-chloropyrimidin-4-yloxy)benzyl)-3-(3-t-butyl-1-p-tolyl-1H-pyrazol-5-yl)urea (187 mg) in ethanol (0.6 mL), N-(3-aminopropyl)-2-pyrrolidinone (0.08 mL) and sodium carbonate (81 mg) were added, and the resulting mixture was stirred at 60 °C for 3 hours. The reaction solution was filtered, and washed with a small amount of ethanol. The obtained filtrate was evaporated under reduced pressure, and the residue was purified by silica gel column chromatography (ethyl acetate/n-hexane=2/1) to obtain the desired product (118 mg, yield: 52%).
¹H-NMR (CDCl₃):δ 8.06 (d 1H J = 5.6 Hz) 7.83 (s 1H) 7.52 (br 1H) 7.35-6.98 (m 8H) 6.39 (s 1H)6.14(d 1HJ=5.6Hz)5.33 (t 1HJ=5.9Hz)4.37(d2HJ=5.9Hz) 3.44-3.03 (m 10H) 2.33 (s 3H) 1.87-1.83 (m 2H) 1.33 (s 9H)
MS (ESI):597 (M+H⁺)

Example 7

### 1-(2-(2-(4-methylpiperazin-1-yl)pyrimidin-4-yloxy)benzyl)-3-(3-t-butyl-1-p-tolyl-1H-pyrazol-5-yl)urea

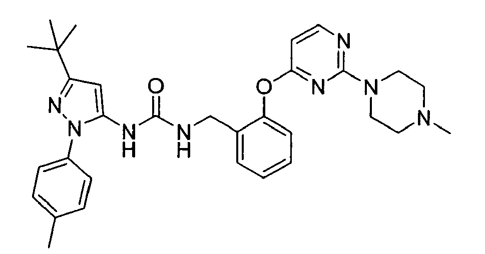

To a solution of 1-(2-(2-chloropyrimidin-4-yloxy)benzyl)-3-(3-t-butyl-1-p-tolyl-1H-pyrazol-5-yl)urea (187 mg) in ethanol (0.6 mL), 1-methylpiperazine (0.065 mL) and sodium carbonate (81 mg) were added, and the resulting mixture was stirred at 60 °C for 1 hour. The reaction solution was filtered, and the washed with a small amount of ethanol. The obtained filtrate was evaporated under reduced pressure, and the residue was purified by silica gel column chromatography (ethyl acetate/n-hexane=2/1) to obtain the desired product (122 mg, yield: 58%). Colorless crystals, mp.149.5 °C
¹H-NMR (CD13):8 8.13 (d 1 H J = 5.6 Hz) 7.33-7.06 (m 8H) 6.20 (s 1 H) 6.01 (s 1H) 5.90 (d 1H J = 5.6 Hz) 5.28 (t 1H J = 6.1 Hz) 4.33 (d 2H J = 6.1 Hz) 3.66 (br 4H) 2.35-2.38 (m 7H) 2.29 (s 3H) 1.32 (s 9H)
MS (ESI):555 (M+H⁺)
Elementary analysis : Calcd.: (C₃₁H₃₈N₈O₂)
C:67.12, H:6.91, N:20.20
Found: C:66.98, H:6.88, N:20.10

Example 8

### 1-(2-(2-chloropyrimidin-4-yloxy)-5-fluorobenzyl)-3-(3-t-butyl-1-p-tolyl-1 H-pyrazol-5-yl)urea

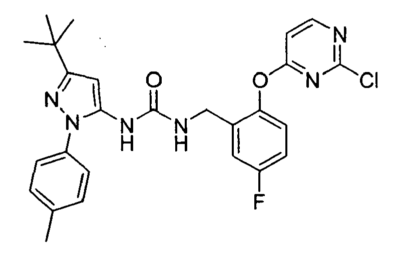

A solution of 2,4-dichloropyrimidine (186 mg) in DMF (2.5 mL) was cooled to 0 °C, and 1-(5-fluoro2-hydroxybenzyl)-3-(3-t-butyl-1-p-tolyl-1H-pyrazol-5-yl)urea (495 mg) and aqueous 1N sodium hydroxide solution (1.25 mL) were added thereto. This reaction mixture was allowed to warm to room temperature and stirred for 3 hours. Saturated ammonium chloride was added to the reaction mixture, which was then extracted with ethyl acetate. The extract was dried over anhydrous sodium sulfate, and then evaporated under reduced pressure. The obtained residue was purified by silica gel column chromatography (ethyl acetate/n-hexane=1/3) to obtain the desired product (362 mg, yield: 57%).
¹H-NMR (CDCl₃):δ 8.44 (d 1H J = 5.6 Hz) 7.32-6.96 (m 7H) 6.82 (d 1H J = 5.6 Hz) 6.22 (s 1H) 6.13 (s 1H) 5.25 (t 1H J = 5.9 Hz) 4.26 (d 2H J = 5.9 Hz) 2.36 (s 3H) 1.33 (s 9H)
MS (ESI):509 (M+H⁺)

Example 9

### 1-(2-(2-morpholinopyrimidin-4-yloxy)-5-fluorobenzyl)-3-(3-t-butyl-1-p-tolyl-1H-pyrazol-5-yl)urea

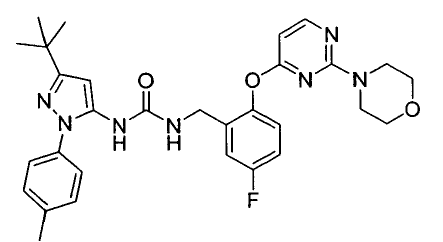

To a solution of 1-(2-(2-chloropyrimidin-4-yloxy)-5-fluorobenzyl)-3-(3-t-butyl-1-p-tolyl-1H-pyrazol-5-yl)urea (41 mg) in ethanol (0.1 mL), morpholine (0.01 mL) and sodium carbonate (25 mg) were added, and the resulting mixture was stirred at 60 °C for 1 hour. The reaction solution was filtered, and washed with a small amount of ethanol. The obtained filtrate was evaporated under reduced pressure, and the residue was purified by silica gel column chromatography (ethyl acetate/n-hexane=2/1) to obtain the desired product (41 mg, yield: 91%).
¹H-NMR (CDCl₃):δ 8.16 (d 1H J = 5.6 Hz) 7.32-6.96 (m 7H) 6.19 (s 1H) 6.00 (d 1H J = 5.6 Hz) 5.99 (s 1H) 5.18 (t 1H J = 5.9 Hz) 4.29 (d 2H J = 5.9 Hz) 3.66-3.58 (m 8H) 2.3 (s 3H) 1.33 (s 9H)
MS (ESI):560 (M+H⁺)

### Example 10

### 1-(2-(2-(4-acetylpiperazin-1-yl)pyrimidin-4-yloxy)benzyl)-3-(3-t-butyl-1-p-tolyl-1 H-pyrazol-5-yl)urea

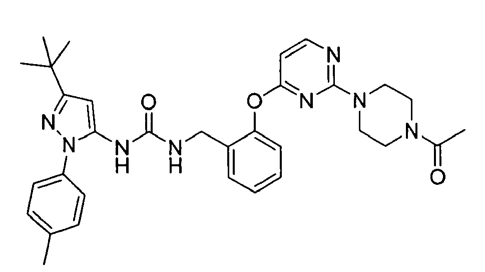

To a solution of 1-(2-(2-chloropyrimidin-4-yloxy)benzyl)-3-(3-t-butyl-1-p-tolyl-1H-pyrazol-5-yl)urea (150 mg) in ethanol (0.5 mL), 1-acetylpiperazine (58 mg) and sodium carbonate (63 mg) were added, and the resulting mixture was stirred at 60 °C for 1 hour. The reaction solution was filtered, and washed with a small amount of ethanol. The obtained filtrate was evaporated under reduced pressure, and the residue was purified by silica gel column chromatography (ethyl acetate/n-hexane = 2/1) to obtain the desired product (95 mg, yield: 53%).
¹H-NMR (CDCl₃):δ 8.16 (d 1H J = 5.6 Hz) 7.33-7.06 (m 8H) 6.20 (s 1H) 6.03 (s 1H) 5.99 (d 1 H J = 5.6 Hz) 5.27 (t 1 H J = 5.9 Hz) 4.34 (d 2H J = 5.9 Hz) 3.66-3.40 (m 8H) 2.38 (s 3H) 2.09 (s 3H) 1.32 (s 9H)
MS (ESI):583 (M+H⁺)

Example 11

### 1-(2-(2-(3-(dimethylamino)propylamino)pyrimidin-4-yloxy)benzyl)-3-(3-t-butyl-1-p-tolyl-1 H-pyrazol-5-yl)urea

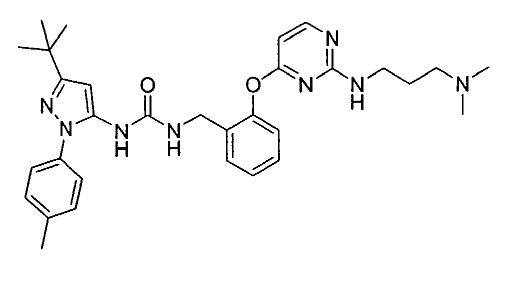

To a solution of 1-(2-(2-chloropyrimidin-4-yloxy)benzyl)-3-(3-t-butyl-1-p-tolyl-1H-pyrazol-5-yl)urea (150 mg) in ethanol (0.5 mL), N,N-dimethyl-1,3-propanediamine (0.06 mL) and sodium carbonate (63 mg) were added, and the resulting mixture was stirred at 60 °C for 3 hours. The reaction solution was filtered, and washed with a small amount of ethanol. The obtained filtrate was evaporated under reduced pressure, and the residue was purified by silica gel column chromatography (ethyl acetate/n-hexane = 2/1) to obtain the desired product (60 mg, yield: 35%).
¹H-NMR (CDCl₃):δ 8.09 (d 1H J = 5.6 Hz) 7.36-7.04 (m 8H) 6.36 (br 1H) 6.23 (s 1H) 5.98 (d 1H J = 5.6 Hz) 5.55 (br 1H) 4.34 (d 2H J = 5.6 Hz) 3.27-3.19 (m 2H) 2.37 (s 3H) 2.24-2.20 (m 2H) 2.12 (br 6H) 1.60 (br 2H) 1.32 (s 9H)
MS (ESI):557 (M+H⁺)

Example 12

### 1-(2-(2-(4-(2-methoxyethyl)piperazin-1-yl)pyrimidin-4-yloxy)benzyl)-3-(3-t-butyl-1-p-tolyl-1H-pyrazol-5-yl)urea

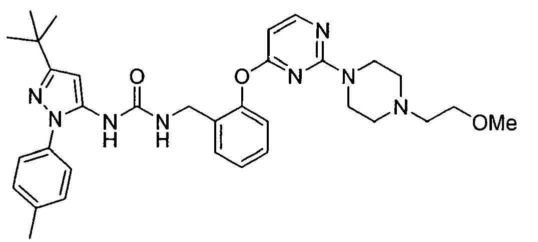

To a solution of 1-(2-(2-chloropyrimidin-4-yloxy)benzyl)-3-(3-t-butyl-1-p-tolyl-1H-pyrazol-5-yl)urea (227 mg) in ethanol (1.0 mL), 1-(2-methoxyethyl)piperazine (80 mg) and N,N-diisopropylethylamine (0.5 mL) were added, and the resulting mixture was stirred at room temperature for 4 days. Saturated aqueous sodium bicarbonate solution was added to the reaction mixture, which was then extracted with ethyl acetate. The extract was washed with water and saturated brine, dried over anhydrous sodium sulfate, and then evaporated under reduced pressure. The obtained residue was purified by amine-silica gel column chromatography (ethyl acetate/n-hexane = 1/1 - 2/1) to obtain the desired product (212 mg, yield: 76.5%).
¹H-NMR (CDCl₃):δ 8.13 (d 1H J = 5.6 Hz) 7.34-7.06 (m 8H) 6.20 (s 1H) 5.99 (s 1H) 5.90 (d 1H J = 5.6 Hz) 5.28 (t 1 H J = 5.9 Hz) 4.34 (d 2H J = 5.9 Hz) 3.68 (br 4H) 3.51 (t 2H J = 5.6 Hz) 3.35 (s 3H) 2.57 (t 2H J = 5.6 Hz) 2.46-2.44 (m 4H) 2.37 (s 3H) 1.32 (s 9H)
MS (ESI):599 (M+H⁺)

Example 13

### 1-(4-(2-(((((3-t-butyl-1-p-tolyl-1H-pyrazol-5-yl)amino)carbonyl)amino)methyl)phenoxy)pyrimidin-2-yl)-piperidin-3-carboxylic amide

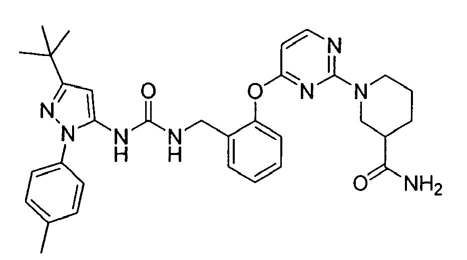

To a solution of 1-(2-(2-chloropyrimidin-4-yloxy)benzyl)-3-(3-t-butyl-1-p-tolyl-1H-pyrazol-5-yl)urea (150 mg) in ethanol (0.5 mL), 3-piperidinecarboxamide (59 mg) and sodium carbonate (63 mg) were added, and the resulting mixture was stirred at 60 °C for 3 hours. The reaction solution was filtered, and then washed with a small amount of ethanol. The obtained filtrate was evaporated under reduced pressure, and the residue was purified by silica gel column chromatography (ethyl acetate/n-hexane = 2/1) to obtain the desired product (50 mg, yield: 28%).
¹H-NMR (CD₃ OD):δ 8.14 (d 1H J = 5.6 Hz) 7.33-7.06 (m 8H) 6.26 (s 1H) 6.14 (d 1H J = 5.6 Hz) 4.32-4.27 (m 2H) 4.24 (s 2H) 2.92-2.81 (m 2H) 2.41 (s 3H) 2.29-2.22 (m 1H) 1.88-1.31 (m 4H) 1.30 (s 9H)
MS (ESI):583 (M+H⁺)

Example 14

### 1-(4-(2-(((((3 -t-butyl-1-p-tolyl-1 H-pyrazol-5-yl)amino)carbonyl)amino)methyl)phenoxy)pyrimidin-2-yl)-piperidin-4-carboxylic amide

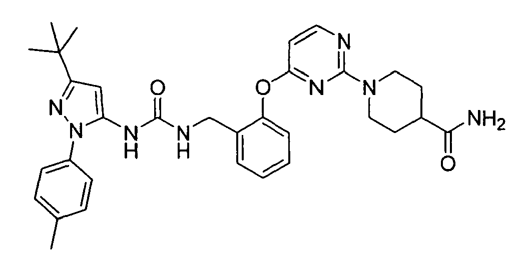

To a solution of 1-(2-(2-chloropyrimidin-4-yloxy)benzyl)-3-(3-t-butyl-1-p-tolyl-1H-pyrazol-5-yl)urea (150 mg) in ethanol (0.5 mL), 4-piperidinecarboxamide (59 mg) and sodium carbonate (63 mg) were added, and the resulting mixture was stirred at 60 °C for 3 hours. The reaction solution was filtered, and washed with a small amount of ethanol. The obtained filtrate was evaporated under reduced pressure, and the residue was purified by silica gel column chromatography (ethyl acetate/n-hexane = 2/1) to obtain the desired product (70 mg, yield: 39%).
¹H-NMR (CD₃ OD):δ 8.13 (d 1H J = 5.6 Hz) 7.34-7.07 (m 8H) 6.26 (s 1H) 6.12 (d 1 H J = 5.6 Hz) 4.46-4.43 (m 2H) 4.24 (s 2H) 2.80-2.73 (m 2H) 2.46-2.40 (m 4H) 1.73-1.68 (m 2H) 1.54-1.44 (m 2H) 1.30 (s 9H)
MS (ESI):583 (M+H⁺)

Example 15

### 1-((3-(2-morpholinopyrimidin-4-yloxy)thiophen-2-yl)methyl)-3-(3-t-butyl-1-p-tolyl-1H-pyrazol-5-yl)urea

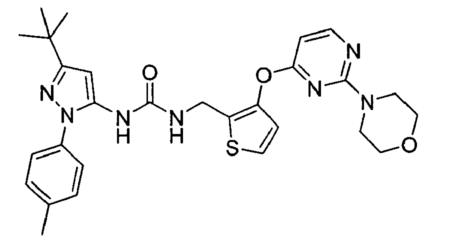

To a solution of 3-(2-morpholinylpyrimidin-4-yloxy)thiophen-2-carboxylic amide (80 mg) in THF (1.3 mL), borane solution in THF (1.0M, 0.78 mL) was added, and the resulting mixture was stirred at 0 °C for 30 minutes. Water (1.5 mL) and diethanol amine (0.075 mL) were added to the reaction mixture, which was then extracted with dichloromethane. The extract was dried over anhydrous sodium sulfate, and evaporated under reduced pressure to obtain (3-(2-morpholino pyrimidin-4-yloxy)thiophen-2-yl)methanamine (47 mg).

This compound was dissolved in DMSO (0.6 mL), and 2,2,2-trichloroethyl 3-t-butyl-1-p-tolyl-1H-pyrazol-5-yl carbamate (60 mg) and diisopropylethylamine (0.04 mL) were added thereto, after which the resulting mixture was stirred at room temperature overnight. Saturated ammonium chloride was added to the reaction mixture, which was then extracted with ethyl acetate. The extract was dried over anhydrous sodium sulfate, and then evaporated under reduced pressure. The obtained residue was purified by silica gel column chromatography (ethyl acetate/n-hexane = 2/1) to obtain the desired product (15 mg, yield: 18%).
¹H-NMR (CDCl₃):δ 8.16 (d 1H J = 5.4 Hz) 7.30-7.20 (m 4H) 7.18 (d 1H J = 5.4 Hz) 6.83 (d 1H J = 5.4 Hz) 6.20 (s 1H) 6.04 (br 1H) 6.03 (d 1H J = 5.4 Hz) 5.26 (t 1H J = 5.6 Hz) 4.45 (d 2H J = 5.6 Hz) 3.69-3.65 (m 8H) 2.37 (s 3H) 1.32 (s 9H)
MS (ESI):548 (M+H⁺)

Example 16

### 1-(2-(6-chloropyridazin-3-yloxy)benzyl)-3-(3-t-butyl-1-p-tolyl-1H-pyrazol-5-yl)urea

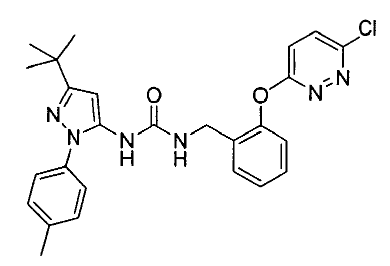

To a mixed solution of 1-(2-hydroxybenzyl)-3-(3-t-butyl-1-p-tolyl-1H-pyrazol-5-yl)urea (112 mg) and 3,6-dichloropyridazine (364 mg) in dimethylformamide (0.5 mL), anhydrous potassium carbonate (210 mg) was added, and the resulting mixture was stirred at room temperature for 4 hours and then at 60 °C for 15 hours. Aqueous 5% citric acid solution was added to the reaction mixture, which was then extracted with ethyl acetate. The extract was dried over anhydrous sodium sulfate, and then evaporated under reduced pressure. The obtained residue was purified by silica gel column chromatography (ethyl acetate/n-hexane = 1/1) to obtain the desired product (98 mg, yield: 68%).
¹H-NMR (CDCl₃):δ 7.46 (d 1H J = 9.0 Hz) 7.36-7.14 (m 8H) 7.07 (d 1H J = 8.1 Hz) 6.25 (s 1H) 6.21 (s 1H) 5.30 (t 1H J = 5.6 Hz) 4.35 (d 2H J = 5.6 Hz) 2.35 (s 3H) 1.33 (s 9H)
MS (ESI):491 493 (M+H⁺)

Example 17

### 1-(2-(2-(methylthio)pyrimidin-4-yloxy)benzyl)-3-(3-t-butyl-1-p-tolyl-1H-pyrazol-5-yl)urea

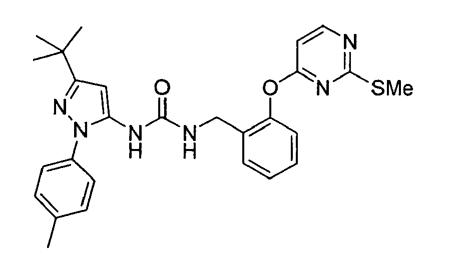

To a mixed solution of 1-(2-hydroxybenzyl)-3-(3-t-butyl-1-p-tolyl-1H-pyrazol-5-yl)urea (23 mg) and 4-chloro-2-methylsulfanylpyrimidine (30 mg) in dimethylformamide (0.2 mL), anhydrous potassium carbonate (15 mg) was added, and the resulting mixture was stirred at room temperature for 22 hours. Aqueous 5% citric acid solution was added to the reaction mixture, which was then extracted with ethyl acetate. The extract was dried over anhydrous sodium sulfate, and then evaporated under reduced pressure. The obtained residue was purified by silica gel column chromatography (ethyl acetate/n-hexane = 3/1) to obtain the desired product (30 mg, yield: 98%).
¹H-NMR (CDCl₃):δ 8.35 (d 1H J = 5.6 Hz) 7.39-7.02 (m 8H) 6.51 (d 1H J = 5.6 Hz) 6.26 (s 1H) 6.09 (s 1H) 5.29 (t 1H J = 5.6 Hz) 4.32 (d 2H J = 5.6 Hz) 2.37 (s 3H) 2.27(s 3H) 1.32 (s 9H)
MS (ESI):503 (M+H⁺)

Example 18

### 1-(2-(2-(3-(diethylamino)propylamino)pyrimidin-4-yloxy)benzyl)-3-(3-t-butyl-1-p-tolyl-1 H-pyrazol-5-yl)urea

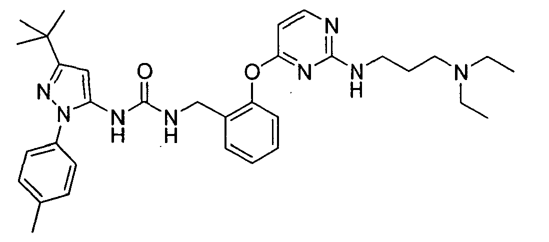

To a solution of 1-(2-(2-chloropyrimidin-4-yloxy)benzyl)-3-(3-t-butyl-1-p-tolyl-1H-pyrazol-5-yl)urea (294 mg) in ethanol (2.0 mL), N,N-diethyl-1,3-propanediamine (109 mg) and sodium carbonate (190 mg) were added, and the resulting mixture was stirred at 60 °C for 11 hours. The reaction mixture was evaporated under reduced pressure, and then the obtained residue was purified by amine-silica gel column chromatography (ethyl acetate/n-hexane = 1/1 - 1 /0) to obtain the desired product (80 mg, yield: 23%).
¹H-NMR (CDCl₃ ):δ8.08 (d 1H J = 5.6 Hz) 7.33-7.04 (m 8H) 6.22 (s 1H) 5.98 (br 1 H) 5.94 (d 1H J = 5.6 Hz) 5.49 (br 1 H) 4.32 (d 2H J = 5.9 Hz) 3.26 (br 2H) 2.46-2.38 (m 6H) 2.36 (s 3H) 1.60 (br 2H) 1.32 (s 9H) 0.95 (t 6H J = 7.1 Hz)
MS (ESI):585 (M+H⁺)
Elementary analysis : Calcd.: (C₃₃H₄₄N₈O₂+0.6H₂O)
C:66.55, H:7.65, N:18.81
Found: C:66.94, H:7.65, N:18.44

Example 19

### 1-(5-chloro-2-((2-chloropyrimidin-4-yl)methylamino)benzyl)-3-(3-t-butyl-1-p-tolyl-1H-pyrazol-5-yl)urea

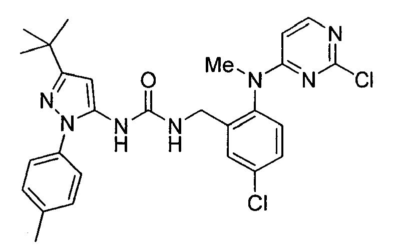

By following the same procedure as described in Example 1, the desired product (6.1 mg, yield: 11%) was obtained from 1-(5-chloro-2-(methylamino)benzyl)-3-(3-t-butyl-1-p-tolyl-1H-pyrazol-5-yl)urea (45 mg) and 2,4-dichloropyrimidine (24 mg).
¹ H-NMR (CD₃ OD):δ 7.94 (d 1H J = 6.4Hz) 7.44-7.22 (m 7H) 6.57 (d 1H J = 6.4 Hz) 6.24 (s 1H) 4.14 (s 2H) 3.82 (s 3H) 2.40 (s 3H) 1.31 (s 9H)
MS (ESI):536, 538 (M-H)

Example 20

### 1-(2-(2-(2-(1-methylpyrrolidin-2-yl)ethylamino)pyrimidin-4-yloxy)benzyl)-3-(3-t-butyl-1-p-tolyl-1H-pyrazol-5-yl)urea

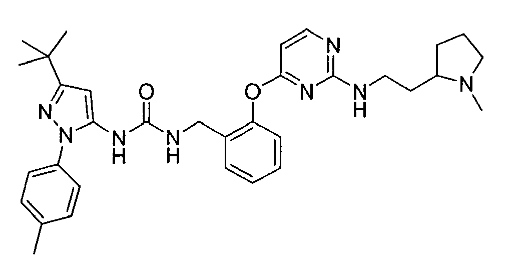

To a solution of 1-(2-(2-chloropyrimidin-4-yloxy)benzyl)-3-(3-t-butyl-1-p-tolyl-1 H-pyrazol-5-yl)urea (143 mg) in ethanol (1.0 mL), 2-(2-aminoethyl)-1-methylpyrrolidine (52 mg) and sodium carbonate (93 mg) were added, and the resulting mixture was stirred at 60 °C for 19 hours. The reaction mixture was evaporated under reduced pressure, and distilled water (3 mL) was added thereto, after which the mixture was extracted with dichloromethane (5 mL×3). The obtained organic layer was dried over anhydrous sodium sulfate, and then evaporated under reduced pressure. The obtained residue was purified by amine-silica gel column chromatography (ethyl acetate/n-hexane = 97/3 - 100/0, ethyl acetate/methanol = 40/1) to obtain the desired product (18 mg, yield: 11%).
¹H-NMR (CDCl₃ ):δ 8.09 (d 1H J = 5.7Hz) 7.38-7.04 (m 8H) 6.23 (s 1H) 6.18 (s 1H) 6.01 (d 1H J = 5.7 Hz) 5.51 (br 1H) 4.36-4.29 (m 3H) 3.25-3.19 (m 2H) 2.95-2.89 (m 2H) 2.37 (s 6H) 2.20 (br 5H) 2.14-2.02 (m 2H) 1.33 (s 9H)
MS (ESI):583 (M+H⁺)

Example 21

### 1-(2-(2-chloropyrimidin-4-yloxy)benzyl)-3-(3-t-pentyl-1-p-tolyl-1 H-pyrazol-5-yl)urea

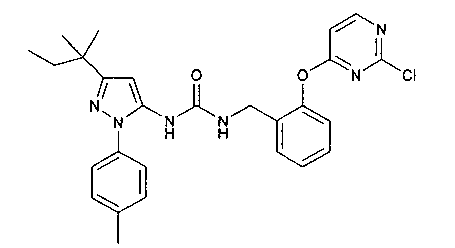

To acetone (14.1 mL), 1-(2-hydroxybenzyl)-3-(3-t-pentyl-1-p-tolyl-1H-pyrazol-5-yl)urea (427 mg) and 2,4-dichloropyrimidine (211 mg) were dissolved. Aqueous 0.1 M sodium hydroxide solution (14.1 mL) was then added thereto, and the resulting mixture was stirred at room temperature for 17 hours. The reaction solution was evaporated under reduced pressure, and the aqueous layer was extracted with dichloromethane (10 mL) three times. The organic layers were combined, dried over anhydrous sodium sulfate, and evaporated under reduced pressure. The obtained residue was purified by silica gel column chromatography (ethyl acetate/n-hexane = 3/7 → 1/1), and washed with hexane to obtain the desired product (472 mg, yield: 86%).
¹H-NMR (CDCl₃ ):8 8.42 (d 1H J = 5.6 Hz) 7.38-7.06 (m 8H) 6.80 (d 1H J = 5.6 Hz) 6.20 (s 1H) 6.04 (s 1H) 5.23 (t 1H J = 5.5 Hz) 4.31 (d 2H J = 5.5 Hz) 2.37 (s 3H) 1.64 (q 2H J = 7.5 Hz) 1.28 (s 6H) 0.80 (t 3H J = 7.5 Hz)
MS (ESI):505 (M+H⁺)

Example 22

### 1-(2-(2-morpholinopyrimidin-4-yloxy)benzyl)-3-(3-t-pentyl-1-p-tolyl-1H-pyrazol-5-yl)urea

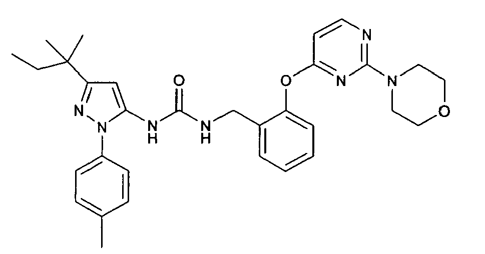

In ethanol (10 mL), 1-(2-(2-chloropyrimidin-4-yloxy)benzyl)-3-(3-t-pentyl-1-p-tolyl-1H-pyrazol-5-yl)urea (445 mg) was dissolved, and morpholine (200 µL) was added thereto, followed by stirring at room temperature for 19 hours. The reaction solution was evaporated under reduced pressure, and the obtained residue was purified by silica gel column chromatography (ethyl acetate/n-hexane=7/13→3/2) to obtain the desired product (445 mg, yield: 91 %).
¹H-NMR (CDCl₃):δ 8.15 (d 1H J = 5.6 Hz) 7.32-7.05 (m 8H) 6.17 (s 1H) 6.02 (s 1H) 5.96 (d 1H J = 5.6 Hz) 5.24 (t 1H J = 6.0 Hz) 4.32 (d 2H J = 6.0 Hz) 3.66-3.60 (m 8H) 2.37 (s 3H) 1.63 (q 2H J = 7.5 Hz) 1.28 (s 6H) 0.80 (t 3H J = 7.5 Hz)

Example 23

### 1-(2-(2-chloropyrimidin-4-yloxy)benzyl)-3-(3-cyclopentyl-1-p-tolyl-1H-pyrazol-5-yl)urea

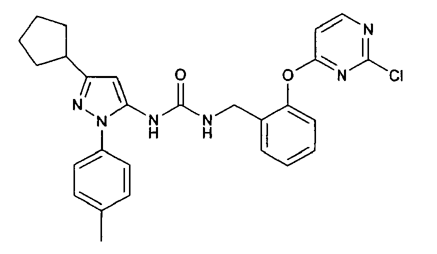

In acetone (2.58 mL), 1-(2-hydroxybenzyl)-3-(3-cyclopentyl-1-p-tolyl-1H-pyrazol-5-yl)urea (77.5 mg) and 2,4-dichloropyrimidine (38.4 mg) were dissolved, and then aqueous 0.1M sodium hydroxide solution (2.58 mL) was added thereto, followed by stirring at room temperature for 16 hours. The reaction mixture was evaporated under reduced pressure, and the obtained residue was purified by silica gel column chromatography (ethyl acetate/n-hexane = 1 /2 → 1/1) to obtain the desired product (67.7 mg, yield: 68%).
¹H-NMR (CDCl₃):δ 8.43 (d 1H J = 5.6 Hz) 7.37-7.06 (m 8H) 6.80 (d 1H J = 5.6 Hz) 6.17 (s 1H) 6.10 (s 1H) 5.29 (t 1H J = 6.0 Hz) 4.31 (d 2H J = 6.0 Hz) 3.11-3.03 (m 1H) 2.37 (s 3H) 2.07-1.65 (m 8H)
MS (ESI):503 (M+H⁺)

Example 24

### 1-(2-(2-morpholinopyrimidin-4-yloxy)benzyl)-3-(3-cyclopentyl-1-p-tolyl-1 H-pyrazol-5-yl)urea

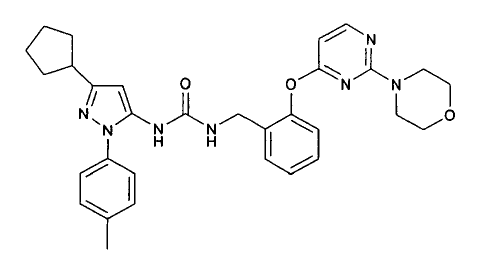

With ethanol (0.8 mL), 1-(2-(2-chloropyrimidin-4-yloxy)benzyl)-3-(3-cyclopentyl-1-p-tolyl-1H-pyrazol-5-yl)urea (52.9 mg) and sodium carbonate (33.4 mg) were mixed, and then morpholine (12.9 µL) was added thereto, followed by stirring at 40 °C for 36 hours. The reaction solution was filtered, and the filtrate was evaporated under reduced pressure. The obtained residue was then purified by silica gel column chromatography (ethyl acetate/n-hexane=2/3 → 2/1) to obtain the desired product (54.2 mg, yield: 93%).
¹H-NMR (CDCl₃):δ 8.14 (d 1H J = 5.6 Hz) 7.32-7.05 (m 8H) 6.22 (s 1H) 6.15 (s 1H) 5.95 (d 1H J = 5.6 Hz) 5.36 (t 1H J = 5.8 Hz) 4.31 (d 2H J = 5.8 Hz) 3.66-3.59 (m 8H) 3.10-3.02 (m 1H) 2.35 (s 3H) 2.09-1.66 (m 8H)
MS (ESI):554 (M+H⁺)

Example 25

### 1-(2-(2-chloropyrimidin-4-yloxy)benzyl)-3-(3-cyclopropyl-1-p-tolyl-1H-pyrazol-5-yl)urea

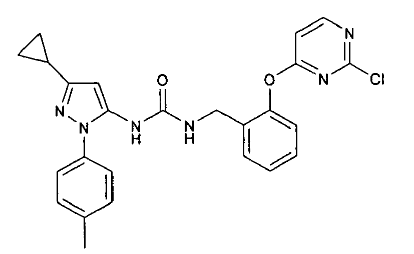

In acetone (2.52 mL), 1-(2-hydroxybenzyl)-3-(3-cyclopropyl-1-p-tolyl-1H-pyrazol-5-yl)urea (70.2 mg) and 2,4-dichloropyrimidine (37.5 mg) were dissolved, and then aqueous 0.1 M sodium hydroxide solution (2.52 mL) was added thereto, followed by stirring at room temperature for 63.5 hours. The reaction solution was evaporated under reduced pressure, and the obtained residue was purified by silica gel column chromatography (ethyl acetate/n-hexane=1/1 → 7/3), and washed with hexane to obtain the desired product (81.5 mg, yield: 89%).
¹H-NMR (CDCl₃ ):δ 8.42 (d 1H J = 5.9 Hz) 7.37-7.05 (m 8H) 6.79 (d 1 H J = 5.9 Hz) 6.21 (s 1H) 6.00 (s 1H) 5.37 (t 1H J = 5.8 Hz) 4.28 (d 2H J = 5.8 Hz) 2.35 (s 3H) 1.95-1.88 (m 1H) 0.95-0.75 (m 4H)
MS (ESI):475 (M+H⁺)

Example 26

### 1-(2-(2-morpholinopyrimidin-4-yloxy)benzyl)-3-(3-cyclopropyl-1-p-tolyl-1H-pyrazol-5-yl)urea

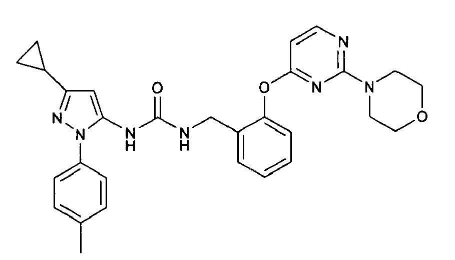

In ethanol (0.8 mL), 1-(2-(2-chloropyrimidin-4-yloxy)benzyl)-3-(3-cyclopropyl-1-p-tolyl-1H-pyrazol-5-yl)urea (49.0 mg) was dissolved, and morpholine (23.5 µL) was added thereto, followed by stirring at room temperature for 85 hours. The reaction solution was evaporated under reduced pressure, and the obtained residue was purified by silica gel column chromatography (ethyl acetate/n-hexane=1/1 → 4/1) to obtain the desired product (44.6 mg, yield: 82%).
¹H-NMR (CDCl₃):δ 8.16 (d 1H J = 5.5 Hz) 7.32-7.06 (m 8H) 6.05 (s 1H) 5.98 (d 1H J = 5.5 Hz) 5.96 (s 1H) 5.27 (t 1H J = 5.9 Hz) 4.32 (d 2H J = 5.9 Hz) 3.65-3.60 (m 8H) 2.37 (s 3H) 1.96-1.89 (m 1H) 0.96-0.75 (m 4H)

Example 27

### 1-(2-(2-chloropyrimidin-4-yloxy)benzyl)-3-(3-neopentyl-1-p-tolyl-1H-pyrazol-5-yl)urea

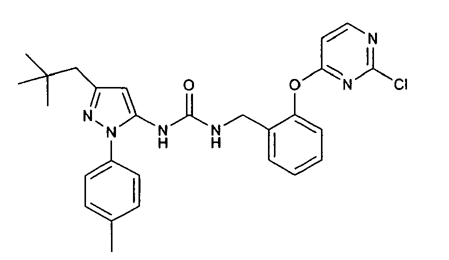

In acetone (2.78 mL), -(2-hydroxybenzyl)-3-(3-neopentyl-1-p-tolyl-1H-pyrazol-5-yl)urea (83.8 mg) and 2,4-dichloropyrimidine (41.4 mg) were dissolved, and then aqueous 0.1 M sodium hydroxide solution (2.78 mL) was added thereto, followed by stirring at room temperature for 16 hours. The reaction solution was evaporated under reduced pressure, and the obtained residue was purified by silica gel column chromatography (ethyl acetate/n-hexane=1/2 → 1/1) to obtain the desired product (82.3 mg, yield: 76%).
¹H-NMR (CDCl₃):δ 8.42 (d 1 H J = 5.7 Hz) 7.38-7.05 (m 8H) 6.80 (d 1H J = 5.7 Hz) 6.15 (s 2H) 5.31 (t 1H J = 5.7 Hz) 4.30 (d 2H J = 5.7 Hz) 2.50 (s 2H) 2.36 (s 3H) 0.97 (s 9H)
MS (ESI):505 (M+H⁺)

Example 28

### 1-(2-(2-morpholinopyrimidin-4-yloxy)benzyl)-3-(3-neopentyl-1-p-tolyl-1 H-pyrazol-5-yl)urea

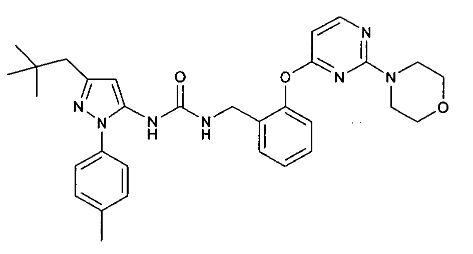

With ethanol (0.8 mL), 1-(2-(2-chloropyrimidin-4-yloxy)benzyl)-3-(3-neopentyl-1-p-tolyl-1H-pyrazol-5-yl)urea (60.4 mg) and sodium carbonate (38.0 mg) were mixed. Morpholine (14.6 µL) was added to the resulting mixture, which was then stirred at 40°C for 36 hours. The reaction solution was filtered, and the filtrate was evaporated under reduced pressure. The obtained residue was purified by silica gel column chromatography (ethyl acetate/n-hexane = 2/3 → 2/1) to obtain the desired product (61.2 mg, yield: 92%)
¹ H-NMR (CDCl₃):δ 8.15 (d, 1H, J = 5.6 Hz), 7.32-7.05 (m, 8H), 6.11 (s, 2H), 5.97 (d, 1H, J = 5.6 Hz), 5.28 (t, 1H, J = 5.9 Hz), 4.32 (d, 2H, J = 5.9 Hz), 3.66-3.59 (m, 8H), 2.50 (s, 2H), 2.37 (s, 3H), 0.97 (s, 9H)
MS (ESI):556 (M+H⁺)

Example 29

### 1-(2-(2-chloropyrimidin-4-yloxy)benzyl)-3-(3-(2-methylfuran-3-yl)-1-p-tolyl-1 H-pyrazol-5-yl)urea

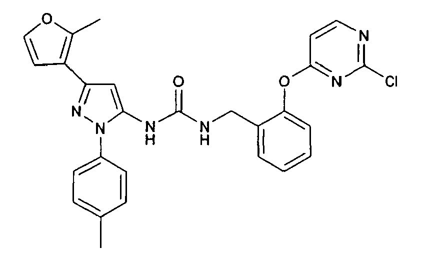

In acetone (2.79 mL), 1-(2-hydroxybenzyl)-3-(3-(2-methylfuran-3-yl)-1-p-tolyl-1H-pyrazol-5-yl)urea (86.5 mg) and 2,4-dichloropyrimidine (41.6 mg) were dissolved, and aqueous 0.1 M sodium hydroxide solution (2.79 mL) was added thereto, followed by stirring at room temperature for 16 hours. The reaction solution was evaporated under reduced pressure, and the obtained residue was purified by silica gel column chromatography (ethyl acetate/n-hexane = 2/3 → 3/2) to obtain the desired product (85.9 mg, yield: 78%).
¹H-NMR (CDCl₃): δ 8.41 (d, 1H, J = 5.6 Hz), 7.38-7.05 (m, 8H), 7.30 (d, 1H, J = 2.0 Hz), 6.80 (d, 1H, J = 5.6 Hz), 6.66 (d, 1H, J = 2.0 Hz), 6.46 (s, 1H), 6.20 (s, 1H), 5.34 (t, 1H, J = 5.7 Hz), 4.32 (d, 2H, J = 5.7 Hz), 2.54 (s, 3H), 2.39 (s, 2H)
MS (ESI):515 (M+H⁺)

Example 30

### 1-(2-(2-morpholinopyrimidin-4-yloxy)benzyl)-3-(3-(2-methylfuran-3-yl)-1-p-tolyl-1H-pyrazol-5-yl)urea

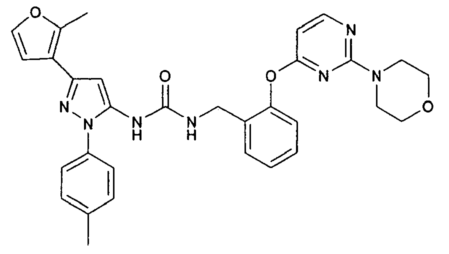

With ethanol (0.8 mL), 1-(2-(2-chloropyrimidin-4-yloxy)benzyl)-3-(3-(2-methylfuran-3-yl)-1-p-tolyl-1H-pyrazol-5-yl)urea (57.7 mg) and sodium carbonate (35.6 mg) were mixed, and morpholine (13.7 µL) was added thereto, followed by stirring at 40°C for 36 hours. The reaction solution was filtered, and the resultant filtrate was evaporated under reduced pressure, followed by purification of the obtained residue by silica gel column chromatography (ethyl acetate/n-hexane = 2/3 → 7/3) to obtain the desired product (53.8 mg, yield: 85%).
¹H-NMR (CDCl₃): δ 8.11 (d, 1H, J = 5.5 Hz), 7.34-7.05 (m, 9H), 6.65 (d, 1H, J = 2.0 Hz), 6.43 (s, 1H), 6.22 (s, 1H), 5.94 (d, 1H, J = 5.5 Hz), 5.38 (t, 1H, J = 5.9 Hz), 4.33 (d, 2H, J = 5.9 Hz), 3.63-3.57 (m, 8H), 2.54 (s, 3H), 2.38 (s, 3H)
MS (ESI):566 (M+H⁺)

Example 31

### 1-(2-(2-chloropyrimidin-4-yloxy)benzyl)-3-(3-cyclohexyl-1-p-tolyl-1H-pyrazol-5-yl)urea

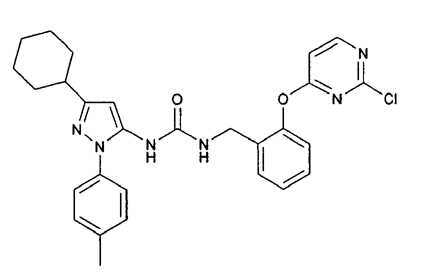

In acetone (2.26 mL), 1-(2-hydroxybenzyl)-3-(3-cyclohexyl)-1-p-tolyl-1H-pyrazol-5-yl)urea (70.3 mg) and 2,4-dichloropyrimidine (33.7 mg) were dissolved, and aqueous 0.1 M sodium hydroxide solution (2.26 mL) was added thereto, followed by stirring at room temperature for 13.5 hours. The reaction solution was evaporated under reduced pressure, and the obtained aqueous layer was extracted 3 times with dichloromethane (5 mL). The organic layers were combined, dried over anhydrous sodium sulfate, and evaporated under reduced pressure. The obtained residue was purified by silica gel column chromatography (ethyl acetate/n-hexane=2/3 → 1/1) to obtain the desired product (71.4 mg, yield: 83%).
¹H-NMR (CDCl₃): δ 8.42 (d, 1H, J = 5.6Hz), 7.36-7.06 (m, 8H), 6.81 (d, 1H, J = 5.6Hz), 6.16 (s, 1H), 6.04 (s, 1H), 5.25 (t, 1H, J = 5.6Hz), 4.33 (d, 2H, J = 5.6Hz), 2.67-2.61 (m, 1H), 2.38 (s, 3H), 2.01-1.26 (m, 10H)
MS (ESI):517 (M+H⁺)

Example 32

### 1-(2-(2-morpholinopyrimidin-4-yloxy)benzyl)-3-(3-cyclohexyl-1-p-tolyl-1H-pyrazol-5-yl)urea

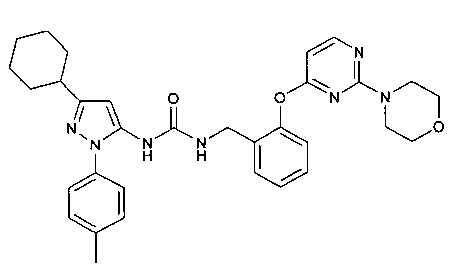

With ethanol (0.7 mL), 1-(2-(2-chloropyrimidin-4-yloxy)benzyl)-3-(3-cyclohexyl-1-p-tolyl-1H-pyrazol-5-yl)urea (59.0 mg) and sodium carbonate (36.3 mg) were mixed, and morpholine (14.0 µL) was added thereto, followed by stirring at 40°C for 45 hours. The reaction solution was filtered, and the resultant filtrate was evaporated under reduced pressure, followed by purification of the obtained residue by silica gel column chromatography (ethyl acetate/n-hexane = 1/1 → ethyl acetate) to obtain the desired product (59.8 mg, yield: 92%).
¹H-NMR (CDCl₃): δ 8.15 (d, 1H, J = 5.6Hz), 7.33-7.06 (m, 8H), 6.13 (s, 1H), 6.02 (s, 1H), 5.97 (d, 1 H, J = 5.6Hz), 5.26 (t, 1 H, J = 6.0Hz), 4.33 (d, 2H, J = 6.0Hz), 3.66-3.61 (m, 8H), 2.66-2.60 (m, 1H), 2.37 (s, 3H), 2.00-1.23 (m, 10H)
MS (ESI):568 (M+H⁺)

Example 33

### 1-(2-(2-chloropyrimidin-4-yloxy)benzyl)-3-(3-t-butyl-1-phenyl-1 H-pyrazol-5 -yl)urea

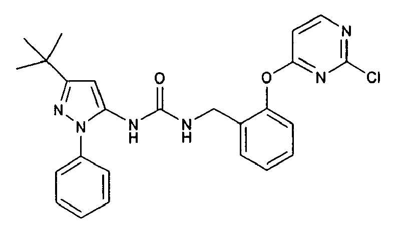

In acetone (2.86 mL), 1-(2-hydroxybenzyl)-3-(3-t-butyl-1-phenyl-1H-pyrazol-5-yl)urea (80.3 mg) and 2,4-dichloropyrimidine (42.7 mg) were dissolved, and aqueous 0.1 M sodium hydroxide solution (2.86 mL) was added thereto, followed by stirring at room temperature for 16 hours. The reaction solution was evaporated under reduced pressure, and the obtained residue was purified by silica gel column chromatography (ethyl acetate/n-hexane = 1/2 → 1/1) to obtain the desired product (83.7 mg, yield: 80%).
¹H-NMR (CDCl₃): δ 8.42 (d, 1H, J = 5.7Hz), 7.44-7.05 (m, 9H), 6.80 (d, 1H, J = 5.7Hz), 6.25 (s, 1H), 6.16 (s, 1H), 5.31 (t, 1H, J = 5.5Hz), 4.31 (d, 2H, J = 5.5Hz), 1.33 (s, 9H)
MS (ESI):477 (M+H⁺)

Example 34

### 1-(2-(2-morpholinopyrimidin-4-yloxy)benzyl)-3-(3-t-butyl-1-phenyl-1 H-pyrazol-5-yl)urea

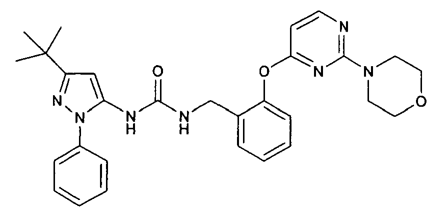

With ethanol (0.8 mL), 1-(2-(2-chloropyrimidin-4-yloxy)benzyl)-3-(3-t-butyl-1-phenyl-1H-pyrazol-5-yl)urea (62.7 mg) and sodium carbonate (55.7 mg) were mixed, and morpholine (16.1 µL) was added thereto, followed by stirring at 40°C for 36 hours. The reaction solution was filtered, and the resultant filtrate was evaporated under reduced pressure, followed by purification of the obtained residue by silica gel column chromatography (ethyl acetate/n-hexane = 1/1 → 2/1) to obtain the desired product (63.3 mg, yield: 91%).
¹H-NMR (CDCl₃): δ 8.15 (d, 1H, J = 5.6Hz), 7.45-7.06 (m, 9H), 6.22 (s, 1H), 6.07 (s, 1H), 5.96 (d, 1 H, J = 5.6Hz), 5.28 (t, 1H, J = 6.0Hz), 4.33 (d, 2H, J = 6.0Hz), 3.66-3.60 (m, 8H), 1.33 (s, 9H)
MS (ESI):528 (M+H⁺)

Example 35

### 1-(2-(2-chloropyrimidin-4-yloxy)benzyl)-3-(3-(furan-2-yl)-1-p-tolyl-1 H-pyrazol-5-yl)urea

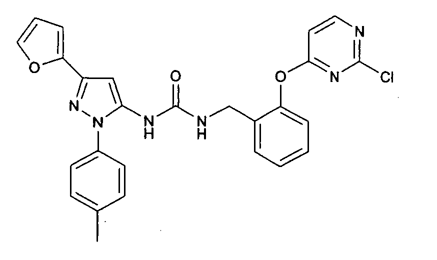

In acetone (2.15 mL), 1-(2-hydroxybenzyl)-3-(3-(furan-2-yl)-1-p-tolyl-1H-pyrazol-5-yl)urea (64.2 mg) and 2,4-dichloropyrimidine (32.0 mg) were dissolved, and aqueous 0.1 M sodium hydroxide solution (2.15 mL) was added thereto, followed by stirring at room temperature for 63.5 hours. The reaction solution was evaporated under reduced pressure, and the obtained residue was purified by silica gel column chromatography (ethyl acetate/n-hexane = 1/1 → 7/3) to obtain the desired product (70.9 mg, yield: 86%).
¹H-NMR (CDCl₃): δ 8.40 (d, 1H, J = 5.6Hz), 7.45 (dd, 1H, J = 0.7, 1.8Hz), 7.39-7.05 (m, 8H), 6.81 (d, 1H, J = 5.6Hz), 6.71 (dd, 1H, J = 0.7, 3.3Hz), 6.62 (s, 1H), 6.47 (dd, 1H, J = 1.8, 3.3Hz), 6.27 (s, 1H), 5.41 (t, 1H, J = 5.7Hz), 4.32 (d, 2H, J = 5.7Hz), 2.38 (s, 3H)
MS (ESI):501 (M+H⁺)

Example 36

### 1-(2-(2-morpholinopyrimidin-4-yloxy)benzyl)-3-(3-(furan-2-yl)-1-p-tolyl-1 H-pyrazol-5-yl)urea

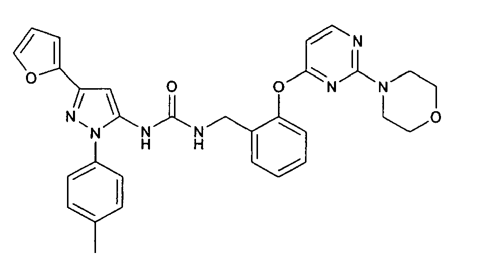

In ethanol (0.8 mL), 1-(2-(2-chloropyrimidin-4-yloxy)benzyl)-3-(3-(furan-2-yl)-1-p-tolyl-1H-pyrazol-5-yl)urea (50.0 mg) was dissolved, and morpholine (22.7 µL) was added thereto, followed by stirring at room temperature for 85 hours. The reaction solution was evaporated under reduced pressure, and the obtained residue was purified by silica gel column chromatography (ethyl acetate/n-hexane = 1/1 → 4/1) to obtain the desired product (47.3 mg, yield: 86%).
¹H-NMR (CDCl₃): δ 8.12 (d, 1H, J = 5.6Hz)), 7.46 (d, 1H, J = 2.0Hz), 7.35-7.06 (m, 8H), 6.71 (d, 1 H, J = 3 .2Hz), 6.57 (s, 1H), 6.47 (dd, 1H, J = 2.0, 3.2Hz), 6.21 (s, 1H), 5.97 (d, 1H, J = 5.6Hz), 5.38 (t, 1H, J = 5.9Hz), 4.34 (d, 2H, J = 5.9Hz), 3.63-3.58 (m, 8H), 2.38 (s, 3H)

Example 37

### 1-(2-(2-chloropyrimidin-4-yloxy)benzyl)-3-(3-t-butyl-1-(6-methylpyridin-3-yl)-1H-pyrazol-5-yl)urea

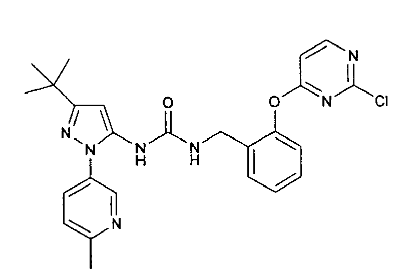

In acetone (1.74 mL), -(2-hydroxybenzyl)-3-(3-t-butyl-1-(6-methylpyridin-3-yl)-1H-pyrazol-5-yl)urea (51.0 mg) and 2,4-dichloropyrimidine (26.0 mg) were dissolved, and aqueous 0.1 M sodium hydroxide solution (1.74 mL) was added thereto, followed by stirring at room temperature for 63.5 hours. The reaction solution was evaporated under reduced pressure, and the obtained residue was purified by silica gel column chromatography (ethyl acetate/n-hexane = 7/3 → ethyl acetate) to obtain the desired product (57.2 mg, yield: 87%).
¹H-NMR (CDCl₃):δ 8.49-7.03 (m, 8H), 8.39 (d, 1H, J = 5.6 Hz), 6.74 (d, 1H, J = 5.6 Hz), 6.35 (s, 1H), 5.70 (t, 1H, J = 5.7 Hz), 4.31 (d, 2H, J = 5.7 Hz), 2.41 (s, 3H), 1.32 (s, 9H)
MS (ESI):492 (M+H⁺)

Example 38

### 1 -(2-(2-morpholinopyrimidin-4-yloxy)benzyl)-3 -(3 -t-butyl-1 -(6-methylpyridin-3 -yl)-1H-pyrazol-5-yl)urea

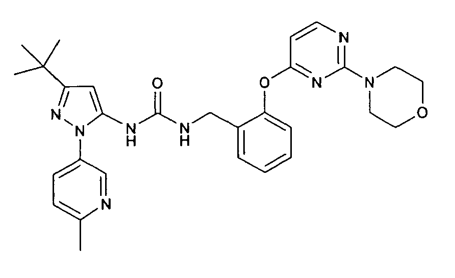

In ethanol (0.8 mL), 1-(2-(2-chloropyrimidin-4-yloxy)benzyl)-3-(3-t-butyl-1-(6-methylpyridin-3-yl)-1H-pyrazol-5-yl)urea (40.6 mg) was dissolved, and morpholine (18.8 µL) was added thereto, followed by stirring at room temperature for 85 hours. The reaction solution was evaporated under reduced pressure, and the obtained residue was purified by silica gel column chromatography (ethyl acetate/n-hexane = 9/1 → ethyl acetate) to obtain the desired product (29.1 mg, yield: 65%).
¹H-NMR (CDCl₃):δ 8.53-6.84 (m, 8H), 8.13 (d, 1H, J = 5.5 Hz), 6.33 (s, 1H), 5.91 (d, 1H, J = 5.5 Hz), 5.51 (t, 1H, J = 5.8 Hz), 4.32 (d, 2H, J = 5.8 Hz), 3.65-3.59 (m, 8H), 2.46 (s, 3H), 1.33 (s, 9H)

Example 39

### 1-(2-(2-chloropyrimidin-4-yloxy)-5-methylbenzyl)-3-(3-t-butyl-1-p-tolyl-1H-pyrazol-5-yl)urea

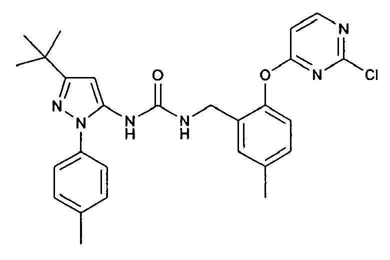

In acetone (2.34 mL), 1-(2-hydroxy-5-methylbenzyl)-3-(3-t-butyl-1-p-tolyl-1H-pyrazol-5-yl)urea (70.6 mg) and 2,4-dichloropyrimidine (34.8 mg) were dissolved, and aqueous 0.1 M sodium hydroxide solution (2.34 mL) was added thereto, followed by stirring at room temperature for 20 hours. The reaction solution was evaporated under reduced pressure, and the obtained residue was purified by silica gel column chromatography (ethyl acetate/n-hexane = 1/4 → 1/1) to obtain the desired product (77.9 mg, yield: 86%).
¹H-NMR (CDCl₃):δ 8.40 (d, 1H, J = 5.7 Hz), 7.30-6.93 (m, 7H), 6.77 (d, 1H, J = 5.7 Hz), 6.24 (s, 1H), 6.07 (s, 1H), 5.28 (t, 1H, J = 5.7 Hz), 4.26 (d, 2H, J = 5.7 Hz), 2.36 (s, 3H), 2.35 (s, 3H), 1.33 (s, 9H)
MS (ESI):505 (M+H⁺)

Example 40

### 1-(2-(2-morpholinopyrimidin-4-yloxy)-5-methylbenzyl)-3-(3-t-butyl-1-p-tolyl-1H-pyrazol-5-yl)urea

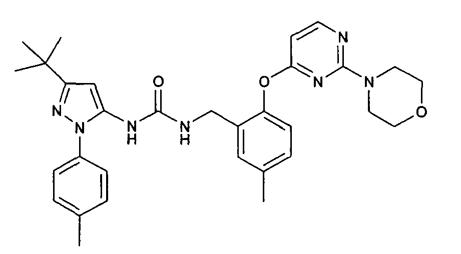

In ethanol (1.0 mL), 1-(2-(2-chloropyrimidin-4-yloxy)-5-methylbenzyl )-3-(3-t-butyl-1-p-tolyl-1H-pyrazol-5-yl)urea (52.6 mg) was dissolved, and morpholine (23.7 µL) was added thereto, followed by stirring at room temperature for 64 hours. The reaction solution was evaporated under reduced pressure, and the obtained residue was purified by silica gel column chromatography (ethyl acetate/n-hexane = 7/13 → 11/9) to obtain the desired product (50.7 mg, yield: 88%).
¹H-NMR (CDCl₃):δ 8.13 (d, 1H, J = 5.6 Hz), 7.30-6.94 (m, 7H), 6.20 (s, 1H), 5.98 (s, 1H), 5.93 (d, 1 H, J = 5.6 Hz), 5.25 (t, 1 H, J = 5.9 Hz), 4.29 (d, 2H, J = 5.9 Hz), 3.66-3.63 (m, 8H), 2.36 (s, 3H), 2.34 (s, 3H), 1.33 (s, 9H)
MS (ESI):556 (M+H⁺)

Example 41

### 1-(2-(2-chloropyrimidin-4-yloxy)-5-(trifluoromethyl)benzyl)-3-(3-t-butyl-1-p-tolyl-1 H-pyrazol-5-yl)urea

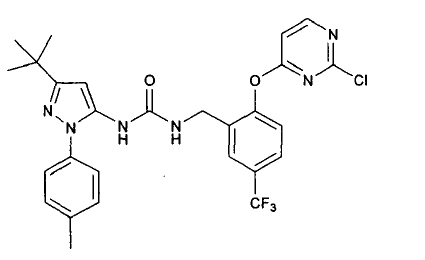

In acetone (2.35 mL), 1-(2-hydroxy-5-(trifluoromethyl)benzyl)-3-(3-t-butyl-1-p-tolyl-1H-pyrazol-5-yl)urea (80.6 mg) and 2,4-dichloropyrimidine (35.0 mg) were dissolved, and aqueous 0.1 M sodium hydroxide solution (2.35 mL) was added thereto, followed by stirring at room temperature for 88 hours. The reaction solution was evaporated under reduced pressure, and the obtained residue was purified by silica gel column chromatography (ethyl acetate/n-hexane = 1/4 → 1/1) to obtain the desired product (56.6 mg, yield: 56%).
¹H-NMR (CDCl₃ ):δ 8.48 (d, 1H, J = 5.6 Hz), 7.65-7.19 (m, 7H), 6.90 (d, 1H, J = 5.6 Hz), 6.21 (s, 1H), 6.09 (s, 1H), 5.30 (t, 1H, J = 5.8 Hz), 4.38 (d, 2H, J = 5.8 Hz), 2.35 (s, 3H), 1.32 (s, 9H)
MS (ESI):559 (M+H⁺)

Example 42

### 1-(2-(2-morpholinopyrimidin-4-yloxy)-5-(trifluoromethyl)benzyl)-3-(3-t-butyl-1-p-tolyl-1H-pyrazol-5-yl)urea

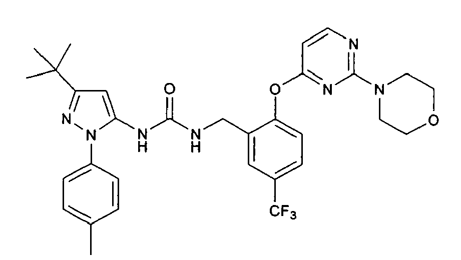

In ethanol (0.8 mL), 1-(2-(2-chloropyrimidin-4-yloxy)-5-(trifluoromethyl)benzyl)-3-(3-t-butyl-1-p-tolyl-1H-pyrazol-5-yl)urea (37.0 mg) was dissolved, and morpholine (15.1 µL) was added thereto, followed by stirring at room temperature for 64 hours. The reaction solution was evaporated under reduced pressure, and the obtained residue was purified by silica gel column chromatography (ethyl acetate/n-hexane = 7/13 → 11/9) to obtain the desired product (28.2 mg, yield: 70%).
¹ H-NMR (CDCl₃ ):δ 8.20 (d, 1H, J = 5.5 Hz), 7.64-7.19 (m, 7H), 6.18 (s, 1H), 6.07 (d, 1H, J = 5.5 Hz), 5.99 (s, 1H), 5.28 (t, 1H, J = 6.0 Hz), 4.41 (d, 2H, J = 6.0 Hz), 3.66-3.58 (m, 8H), 2.36 (s, 3H), 1.33 (s, 9H)
MS (ESI):610 (M+H⁺)

Example 43

### 1-(2-(2-chloropyrimidin-4-yloxy)-5-chlorobenzyl)-3-(3-t-butyl-1-p-tolyl-1H-pyrazol-5-yl)urea

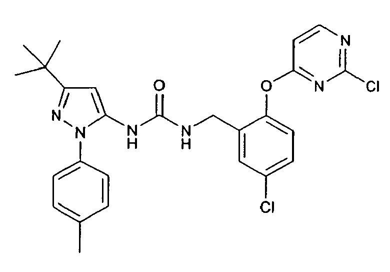

In acetone (4.28mL), 1-(2-hydroxy-5-chlorobenzyl)-3-(3-t-butyl-1-p-tolyl-1H-pyrazol-5-yl)urea (136 mg) and 2,4-dichloropyrimidine (63.7 mg) were dissolved, and aqueous 0.1 M sodium hydroxide solution (4.28 mL) was added thereto, followed by stirring at room temperature for 16 hours. The reaction solution was evaporated under reduced pressure, and the obtained residue was purified by silica gel column chromatography (ethyl acetate/n-hexane = 1/4 → 9/11) to obtain the desired product (142 mg, yield: 82%).
¹H-NMR (CDCl₃):δ 8.45 (d, 1H, J = 5.6 Hz), 7.34-7.01 (m, 7H), 6.84 (d, 1H, J = 5.6 Hz), 6.23 (s, 1H), 6.08 (s, 1H), 5.25 (t, 1H, J = 5.9 Hz), 4.28 (d, 2H, J = 5.9 Hz), 2.36 (s, 3H), 1.33 (s, 9H)
MS (ESI):525 (M+H⁺)

Example 44

### 1-(2-(2-morpholinopyrimidin-4-yloxy)-5-chlorobenzyl)-3-(3-t-butyl-1-p-tolyl-1H-pyrazol-5-yl)urea

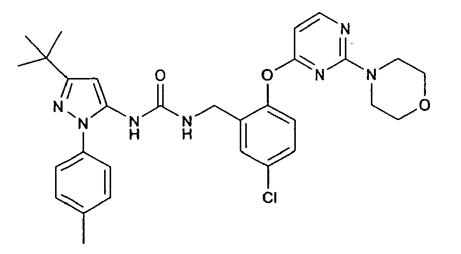

In ethanol (3.0 mL), 1-(2-(2-chloropyrimidin-4-yloxy)-5-chlorobenzyl)-3-(3-t-butyl-1-p-tolyl-1H-pyrazol-5-yl)urea (114 mg) was dissolved, and morpholine (49.3 µL) was added thereto, followed by stirring at room temperature for 18 hours. The reaction solution was evaporated under reduced pressure, and the obtained residue was purified by silica gel column chromatography (ethyl acetate/n-hexane = 7/13 → 11/9) to obtain the desired product (105 mg, yield: 84%).
¹H-NMR (CDCl₃ ):δ8.17 (d, 1H, J = 5.5 Hz), 7.32-7.00 (m, 7H), 6.19 (s, 1H), 6.02 (s, 1H), 6.02 (d, 1H, J = 5.5 Hz), 5.22 (t, 1H, J = 6.1 Hz), 4.30 (d, 2H, J = 6.1 Hz), 3.67-3.59 (m, 8H), 2.37 (s, 3H), 1.33 (s, 9H)
MS (ESI):576 (M+H⁺)

Example 45

### 1-(3-chloro-2-(2-chloropyrimidin-4-yloxy)-5-fluorobenzyl)-3-(3-t-butyl-1-p-tolyl-1 H-pyrazol-5-yl)urea

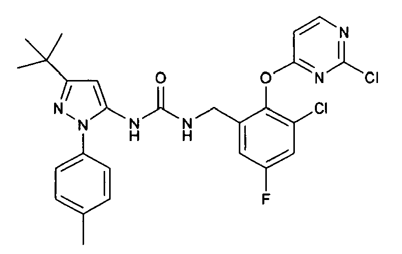

In acetone (8.3 mL), 1-(3-chloro-5-fluoro-2-hydroxybenzyl)-3-(3-t-butyl-1-p-tolyl-1H-pyrazol-5-yl)urea (275 mg) and 2,4-dichloropyrimidine (124 mg) were dissolved, and aqueous 0.1 M sodium hydroxide solution (8.3 mL) was added thereto, followed by stirring at room temperature for 41 hours. The reaction solution was evaporated under reduced pressure, and the obtained aqueous layer was extracted 3 times with dichloromethane (10 mL). The organic layers were combined, dried over anhydrous sodium sulfate, and evaporated under reduced pressure. The obtained residue was purified by silica gel column chromatography (ethyl acetate/n-hexane = 3/7 → 1/1), and was washed with hexane to obtain the desired product (262 mg, yield: 76%).
¹H-NMR (CDCl₃):δ 8.48 (d, 1H, J = 5.6 Hz), 7.33-6.86 (m, 6H), 6.92 (d, 1H, J = 5.6 Hz), 6.22 (s, 1H), 6.15 (s, 1H), 5.24 (t, 1H, J = 6.0 Hz), 4.27 (d, 2H, J = 6.0 Hz), 2.37 (s, 3H), 1.33 (s, 9H)
MS (ESI):543 (M+H⁺)

Example 46

### 1-(3-chloro-2-(2-morpholinopyrimidin-4-yloxy)-5-fluorobenzyl)-3-(3-t-butyl-1-p-tolyl-1H-pyrazol-5-yl)urea

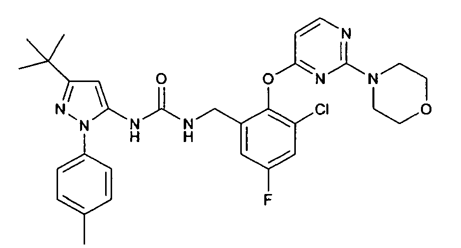

In ethanol (5 mL), 1-(3-chloro-2-(2-chloropyrimidin-4-yloxy)-5-fluorobenzyl)-3-(3-t-butyl-1-p-tolyl-1H-pyrazol-5-yl)urea (239 mg) was dissolved, and morpholine (100 µL) was added thereto, followed by stirring at room temperature for 19 hours. The reaction solution was evaporated under reduced pressure, and the obtained residue was purified by silica gel column chromatography (ethyl acetate/n-hexane = 2/3 → 3/2) to obtain the desired product (249 mg, yield: 96%).
¹H-NMR (CDCl3 ):δ 8.20 (d, 1H, J = 5.6 Hz), 7.33-6.86 (m, 6H), 6.19 (s, 1H), 6.11 (d, 1H, J = 5.6 Hz), 6.05 (s, 1H), 5.17 (t, 1H, J = 6.1 Hz), 4.27 (d, 2H, J = 6.1 Hz), 3.64-3.53 (m, 8H), 2.37 (s, 3H), 1.34 (s, 9H)
MS (ESI):594 (M+H⁺)

Example 47

### 1-(2-(2-chloropyrimidin-4-yloxy)-5-fluorobenzyl)-3-(3-t-pentyl-1-p-tolyl-1 H-pyrazol-5-yl)urea

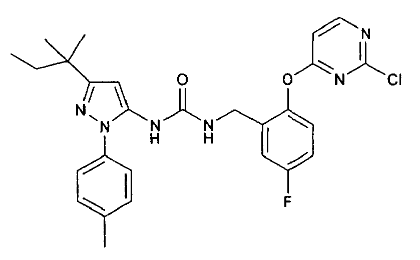

In acetone (14.4 mL), 1-(5-fluoro-2-hydroxybenzyl)-3-(3-t-pentyl-1-p-tolyl-1 H-pyrazol-5-yl)urea (454 mg) and 2,4-dichloropyrimidine (214 mg) were dissolved, and aqueous 0.1 M sodium hydroxide solution (14.4 mL) was added thereto, followed by stirring at room temperature for 17 hours. The reaction solution was evaporated under reduced pressure, and the obtained aqueous layer was extracted 3 times with dichloromethane (10 mL). The organic layers were combined, dried over anhydrous sodium sulfate, and evaporated under reduced pressure. The obtained residue was purified by silica gel column chromatography (ethyl acetate/n-hexane = 3/7 → 1/1) to obtain the desired product (529 mg, yield: 92%).
¹H-NMR (CDCl₃ ):δ 8.42 (d, 1H, J = 5.6 Hz), 7.30-6,94 (m, 7H), 6.81 (d, 1H, J = 5.6 Hz), 6.26 (s, 1H), 6.19 (s, 1H), 5.32 (t, 1H, J = 6.0 Hz), 4.23 (d, 2H, J = 6.0 Hz), 2.34 (s, 3H), 1.64 (q, 2H, J = 7.4 Hz), 1.28 (s, 6H), 0.81 (t, 3H, J = 7.4 Hz)
MS (ESI):523 (M+H⁺)

Example 48

### 1-(2-(2-morpholinopyrimidin-4-yloxy)-5-fluorobenzyl)-3-(3-t-pentyl-l-p-tolyl-1H-pyrazol-5-yl)urea

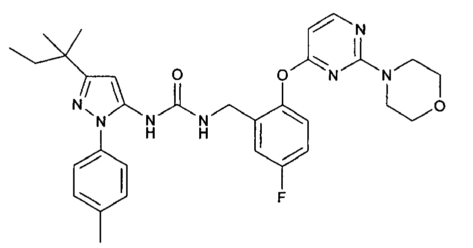

In ethanol (10 mL), 1-(2-(2-chloropyrimidin-4-yloxy)-5-fluorobenzyl)-3-(3-t-pentyl-1-p-tolyl-1H-pyrazol-5-yl)urea (481 mg) was dissolved, and morpholine (209 µL) was added thereto, followed by stirring at room temperature for 19 hours. The reaction solution was evaporated under reduced pressure, and the obtained residue was purified by silica gel column chromatography (ethyl acetate/n-hexane = 7/13 → 3/2) to obtain the desired product (481 mg, yield: 91 %).
¹H-NMR (CDCl₃ ):δ 8.15 (d, 1 H, J = 5.4 Hz), 7.31-6.95 (m, 7H), 6.17 (s, 1 H), 6.12 (s, 1 H), 5.99 (d, 1 H, J = 5.4 Hz), 5.21 (t, 1 H, J = 6.0 Hz), 4.27 (d, 2H, J = 6.0 Hz), 3.66-3.58 (m, 8H), 2.36 (s, 3H), 1.64 (q, 2H, J = 7.5 Hz), 1.28 (s, 6H), 0.81 (t, 3H, J = 7.5 Hz)
MS (ESI):574 (M+H⁺)

Example 49

### 1-(2-(2-chloropyrimidin-4-yloxy)benzyl)-3-(3-t-butyl-1-(4-chlorophenyl)-1H-pyrazol-5-yl)urea

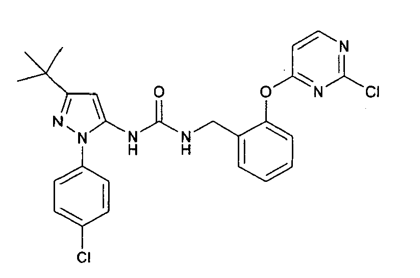

In acetone (8.73 mL), 1-(2-hydroxybenzyl)-3-(3-t-butyl-1-(4-chlorophenyl)-1H-pyrazol-5-yl)urea (268 mg) and 2,4-dichloropyrimidine (130 mg) were dissolved, and aqueous 0.1 M sodium hydroxide solution (8.73 mL) was added thereto, followed by stirring at room temperature for 16 hours. The reaction solution was evaporated under reduced pressure, and the aqueous layer was extracted 3 times with dichloromethane (10 mL). The organic layers were combined, dried over anhydrous sodium sulfate, and evaporated under reduced pressure. The obtained residue was purified by silica gel column chromatography (ethyl acetate/n-hexane = 3/7 → 1/1), and was washed with hexane to obtain the desired product (289 mg, yield: 84%).
¹H-NMR (CDCl₃ ):δ 8.41 (d, 1H, J = 5.6 Hz), 7.40-7.04 (m, 8H), 6.77 (d, 1H, J = 5.6 Hz), 6.44 (s, 1H), 6.22 (s, 1H), 5.37 (t, 1H, J = 5.6 Hz), 4.25 (d, 2H, J = 5.6 Hz), 1.31 (s, 9H)

Example 50

### 1-(2-(2-morpholinopyrimidin-4-yloxy)benzyl)-3-(3-t-butyl-1-(4-chlorophenyl)-1H-pyrazol-5-yl)urea

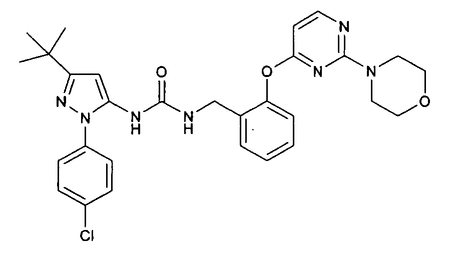

In ethanol (5 mL), 1-(2-(2-chloropyrimidin-4-yloxy)benzyl)-3-(3-t-butyl-1-(4-chlorophenyl)-1H-pyrazol-5-yl)urea (249 mg) was dissolved, and morpholine (111 µL) was added thereto, followed by stirring at room temperature for 13 hours. The reaction solution was evaporated under reduced pressure, and the obtained residue was purified by silica gel column chromatography (ethyl acetate/n-hexane = 3/7 → 11/9) to obtain the desired product (254 mg, yield: 93%).
¹H-NMR (CDCl₃ ):δ8.14 (d, 1H, J = 5.5 Hz), 7.40-7.05 (m, 8H), 6.28 (s, 1H), 6.21 (s, 1H), 5.94 (d, 1H, J = 5.5 Hz), 5.27 (t, 1H, J = 6.0 Hz), 4.30 (d, 2H, J = 6.0 Hz), 3.65-3.60 (m, 8H), 1.32 (s, 9H)
MS (ESI):562 (M+H⁺)

Example 51

### 1-(2-(2-chloropyrimidin-4-yloxy)benzyl)-3-(3-t-butyl-1-(pyridin-3-yl)-1H-pyrazol-5-yl)urea

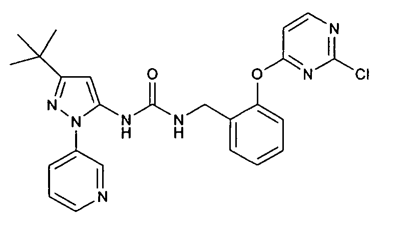

In acetone (8.45 mL), 1-(2-hydroxybenzyl)-3-(3-t-butyl-1-(pyridin-3-yl)-1H-pyrazol-5-yl)urea (237 mg) and 2,4-dichloropyrimidine (126 mg) were dissolved, and aqueous 0.1 M sodium hydroxide solution (8.45 mL) was added thereto, followed by stirring at room temperature for 17 hours. The precipitated solids were filtered, and was washed sequentially with distilled water and acetone to obtain the desired product (232 mg, yield: 75%).
¹H-NMR (CDCl₃):δ 8.67-7.83 (m, 3H), 8.38 (d, 1H, J = 5.7 Hz), 7.35-7.03 (m, 5H), 7.12 (s, 1H), 6.76 (d, 1H, J = 5.7 Hz), 6.33 (s, 1H), 5.71 (t, 1H, J = 5.9 Hz), 4.30 (d, 2H, J = 5.9 Hz), 1.32 (s, 9H)

Example 52

### 1-(2-(2-morpholinopyrimidin-4-yloxy)benzyl)-3-(3-t-butyl-1-(pyridin-3-yl)-1 H-pyrazol-5-yl)urea

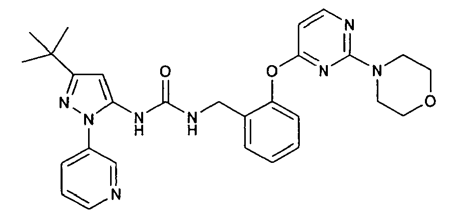

In ethanol (5 mL), 1-(2-(2-chloropyrimidin-4-yloxy)benzyl)-3-(3-t-butyl-1-(pyridin-3-yl)-1H-pyrazol-S-yl)urea (205 mg) was dissolved, and morpholine (97.5 µL) was added thereto, followed by stirring at 40°C for 15 hours. The reaction solution was evaporated under reduced pressure, and the obtained residue was purified by silica gel column chromatography (ethyl acetate/n-hexane = 1/1 → ethyl acetate) to obtain the desired product (212 mg, yield: 94%).
¹H-NMR (CDCl₃):δ 8.71-7.82 (m, 3H), 8.12 (d, 1H, J = 5.5 Hz), 7.33-7.04 (m, 5H), 6.94 (s, 1H), 6.33 (s, 1H), 5.92 (d, 1H, J = 5.5 Hz), 5.59 (t, 1H, J = 5.9 Hz), 4.31 (d, 2H, J = 5.9 Hz), 3.65-3.58 (m, 8H), 1.33 (s, 9H)
MS (ESI):529 (M+H⁺)

Example 53

### 1-(2-(2-chloropyrimidin-4-yloxy)benzyl)-3-(3-t-butyl-1-(4-methoxy-3-methylphenyl)-1 H-pyrazol-5-yl)urea

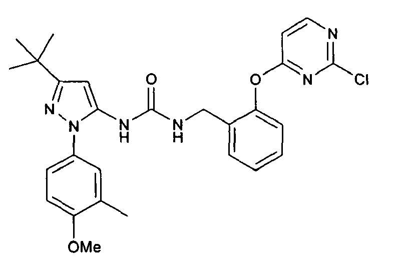

In acetone (26.1 mL), 1-(2-hydroxybenzyl)-3-(3-t-butyl-1-(4-methoxy-3-methylphenyl)-1H-pyrazol-5-yl)urea (819 mg) and 2,4-dichloropyrimidine (388 mg) were dissolved, and aqueous 0.1 M sodium hydroxide solution (26.1 mL) was added thereto, followed by stirring at room temperature for 17 hours and then at 40°C for 21 hours. DMSO (100 mL) and 2,4-dichloropyrimidine (190 mg) were added thereto, and the resulting mixture was stirred at 50°C for 14 hours. Thereafter, 2,4-dichloropyrimidine (190 mg) was added thereto, and the resulting mixture was stirred at 60°C for 4 hours. The reaction solution was evaporated under reduced pressure, and distilled water (15 mL) was added to the obtained residue, followed by extraction of the resulting mixture 3 times with dichloromethane (15 mL). The organic layers were combined, dried over anhydrous sodium sulfate, and evaporated under reduced pressure. The obtained residue was purified by silica gel column chromatography (ethyl acetate/n-hexane = 3/7 → 1/1 → 7/3) to obtain the desired product (501 mg, yield: 48%).
¹H-NMR (CDCl₃):δ 8.41 (d, 1H, J = 5.9 Hz), 7.35-6.80 (m, 7H), 6.80 (d, 1H, J = 5.9 Hz), 6.22 (s, 1H), 6.20 (s, 1H), 5.38 (t, 1H, J = 5.7 Hz), 4.31 (d, 2H, J = 5.7 Hz), 3.84 (s, 3H), 2.20 (s, 3H), 1.32 (s, 9H)

Example 54

### 1-(2-(2-morpholinopyrimidin-4-yloxy)benzyl)-3-(3-t-butyl-1-(4-methoxy-3-methylphenyl)-1H-pyrazol-5-yl)urea

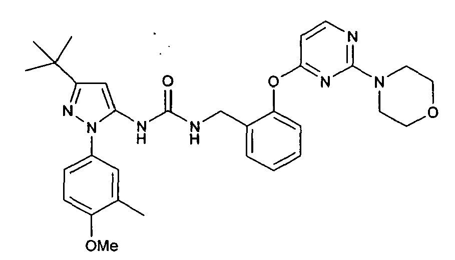

In ethanol (10 mL), 1-(2-(2-chloropyrimidin-4-yloxy)benzyl)-3-(3-t-butyl-1-(4-methoxy-3-methylphenyl)-1H-pyrazol-5-yl)urea (474 mg) was dissolved, and morpholine (207 µL) was added thereto, followed by stirring at room temperature for 19 hours. The reaction solution was evaporated under reduced pressure, and the obtained residue was purified by silica gel column chromatography (ethyl acetate/n-hexane = 9/11 → 7/3) to obtain the desired product (435 mg, yield: 84%).
¹H-NMR (CDCl₃):δ 8.15 (d, 1H, J = 5.5 Hz), 7.32-6.79 (m, 7H), 6.18 (s, 1H), 6.06 (s, 1H), 5.96 (d, 1H, J = 5.5 Hz), 5.30 (t, 1H, J = 5.9 Hz), 4.32 (d, 2H, J = 5.9 Hz), 3.84 (s, 3H), 3.65-3.61 (m, 8H), 2.20 (s, 3H), 1.32 (s, 9H)

Example 55

### 1-(2-(2-chloropyrimidin-4-yloxy)benzyl)-3-(3-t-butyl-1-(3-methoxy-4-methylphenyl)-1H-pyrazol-5 -yl)urea

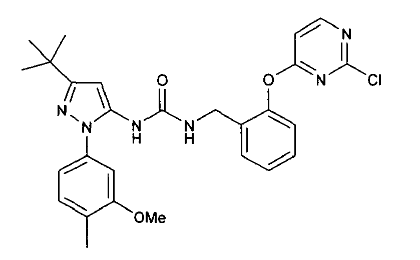

In a mixture of acetone (6.0 mL) and DMSO (40 mL), 1-(2-hydroxybenzyl)-3-(3-t-butyl-1-(3-methoxy-4-methylphenyl)-1H-pyrazol-5-yl)urea (824 mg) and 2,4-dichloropyrimidine (601 mg) were dissolved, and aqueous 0.1 M sodium hydroxide solution (30.3 mL) was added thereto, followed by stirring at 60°C for 4 hours. The reaction solution was evaporated under reduced pressure, and distilled water (15 mL) was added to the obtained residue. The resulting mixture was extracted 3 times with dichloromethane (15 mL). The organic layers were combined, dried over anhydrous sodium sulfate, and evaporated under reduced pressure. The obtained residue was purified by silica gel column chromatography (ethyl acetate/n-hexane = 3/7 → 3/2) to obtain the desired product (893 mg, yield: 85%).
¹H-NMR (CDCl₃):δ 8.40 (d, 1H, J = 5.6 Hz), 7.37-6.84 (m, 7H), 6.79 (d, 1H, J = 5.6 Hz), 6.24 (s, 2H), 5.31 (t, 1H, J = 5.8 Hz), 4.29 (d, 2H, J = 5.8 Hz), 3.77 (s, 3H), 2.20 (s, 3H), 1.33 (s, 9H)
MS (ESI):521 (M+H⁺)

Example 56

### 1-(2-(2-morpholinopyrimidin-4-yloxy)benzyl)-3-(3-t-butyl-1-(3-methoxy-4-methylphenyl)-1H-pyrazol-5-yl)urea

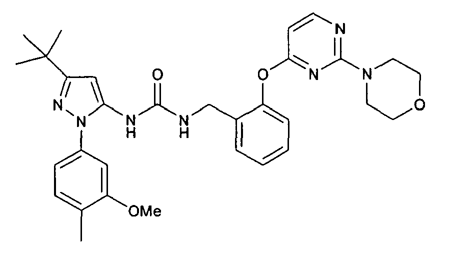

In ethanol (19 mL), 1-(2-(2-chloropyrimidin-4-yloxy)benzyl)-3-(3-t-butyl-1-(3-methoxy-4-methylphenyl)-1H-pyrazol-5-yl)urea (863 mg) was dissolved, and morpholine (377 µL) was added thereto, followed by stirring at room temperature for 19 hours. The reaction solution was evaporated under reduced pressure, and the obtained residue was purified by silica gel column chromatography (ethyl acetate/n-hexane = 9/11 → 7/3) to obtain the desired product (884 mg, yield: 93%).
¹H-NMR (CDCl₃):δ 8.14 (d, 1H, J = 5.5 Hz), 7.32-6.84 (m, 7H), 6.24 (s, 1H), 6.21 (s, 1H), 5.95 (d, 1H, J = 5.5 Hz), 5.33 (t, 1H, J = 6.0 Hz), 4.31 (d, 2H, J = 6.0 Hz), 3.76 (s, 3H), 3.66-3.60 (m, 8H), 2.21 (s, 3H), 1.33 (s, 9H)

Example 57

### 1-(2-(2-chloropyrimidin-4-yloxy)benzyl)-3-(1-(3-(benzyloxy)-4-methylphenyl)-3-t-butyl-1 H-pyrazol-5-yl)urea

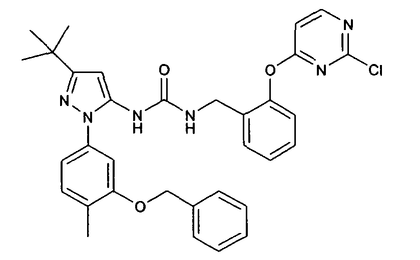

In acetone (12 mL), 1-(2-hydroxybenzyl)-3-(1-(3-(benzyloxy)-4-methylphenyl)-3-t-butyl-1H-pyrazol-5-yl)urea (414 mg) and 2,4-dichloropyrimidine (165 mg) were dissolved, and aqueous 0.1 M sodium hydroxide solution (11.1 mL) was added thereto, followed by stirring at room temperature for 13 hours. The reaction solution was evaporated under reduced pressure, and the obtained residue was purified by silica gel column chromatography (ethyl acetate/n-hexane = 1/3 - 1/1) to obtain the desired product (397 mg, yield: 78%).
¹H-NMR (CDCl₃):δ 8.39 (d, 1H, J = 5.6 Hz), 7.42-6.87 (m, 12H), 6.77 (d, 1H, J = 5.6 Hz), 6.24 (s, 1H), 6.22 (s, 1H), 5.23 (t, 1H, J = 5.9 Hz), 5.02 (s, 2H), 4.28 (d, 2H, J = 5.9 Hz), 2.27 (s, 3H), 1.33 (s, 9H)

Example 58

### 1-(2-(2-morpholinopyrimidin-4-yloxy)benzyl)-3-(1-(3-(benzyloxy)-4-methylphenyl)-3-t-butyl-1H-pyrazol-5-yl)urea

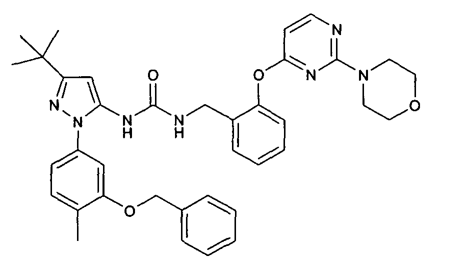

In ethanol (10 mL), 1-(2-(2-chloropyrimidin-4-yloxy)benzyl)-3-(1-(3-(benzyloxy)-4-methylphenyl)-3-t-butyl-1H-pyrazol-5-yl)urea (372 mg) was dissolved, and morpholine (142 µL) was added thereto, followed by stirring at room temperature for 12 hours and then at 40°C for 5 hours. The reaction solution was evaporated under reduced pressure, and the obtained residue was purified by silica gel column chromatography (ethyl acetate/n-hexane = 1/3 → 1/1) to obtain the desired product (349 mg, yield: 86%).
¹H-NMR (CDCl₃):δ 8.14 (d, 1H, J = 5.5 Hz), 7.42-6.88 (m, 12H), 6.22 (s, 1H), 6.11 (s, 1H), 5.95 (d, 1H, J = 5.5 Hz), 5.18 (t, 1H, J = 5.9 Hz), 5.03 (s, 2H), 4.31 (d, 2H, J = 5.9 Hz), 3.64-3.60 (m, 8H), 2.28 (s, 3H), 1.33 (s, 9H)
MS (ESI):648 (M+H⁺)

Example 59

### 1-(2-(2-morpholinopyrimidin-4-yloxy)benzyl)-3-(3-t-butyl-1-(3-hydroxy-4-methylphenyl)-1H-pyrazol-5-yl)urea

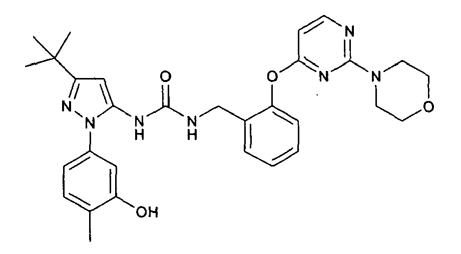

To methanol (3 mL), 1-(2-(2-morpholinopyrimidin-4-yloxy)benzyl)-3-(1-(3-(benzyloxy)-4-methylphenyl)-3-t-butyl-1H-pyrazol-5-yl)urea (160 mg) and palladium carbon (15.0 mg) were added, and the resulting mixture was stirred for 10 hours at room temperature under hydrogen atmosphere at 1 atm. The reaction solution was filtered through Celite, and the resultant filtrate was evaporated under reduced pressure. The obtained residue was purified by silica gel column chromatography (ethyl acetate/n-hexane = 7/3 → 3/2) to obtain the desired product (138 mg, quantitative).
¹H-NMR (CDCl₃):δ 8.29 (br, 1H), 8.16 (d, 1H, J = 5.6 Hz), 7.36-6.65 (m, 7H), 6.27 (s, 1H), 6.19 (s, 1H), 5.99 (d, 1H, J = 5.6 Hz), 5.13 (t, 1H, J = 5.6 Hz), 4.31 (d, 2H, J = 5.6 Hz), 3.58-3.57 (m, 8H), 2.17 (s, 3H), 1.30 (s, 9H)
MS (ESI):558 (M+H⁺)

Example 60

### 1-(2-(2-chloropyrimidin-4-yloxy)benzyl)-3-(2-t-butyl-4-p-tolyloxazol-5-yl)urea

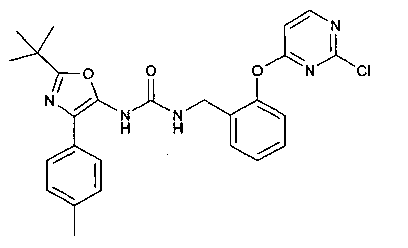

In acetone (2.5 mL), 1-(2-hydroxybenzyl)-3-(2-t-butyl-4-p-tolyloxazol-5-yl)urea (72.3 mg) and 2,4-dichloropyrimidine (36.9 mg) were dissolved, and aqueous 0.1 M sodium hydroxide solution (2.48 mL) was added thereto, followed by stirring at room temperature for 13 hours. The reaction solution was evaporated under reduced pressure, and the obtained residue was purified by silica gel column chromatography (ethyl acetate/n-hexane = 1/4 → 2/3) to obtain the desired product (49.0 mg, yield: 52%).
¹H-NMR (CDCl₃):δ 8.39 (d, 1H, J = 5.6 Hz), 7.72-7.16 (m, 8H), 6.70 (d, 1H, J = 5.6 Hz), 6.16 (s, 1H), 5.09 (t, 1H, J = 6.3 Hz), 4.32 (d, 2H, J = 6.3 Hz), 2.36 (s, 3H), 1.39 (s, 9H)
MS (ESI):492 (M+H⁺)

Example 61

### 1-(2-(2-morpholinopyrimidin-4-yloxy)benzyl)-3-(2-t-butyl-4-p-tolyloxazol-5-yl)urea

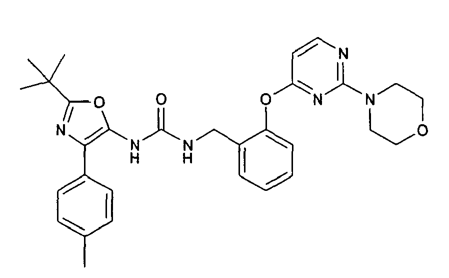

In ethanol (1.5 mL), 1-(2-(2-chloropyrimidin-4-yloxy)benzyl)-3-(2-t-butyl-4-p-tolyloxazol-5-yl)urea (49.0 mg) was dissolved, and morpholine (22.7 µL) was added thereto, followed by stirring at room temperature for 12 hours. The reaction solution was evaporated under reduced pressure, and the obtained residue was purified by silica gel column chromatography (ethyl acetate/n-hexane = 2/3 → 13/7) to obtain the desired product (39.1 mg, yield: 72%).
¹H-NMR (CDCl₃):δ 8.11 (d, 1H, J = 5.5 Hz), 7.73-6.99 (m, 8H), 6.15 (s, 1H), 5.87 (d, 1H, J = 5.5 Hz), 5.09 (t, 1H, J = 6.0 Hz), 4.33 (d, 2H, J = 6.0 Hz), 3.66-3.59 (m, 8H), 2.36 (s, 3H), 1.39 (s, 9H)

Example 62

### 1-(2-(2-chloropyrimidin-4-yloxy)benzyl)-3-(5-t-butyl-1,3,4-thiadiazol-2-yl)urea

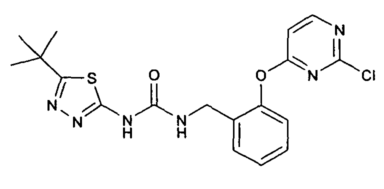

In acetone (6.83 mL), 1-(2-hydroxybenzyl)-3-(5-t-butyl-1,3,4-thiadiazol-2-yl)urea (161 mg) and 2,4-dichloropyrimidine (102 mg) were dissolved, and aqueous 0.1 M sodium hydroxide solution (6.83 mL) was added thereto, followed by stirring at room temperature for 89 hours. The reaction solution was evaporated under reduced pressure, and the obtained residue was purified by silica gel column chromatography (ethyl acetate/n-hexane = 3/1 → ethyl acetate) to obtain the desired product (208 mg, yield: 95%).
¹H-NMR (CDCl₃):δ 8.35 (d, 1H, J = 5.6 Hz), 7.53-7.10 (m, 4H), 6.80 (d, 1H, J = 5.6 Hz), 4.48 (d, 2H, J = 5.6 Hz), 1.38 (s, 9H)
MS (ESI):419 (M+H⁺)

Example 63

### 1-(2-(2-morpholinopyrimidin-4-yloxy)benzyl)-3-(5-t-butyl-1,3,4-thiadiazol-2-yl)urea

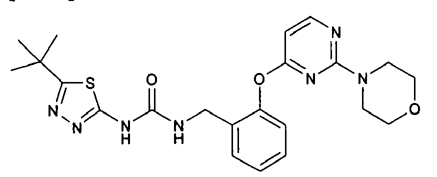

In ethanol (5.0 mL), 1-(2-(2-chloropyrimidin-4-yloxy)benzyl)-3-(5-t-butyl-1,3,4-thiadiazol-2-yl)urea (145 mg) was dissolved, and morpholine (78.7 µL) was added thereto, followed by stirring at room temperature for 19 hours. The reaction solution was evaporated under reduced pressure, and the obtained residue was purified by silica gel column chromatography (ethyl acetate/n-hexane = 1/1 → ethyl acetate) to obtain the desired product (98.6 mg, yield: 61%).
¹H-NMR (CDCl₃):δ 8.10 (d, 1H, J = 5.5 Hz), 7.50-7.08 (m, 4H), 6.06 (d, 1H, J = 5.5 Hz), 4.49 (d, 2H, J = 5.6 Hz), 3.63-3.59 (m, 8H), 1.37 (s, 9H)
MS (ESI):470 (M+H⁺)

Example 64

### 1-(2-(2-chloropyrimidin-4-yloxy)benzyl)-3-(5-(furan-2-yl)-1,3,4-oxadiazol-2-yl)urea

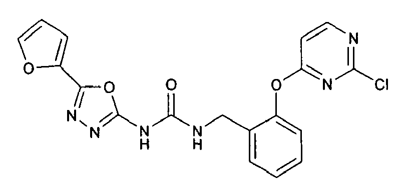

In acetone (8.40 mL), 1-(2-hydroxybenzyl)-3-(5-(furan-2-yl)-1,3,4-oxadiazol-2-yl)urea (194 mg) and 2,4-dichloropyrimidine (125 mg) were dissolved, and aqueous 0.1 M sodium hydroxide solution (8.40 mL) was added thereto, followed by stirring at room temperature for 89 hours. To this mixture, 2,4-dichloropyrimidine (62.0 mg) was added, and the resulting mixture was stirred at 40°C for 54 hours. Subsequently, 2,4-dichloropyrimidine (62.0 mg) was added to the above mixture, and then the resulting mixture was stirred at 40°C for 44 hours. The reaction solution was evaporated under reduced pressure, and the obtained residue was filtered and washed with methanol to obtain the desired product (136 mg, yield: 51%).
¹H-NMR (CD₃ OD):δ 8.47 (d, 1H, J = 5.7 Hz), 7.79 (dd, 1H, J = 0.7, 1.8 Hz), 7.55-7.14 (m, 5H), 7.07 (d, 1 H, J = 5.7 Hz), 6.68 (dd, 1H, J = 1.8, 3.5 Hz), 4.47 (s, 2H)
MS (ESI):413 (M+H⁺)

Example 65

### 1-(2-(2-morpholinopyrimidin-4-yloxy)benzyl)-3-(5-(furan-2-yl)-1,3,4-oxadiazol-2-yl)urea

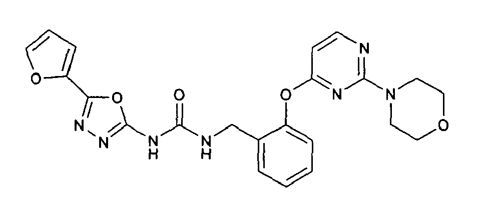

In ethanol (4.0 mL), 1-(2-(2-chloropyrimidin-4-yloxy)benzyl)-3-(5-(furan-2-yl)-1,3,4-oxadiazol-2-yl)urea (99.5 mg) was dissolved, and morpholine (54.8 µL) was added thereto, followed by stirring at room temperature for 16 hours and then at 60°C for 21 hours. The reaction solution was evaporated under reduced pressure, and the obtained residue was washed with methanol and ether to obtain the desired product (119 mg, quantitative).
¹H-NMR (CD₃ OD):δ 8.11 (d, 1H, J = 5.6 Hz), 7.80 (dd, 1H, J = 0.7,1.8 Hz), 7.50-7.08 (m, 4H), 7.14 (dd, 1H, J = 0.7, 3.5 Hz), 6.68 (dd, 1H, J = 1.8, 3.5 Hz), 6.25 (d, 1H, J = 5.6 Hz), 4.46 (s, 2H), 3.88-3.20 (m, 8H)
MS (ESI):464 (M+H⁺)

Example 66

### 1-(2-(2-chloropyrimidin-4-yloxy)benzyl)-3-(4-t-butylthiazol-2-yl)urea

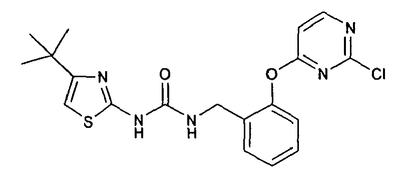

In acetone (9.02 mL), 1-(2-hydroxybenzyl)-3-(4-t-butylthiazol-2-yl)urea (212 mg) and 2,4-dichloropyrimidine (134 mg) were dissolved, and aqueous 0.1 M sodium hydroxide solution (9.02 mL) was added thereto, followed by stirring at room temperature for 89 hours. The reaction solution was evaporated under reduced pressure, and the obtained residue was purified by silica gel column chromatography (ethyl acetate/n-hexane = 2/3 → 13/7) to obtain the desired product (228 mg, yield: 79%).
¹H-NMR (CDCl₃):δ 8.39 (d, 1H, J = 5.6 Hz), 7.53-7.10 (m, 4H), 6.81 (d, 1H, J = 5.6 Hz), 6.31 (s, 1H), 4.47 (d, 2H, J = 5.6 Hz), 1.22 (s, 9H)
MS (ESI):418 (M+H⁺)

Example 67

### 1-(2-(2-monopholinopyrimidin-4-yloxy)benzyl)-3-(4-t-butylthiazol-2-yl)urea

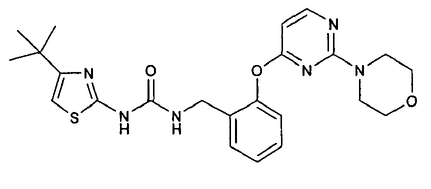

In ethanol (5.0 mL), 1-(2-(2-chloropyrimidin-4-yloxy)benzyl)-3-(4-t-butylthiazol-2-yl)urea (147 mg) was dissolved, and morpholine (79.9 µL) was added thereto, followed by stirring at room temperature for 19 hours. The reaction solution was evaporated under reduced pressure, and the obtained residue was purified by silica gel column chromatography (ethyl acetate/n-hexane = 7/3 → 3/2) to obtain the desired product (147 mg, yield: 89%).
¹H-NMR (CDCl₃):δ 8.15 (d, 1H, J = 5.6 Hz), 7.52-7.09 (m, 4H), 6.31 (s, 1H), 6.07 (d, 1H, J = 5.6 Hz), 4.49 (d, 2H, J = 5.6 Hz), 3.67-3.60 (m, 8H), 1.22 (s, 9H)
MS (ESI):470 (M+H⁺)

Example 68

### 1-(2-(2-chloropyrimidin-4-yloxy)benzyl)-3-(4-methyloxazol-2-yl)urea

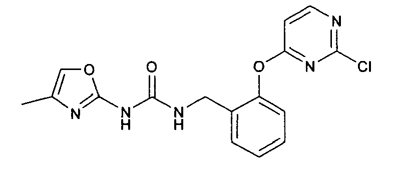

In acetone (5.26 mL), 1-(2-hydroxybenzyl)-3-(4-methyloxazol-2-yl)urea (100 mg) and 2,4-dichloropyrimidine (78.3 mg) were dissolved, and aqueous 0.1 M sodium hydroxide solution (5.26 mL) was added thereto, followed by stirring at room temperature for 64 hours. The reaction solution was evaporated under reduced pressure, and the obtained residue was purified by silica gel column chromatography (ethyl acetate/n-hexane =1/1 → 7/3 → ethyl acetate) to obtain the desired product (29.1 mg, yield: 20%).
¹H-NMR (CDCl₃):δ 8.80 (t, 1H, J = 5.9 Hz), 8.39 (d, 1H, J = 5.7 Hz), 8.14 (br, 1H), 7.54-7.10 (m, 4H), 6.99 (d, 1H, J = 1.0 Hz), 6.81 (d, 1H, J = 5.7 Hz), 4.51 (d, 2H, J = 5.9 Hz), 2.05 (d, 3H, J = 1.0 Hz)
MS (ESI):360 (M+H⁺)

Example 69

### 1-(2-(2-morpholinopyrimidin-4-yloxy)benzyl)-3-(4-methyloxazol-2-yl)urea

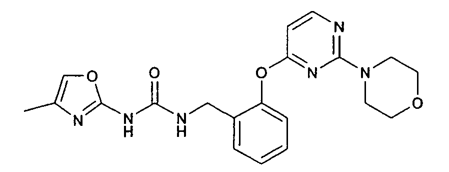

In ethanol (1.0 mL), 1-(2-(2-chloropyrimidin-4-yloxy)berizyl)-3-(4-methyloxazol-2-yl)urea (21.5 mg) was dissolved, and morpholine (13.6 µL) was added thereto, followed by stirring at room temperature for 12 hours and then at 40°C for 8 hours. The reaction solution was evaporated under reduced pressure, and the obtained residue was purified by silica gel column chromatography (ethyl acetate/n-hexane = 3/7 → 7/3) to obtain the desired product (19.1 mg, yield: 78%).
¹H-NMR (CDCl₃):δ 8.79 (t, 1H, J = 6.1 Hz), 8.16 (d, 1H, J = 5.6 Hz), 7.89 (br, 1H), 7.51-7.08 (m, 4H), 6.99 (d, 1H, J = 1.2 Hz), 6.10 (d, 1H, J = 5.6 Hz), 4.51 (d, 2H, J = 6.1 Hz), 3.67-3.60 (m, 8H), 2.04 (d, 3H, J = 1.2 Hz)
MS (ESI):411 (M+H⁺)

Example 70

### 1-(2-(2-chloropyrimidin-4-yloxy)benzyl)-3-(5-(2-hydroxypropan-2-yl)-4-methyloxazol-2-yl)urea

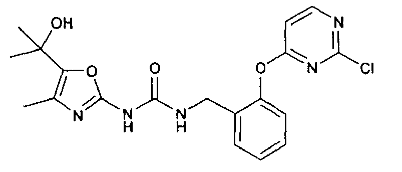

In acetone (5.26 mL), 1-(2-hydroxybenzyl)-3-(5-(2-hydroxypropan-2-yl)-4-methyloxazol-2-yl)urea (100 mg) and 2,4-dichloropyrimidine (78.3 mg) were dissolved, and aqueous 0.1 M sodium hydroxide solution (5.26 mL) was added thereto, followed by stirring at room temperature for 64 hours. The reaction solution was evaporated under reduced pressure, and the obtained residue was purified by silica gel column chromatography (ethyl acetate/n-hexane = 1/1 → 7/3 → ethyl acetate) to obtain the desired product (83.2 mg, yield: 49%).
¹H-NMR (CDCl₃ ):δ 8.71 (t, 1H, J = 5.9 Hz), 8.37 (d, 1H, J = 5.7 Hz), 7.72 (br, 1H), 7.53-7.08 (m, 4H), 6.78 (d, 1H, J = 5.7 Hz), 4.49 (d, 2H, J = 5.9 Hz), 2.15 (s, 3H), 1.57 (s, 6H)
MS (ESI):418 (M+H⁺)

Example 71

### 1-(2-(2-morpholinopyrimidin-4-yloxy)benzyl)-3-(5-(2-hydroxypropan-2-yl)-4-methyloxazol-2-yl)urea

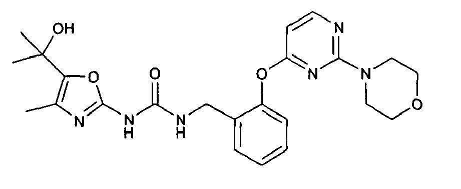

In ethanol (2.5 mL), 1-(2-(2-chloropyrimidin-4-yloxy)benzyl)-3-(5-(2-hydroxypropan-2-yl)-4-methyloxazol-2-yl)urea (66.7 mg) was dissolved, and morpholine (36.3 µL) was added thereto, followed by stirring at room temperature for 21 hours. The reaction solution was evaporated under reduced pressure, and the obtained residue was purified by silica gel column chromatography (ethyl acetate → ethyl acetate/methanol = 9/1) to obtain the desired product (68.2 mg, yield: 91%). ¹H-NMR (CDCl₃):δ 8.70 (t, 1H, J = 6.1 Hz), 8.10 (d, 1H, J = 5.6 Hz), 7.74 (br, 1H), 7.49-7.06 (m, 4H), 6.05 (d, 1H, J = 5.6 Hz), 4.49 (d, 2H, J = 6.1 Hz), 3.67-3.59 (m, 8H), 2.13 (s, 3H), 1.56 (s, 6H)
MS (ESI):469 (M+H⁺)

Example 72

### N-(1-(4-(2-(3-(5-t-butyl-2-p-toluyl-2H-pyrazol-3-yl)ureidomethyl)phenoxy)pyrimidin-2-yl)pyrrolidin-3-yl)-N-methylacetamide

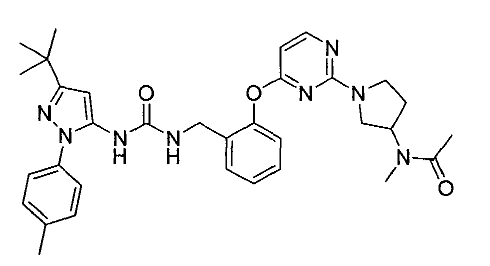

N-methyl-N-pyrrolidin-3-ylacetamide (105 mg) and N,N-diisopropylethylamine (0.50 mL) were added to a solution of 1-(2-(2-chloropyrimidin-4-yloxy)benzyl)-3-(3-t-butyl-1-p-tolyl-1H-pyrazol-5-yl)urea (210 mg) in ethanol (2.0 mL), and the resulting mixture was stirred at room temperature for 2 days. Aqueous 5% citric acid solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by silica gel column chromatography (ethyl acetate) to obtain the desired product (160 mg, yield: 62.5%).
¹H-NMR (CDCl₃):δ 8.16 (d, 1H, J=6.0Hz), 7.35-7.16 (m, 6H), 7.07 (d, 1H, J=6.0Hz), 6.23 (s, 1H), 5.98 (bs, 1H), 5.33 (bs, 1H), 4.37 (m, 4H), 3.64 (br, 2H), 2.87 (s, 3H), 2.82 (s, 1H), 2.37 (s, 3H), 2.01 (br, 3H), 1.32 (s, 9H), 1.29 (m, 2H)
MS (ESI):597 (M+H⁺), 595 (M-H⁺)

Example 73

### 1-(5-t-butyl-2-p-tolyl-2H-pyrazol-3-yl)-3-(2-(2-(3-dimethylaminopropyl)methylamino)pyrimidin-4-yloxy)benzyl)-urea

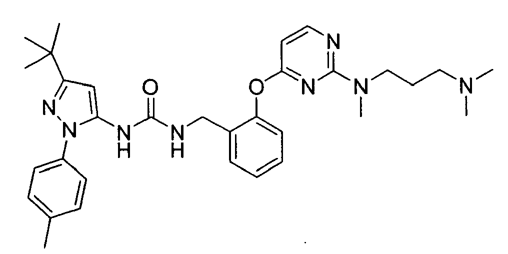

N,N,N'-trimethylpropan-1,3-diamine (76 mg) and N,N-diisopropylethylamine (0.50 mL) were added to a solution of 1-(2-(2-chloropyrimidin-4-yloxy)benzyl)-3-(3-t-butyl-1-p-tolyl-1H-pyrazol-5-yl)urea (229 mg) in ethanol (1.0 mL), and the resulting mixture was stirred at room temperature for 3 days. Aqueous 5% citric acid solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by silica gel column chromatography (ethyl acetate) to obtain the desired product (104 mg, yield: 39%).
¹H-NMR (CDCl₃):δ 8.16 (d, 1H, J=5.4Hz), 7.35-7.16 (m, 6H), 7.04 (d, 1H, J=7.6Hz), 6.26 (s, 1H), 6.04 (s, 1H), 5.85 (br, 1H), 4.35 (d, 2H, J=5.8Hz), 3.17 (br, 2H), 3.03 (s, 3H), 2.37 (s, 3H), 2.01 (bs, 8H), 1.50 (bs, 2H), 1.33 (s, 9H)
MS (ESI):571 (M+H⁺), 569 (M-H⁺)

Example 74

### 1-(4-(2-(3-(5-t-butyl-2-p-tolyl-2H-pyrazol-3-yl)-ureidomethyl)phenoxy)pyrimidin-2-yl)piperidin-3-carboxylic acid diethylamide

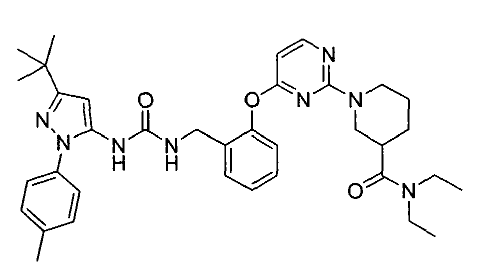

Piperidin-3-carboxylic acid diethylamide (97 mg) and N,N-diisopropylethylamine (0.10 mL) were added to a solution of 1-(2-(2-chloropyrimidin-4-yloxy) benzyl)-3-(3-t-butyl-1-p-tolyl-1H-pyrazol-5-yl)urea (205 mg) in ethanol (1.0 mL), and the resulting mixture was stirred at room temperature for 4 days. Saturated aqueous sodium bicarbonate solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by silica gel column chromatography (ethyl acetate/n-hexane = 2/1) to obtain the desired product (256 mg, yield: 96%).
¹H-NMR (CDCl₃):δ 8.12 (d, 1H, J = 5.6 Hz), 7.33-7.16 (m, 7H), 7.05 (d, 1H, J =7.1 Hz), 6.39 (s, 1H), 6.24 (s, 1H), 5.97 (d, 1H, J=5.6 Hz), 5.39 (t, 1H, J=5.8 Hz), 4.40-4.20 (m, 4H), 3.40-2.90 (m, 6H), 2.48 (m, 1H), 2.36 (s, 3H), 1.78 (m, 2H), 1.65 (m, 1H), 1.39 (m, 1H), 1.32 (s, 9H), 1.10 (t, 3H, J=5.7 Hz), 1.01 (t, 3H, J=7.1 Hz)
MS (ESI):639 (M+H⁺), 637 (M-H⁺)

Example 75

### 1-(4-(2-(3-(5-t-butyl-2-p-tolyl-2H-pyrazol-3-yl)-ureidomethyl)-4-fluorophenoxy)pyrimidin-2-yl)piperidin-3-carboxylic acid diethylamide

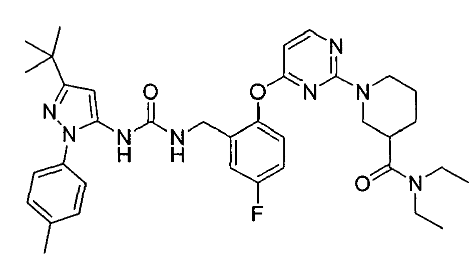

Piperidin-3-carboxylic acid diethylamide (100 mg) and N,N-diisopropylethylamine (0.10 mL) were added to a solution of 1-(2-(2-chloropyrimidin-4-yloxy)-5-fluorobenzyl)-3-(3-t-butyl-1-p-tolyl-1 H-pyrazol-5-yl)urea (174 mg) in ethanol (1.0 mL), and the resulting mixture was stirred at room temperature for 4 days. Saturated aqueous sodium bicarbonate solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by silica gel column chromatography (ethyl acetate/n-hexane = 2/1) to obtain the desired product (173 mg, yield: 77%).
¹H-NMR (CDCl₃):δ 8.12 (d, 1H, J = 5.5 Hz), 7.31 (d, 2H, J = 8.4 Hz), 7.19 (d, 2H, J = 8.4 Hz), 7.03-6.90 (m, 3H), 6.51 (s, 1H), 6.25 (s, 1H), 6.00 (d, 1H, J=5.5 Hz), 5.41 (t, 1H, J=5.9 Hz), 4.37-4.08 (m, 4H), 3.37-2.98 (m, 6H), 2.48 (m, 1H), 2.36 (s, 3H), 1.78-1.70 (m, 2H), 1.67 (m, 1H), 1.40 (m, 1H), 1.33 (s, 9H), 1.11 (t, 3H, J=7.1Hz), 1.01 (t, 3 H, J=7.1 Hz)
MS (ESI):657 (M+H⁺), 655 (M-H⁺)

Example 76

### 1-(5-t-butyl-2-p-tolyl-2H-pyrazol-3-yl)-3-(5-fluoro-2-(2-(2-hydroxymethylmorpholin-4-yl)pyrimidin-4-yloxy)benzyl)urea

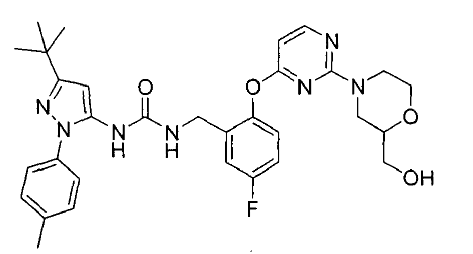

Morpholin-2-ylmethanol (32 mg) and N,N-diisopropylethylamine (0.1 mL) were added to a solution of 1-(2-(2-chloropyrimidin-4-yloxy)-5-fluorobenzyl)-3-(3-t-butyl-1-p-tolyl-1H-pyrazol-5-yl)urea (114 mg) in ethanol (2.0 mL), and the resulting mixture was stirred at room temperature for 1 day. Saturated aqueous sodium bicarbonate solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by silica gel column chromatography (ethyl acetate/n-hexane = 1/1 to 1/0) to obtain the desired product (116 mg, yield: 88%).
¹H-NMR (CDCl₃):δ 8.16 (d, 1H, J = 5.6 Hz), 7.33 (d, 2H, J = 8.2 Hz), 7.23 (d, 2H, J = 8.2 Hz), 7.03-6.91 (m, 3H), 6.21 (s, 1H), 6.11 (bs, 1H), 6.03 (d, 1H, J=5.6 Hz), 5.24 (t, 1H, J=6.2 Hz), 4.30 (m, 2H), 4.25 (br, 2H), 3.92 (m, 1H), 3.52 (m, 4H), 2.98 (t, 1H, J=7.7Hz), 2.75 (m, 1H), 2.38 (s, 3H), 2.20 (br, 1H), 1.33 (s, 9H)
MS (ESI):590 (M+H⁺), 588 (M-H⁺)

Example 77

### 1-(5-t-butyl-2-p-tolyl-2H-pyrazol-3-yl)-3-(2-(2-chloro-5-nitropyrimidin-4-yloxy)benzyl)urea

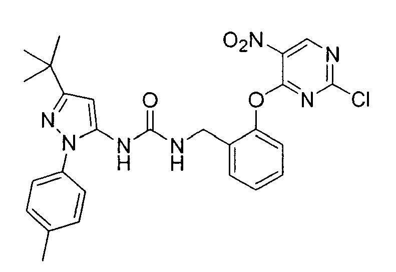

Sodium hydroxide (130 mg) was added to a solution of 1-(2-hydroxybenzyl)-3-(3-t-butyl-1-p-tolyl-1H-pyrazol-5-yl)urea (995 mg) and 2,4-dichloro-5-nitropyrimidin (595 mg) in acetone (10 mL) and water (6.0 mL), and the resulting mixture was stirred at room temperature for 20 hours. Aqueous 5% citric acid solution was added to the reaction mixture, and the resulting mixture was extracted with ethyl acetate. The extract was dried over anhydrous sodium sulfate, and was evaporated under reduced pressure. The obtained residue was purified by silica gel column chromatography (ethyl acetate/n-hexane = 1/4 → 3/2) to obtain the desired product (690 mg, yield: 49%).
¹H-NMR (CDCl₃):δ 9.14 (s, 1H), 7.45-7.15 (m, 8H), 6.20 (s, 1Hz), 6.03 (s, 1H), 5.40 (t, 1H, J = 6.1 Hz), 4.33 (d, 2H, J = 6.1 Hz), 2.35 (s, 3H), 1.32 (s, 9H)
MS (ESI):536, 538 (M+H⁺), 534, 536 (M-H⁺)

Example 78

### 1-(5-t-butyl-2-p-tolyl-2H-pyrazol-3-yl)-3-(2-(6-(2-(1-methylpyrrolidin-2-yl)ethylamino)pyrimidin-4-yloxy)benzyl)urea

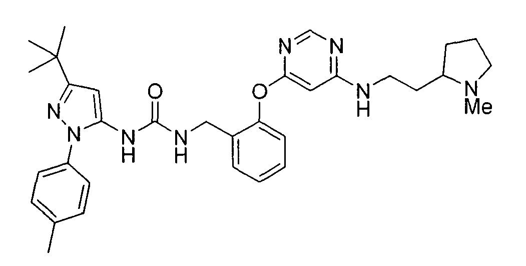

To a solution of 1-(2-(6-chloropyrimidin-4-yloxy)benzyl)-3-(3-t-butyl-1-p-tolyl-1H-pyrazol-5-yl)urea (191 mg) in ethanol (2.0 mL), 2-(1-methylpyrrolidin-2-yl)ethylamine (141 mg) and N,N-diisopropylethylamine (0.050 mL) were added, and the resulting mixture was stirred at room temperature for 3.5 hours and then at 65°C for 22 hours. Saturated aqueous sodium bicarbonate solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by amine silica gel column chromatography (ethyl acetate) to obtain the desired product (151 mg, yield: 67%).
¹H-NMR (CDCl₃):δ 8.04 (s, 1H), 7.36-7.17 (m, 7H), 7.03 (d, 1H, J=7.4Hz), 6.21 (s, 1H), 6.12 (bs, 2H), 5.67 (s, 1H), 5.49 (t, 1H, J=5.9Hz), 4.34 (d, 2H, J=5.9Hz), 3.30 (br, 2H), 3.07 (m, 1H), 2.37 (s, 3H), 2.32 (s, 3H), 2.27 (m, 1H), 2.20-1.70 (m, 6H), 2.15 (m, 1H), 1.32 (s, 9H)
MS (ESI):583 (M+H⁺)

Example 79

### 1-(5-t-butyl-2-p-tolyl-2H-pyrazol-3-yl)-3-(2-(6-(3-(2-oxopyrrolidin-1-yl)propylamino)pyrimidin-4-yloxy)benzyl)urea

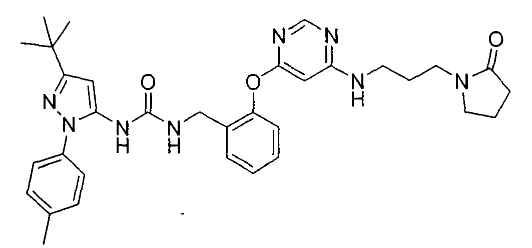

To a solution of 1-(2-(6-chloropyrimidin-4-yloxy)benzyl)-3-(3-t-butyl-1-p-tolyl-1H-pyrazol-5-yl)urea (400 mg) in ethanol (2.6 mL), 1-(3-aminopropyl)pyrrolidin-2-one (1.38 mmol) and N,N-diisopropylethylamine (0.428 mL) were added, and the resulting mixture was stirred at 50°C for 7 hours.
Saturated aqueous sodium bicarbonate solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by reverse-phase HPLC to obtain the desired product (186 mg, yield: 38%).
¹H-NMR (CDCl₃):δ 8.11 (s, 1H), 7.46 (dd, 1H, J=1.5, 7.6 Hz), 7.34-7.25 (m, 3H), 7.21 (dt, 1H, J=1.2, 7.5 Hz), 7.16 (d, 2H, J=8.0 Hz), 7.03 (dt, 1H, J=1.2, 8.0 Hz), 6.98 (s, 1H), 6.29 (s, 1H), 5.77 (s, 1H), 5.59 (s, 1H), 5.51 (t, 1H, J=6.0 Hz), 4.34 (d, 2H, J=6.0 Hz), 3.33 (t, 2H, J=7.1Hz), 3.29 (br, 2H), 3.24 (t, 2H, J=6.4Hz), 2.35 (s, 3H), 2.22 (t, 2H, J=8.1Hz), 1.99 (m, 2H), 1.73 (m, 2H), 1.32 (s, 9H)
MS (ESI):597 (M+H⁺), 595 (M-H⁺)

Example 80

### 1-(5-t-butyl-2-p-tolyl-2H-pyrazol-3-yl)-3-(2-(6-(4-methylpiperazin-1-yl)pyrimidin-4-yloxy)benzyl)urea

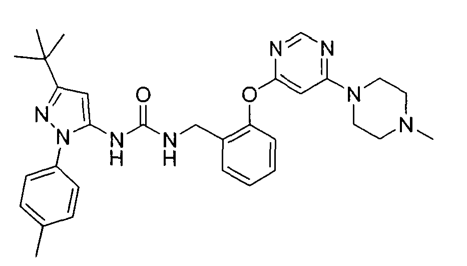

To a solution of 1-(2-(6-chloropyrimidin-4-yloxy)benzyl)-3-(3-t-butyl-1-p-tolyl-1H-pyrazol-5-yl)urea (400 mg) in ethanol (2.6 mL), 1-methylpiperazine (1.38 mmol) and N,N-diisopropylethylamine (0.428 mL) were added, and the resulting mixture was stirred at 50°C for 7 hours. Saturated aqueous sodium bicarbonate solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by reverse-phase HPLC to obtain the desired product (385 mg, yield: 85%).
¹H-NMR (CDCl₃):δ 8.07 (s, 1H), 7.36-7.17 (m, 7H), 7.02 (d, 1H, J=8.1 Hz), 6.21 (s, 1H), 5.99 (bs, 1H), 5.93 (s, 1H), 5.45 (t, 1H, J=5.6Hz), 4.34 (d, 2H, J=5.6Hz), 3.62 (br, 4H), 2.45 (t, 4H, J=5.1Hz), 2.37 (s, 3H), 2.34 (s, 3H), 1.33 (s, 9H)
MS (ESI):555 (M+H⁺)

Example 81

### 1-(6-(2-(3-(5-t-butyl-2-p-tolyl-2H-pyrazol-3-yl)-ureidomethyl)phenoxy)pyrimidin-4-yl)piperidin-4-carboxylic amide

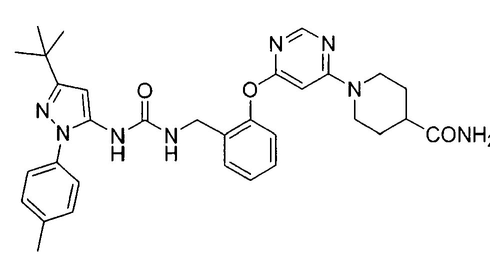

Piperidin-4-carboxylic amide (1.38 mmol) and N,N-diisopropylethylamine (0.428 mL) were added to a solution of 1-(2-(6-chloropyrimidin-4-yloxy)benzyl)-3-(3-t-butyl-1-p-tolyl-1H-pyrazol-5-yl)urea (400 mg) in ethanol (2.6 mL), and the resulting mixture was stirred at 50°C for 7 hours. Saturated aqueous sodium bicarbonate solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by reverse-phase HPLC to obtain the desired product (326 mg, yield: 69%).
¹H-NMR (CDCl₃):δ 8.06 (s, 1H), 7.34-7.14 (m, 7H), 7.02 (d, 1H, J=8.0 Hz), 6.69 (bs, 1H), 6.49 (bs, 1H), 6.24 (s, 1H), 5.82 (bs, 1H), 5.75 (m, 1H), 5.33 (s, 1H), 4.35 (dd, 1H, J=6.0, 14.8 Hz), 4.19 (dd, 1H, J=5.2, 14.8 Hz), 4.00 (m, 2H), 3.32 (m, 1H), 3.07 (m, 1H), 2.33 (s, 3H), 2.12 (m, 1H), 1.88 (m, 2H), 1.66 (m, 1H), 1.45 (m, 1H), 1.31 (s, 9H),
MS (ESI):583 (M+H⁺), 581 (M-H⁺)

Example 82

### 1-(6-(2-(3-(5-t-butyl-2-p-tolyl-2H-pyrazol-3-yl)-ureidomethyl)phenoxy)pyrimidin-4-yl)piperidin-3-carboxylic amide

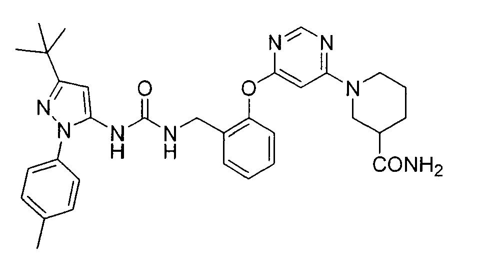

Piperidin-3-carboxylic amide (1.38 mmol) and N,N-diisopropylethylamine (0.428 mL) were added to a solution of 1-(2-(6-chloropyrimidin-4-yloxy)benzyl)-3-(3-t-butyl-1-p-tolyl-1H-pyrazol-5-yl)urea (400 mg) in ethanol (2.6 mL), and the resulting mixture was stirred at 50°C for 7 hours. Saturated aqueous sodium bicarbonate solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by reverse-phase HPLC to obtain the desired product (414 mg, yield: 87%).
¹H-NMR (CDCl₃):δ 8.08 (s, 1H), 7.34-7.15 (m, 7H), 7.02 (d, 1H, J=8.0 Hz), 6.27 (bs, 1H), 6.21 (bs, 1H), 5.92 (s, 1H), 5.54 (s, 1H), 5.52 (t, 1H, J=5.6 Hz), 4.33 (s, 1H), 4.32 (d, 2H, J=5.6 Hz), 3.76 (m, 2H), 2.95 (m, 2H), 2.42 (m, 1H), 2.36 (s, 3H), 1.89 (m, 2H), 1.69 (m, 2H), 1.32 (s, 9H),
MS (ESI):583 (M+H⁺)

Example 83

### 1-(5-t-butyl-2-p-tolyl-2H-pyrazol-3-yl)-3-(2-(6-(2,3-dihydroxypropyl)methylamino)pyrimidin-4-yloxy)benzyl)urea

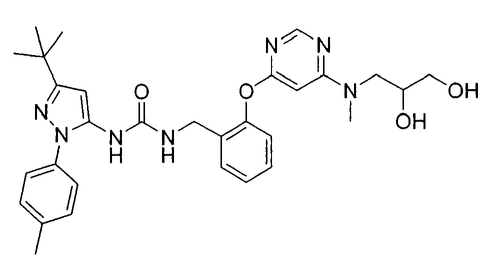

To a solution of 1-(2-(6-chloropyrimidin-4-yloxy)benzyl)-3-(3-t-butyl-1-p-tolyl-1H-pyrazol-5-yl)urea (400 mg) in ethanol (2.6 mL), 3-methylaminopropan-1,2-diol (1.38 mmol) and N,N-diisopropylethylamine (0.428 mL) were added and the resulting mixture was stirred at 50°C for 7 hours. Saturated aqueous sodium bicarbonate solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by reverse-phase HPLC to obtain the desired product (320 mg, yield: 70%).
¹H-NMR (CDCl₃):δ 8.05 (s, 1H), 7.36-7.17 (m, 7H), 7.03 (d, 1H, J=8.0 Hz), 6.21 (s, 1H), 6.04 (bs, 1H), 5.87 (s, 1H), 5.40 (t, 1H, J=5.6 Hz), 4.35 (d, 2H, J=5.6 Hz), 3.83 (m, 1H), 3.56-3.40 (m, 4H), 3.02 (s, 3H), 2.37 (s, 3H), 1.32 (s, 9H)
MS (ESI):560 (M+H⁺)

Example 84

### 1-(5-t-butyl-2-p-tolyl-2H-pyrazol-3-yl)-3-(2-(6-(4-hydroxymethylpiperidin-1-yl)pyrimidin-4-yloxy)benzyl)urea

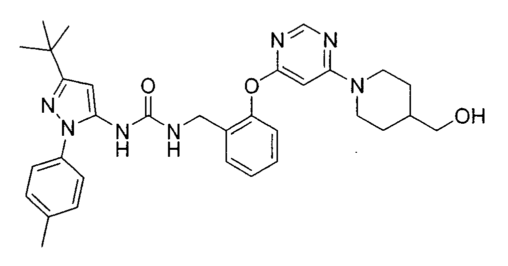

Piperidin-4-ylmethanol (1.38 mmol) and N,N-diisopropylethylamine (0.428 mL) were added to a solution of 1-(2-(6-chloropyrimidin-4-yloxy)benzyl)-3-(3-t-butyl-1-p-tolyl-1H-pyrazol-5-yl)urea (400 mg) in ethanol (2.6 mL), and the resulting mixture was stirred at 50°C for 7 hours. Saturated aqueous sodium bicarbonate solution was added to the reaction mixture, and the resulting mixture was extracted with ethyl acetate. The extract was washed with water and saturated brine, and was dried over anhydrous sodium sulfate, followed by evaporation under reduced pressure. The obtained residue was purified by reverse-phase HPLC to obtain the desired product (400 mg, yield: 86%).
¹H-NMR (CDCl₃):δ 8.07 (s, 1H), 7.35-7.03 (m, 7H), 7.02 (d, 1H, J=8.0 Hz), 6.21 (s, 1H), 6.06 (bs, 1H), 5.94 (s, 1H), 5.46 (t, 1H, J=5.8 Hz), 4.35 (bs, 1H), 4.34 (d, 2H, J=5.8 Hz), 3.52 (t, 2H, J=5.6 Hz), 2.85 (t, 2H, J=6.5 Hz), 2.37 (s, 3H), 1.90-1.80 (m, 4H), 1.33 (s, 9H), 1.25-1.15 (m, 1H)
MS (ESI):570 (M+H⁺)

Example 85

### 1-(2-(2-chloropyrimidin-4-yloxy)-3-methyl-5-fluorobenzyl)-3-(3-t-butyl-1-p-tolyl-1H-pyrazol-5-yl)urea

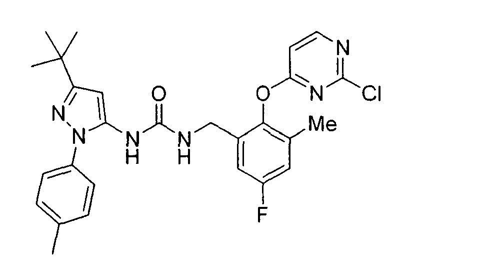

A solution of 2,4-dichloropyrimidine (0.95 g) in acetone (10 mL) was cooled to 0°C, and 1-(2-hydroxy-3-methyl-5-fluorobenzyl)-3-(3-t-butyl-1-p-tolyl-1H-pyrazol-5-yl)urea (2.31 g) and aqueous 1N sodium hydroxide solution (7.0 mL) were added thereto. This reaction mixture was allowed to warm to room temperature, and the reaction mixture was stirred for 24 hours. The reaction mixture was poured into aqueous 5% citric acid solution, and the resulting mixture was extracted with ethyl acetate. The extract was washed with water and saturated brine, and was dried over anhydrous sodium sulfate, followed by evaporation under reduced pressure. The obtained residue was purified by silica gel column chromatography (ethyl acetate/n-hexane = 1/2) to obtain the desired product (2.94 mg, yield: 100%).
¹H-NMR (CDCl₃):δ 8.43 (d, 1H, J = 5.6 Hz), 7.31 (d, 2H, J = 8.3 Hz), 7.21 (d, 2H, J = 8.3 Hz), 6.89 (dd, 1H, J = 3.2, 8.6 Hz), 6.80 (d, 1H, J = 5.6 Hz), 6.77 (dd, 1H, J = 3.2, 8.6 Hz), 6.61 (s, 1H), 6.23 (s, 1H), 5.34 (t, 1H, J = 5.9 Hz), 4.28 (d, 2H, J = 5.9 Hz), 2.36 (s, 3H), 2.08 (s, 3H), 1.25 (s, 9H)
MS (ESI):523, 525 (M+H⁺), 521, 523 (M-H⁺)

Example 86

### 1-(2-(2-chloropyrimidin-4-yloxy)-3,5-difluorobenzyl)-3-(3-t-butyl-1-p-tolyl-1H-pyrazol-5-yl)urea

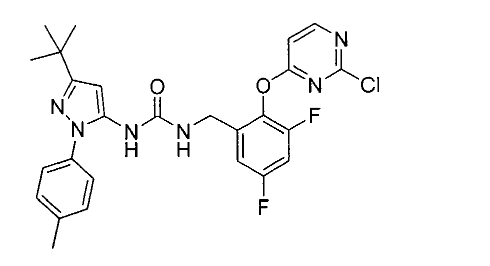

A solution of 2,4-dichloropyrimidine (0.91 g) in acetone (10 mL) was cooled to 0°C, and 1-(2-hydroxy-3,5-difluorobenzyl)-3-(3-t-butyl-1-p-tolyl-1H-pyrazol-5-yl)urea (1.70 g) and aqueous 1N sodium hydroxide solution (6.0 mL) were added thereto. This reaction mixture was allowed to warm to room temperature, and the reaction mixture was stirred for 24 hours. The reaction mixture was poured into aqueous 5% citric acid solution, and the resulting mixture was extracted with ethyl acetate. The extract was washed with water and saturated brine, and was dried over anhydrous sodium sulfate, followed by evaporation under reduced pressure. The obtained residue was purified by silica gel column chromatography (ethyl acetate/n-hexane = 1/2) to obtain the desired product (2.07 g, yield: 94%).
¹H-NMR (CDCl₃):δ 8.47 (d, 1H, J = 5.6 Hz), 7.31 (d, 2H, J = 8.0 Hz), 7.22 (d, 2H, J = 8.0 Hz), 6.93 (d, 1H, J = 5.6 Hz), 6.87 (m, 1H), 6.75 (m, 1H), 6.21 (s, 2H), 5.22 (t, 1H, J = 6.0 Hz), 4.31 (d, 2H, J = 6.0 Hz), 2.37 (s, 3H), 1.33 (s, 9H)
MS (ESI):526, 528 (M+H⁺), 524, 526 (M-H⁺)

Example 87

### 1-(2-(2-morphol inopyrimidin-4-yloxy)-3-methyl-5-fluorobenzyl)-3-(3-t-butyl-1-p-tolyl-1H-pyrazol-5-yl)urea

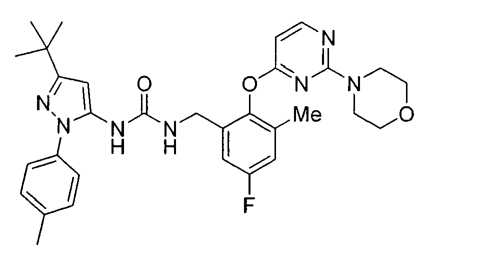

To a solution of 1-(2-(2-chloropyrimidin-4-yloxy)-3-methyl-5-fluorobenzyl)-3-(3-t-butyl-1-p-tolyl-1H-pyrazol-5-yl)urea (342 mg) in ethanol (2.0 mL), morpholine (0.50 mL) was added, and the resulting mixture was stirred at room temperature for 6 hours. The reaction mixture was poured into aqueous 5% citric acid solution, and the resulting mixture was extracted with ethyl acetate. The extract was washed with water and saturated brine, and was dried over anhydrous sodium sulfate, followed by evaporation under reduced pressure. The obtained residue was purified by silica gel column chromatography (ethyl acetate/n-hexane = 1/1 to 1/0) to obtain the desired product (192 mg, yield: 51 %).
¹H-NMR (CDCl₃):δ 8.15 (d, 1H, J = 5.6 Hz), 7.32 (d, 2H, J = 8.2 Hz), 7.23 (d, 2H, J = 8.2 Hz), 6.86 (dd, 1H, J = 3.2, 8.8 Hz), 6.80 (dd, 1H, J = 2.8, 8.6 Hz), 6.21 (s, 1H), 5.98 (d, 1H, J = 5.6 Hz), 5.97 (s, 1H), 5.16 (t, 1H, J = 6.1 Hz), 4.23 (d, 2H, J = 6.1 Hz), 3.65 (m, 4H), 3.56 (br, 4H), 2.38 (s, 3H), 2.08 (s, 3H), 1.34 (s, 9H).
MS (ESI):574 (M+H⁺), 572 (M-H⁺)

Example 88

### 1-(2-(2-morpholinopyrimidin-4-yloxy)-3,5-difluorobenzyl)-3-(3-t-butyl-1-p-tolyl-1H-pyrazol-5-yl)urea

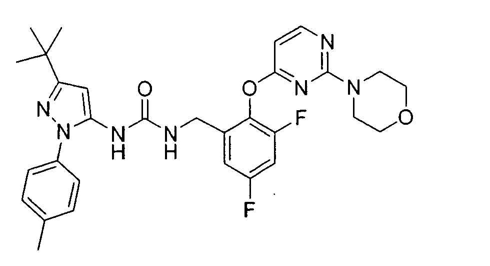

To a solution of 1-(2-(2-chloropyrimidin-4-yloxy)-3,5-difluorobenzyl)-3-(3-t-butyl-1-p-tolyl-1H-pyrazol-5-yl)urea (272 mg) in ethanol (2.0 mL), morpholine (0.50 mL) was added, and the resulting mixture was stirred at room temperature for 24 hours. The reaction mixture was poured into aqueous 5% citric acid solution, and the resulting mixture was extracted with ethyl acetate. The extract was washed with water and saturated brine, and was dried over anhydrous sodium sulfate, followed by evaporation under reduced pressure. The obtained residue was purified by silica gel column chromatography (ethyl acetate/n-hexane = 1/1 to 1/0) to obtain the desired product (226 mg, yield: 76%).
¹H-NMR (CDCl₃):δ 8.20 (d, 1H, J = 5.6 Hz), 7.31 (d, 2H, J = 8.5 Hz), 7.21 (d, 2H, J = 8.5 Hz), 6.84 (m, 1H), 6.77 (m, 1H), 6.19 (s, 1H), 6.14 (d, 1H, J = 5.6 Hz), 6.01 (s, 1H), 5.16 (t, 1H, J = 6.2 Hz), 4.31 (d, 2H, J = 6.2 Hz), 3.63 (m, 4H), 3.54 (br, 4H), 2.38 (s, 3H), 1.34 (s, 9H)
MS (ESI):578 (M+H⁺), 576 (M-H⁺)

Example 89

### 1-(2-((2-chloropyrimidin-4-yl)methyl)benzyl)-3-(3-t-butyl-1-p-tolyl-1H-pyrazol-5-yl)urea

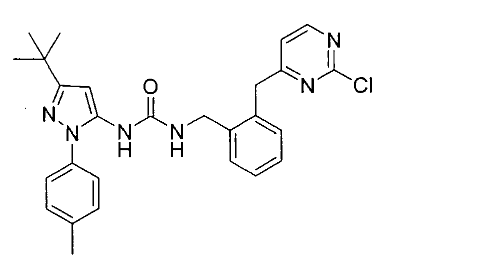

To a solution of (2-((2-chloropyrimidin-4-yl)methyl)phenyl)methanamine (106 mg) in acetonitrile (5 mL), 2,2,2-trichloroethyl 3-t-butyl-1-p-tolyl-1H-pyrazol-5-yl carbamate (220 mg) and diisopropylethylamine (158 µL) were added, and the resulting mixture was stirred at 60°C for 2 hours. To the reaction solution, aqueous 1N sodium hydroxide solution was added, and the resulting mixture was extracted with ethyl acetate. The organic layer was washed with saturated aqueous sodium bicarbonate solution and saturated brine, and was dried over magnesium sulfate.
The organic layer was filtered, and the obtained filtrate was evaporated under reduced pressure. The resultant residue was purified by silica gel column chromatography (ethyl acetate/n-hexane = 3/1) to obtain the desired product (74 mg, yield: 33%).
¹H-NMR (CDCl₃):δ 8.48 (d, 1H, J = 4.9 Hz), 7.33-7.35 (m, 1H), 7.22-7.27 (m, 4H), 7.12-7.18 (m, 4H), 6.38 (brs, 1H), 6.35 (s, 1H), 6.28 (brs, 1H), 4.45 (d, 2H, J = 5.4 Hz), 4.08 (s, 2H), 2.35(s, 3H), 1.32(s, 9H)
MS (ESI):489 (M+H⁺)

Example 90

### 1-(2-((2-morpholinopyrimidin-4-yl)methyl)benzyl)-3-(3-t-butyl-1-p-tolyl-1 H-pyrazol-5-yl)urea

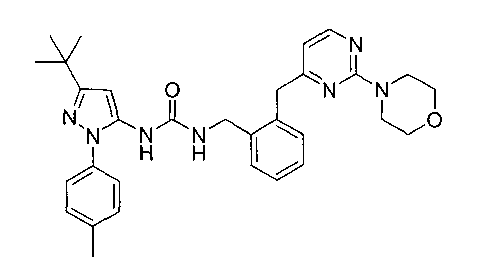

Morpholine (22 µL) and diisopropylethylamine (21 µL) were added to a solution of 1-(2-((2-chloropyrimidin-4-yl)methyl)benzyl)-3-(3-t-butyl-1-p-tolyl-1H-pyrazol-5-yl)urea (17 mg) in acetonitrile (2 mL), and the resulting mixture was stirred at 60°C for 2hours. Saturated aqueous sodium bicarbonate solution was added to the reaction solution, and the resulting mixture was extracted with ethyl acetate. The organic layer was washed with saturated brine, and was dried over magnesium sulfate. The organic layer was filtered, and the obtained filtrate was evaporated under reduced pressure. The resultant residue was purified by silica gel column chromatography (ethyl acetate/n-hexane = 5/1) to obtain the desired product (16 mg, yield: 86%).
¹H-NMR (CDCl₃):δ 8.14 (d, 1H, J = 5.1 Hz), 7.18-7.30(m, 8H), 6.26 (d, 1H, J = 4.9), 6.22 (s, 1H), 6.00 (s, 1H), 5.25 (brs, 1H), 4.44 (d, 2H, J = 5.6 Hz), 3.93 (s, 2H), 3.69 (s, 8H), 2.37 (s, 3H), 1.32(s, 9H)
MS (ESI):540 (M+H⁺)

Example 91

### 1-(2-(6-chloropyrimidin-4-yloxy)-5-fluorobenzyl)-3-(3-t-butyl-1-p-tolyl-1H-pyrazol-5-yl)urea

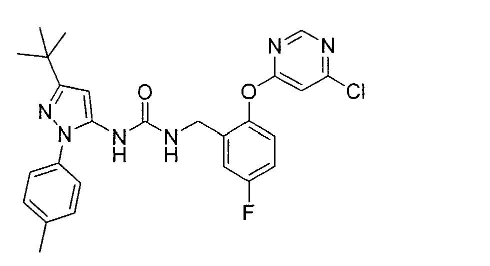

A solution of 4,6-dichloropyrimidine (254 mg) in DMF (3.5 mL) was cooled to 0°C, and 1-(5-fluoro-2-hydroxybenzyl)-3-(3-t-butyl-1-p-tolyl-1H-pyrazol-5-yl)urea (675 mg) and 1N aqueous sodium hydroxide solution (1.8 mL) were added thereto. This reaction mixture was heated to 50°C, and the reaction mixture was stirred overnight. Saturated ammonium chloride was added to the reaction mixture, and the resulting mixture was extracted with ethyl acetate. The extract was dried over anhydrous sodium sulfate, and was evaporated under reduced pressure. The obtained residue was purified by silica gel column chromatography (ethyl acetate/n-hexane = 1/2) to obtain the desired product (648 mg, yield: 75%).
¹H-NMR (CDCl₃):δ 8.42 (s, 1H), 7.31-6.94 (m, 8H), 6.18 (s, 1H), 6.07 (s, 1H), 5.18 (t, 1H, J = 5.9 Hz), 4.25 (d, 2H, J = 5.9 Hz), 2.37 (s, 3H), 1.34 (s, 9H)
MS (ESI):509 (M+H⁺)

Example 92

### 1-(5-fluoro-2-(6-morpholinopyrimidin-4-yloxy)benzyl)-3-(3-t-butyl-1-p-tolyl-1 H-pyrazol-5-yl)urea

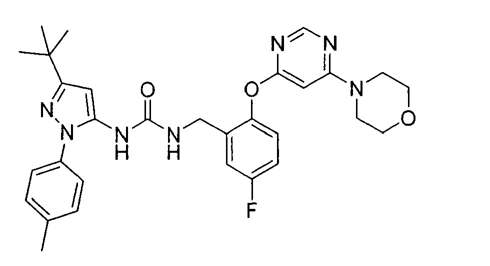

Morpholine (0.03 mL) and sodium carbonate (20 mg) were added to a solution of 1-(2-(6-chloropyrimidin-4-yloxy)-5-fluorobenzyl)-3-(3-t-butyl-1-p-tolyl-1H-pyrazol-5-yl)urea (93 mg) in ethanol (0.5 mL), and the resulting mixture was stirred at room temperature overnight. The reaction solution was filtered, and washed with a small amount of ethanol. The obtained filtrate was evaporated under reduced pressure, and the resultant residue was purified by silica gel column chromatography (ethyl acetate/n-hexane = 1/2) to obtain the desired product (83 mg, yield: 81%).
¹H-NMR (CDCl₃):8 8.07 (s, 1H), 7.29-6.98 (m, 7H), 6.20 (s, 1H), 6.06 (s, 1H), 5.91 (s, 1H), 5.41 (t, 1H, J = 5.9 Hz), 4.28 (d, 2H, J = 5.9 Hz), 3.77-3.74 (m, 4H), 3.58-3.56 (m, 4H), 2.37 (s, 3H), 1.33 (s, 9H)
MS (ESI):560 (M+H⁺)

Example 93

### 1-(2-(2-chloro-5-methylpyrimidin-4-yloxy)benzyl)-3-(3-t-butyl-1-p-tolyl-1H-pyrazol-5-yl)urea

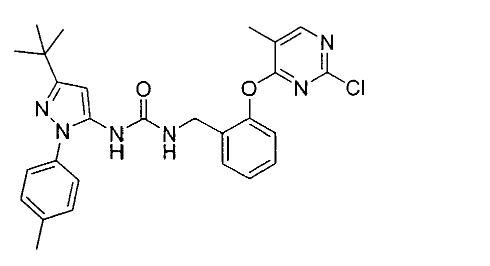

A solution of 2,4-dichloro-5-methylpyrimidine (82 mg) in acetone (1mL) was cooled to 0°C, and 1-(2-hydroxybenzyl)-3-(3-t-butyl-1-p-tolyl-1H-pyrazol-5-yl)urea (189 mg) and aqueous 1N sodium hydroxide solution (0.6 mL) were added thereto. This reaction mixture was allowed to warm to room temperature and stirred overnight. Saturated ammonium chloride was added to the reaction mixture, and the resulting mixture was extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (168 mg, yield: 67%).
¹H-NMR (CDCl₃):δ 8.25 (s, 1H), 7.37-7.04 (m, 8H), 6.23 (s, 1H), 6.05 (s, 1H), 5.22 (t, 1H, J = 5.6 Hz), 4.31 (d, 2H, J = 5.6 Hz), 2.35 (s, 3H), 2.29 (s, 3H), 1.32 (s, 9H)
MS (ESI):505 (M+H⁺)

Example 94

### 1-(2-(5-methyl-2-morpholinopyrimidin-4-yloxy)benzyl)-3-(3-t-butyl-1-p-tolyl-1H-pyrazol-5-yl)urea

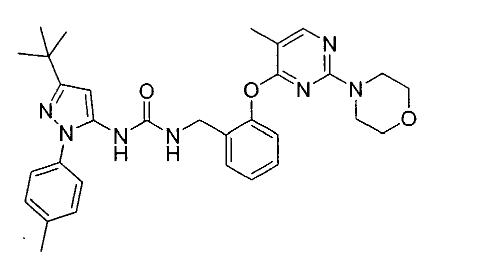

Morpholine (0.03 mL) and sodium carbonate (28 mg) were added to a solution of 1-(2-(2-chloro-5-methylpyridin-4-yloxy)benzyl)-3-(3-t-butyl-1-p-tolyl-1H-pyrazol-5-yl)urea (130 mg) in ethanol (3 mL), and the resulting mixture was stirred at 60°C for 3 hours. The reaction solution was filtered, and washed with a small amount of ethanol. The obtained filtrate was evaporated under reduced pressure, and the resultant residue was purified by silica gel column chromatography (ethyl acetate/n-hexane = 1/2) to obtain the desired product (141 mg, yield: 99%). ¹H-NMR (CDCl₃):δ 8.01 (s, 1H), 7.31-7.08 (m, 8H), 6.18 (s, 1H), 5.94 (s, 1H), 5.13 (t, 1H, J = 5.9 Hz), 4.34 (d, 2H, J = 5.9 Hz), 3.60-3.58 (m, 4H), 3.44-3.42 (m, 4H), 2.37 (s, 3H), 2.10 (s, 3H), 1.31 (s, 9H)
MS (ESI):556 (M+H⁺)

Example 95

### 1-(2-(2-chloro-6-methylpyrimidin-4-yloxy)benzyl)-3-(3-t-butyl-1-p-tolyl-1 H-pyrazol-5-yl)urea

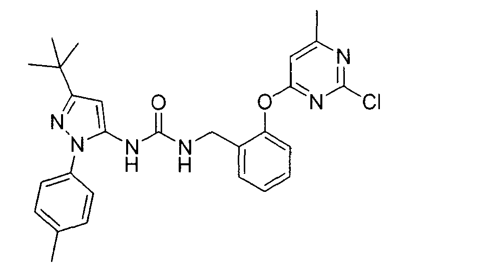

A solution of 2,4-dichloro-6-methylpyrimidine (82 mg) in acetone (1mL) was cooled to 0°C, and 1-(2-hydroxybenzyl)-3-(3-t-butyl-1-p-tolyl-1H-pyrazol-S-yl)urea (189 mg) and aqueous 1N sodium hydroxide solution (0.6 mL) were added thereto. This reaction mixture was allowed to warm to room temperature and stirred overnight. Saturated ammonium chloride was added to the reaction mixture, and the resulting mixture was extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (156 mg, yield: 62%).
¹H-NMR (CDCl₃):δ 7.36-7.03 (m, 8H), 6.62 (s, 1H), 6.25 (s, 1H), 6.06 (s, 1H), 5.28 (t, 1H, J = 5.6 Hz), 4.31 (d, 2H, J = 5.6 Hz), 2.46 (s, 3H), 2.37 (s, 3H), 1.32 (s, 9H)
MS (ESI):505 (M+H⁺)

Example 96

### 1-(2-(6-chloro-2-methylpyrimidin-4-yloxy)benzyl)-3-(3-t-butyl-1-p-tolyl-1H-pyrazol-5-yl)urea

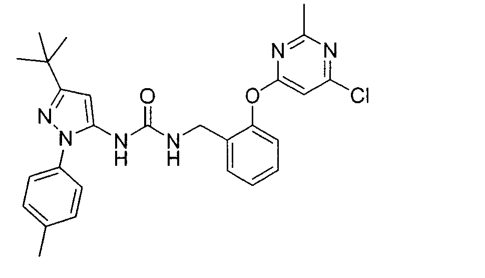

A solution of 4,6-dichloro-2-methylpyrimidine (82 mg) in acetone (1mL) was cooled to 0°C, and 1-(2-hydroxybenzyl)-3-(3-t-butyl-1-p-tolyl-1H-pyrazol-5-yl)urea (189 mg) and aqueous 1N sodium hydroxide solution (0.6 mL) were added thereto. This reaction mixture was allowed to warm to room temperature and stirred overnight. Saturated ammonium chloride was added to the reaction mixture, and the resulting mixture was extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (226 mg, yield: 90%).
¹H-NMR (CDCl₃):δ 7.36-7.04 (m, 8H), 6.61 (s, 1H), 6.20 (s, 1H), 6.04 (s, 1H), 5.23 (t, 1H, J = 5.9 Hz), 4.31 (d, 2H, J = 5.9 Hz), 2.48 (s, 3H), 2.37 (s, 3H), 1.32 (s, 9H)
MS (ESI):505 (M+H⁺)

Example 97

### 1-(2-(6-chloro-5-methylpyrimidin-4-yloxy)benzyl)-3-(3-t-butyl-1-p-tolyl-1 H-pyrazol-5-yl)urea

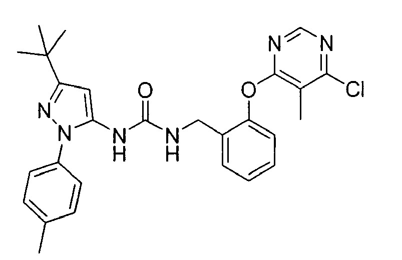

A solution of 4,6-dichloro-5-methylpyrimidine (82 mg) in acetone (1mL) was cooled to 0°C, and 1-(2-hydroxybenzyl)-3-(3-t-butyl-1-p-tolyl-1H-pyrazol-5-yl)urea (189 mg) and aqueous 1N sodium hydroxide solution (0.6 mL) were added thereto. This reaction mixture was allowed to warm to room temperature and stirred overnight. Saturated ammonium chloride was added to the reaction mixture, and the resulting mixture was extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (166 mg, yield: 66%).
¹H-NMR (CDCl₃):δ 8.19 (s, 1H), 7.37-7.02 (m, 8H), 6.14 (s, 1H), 5.95 (s, 1H), 5.11 (t, 1H, J = 5.6 Hz), 4.31 (d, 2H, J = 5.6 Hz), 2.39 (s, 3H), 2.37 (s, 3H), 1.32 (s, 9H)
MS (ESI):505 (M+H⁺)

Example 98

### 1-(2-(6-methyl-2-morpholinopyrimidin-4-yloxy)benzyl)-3-(3-t-butyl-1-p-tolyl-1 H-pyrazol-5-yl)urea

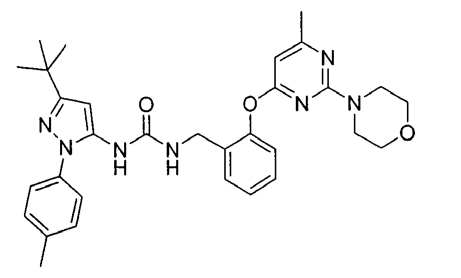

Morpholine (0.03 mL) and sodium carbonate (28 mg) were added to a solution of 1-(2-(2-chloro-6-methylpyrimidin-4-yloxy)benzyl)-3-(3-t-butyl-1-p-tolyl-1H-pyrazol-5-yl)urea (130 mg) in ethanol (0.3 mL), and the resulting mixture was stirred at 60°C for 3 hours. The reaction solution was filtered, and washed with a small amount of ethanol. The obtained filtrate was evaporated under reduced pressure, and the resultant residue was purified by silica gel column chromatography (ethyl acetate/n-hexane = 1/2) to obtain the desired product (132 mg, yield: 92%).
¹H-NMR (CDCl₃):δ 7.33-7.05 (m, 8H), 6.20 (s, 1H), 5.95 (s, 1H), 5.81 (s, 1H), 5.25 (t, 1H, J = 5.9 Hz), 4.33 (d, 2H, J = 5.9 Hz), 3.66-3.61 (m, 8H), 2.37 (s, 3H), 2.25 (s, 3H), 1.32 (s, 9H)
MS (ESI):556 (M+H⁺)

Example 99

### 1-(2-(2-methyl-6-morpholinopyrimidin-4-yloxy)benzyl)-3-(3-t-butyl-1-p-tolyl-1 H-pyrazol-5-yl)urea

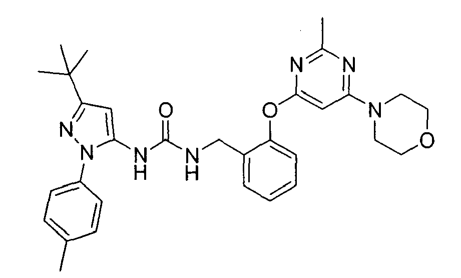

Morpholine (0.03 mL) and sodium carbonate (28 mg) were added to a solution of 1-(2-(6-chloro-2-methylpyrimidin-4-yloxy)benzyl)-3-(3-t-butyl-1-p-tolyl-1H-pyrazol-5-yl)urea (130 mg) in ethanol (0.3 mL), and the resulting mixture was stirred at 60°C for 3 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (91 mg, yield: 64%).
¹H-NMR (CDCl₃):δ 7.36-7.00 (m, 8H), 6.23 (s, 1H), 6.16 (s, 1H), 5.55 (s, 1H), 5.52 (t, 1H, J = 5.6 Hz), 4.32 (d, 2H, J = 5.6 Hz), 3.71-3.69 (m, 4H), 3.51-3.49 (m, 4H), 2.36 (s, 3H), 2.28 (s, 3H), 1.31 (s, 9H)
MS (ESI):556 (M+H⁺)

Example 100

### 1-(2-(5-methyl-6-morpholinopyrimidin-4-yloxy)benzyl)-3-(3-t-butyl-1-p-tolyl-1H-pyrazol-5-yl)urea

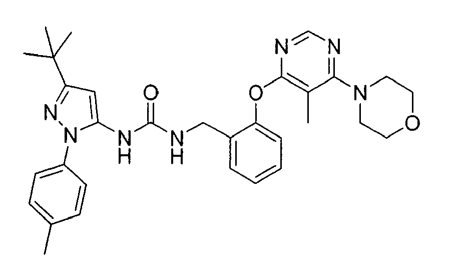

Morpholine (0.03 mL) and sodium carbonate (28 mg) were added to a solution of 1-(2-(6-chloro-5-methylpyrimidin-4-yloxy)benzyl)-3-(3-t-butyl-1-p-tolyl-1H-pyrazol-5-yl)urea (130 mg) in ethanol (0.3 mL), and the resulting mixture was stirred at 60°C overnight. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (90 mg, yield: 63%).
¹H-NMR (CDCl₃):δ 8.03 (s, 1H), 7.34-6.97 (m, 8H), 6.16 (s, 1H), 5.98 (s, 1H), 5.30 (t, 1H, J = 5.4 Hz), 4.32 (d, 2H, J = 5.4 Hz), 3.83-3.81 (m, 4H), 3.41-3.38 (m, 4H), 2.36 (s, 3H), 2.19 (s, 3H), 1.31 (s, 9H)
MS (ESI):556 (M+H⁺)

Example 101

### (R)-1-(5-fluoro-2-(2-(2-(hydroxymethyl)pyrrolidin-1-yl)pyrimidin-4-yloxy)benzyl)-3-(3-t-butyl-1-p-tolyl-1H-pyrazol-5-yl)urea

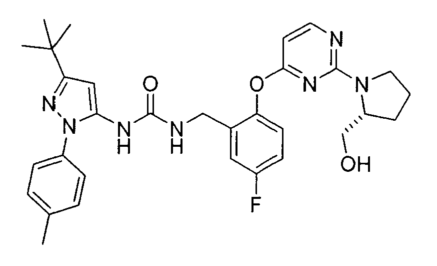

(R)-(-)-2-pyrrolidinemethanol (0.015 mL) and sodium carbonate (32 mg) were added to a solution of 1-(2-(2-chloropyrimidin-4-yloxy)-5-fluorobenzyl)-3-(3-t-butyl-1-p-tolyl-1H-pyrazol-5-yl)urea (51 mg) in ethanol (0.1 mL), and the resulting mixture was stirred at 60°C for 2 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (57 mg, yield: 99%).
¹H-NMR (CDCl₃):δ 8.10 (brs, 1H), 7.32-6.97 (m, 7H), 6.45 (brs, 1H), 6.24 (s, 1H), 6.07 (brs, 1H), 5.49 (brs, 1H), 4.30 (brs, 2H), 3.65-3.25 (m, 4H), 2.36 (s, 3H), 2.04-1.58 (m, 6H), 1.32 (s, 9H)
MS (ESI):574 (M+H⁺)

Example 102

### (S)-1-(5-fluoro-2-(2-(2-(hydroxymethyl)pyrrolidin-1-yl)pyrimidin-4-yloxy)benzyl)-3-(3-t-butyl-1-p-tolyl-1H-pyrazol-5-yl)urea

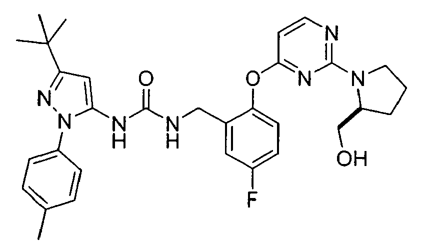

(S)-(+)-2-pyrrolidinemethanol (0.015 mL) and sodium carbonate (32 mg) were added to 1-(2-(2-chloropyrimidin-4-yloxy)-5 -fluorobenzyl)-3 -(3 -t-butyl-1-p-tolyl-1H-pyrazol-5-yl)urea (51 mg) in ethanol (0.1 mL), and the resulting mixture was stirred at 60°C for 2 hours. The reaction solution was filtered, and washed with a small amount of ethanol. The obtained filtrate was evaporated under reduced pressure, and the resultant residue was purified by silica gel column chromatography (ethyl acetate/n-hexane = 3/2) to obtain the desired product (57 mg, yield: 99%).
¹H-NMR (CDCl₃):δ 8.12 (brs, 1H), 7.32-6.97 (m, 7H), 6.46 (brs, 1H), 6.24 (s, 1H), 6.07 (brs, 1H), 5.52 (brs, 1H), 4.29 (brs, 2H), 3.60-3.17 (m, 4H), 2.36 (s, 3H), 2.04-1.56 (m, 6H), 1.32 (s, 9H).
MS (ESI):574 (M+H⁺).

Example 103

### (S)-methyl-1-(4-(2-((3-(3-t-butyl-1-p-tolyl-1 H-pyrazol-5-yl)ureido)methyl)-4-fluorophenoxy)pyrimidin-2-yl)pyrrolidin-2-carboxylate

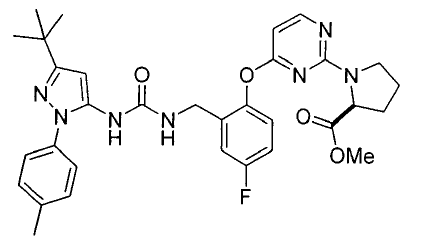

Triethylamine (0.02 mL) was added to a solution of L-proline methyl ester hydrochloric acid salt (25 mg) in ethanol (0.1 mL), and the resulting mixture was stirred. Thereafter, 1-(2-(2-chloropyrimidin-4-yloxy)-5-fluorobenzyl)-3-(3-t-butyl-1-p-tolyl-1H-pyrazol-5-yl)urea (51 mg) and sodium carbonate (32 mg) were added thereto, and the resulting mixture was stirred at 60°C for 2 hours. The reaction solution was filtered, and washed with a small amount of ethanol. The obtained filtrate was evaporated under reduced pressure, and the resultant residue was purified by silica gel column chromatography (ethyl acetate/n-hexane = 3/2) to obtain the desired product (88 mg, yield: 73%).
¹H-NMR (CDCl₃):δ 8.18 (d, 1H, J = 5.4 Hz), 7.31-6.95 (m, 7H), 6.90 (s, 1H), 6.37 (s, 1H), 6.28 (d, 1H, J = 5.4 Hz), 4.88 (t, 1H, J = 6.1 Hz), 4.27 (d, 2H, J = 6.1 Hz), 4.00-3.97 (m, 1H), 3.57-3.51 (m, 2H), 3.27 (s, 3H), 2.33 (s, 3H), 2.19-1.72 (m, 4H), 1.33 (s, 9H).
MS (ESI):602 (M+H⁺).

Example 104

### 1-(5-fluoro-2-(2-(4-oxopiperidin-1-yl)pyrimidin-4-yloxy)benzyl)-3-(3-t-butyl-1-p-tolyl-1H-pyrazol-5-yl)urea

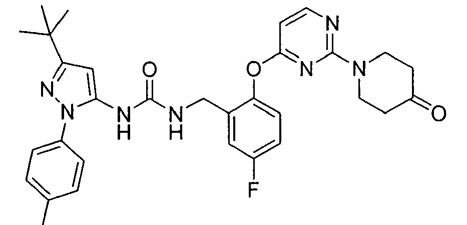

Triethylamine (0.02 mL) was added to a solution of 4-piperidone monohydrate hydrochloric acid salt (26 mg) in ethanol (0.1 mL), and the resulting mixture was stirred. Thereafter, 1-(2-(2-chloropyrimidin-4-yloxy)-5-fluorobenzyl)-3-(3-t-butyl-1-p-tolyl-1H-pyrazol-5-yl)urea (51 mg) and sodium carbonate (32 mg) were added thereto, and the resulting mixture was stirred at 60°C for 2 hours. The reaction solution was filtered, and washed with a small amount of ethanol. The obtained filtrate was evaporated under reduced pressure, and the resultant residue was purified by silica gel column chromatography (ethyl acetate/n-hexane = 3/2) to obtain the desired product (46 mg, yield: 80%).
¹H-NMR (CDCl₃):δ 8.20 (d, 1H, J = 5.6 Hz), 7.32-6.95 (m, 7H), 6.19 (s, 1H), 6.07 (s, 1H), 6.06 (d, 1H, J = 5.6 Hz), 5.20 (t, 1H, J = 5.9 Hz), 4.30 (d, 2H, J = 5.9 Hz), 3.91 (brs, 4H), 2.41-2.38 (m, 4H), 2.37 (s, 3H), 1.33 (s, 9H).
MS (ESI):572 (M+H⁺).

Example 105

### 1-(2-(2,6-dimethoxypyrimidin-4-yloxy)benzyl)-3-(3-t-butyl-1-p-tolyl-1 H-pyrazol-5-yl)urea

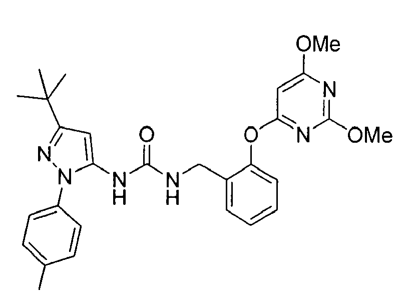

A solution of 6-chloro-2,4-dimethoxypyrimidine (88 mg) in acetone (1mL) was cooled to 0°C, and 1-(2-hydroxybenzyl)-3 -(3 -t-butyl-1-p-tolyl-1H-pyrazol-5-yl)urea (189 mg) and aqueous 1N sodium hydroxide solution (0.6 mL) were added thereto. This reaction mixture was allowed to warm to room temperature and stirred overnight., followed by further stirring of the reaction mixture at 60°C for 6hours. Saturated ammonium chloride was added to the reaction mixture, which was then extracted with ethyl acetate. The extract was dried over anhydrous sodium sulfate, and was evaporated under reduced pressure. The obtained residue was purified by silica gel column chromatography (ethyl acetate/n-hexane = 2/3) to obtain the desired product (21 mg, yield: 8%).
¹H-NMR (CDCl₃):δ 7.33-7.04 (m, 8H), 6.33 (s, 1H), 6.23 (s, 1H), 5.67 (s, 1H), 5.46 (t, 1H, J = 5.9 Hz), 4.34 (d, 2H, J = 5.9 Hz), 3.93 (s, 3H), 3.81 (s, 3H), 2.37 (s, 3H), 1.32 (s, 9H).
MS (ESI):517 (M+H⁺).

Example 106

### 1-(2-(2-(4-oxopiperidin-1-yl)pyrimidin-4-yloxy)benzyl)-3-(3 -t-butyl-1-p-tolyl- H-pyrazol-5-yl)urea

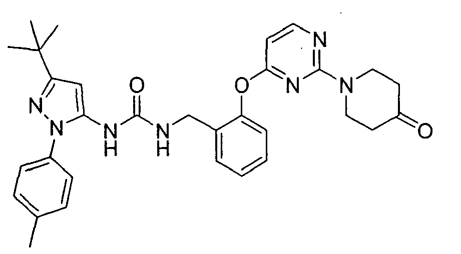

Triethylamine (0.45 mL) was added to a solution of 4-piperidone monohydrate hydrochloric acid salt (515 mg) in ethanol (2 mL), and the resulting mixture was stirred. Thereafter, 1-(2-(2-chloropyrimidin-4-yloxy)benzyl)-3-(3-t-butyl-1-p-tolyl-1H-pyrazol-5-yl)urea (982 mg) and sodium carbonate (212 mg) were added thereto, and the resulting mixture was stirred at 60°C for 2 hours. The reaction solution was filtered, and washed with a small amount of ethanol. The obtained filtrate was evaporated under reduced pressure, and the resultant residue was purified by silica gel column chromatography (ethyl acetate/n-hexane = 1/2 → 2/1) to obtain the desired product (996 mg, yield: 90%).
¹H-NMR (CDCl₃):δ 8.19 (d, 1H, J = 5.6 Hz), 7.33-7.06 (m, 8H), 6.20 (s, 1H), 6.03 (d, 1H, J = 5.6 Hz), 5.97 (s, 1H), 5.23 (t, 1H, J = 5.9 Hz), 4.35 (d, 2H, J = 5.9 Hz), 3.93-3.90 (m, 4H), 2.42-2.39 (m, 4H), 2.37 (s, 3H), 1.32 (s, 9H).
MS (ESI):554 (M+H⁺).

Example 107

### 1-(2-(2-chloropyrimidin-4-yloxy)benzyl)-3-(3-t-butyl-1-(4-isopropylphenyl)-1 H-pyrazol-5-yl)urea

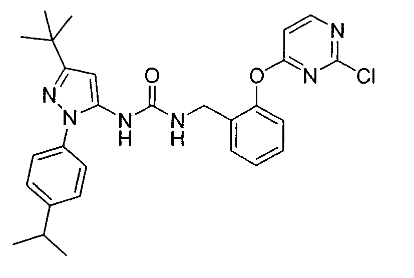

A solution of 2,4-dichloropyrimidine (149 mg) in acetone (1.1 mL) was cooled to 0°C, and 1-(2-hydroxybenzyl)-3-(3-t-butyl-1-(4-isopropylphenyl)-1H-pyrazol-5 -yl)urea (407 mg) and aqueous 1N sodium hydroxide solution (1.1 mL) were added thereto. This reaction mixture was allowed to warm to room temperature and stirred overnight. Saturated ammonium chloride was added to the reaction mixture, which was then extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (378 mg, yield: 73%).
¹H-NMR (CDCl₃):δ 8.43 (d, 1H, J = 5.6 Hz), 7.38-7.06 (m, 8H), 6.81 (d, 1H, J = 5.6 Hz), 6.24 (s, 1H), 6.08 (s, 1H), 5.29 (t, 1H, J = 5.9 Hz), 4.33 (d, 2H, J = 5.9 Hz), 2.93 (dq, 1H, J = 6.8, 6.8 Hz), 1.32 (s, 9H), 1.24 (d, 6H, J = 6.8 Hz).
MS (ESI):519 (M+H⁺).

Example 108

### 1-(2-(2-morpholinopyrimidin-4-yloxy)benzyl)-3-(3-t-butyl-1-(4-isopropylphenyl)-1 H-pyrazol-5-yl)urea

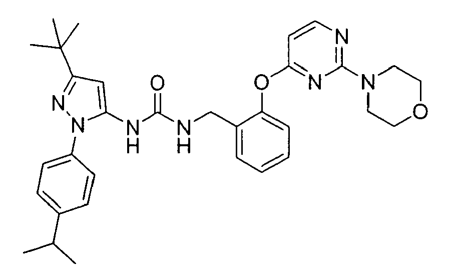

Morpholine (0.035 mL) and sodium carbonate (27 mg) were added to a solution of 1-(2-(2-chloropyrimidin-4-yloxy)benzyl)-3-(3-t-butyl-1-(4-isopropylphenyl)-1H-pyrazol-5-yl)urea (130 mg) in ethanol (0.5 mL), and the resulting mixture was stirred at 60°C for 5 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (141 mg, yield: 99%).
¹H-NMR (CDCl₃):δ 8.15 (d, 1H, J = 5.6 Hz), 7.36-7.07 (m, 8H), 6.20 (s, 1H), 5.97 (d, 1H, J = 5.6 Hz), 5.96 (s, 1 H), 5.26 (t, 1 H, J = 5.9 Hz), 4.35 (d, 2H, J = 5.9 Hz), 3.67-3.60 (m, 8H), 2.94 (dq, 1H, J = 6.8, 6.8 Hz), 1.33 (s, 9H), 1.25 (d, 6H, J = 6.8 Hz).
MS (ESI):570 (M+H⁺).

Example 109

### 1-(2-(2-chloropyrimidin-4-yloxy)benzyl)-3-(3-t-butyl-1-(4-methoxyphenyl)-1H-pyrazol-5-yl)urea

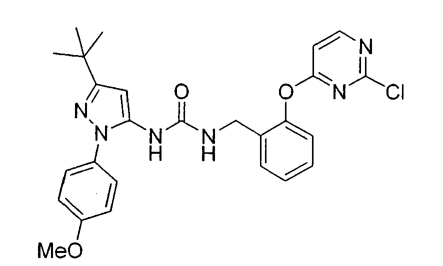

A solution of 2,4-dichloropyrimidine (149 mg) in acetone (1.1 mL) was cooled to 0°C, and 1-(2-hydroxybenzyl)-3-(3-t-butyl-1-(4-methoxylphenyl)-1H-pyrazol-5-yl)urea (395 mg) and aqueous 1N sodium hydroxide solution (1.1 mL) were added thereto. This reaction mixture was allowed to warm to room temperature and stirred overnight. Saturated ammonium chloride was added to the reaction mixture, which was then extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (438 mg, yield: 86%).
¹ H-NMR (CDCl₃):δ 8.42 (d, 1H, J = 5.9 Hz), 7.38-6.89 (m, 8H), 6.80 (d, 1H, J = 5.9 Hz), 6.21 (s, 1 H), 6.07 (s, 1H), 5.27 (t, 1 H, J = 5.9 Hz), 4.31 (d, 2H, J = 5.9 Hz), 3.82 (s, 3H), 1.32 (s, 9H).
MS (ESI):507 (M+H⁺).

Example 110

### 1-(2-(2-morpholinopyrimidin-4-yloxy)benzyl)-3-(3-t-butyl-1-(4-methoxyphenyl)-1 H-pyrazol-5-yl)urea

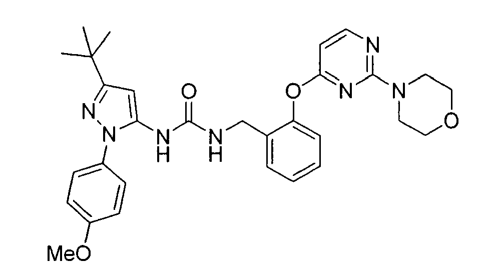

Morpholine (0.065 mL) and sodium carbonate (53 mg) were added to a solution of 1-(2-(2-chloropyrimidin-4-yloxy)benzyl)-3-(3-t-butyl-1-(4-methoxyphenyl)-1H-pyrazol-5-yl)urea (225 mg) in ethanol (1 mL), and the resulting mixture was stirred at 60°C for 3 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (256 mg, yield: 91%).
¹ H-NMR (CDCl₃ ):δ 8.15 (d, 1H, J = 5.6 Hz), 7.33-6.89 (m, 8H), 6.18 (s, 1H), 5.97 (d, 1H, J = 5.6 Hz), 5.96 (s, 1H), 5.24 (t, 1H, J = 5.9 Hz), 4.33 (d, 2H, J = 5.9 Hz), 3.82 (s, 3H), 3.66-3.59 (m, 8H), 1.32 (s, 9H).
MS (ESI):558 (M+H⁺).

Example 111

### Methyl 3-(5-(3-(2-(2-chloropyrimidin-4-yloxy)benzyl)ureido)-3-t-butyl-1H-pyrazol-1-yl) benzoate

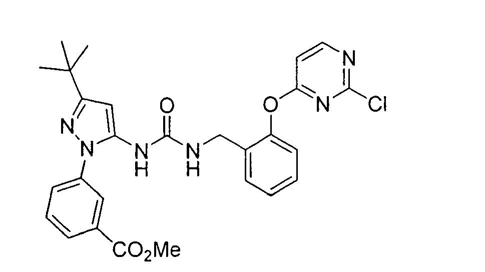

A solution of 2,4-dichloropyrimidine (127 mg) in acetone (0.9 mL) was cooled to 0°C, and methyl 3-(5-(3-(2-hydroxybenzyl)ureido)-3-t-butyl-1H-pyrazol-1-yl) benzoate (360 mg) and aqueous 1N sodium hydroxide solution (0.9 mL) were added thereto. This reaction mixture was allowed to warm to room temperature and stirred overnight. Saturated ammonium chloride was added to the reaction mixture, which was then extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (287 mg, yield: 63%).
¹H-NMR (CDCl₃):δ 8.41 (d, 1H, J = 5.6 Hz), 8.11-7.04 (m, 8H), 6.79 (d, 1H, J = 5.6 Hz), 6.29 (s, 1H), 6.27 (s, 1H), 5.37 (t, 1H, J = 5.9 Hz), 4.30 (d, 2H, J = 5.9 Hz), 3.89 (s, 3H), 1.33 (s, 9H).
MS (ESI):535 (M+H⁺).

Example 112

### Methyl 3-(5-(3-(2-(2-morpholinopyrimidin-4-yloxy)benzyl)ureido)-3-t-butyl-1H-pyrazol-1-yl) benzoate

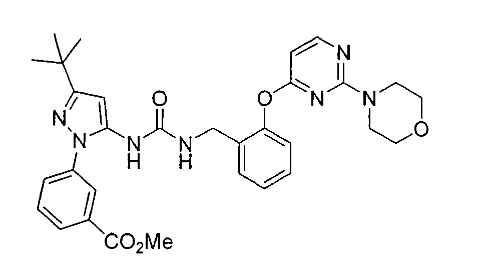

Morpholine (0.055 mL) and sodium carbonate (47 mg) were added to a solution of methyl 3-(5-(3-(2-(2-chloropyrimidin-4-yloxy)benzyl)ureido)-3-t-butyl-1H-pyrazol-1-yl) benzoate (235 mg) in ethanol (1 mL), and the resulting mixture was stirred at 60°C for 2 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (231 mg, yield: 90%). ¹ H-NMR (CDCl₃ ):δ 8.14 (d, 1H, J = 5.6 Hz), 8.13-7.05 (m, 8H), 6.25 (s, 1H), 6.10 (s, 1H), 5.96 (d, 1H, J = 5.6 Hz), 5.31 (t, 1H, J = 5.9 Hz), 4.33 (d, 2H, J = 5.9 Hz), 3.91 (s, 3H), 3.67-3.59 (m, 8H), 1.33 (s, 9H).
MS (ESI):586 (M+H⁺).

Example 113

### 1-(2-(2-chloropyrimidin-4-yloxy)benzyl)-3-(3-t-butyl-1-(3-methoxyphenyl)-1H-pyrazol-5-yl)urea

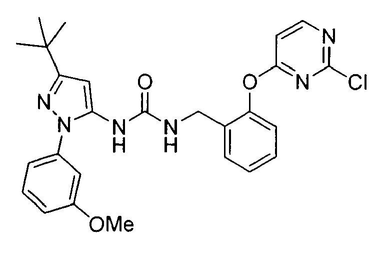

A solution of 2,4-dichloropyrimidine (285 mg) in acetone (2 mL) was cooled to 0°C, and 1-(2-hydroxybenzyl)-3-(3-t-butyl-1-(3-methoxyphenyl)-1H-pyrazol-5-yl)urea (753 mg) and aqueous 1N sodium hydroxide solution (2 mL) were added thereto. This reaction mixture was allowed to warm to room temperature and stirred overnight. Saturated ammonium chloride was added to the reaction mixture, and the resulting mixture was extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (915 mg, yield: 95%).
¹H-NMR (CDCl₃):δ 8.41 (d, 1H, J = 5.9 Hz), 7.37-6.85 (m, 8H), 6.80 (d, 1H, J = 5.9 Hz), 6.25 (s, 1 H), 6.24 (s, 1 H), 5.32 (t, 1 H, J = 5.9 Hz), 4.30 (d, 2H, J = 5.9 Hz), 3.78 (s, 3H), 1.33 (s, 9H).
MS (ESI):507 (M+H⁺).

Example 114

### 1-(2-(2-morpholinopyrimidin-4-yloxy)benzyl)-3-(3-t-butyl-1-(3-methoxyphenyl)-1 H-pyrazol-5-yl)urea

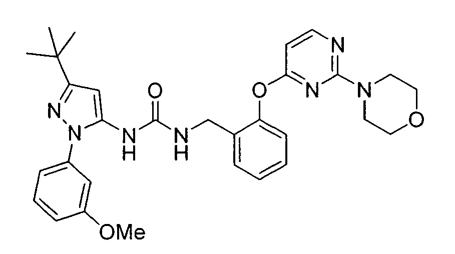

Morpholine (0.20 mL) and sodium carbonate (165 mg) were added to a solution of 1-(2-(2-chloropyrimidin-4-yloxy)benzyl)-3-(3-t-butyl-1-(3-methoxyphenyl)-1H-pyrazol-5-yl)urea (786 mg) in ethanol (3 mL), and the resulting mixture was stirred at 60°C for 2 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (845 mg, yield: 98%).
¹ H-NMR (CDCl₃):δ 8.15 (d, 1H, J = 5.6 Hz), 7.33-6.87 (m, 8H), 6.22 (s, 1H), 6.05 (s, 1H), 5.97 (d, 1H, J = 5.6 Hz), 5.25 (t, 1H, J = 5.9 Hz), 4.34 (d, 2H, J = 5.9 Hz), 3.79 (s, 3H), 3.66-3.59 (m, 8H), 1.33 (s, 9H).
MS (ESI):558 (M+H⁺).

Example 115

### 1-(2-(2-morpholinopyrimidin-4-yloxy)benzyl)-3-(3-t-butyl-1-(3-(2-hydroxyethoxy)phenyl)-1 H-pyrazol-5-yl)urea

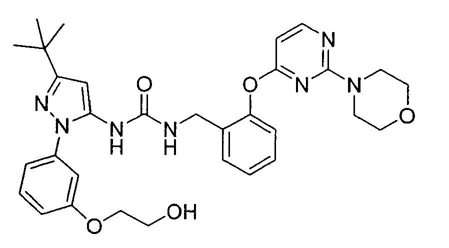

Morpholine (0.012 mL) and sodium carbonate (9.3 mg) were added to a solution of 1-(2-(2-chloropyrimidin-4-yloxy)benzyl)-3-(3-t-butyl-1-(3-(2-hydroxyethoxy)phenyl)-1H-pyrazol-5-yl)urea (47 mg) in ethanol (0.3 mL), and the resulting mixture was stirred at 60°C for 6 hours. The reaction solution was filtered, and washed with a small amount of ethanol. The obtained filtrate was evaporated under reduced pressure, and the resultant residue was purified by silica gel column chromatography (ethyl acetate/n-hexane = 2/1) to obtain the desired product (39 mg, yield: 76%).
¹H-NMR (CDCl₃):δ 8.15 (d, 1H, J = 5.6 Hz), 7.35-6.86 (m, 8H), 6.25 (s, 1H), 6.22 (s, 1H), 5.96 (d, 1H, J = 5.6 Hz), 5.40 (t, 1H, J = 5.9 Hz), 4.33 (d, 2H, J = 5.9 Hz), 4.06 (t, 2H, J = 4.4 Hz), 3.93 (brs, 2H), 3.66-3.58 (m, 8H), 2.51 (brs, 1H), 1.32 (s, 9H).
MS (ESI):588 (M+H⁺).

Example 116

### Sodium 3-(5-(3-(2-(2-morpholinopyrimidin-4-yloxy)benzyl)ureido)-3-t-butyl-1H-pyrazol-1-yl) benzoate

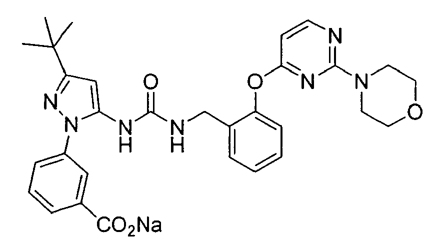

Methyl 3-(5-(3-(2-(2-morpholinopyrimidin-4-yloxy)benzyl)ureido)-3-t-butyl-1H-pyrazol-1-yl) benzoate (58 mg) was dissolved in THF (1.0 mL), and aqueous 0.1 M sodium hydroxide solution (1.0 mL) was added thereto, followed by stirring at room temperature overnight. Water was added to the reaction solution, which was then extracted with ether. The obtained aqueous layer was evaporated under reduced pressure to obtain the desired product (58 mg, yield: 99%).
¹H-NMR (CD₃ OD):δ 8.15 (d, 1H, J = 5.6 Hz), 8.00-7.05 (m, 8H), 6.32 (s, 1H), 6.18 (d, 1H, J = 5.6 Hz), 4.25 (s, 2H), 3.58-3.48 (m, 8H), 1.31 (s, 9H).
MS (ESI):570 (M-Na⁺).

Example 117

### 1-(2-(2-morpholinopyrimidin-4-yloxy)benzyl)-3-(3-t-butyl-1-(3-hydroxyphenyl)-1H-pyrazol-5-yl)urea

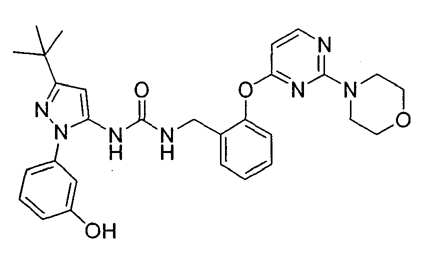

Into a 50 mL eggplant-type flask, in which the atmosphere had been replaced with argon, 1-(2-(2-morpholinopyrimidin-4-yloxy)benzyl)-3-(3-t-butyl-1-(3-methoxyphenyl)-1H-pyrazol-5-yl)urea (223 mg) and dry dichloromethane (5 mL) were added, followed by cooling the resulting mixture to 0°C. To this solution, 1.0M BBr₃ solution in dichloromethane (5 mL) was added by dropwise. The resulting mixture was allowed to warm to room temperature, and was stirred for 2 hours. To the reaction solution, aqueous 15% ethylenediamine solution (25 mL) was added, and then aqueous 6M hydrochloric acid solution was added to acidify the solution. The obtained solution was separated into an organic layer and an aqueous layer, and then the aqueous layer was extracted 3 times with ethyl acetate (20 mL). All of the obtained the organic layers were dried over anhydrous sodium sulfate, and evaporated under reduced pressure. The obtained residue was purified by silica gel column chromatography (ethyl acetate/n-hexane = 3/7 → 7/3) to obtain the desired product (35 mg, yield: 16%).
¹H-NMR (CDCl₃):δ 8.13 (d, 1H, J = 5.9 Hz), 7.35-6.73 (m, 8H), 6.37 (s, 1H), 6.29 (s, 1H), 5.97 (d, 1H, J = 5.9 Hz), 5.29 (t, 1H, J = 5.6 Hz), 4.30 (d, 2H, J = 5.6 Hz), 3.60-3.54 (m, 8H), 1.31 (s, 9H).
MS (ESI):544 (M+H⁺).

Example 118

### 1-(2-(2-chloropyrimidin-4-yloxy)benzyl)-3-(3-t-butyl-1-(4-ethylphenyl)-1 H-pyrazol-5-yl)urea

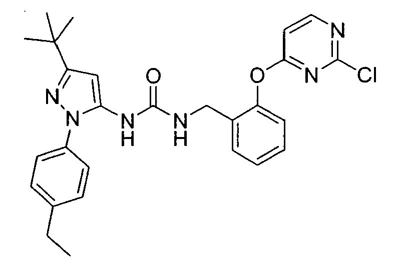

A solution of 2,4-dichloropyrimidine (134 mg) in acetone (0.9 mL) was cooled to 0°C, and 1-(2-hydroxybenzyl)-3-(3-t-butyl-1-(4-ethylphenyl)-1H-pyrazol-5-yl)urea (352 mg) and aqueous 1N sodium hydroxide solution (0.9 mL) were added thereto. This reaction mixture was allowed to warm to room temperature and stirred overnight. Saturated ammonium chloride was added to the reaction mixture, which was then extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (405 mg, yield: 89%).
¹H-NMR (CDCl₃):δ 8.42 (d, 1H, J = 5.6 Hz), 7.37-7.05 (m, 8H), 6.80 (d, 1H, J = 5.6 Hz), 6.23 (s, 1H), 6.09 (s, 1H), 5.29 (t, 1H, J = 5.9 Hz), 4.32 (d, 2H, J = 5.9 Hz), 2.66 (q, 2H, J = 7.6 Hz), 1.33 (s, 9H), 1.23 (t, 3H, J = 7.6 Hz).
MS (ESI):505 (M+H⁺).

Example 119

### 1-(2-(2-morpholinopyrimidin-4-yloxy)benzyl)-3-(3-t-butyl-1-(4-ethylphenyl)-1 H-pyrazol-5-yl)urea

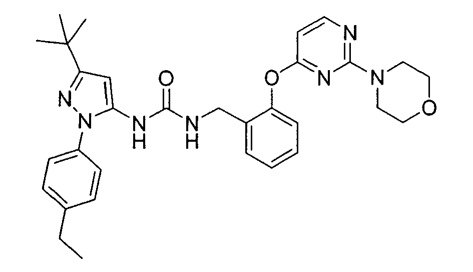

Morpholine (0.046 mL) and sodium carbonate (38 mg) were added to a solution of 1-(2-(2-chloropyrimidin-4-yloxy)benzyl)-3-(3-t-butyl-1-(4-ethylphenyl)-1H-pyrazol-5-yl)urea (177 mg) in ethanol (1 mL), and the resulting mixture was stirred at 60°C for 2 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (158 mg, yield: 81%).
¹H-NMR (CDCl₃):δ 8.15 (d, 1H, J = 5.6 Hz), 7.34-7.06 (m, 8H), 6.20 (s, 1H), 5.97 (s, 1 H), 5.97 (d, 1H, J = 5.6 Hz), 5.25 (t, 1H, J = 5.9 Hz), 4.34 (d, 2H, J=5.9 Hz), 3.67-3.59 (m, 8H), 2.67 (q, 2H, J = 7.6 Hz), 1.33 (s, 9H), 1.24 (t, 3H, J = 7.6 Hz).
MS (ESI):556 (M+H⁺).

Example 120

### 1-(2-(2-chloropyrimidin-4-yloxy)benzyl)-3-(3-t-butyl-1-(3-(dimethylamino)phenyl)-1H-pyrazol-5-yl)urea

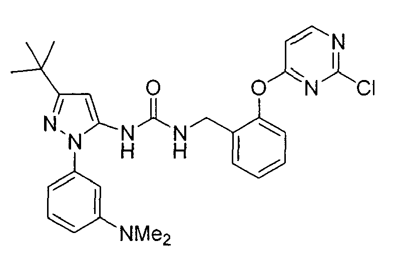

A solution of 2,4-dichloropyrimidine (194 mg) in acetone (1.5 mL) was cooled to 0°C, and 1-(2-hydroxybenzyl)-3-(3-t-butyl-1-(3-(dimethylamino)phenyl)-1H-pyrazol-5-yl)urea (530 mg) and aqueous 1N sodium hydroxide solution (1.5 mL) were added thereto. This reaction mixture was allowed to warm to room temperature and stirred overnight. Saturated ammonium chloride was added to the reaction mixture, which was then extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (533 mg, yield: 79%).
¹H-NMR (CDCl₃):δ 8.41 (d, 1H, J = 5.6 Hz), 7.36-7.05 (m, 5H), 6.79 (d, 1H, J = 5.6 Hz), 6.71-6.65 (m, 3H), 6.25 (s, 2H), 5.33 (t, 1H, J = 5.9 Hz), 4.31 (d, 2H, J = 5.9 Hz), 2.93 (s, 6H), 1.33 (s, 9H).
MS (ESI):520 (M+H⁺).

Example 121

### 1-(2-(2-morpholinopyrimidin-4-yloxy)benzyl)-3-(3-t-butyl-1-(3-(dimethylamino)phenyl)-1H-pyrazol-5-yl)urea

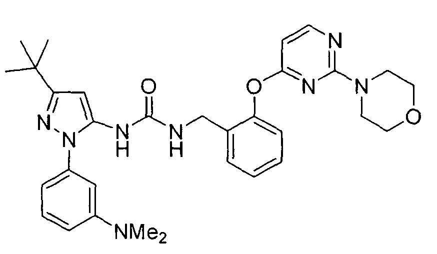

Morpholine (0.040 mL) and sodium carbonate (32 mg) were added to a solution of 1-(2-(2-chloropyrimidin-4-yloxy)benzyl)-3-(3-t-butyl-1-(3-(dimethylamino)phenyl)- 1 H-pyrazol-5 -yl)urea (156 mg) in ethanol (1 mL), and the resulting mixture was stirred at 60°C for 3 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (163 mg, yield: 95%).
¹H-NMR (CDCl₃):δ 8.15 (d, 1H, J = 5.6 Hz), 7.36-6.67 (m, 8H), 6.22 (s, 1H), 6.14 (s, 1H), 5.97 (d, 1H, J = 5.6 Hz), 5.29 (t, 1H, J = 5.9 Hz), 4.35 (d, 2H, J = 5.9 Hz), 3.67-3.59 (m, 8H), 2.94 (s, 6H), 1.33 (s, 9H).
MS (ESI):571 (M+H⁺).

Example 122

### 1 -(2-(2-chloropyrimidin-4-yloxy)benzyl)-3-(3-t-butyl-1-(3-(methylthio)phenyl)-1H-pyrazol-5-yl)urea

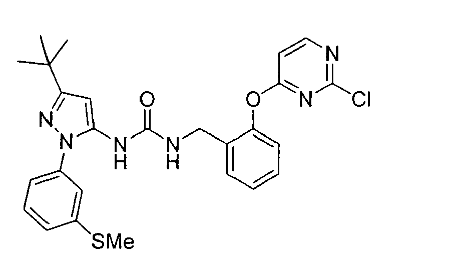

A solution of 2,4-dichloropyrimidine (67 mg) in acetone (0.5 mL) was cooled to 0°C, and 1-(2-hydroxybenzyl)-3-(3-t-butyl-1-(3-(methylthio)phenyl)-1H-pyrazol-5-yl)urea (183 mg) and aqueous 1N sodium hydroxide solution (0.5 mL) were added thereto. This reaction mixture was allowed to warm to room temperature and stirred overnight. Saturated ammonium chloride was added to the reaction mixture, and the resulting mixture was extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (183 mg, yield: 78%).
¹H-NMR (CDCl₃):δ 8.42 (d, 1H, J = 5.9 Hz), 7.37-7.05 (m, 8H), 6.80 (d, 1H, J = 5.9 Hz), 6.25 (s, 1H), 6.20 (s, 1H), 5.33 (t, 1H, J = 5.9 Hz), 4.31 (d, 2H, J = 5.9 Hz), 2.45 (s, 3H), 1.33 (s, 9H).
MS (ESI):523 (M+H⁺).

Example 123

### 1-(2-(2-morpholinopyrimidin-4-yloxy)benzyl)-3-(3-t-butyl-1-(3-(methylthio)phenyl)-1H-pyrazol-5-yl)urea

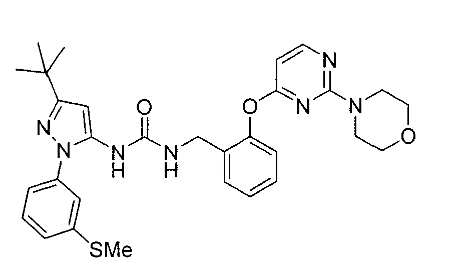

Morpholine (0.035 mL) and sodium carbonate (27 mg) were added to a solution of 1-(2-(2-chloropyrimidin-4-yloxy)benzyl)-3-(3-t-butyl-1-(3-(methylthio)phenyl)-1H-pyrazol-5-yl)urea (130 mg) in ethanol (1 mL), and the resulting mixture was stirred at 60°C for 3 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (142 mg, yield: 99%).
¹H-NMR (CDCl₃):δ 8.15 (d, 1H, J = 5.6 Hz), 7.33-7.06 (m, 8H), 6.23 (s, 1H), 6.09 (s, 1 H), 5.96 (d, 1H, J = 5.6 Hz), 5.30 (t, 1H, J = 5.9 Hz), 4.34 (d, 2H, J = 5.9 Hz), 3.67-3.59 (m, 8H), 2.45 (s, 3H), 1.33 (s, 9H).
MS (ESI):574 (M+H⁺).

Example 124

### 1-(2-(2-chloropyrimidin-4-yloxy)benzyl)-3-(3-t-butyl-1-(3-(methylsulfonyl)phenyl)-1H-pyrazol-5-yl)urea

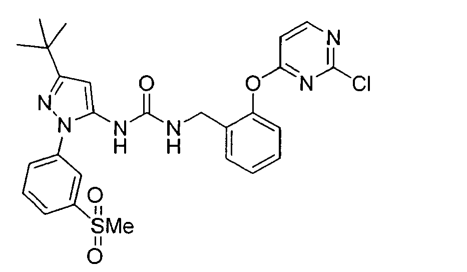

A solution of 2,4-dichloropyrimidine (41 mg) in acetone (0.3 mL) was cooled to 0°C, and 1-(2-hydroxybenzyl)-3-(3-t-butyl-1-(3-(methylsulfonyl)phenyl)-1H-pyrazol-5-yl)urea (123 mg) and aqueous 1N sodium hydroxide solution (0.3 mL) were added thereto. This reaction mixture was allowed to warm to room temperature and stirred overnight. Saturated ammonium chloride was added to the reaction mixture, and the resulting mixture was extracted with ethyl acetate. The extract was dried over anhydrous sodium sulfate, and was evaporated under reduced pressure. The obtained residue was purified by silica gel column chromatography (ethyl acetate/n-hexane = 1/1) to obtain the desired product (100 mg, yield: 65%). ¹H-NMR (CDCl₃ ):δ 8.42 (d, 1H, J = 5.6 Hz), 8.06-7.05 (m, 8H), 6.81 (d, 1H, J = 5.6 Hz), 6.53 (s, 1H), 6.32 (s, 1H), 5.60 (t, 1H, J = 5.6 Hz), 4.31 (d, 2H, J = 5.6 Hz), 3.04 (s, 3H), 1.33 (s, 9H).
MS (ESI):555 (M+H⁺).

Example 125

### 1-(2-(2-morpholinopyrimidin-4-yloxy)benzyl)-3-(3-t-butyl-1-(3-(methylsulfonyl)phenyl)-1H-pyrazol-5-yl)urea

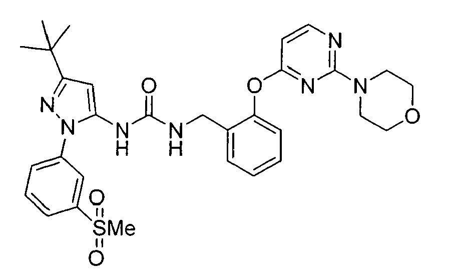

Morpholine (0.015 mL) and sodium carbonate (11 mg) were added to a solution of 1-(2-(2-chloropyrimidin-4-yloxy)benzyl)-3-(3-t-butyl-1-(3-(methylsulfonyl)phenyl)-1H-pyrazol-5-yl)urea (56 mg) in ethanol (0.4 mL), and the resulting mixture was stirred at 60°C for 3 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (50 mg, yield: 82%).
¹H-NMR (CDCl₃):δ 8.15 (d, 1H, J = 5.6 Hz), 8.06-7.04 (m, 8H), 6.66 (s, 1H), 6.34 (s, 1H), 5.95 (d, 1H, J = 5.6 Hz), 5.75 (t, 1H, J = 5.9 Hz), 4.31 (d, 2H, J = 5.9 Hz), 3.68-3.59 (m, 8H), 3.05 (s, 3H), 1.33 (s, 9H).
MS (ESI):606 (M+H⁺).

Example 126

### 1-(2-(2-chloropyrimidin-4-yloxy)benzyl)-3-(3-t-butyl-1-(3-(trifluoromethylsulfonyl)phenyl)-1 H-pyrazol-5-yl)urea

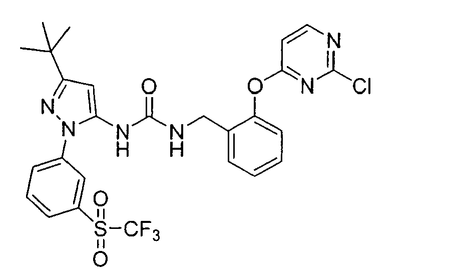

A solution of 2,4-dichloropyrimidine (224 mg) in acetone (1.5 mL) was cooled to 0°C, and 1-(2-hydroxybenzyl)-3-(3-t-butyl-1-(3-(trifluoromethylsulfonyl)phenyl)-1H-pyrazol-5-yl)urea (745 mg) and aqueous 1N sodium hydroxide solution (1.5 mL) were added thereto. This reaction mixture was allowed to warm to room temperature and stirred overnight. Saturated ammonium chloride was added to the reaction mixture, and the resulting mixture was extracted with ethyl acetate. The extract was dried over anhydrous sodium sulfate, and was evaporated under reduced pressure. The obtained residue was purified by silica gel column chromatography (ethyl acetate/n-hexane = 1/2) to obtain the desired product (690 mg, yield: 76%).
¹H-NMR (CDCl₃):δ 8.42 (d, 1H, J = 5.6 Hz), 8.22-7.04 (m, 8H), 6.80 (s, 1H), 6.79 (d, 1H, J = 5.6 Hz), 6.24 (s, 1H), 5.50 (t, 1H, J = 5.9 Hz), 4.26 (d, 2H, J = 5.9 Hz), 1.32 (s, 9H).
MS (ESI):609 (M+H⁺).

Example 127

### 1-(2-(2-morpholinopyrimidin-4-yloxy)benzyl)-3-(3-t-butyl-1-(3-(trifluoromethylsulfonyl)phenyl)-1H-pyrazol-5-yl)urea

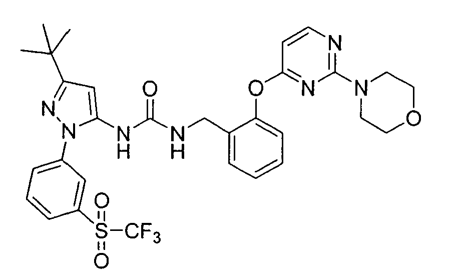

Morpholine (0.030 mL) and sodium carbonate (23 mg) were added to a solution of 1-(2-(2-morpholinopyrimidin-4-yloxy)benzyl)-3-(3-t-butyl-1-(3-(trifluoromethylsulfonyl)phenyl)-1H-pyrazol-5-yl)urea (134 mg) in ethanol (0.5 mL), and the resulting mixture was stirred at 60°C for 3 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (128 mg, yield: 88%).
¹H-NMR (CDCl₃):δ 8.23 (s, 1H), 8.15 (d, 1H, J = 5.6 Hz), 8.06-7.05 (m, 7H), 6.44 (s, 1H), 6.27 (s, 1H), 5.95 (d, 1H, J = 5.6 Hz), 5.44 (t, 1H, J = 5.9 Hz), 4.31 (d, 2H, J = 5.9 Hz), 3.67-3.59 (m, 8H), 1.33 (s, 9H).
MS (ESI):660 (M+H⁺).

Example 128

### 1-(2-(2-(butylamino)pyrimidin-4-yloxy)benzyl)-3-(3-t-butyl-1-p-tolyl-1 H-pyrazol-5-yl)urea

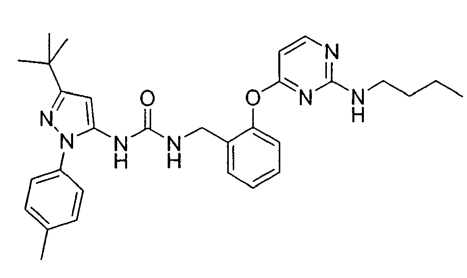

To a solution of 1-(2-(2-chloropyrimidin-4-yloxy)benzyl)-3-(3-t-butyl-1-p-tolyl-1H-pyrazol-5-yl)urea (30 mg) in ethanol (0.2 mL), n-butylamine (24 mg) and sodium carbonate (6 mg) were added and the resulting mixture was stirred at 60°C for 3 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (3 mg, yield: 9%).
¹H-NMR (CD₃ OD):δ 8.04 (d, 1H, J = 5.6 Hz), 7.33-7.06 (m, 8H), 6.27 (s, 1H), 6.12 (d, 1H, J = 5.6 Hz), 4.23 (s, 2H), 3.12 (brs, 2H), 2.40 (s, 3H), 1.37-0.84 (m, 16H)
MS (ESI):528 (M+H⁺)

Example 129

### 1-(2-(2-(cyclohexyl(methyl)amino)pyrimidin-4-yloxy)benzyl)-3-(3-t-butyl-1-p-tolyl-1 H-pyrazol-5-yl)urea

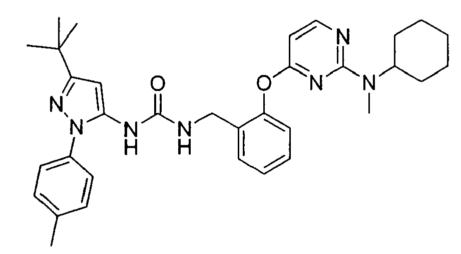

N-methylcyclohexylamine (12 mg) and sodium carbonate (6 mg) were added to a solution of 1-(2-(2-chloropyrimidin-4-yloxy)benzyl)-3-(3-t-butyl-1-p-tolyl-1H-pyrazol-5-yl)urea (30 mg) in ethanol (0.2 mL), and the resulting mixture was stirred at 60°C for 3 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (18 mg, yield: 51%).
¹ H-NMR (CD₃ OD):δ 8.11 (d, 1H, J = 5.6 Hz), 7.33-7.07 (m, 8H), 6.27 (s, 1H), 6.12 (d, 1H, J = 5.6 Hz), 4.24 (s, 2H), 4.10 (brs, 1H), 2.82 (s, 3H), 2.40 (s, 3H), 1.69 (brs, 2H), 1.48 (brs, 3H), 1.40-1.38 (m, 2H), 1.32-1.28(m, 10H), 1.09 (brs, 2H)
MS (ESI):568 (M+H⁺)

Example 130

### 1-(2-(2-(4-oxopiperidin-1-yl)pyrimidin-4-yloxy)benzyl)-3-(1-(3-(benzyloxy)-4-methylphenyl)-3-t-butyl-1H-pyrazol-5-yl)urea

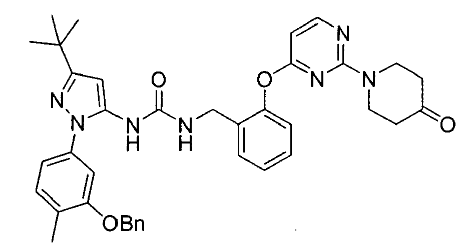

Triethylamine (0.42 mL) was added to a solution of 4-piperidone monohydrate hydrochloric acid salt (515 mg) in ethanol (2 mL), and the resulting mixture was stirred. Thereafter, 1-(2-(2-chloropyrimidin-4-yloxy)benzyl)-3-(1-(3-(benzyloxy)-4-methylphenyl)-3-t-butyl-1H-pyrazol-5-yl)urea (1194 mg) and sodium carbonate (212 mg) were added thereto, and the resulting mixture was stirred at 60°C for 5 hours. The reaction solution was filtered, and washed with a small amount of ethanol. The obtained filtrate was evaporated under reduced pressure, and the resultant residue was purified by silica gel column chromatography (ethyl acetate/n-hexane = 1/1) to obtain the desired product (1.10 g, yield: 83%).
¹H-NMR (CDCl₃):δ 8.18 (d, 1H, J = 5.6 Hz), 7.43-6.89 (m, 12H), 6.22 (s, 1H), 6.05 (s, 1H), 6.02 (d, 1H, J = 5.6 Hz), 5.14 (t, 1H, J = 6.1 Hz), 5.04 (s, 2H), 4.34 (d, 2H, J = 6.1 Hz), 3.93-3.89 (m, 4H), 2.41-2.38 (m, 4H), 2.28 (s, 3H), 1.33 (s, 9H)
MS (ESI):660 (M+H⁺)

Example 131

### 1-(2-(2-(4-oxopiperidin-1-yl)pyrimidin-4-yloxy)benzyl)-3-(3-t-butyl-1-(3-hydroxy-4-methylphenyl)-1H-pyrazol-5-yl)urea

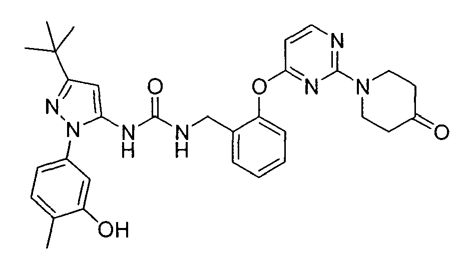

Palladium carbon (17 mg) was added to a solution of 1-(2-(2-(4-oxopiperidin-1-yl)pyrimidin-4-yloxy)benzyl)-3-(1-(3-(benzyloxy)-4-methylphenyl)-3-t-butyl-1H-pyrazol-5-yl)urea (165 mg) in methanol (1 mL), and the resulting mixture was stirred overnight under hydrogen atmosphere at room temperature. The reaction solution was filtered through Celite, and the filtrate was evaporated under reduced pressure. The resultant residue was purified by silica gel column chromatography (ethyl acetate/n-hexane = 3/7 → 7/3) to obtain the desired product (153 mg, yield: 99%).
¹H-NMR(CDCl₃):δ 8.20 (d, 1H, J = 5.6 Hz), 8.11 (br, 1H), 7.35-6.69 (m, 7H), 6.24 (s, 1H), 6.20 (s, 1H), 6.04 (d, 1H, J = 5.6 Hz), 5.17 (t, 1H, J = 5.6 Hz), 4.33 (d, 2H, J = 5.6 Hz), 3.90-3.86 (m, 4H), 2.38-2.35 (m, 4H), 2.17 (s, 3H), 1.30 (s, 9H)
MS (ESI):570 (M+H⁺)

Examples 132-260

### 1-(2-(2-substituted pyrimidin-4-yloxy)benzyl)-3-(3-t-butyl-1-p-tolyl-1H-pyrazol-5-yl)urea

Specific examples of the compounds represented by Formula (Ia) described above were synthesized from 1-(2-(2-chloropyrimidin-4-yloxy)benzyl)-3-(3-t-butyl-1-p-tolyl-1H-pyrazol-5-yl)urea by the same procedure as described in Example 2.

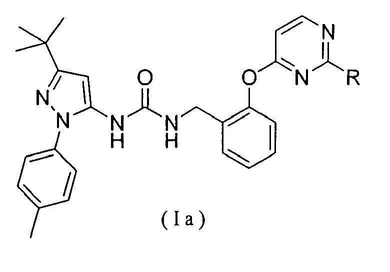

**[Table 1-1]**

| | | | | | | |
|---|---|---|---|---|---|---|
| 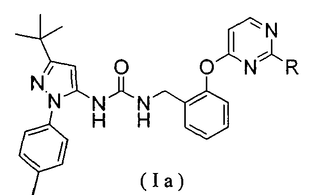 | | | | | | |

| Example No. | R | M/S Found (M+H) | | Example No. | R | M/S Found (M+H) |
|---|---|---|---|---|---|---|
| 132 | 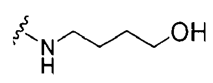 | 544 | | 133 | 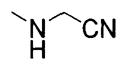 | 511 |
| 134 | 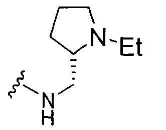 | 583 | | 135 | 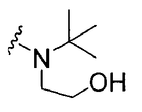 | 572 |
| 136 | 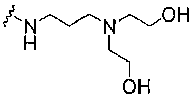 | 617 | | 137 | 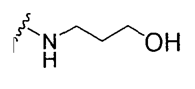 | 530 |
| 138 | 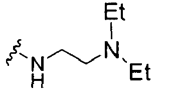 | 571 | | 139 | 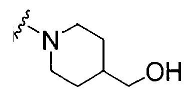 | 570 |
| 140 | 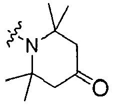 | 610 | | 141 | 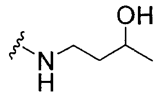 | 544 |
| 142 | 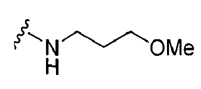 | 544 | | 143 | 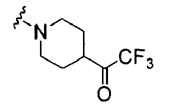 | 636 |
| 144 | 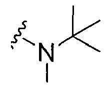 | 542 | | 145 | 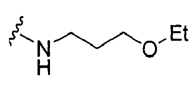 | 558 |
| 146 | 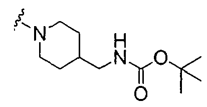 | 669 | | 147 | 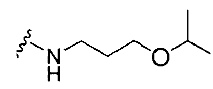 | 572 |
| 148 | 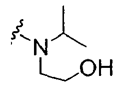 | 558 | | 149 | 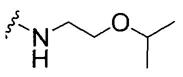 | 558 |

**[Table 1-2]**

| | | | | | | |
|---|---|---|---|---|---|---|
| 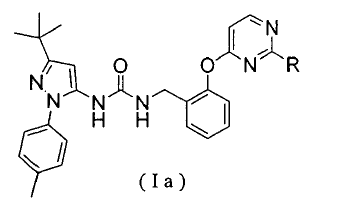 | | | | | | |

| Example No. | R | MS Found (M+H) | | Example No. | R | MS Found (M+H) |
|---|---|---|---|---|---|---|
| 150 | 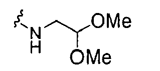 | 576 | | 151 | 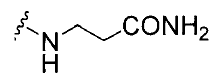 | 543 |
| 152 | 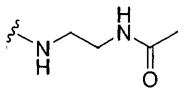 | 557 | | 153 | 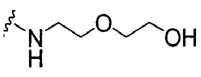 | 560 |
| 154 | 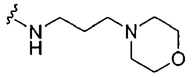 | 599 | | 155 | 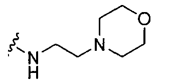 | 585 |
| 156 | 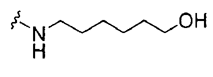 | 572 | | 157 | 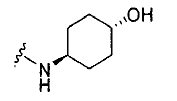 | 570 |
| 158 | 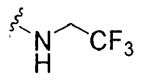 | 554 | | 159 | 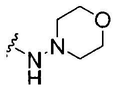 | 557 |
| 160 | 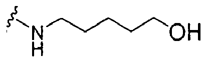 | 558 | | 161 | 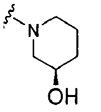 | 556 |
| 162 | 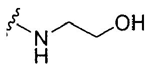 | 516 | | 163 | 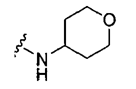 | 556 |
| 164 | 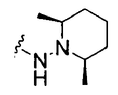 | 583 | | 165 | 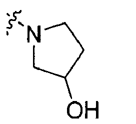 | 542 |
| 166 | 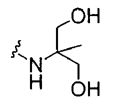 | 560 | | 167 | 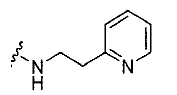 | 577 |
| 168 | 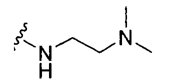 | 543 | | 169 | 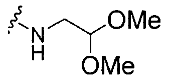 | 560 |

**[Table 1-3]**

| | | | | | | |
|---|---|---|---|---|---|---|
| 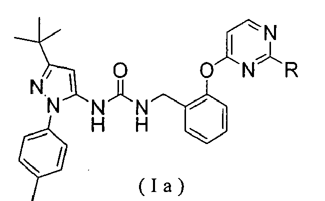 | | | | | | |

| Example No. | R | MS Found (M+H) | | Example MS No. | R | Found (M+H) |
|---|---|---|---|---|---|---|
| 170 | 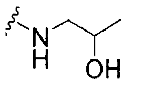 | 530 | | 171 | 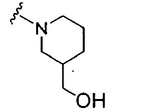 | 570 |
| 172 | 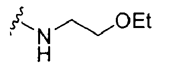 | 544 | | 173 | 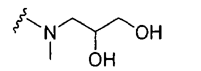 | 560 |
| 174 | 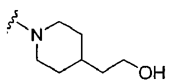 | 584 | | 175 | 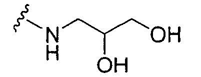 | 546 |
| 176 | 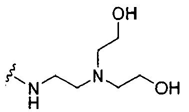 | 603 | | 177 | 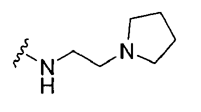 | 569 |
| 178 | 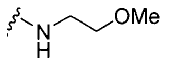 | 582 | | 179 | 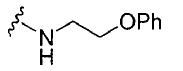 | 592 |
| 180 | 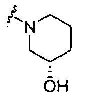 | 556 | | 181 | 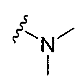 | 500 |
| 182 | 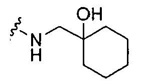 | 584 | | 183 | 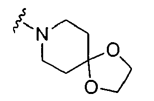 | 598 |
| 184 | 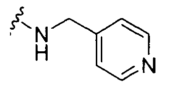 | 616 | | 185 | 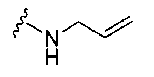 | 512 |
| 186 | 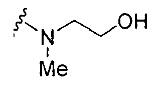 | 530 | | 187 | 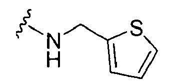 | 568 |
| 188 | 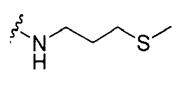 | 560 | | 189 | 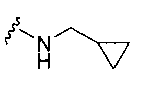 | 526 |

**[Table 1-4]**

| | | | | | | |
|---|---|---|---|---|---|---|
| 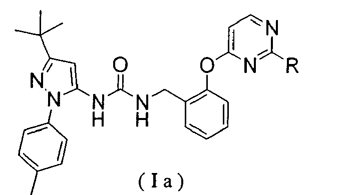 | | | | | | |

| Example No. | R | MS Found (M+H) | | Example No. | R | MS Found (M+H) |
|---|---|---|---|---|---|---|
| 190 | 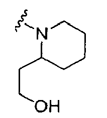 | 584 | | 191 | 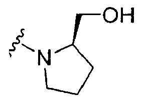 | 556 |
| 192 | 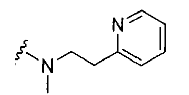 | 591 | | 193 | 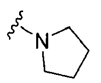 | 526 |
| 194 | 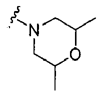 | 570 | | 195 | 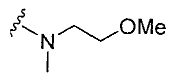 | 544 |
| 196 | 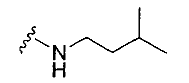 | 542 | | 197 | 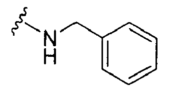 | 562 |
| 198 | 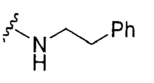 | 576 | | 199 | 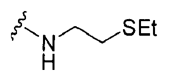 | 560 |
| 200 | 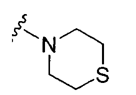 | 558 | | 201 | 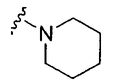 | 540 |
| 202 | 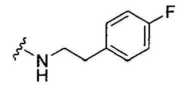 | 594 | | 203 | 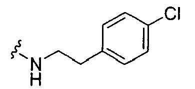 | 610 |
| 204 | 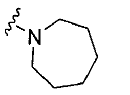 | 554 | | 205 | 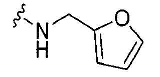 | 552 |
| 206 | 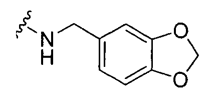 | 506 | | 207 | 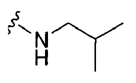 | 528 |
| 208 | 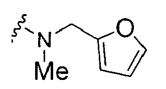 | 566 | | 209 | 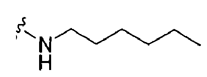 | 556 |

**[Table 1-5]**

| | | | | | | |
|---|---|---|---|---|---|---|
| 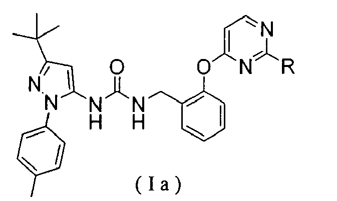 | | | | | | |

| Example No. | R | MS Found (M+H) | | Example No. | R | MS Found (M+H) |
|---|---|---|---|---|---|---|
| 210 | 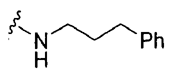 | 590 | | 211 | 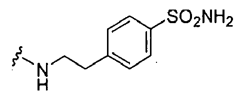 | 655 |
| 212 | 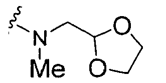 | 572 | | 213 | 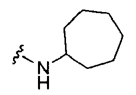 | 568 |
| 214 | 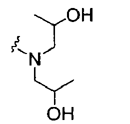 | 588 | | 215 | 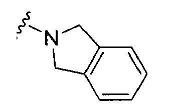 | 574 |
| 216 | 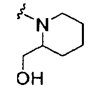 | 570 | | 217 | 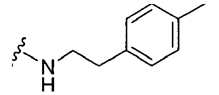 | 590 |
| 218 | 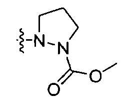 | 585 | | 219 | 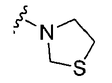 | 544 |
| 220 | 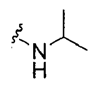 | 514 | | 221 | 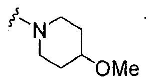 | 570 |
| 222 | 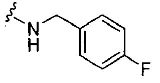 | 580 | | 223 | 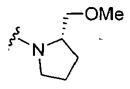 | 570 |
| 224 | 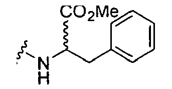 | 634 | | 225 | 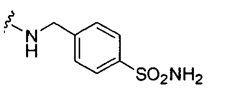 | 641 |
| 226 | 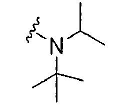 | 570 | | 227 | 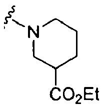 | 612 |
| 228 | 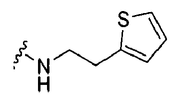 | 582 | | 229 | 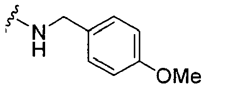 | 592 |
| 230 | 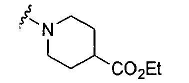 | 612 | | 231 | 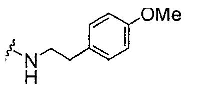 | 606 |

**[Table 1-6]**

| | | | | | | |
|---|---|---|---|---|---|---|
| 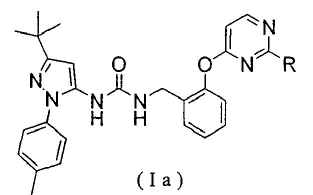 | | | | | | |

| Example No. | R | MS Found (M+H) | | Example | R | MS Found (M+H) |
|---|---|---|---|---|---|---|
| 232 | 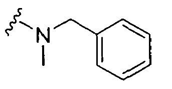 | 576 | | 233 | 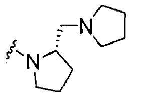 | 609 |
| 234 | 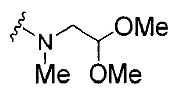 | 574 | | 235 | 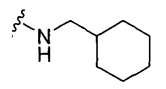 | 568 |
| 236 | 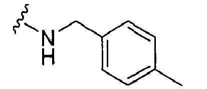 | 576 | | 237 | 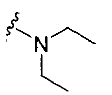 | 528 |
| 238 | 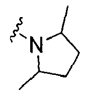 | 554 | | 239 | 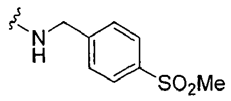 | 640 |
| 240 | 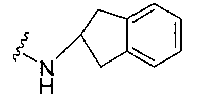 | 588 | | 241 | 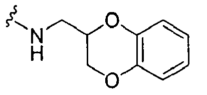 | 620 |
| 242 | 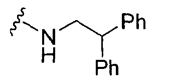 | 652 | | 243 | 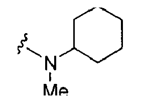 | 568 |
| 244 | 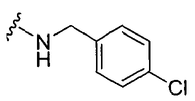 | 596 | | 245 | 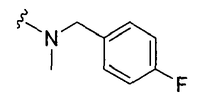 | 594 |
| 246 | 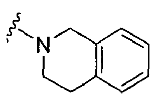 | 588 | | 247 | 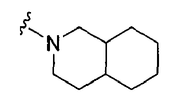 | 594 |
| 248 | 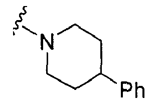 | 616 | | 249 | 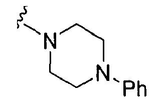 | 617 |
| 250 | 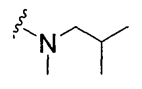 | 588 | | 251 | 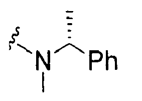 | 590 |

**[Table 1-7]**

| | | | | | | |
|---|---|---|---|---|---|---|
| 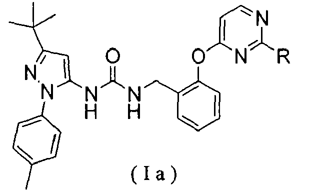 | | | | | | |

| Example No. | R | MS Found (M+H) | | Example No. | R | MS Found (M+H) |
|---|---|---|---|---|---|---|
| 252 | 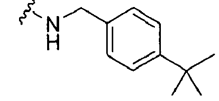 | 590 | | 253 | 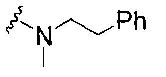 | 590 |
| 254 | 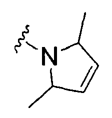 | 552 | | 255 | 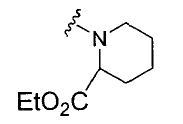 | 612 |
| 256 | 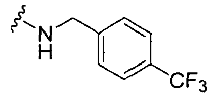 | 630 | | 257 | 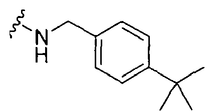 | 618 |
| 258 | 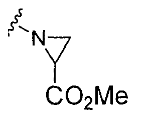 | 556 | | 259 | 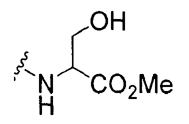 | 574 |
| 260 | 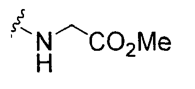 | 544 | | | | |

Examples 261-392

### 1-(2-(6-substituted pyrimidin-4-yloxy)benzyl)-3-(3-t-butyl-1-p-tolyl-1H-pyrazol-5-yl)urea

Specific examples of the compounds represented by Formula (Ib) described above were synthesized from 1-(2-(6-chloropyrimidin-4-yloxy)benzyl)-3-(3-t-butyl-1-p-tolyl-1H-pyrazol-5-yl)urea by the same procedure as described in Example 5.

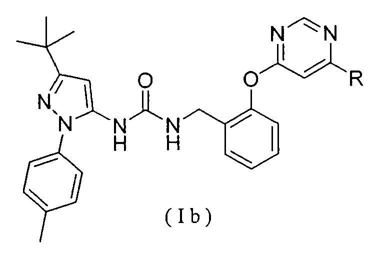

**[Table 2-1]**

| | | | | | | |
|---|---|---|---|---|---|---|
| 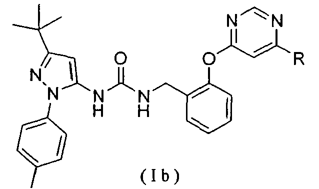 | | | | | | |

| Example No. | R | MS Found (M+H) | | Example No. | R | MS Found (M+H) |
|---|---|---|---|---|---|---|
| 261 | 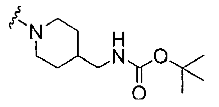 | 544 | | 262 | 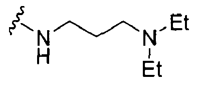 | 585 |
| 263 | 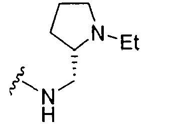 | 583 | | 264 | 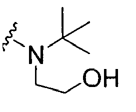 | 572 |
| 265 | 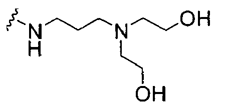 | 617 | | 266 | 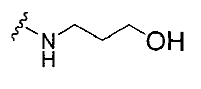 | 530 |
| 267 | 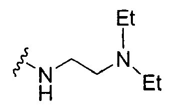 | 571 | | 268 | 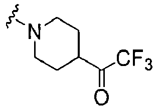 | 636 |
| 269 | 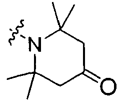 | 610 | | 270 | 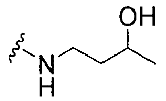 | 544 |
| 271 | 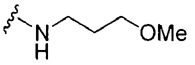 | 544 | | 272 | 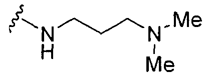 | 557 |
| 273 | 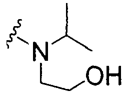 | 558 | | 274 | 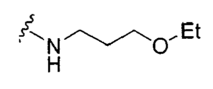 | 558 |
| 275 | 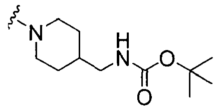 | 669 | | 276 | 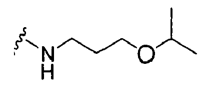 | 572 |
| 277 | 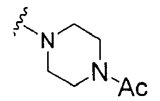 | 583 | | 278 | 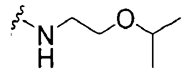 | 558 |

**[Table 2-2]**

| | | | | | | |
|---|---|---|---|---|---|---|
| 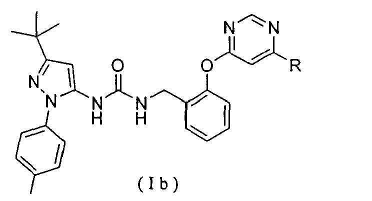 | | | | | | |

| Example No. | R | MS Found (M+H) | | Example No. | R | MS Found (M+H) |
|---|---|---|---|---|---|---|
| 279 | 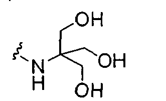 | 576 | | 280 | 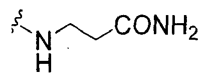 | 543 |
| 281 | 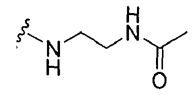 | 557 | | 282 | 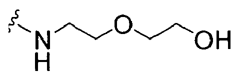 | 560 |
| 283 | 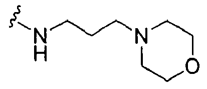 | 599 | | 284 | 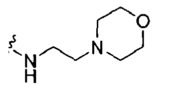 | 585 |
| 285 | 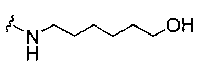 | 572 | | 286 | 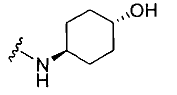 | 570 |
| 287 | 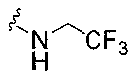 | 554 | | 288 | 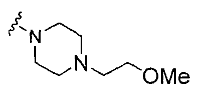 | 599 |
| 289 | 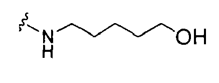 | 558 | | 290 | 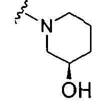 | 556 |
| 291 | 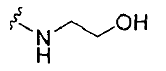 | 516 | | 292 | 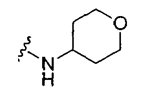 | 556 |
| 293 | 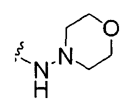 | 557 | | 294 | 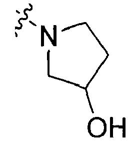 | 542 |
| 295 | 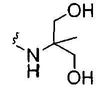 | 560 | | 296 | 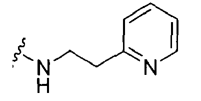 | 577 |
| 297 | 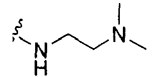 | 543 | | 298 | 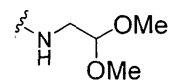 | 560 |

**[Table 2-3]**

| | | | | | | |
|---|---|---|---|---|---|---|
| 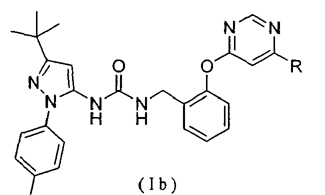 | | | | | | |

| Example No. | R | MS Found (M+H) | | Example No. | R | MS Found (M+H) |
|---|---|---|---|---|---|---|
| 299 | 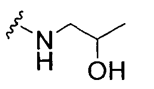 | 530 | | 300 | 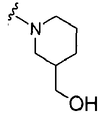 | 570 |
| 301 | 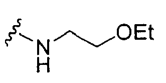 | 544 | | 302 | 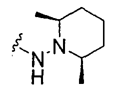 | 583 |
| 303 | 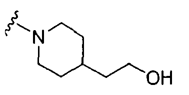 | 584 | | 304 | 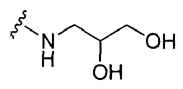 | 546 |
| 305 | 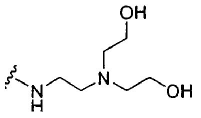 | 603 | | 306 | 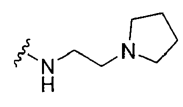 | 569 |
| 307 | 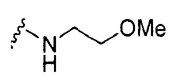 | 582 | | 308 | 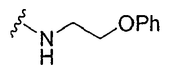 | 592 |
| 309 | 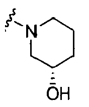 | 556 | | 310 | 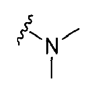 | 500 |
| 311 | 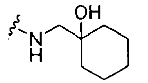 | 584 | | 312 | 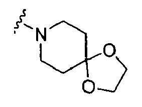 | 598 |
| 313 | 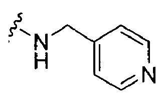 | 616 | | 314 | 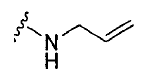 | 512 |
| 315 | 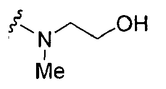 | 530 | | 316 | 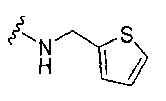 | 568 |
| 317 | 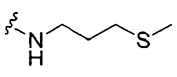 | 560 | | 318 | 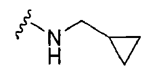 | 526 |

**[Table 2-4]**

| | | | | | | |
|---|---|---|---|---|---|---|
| 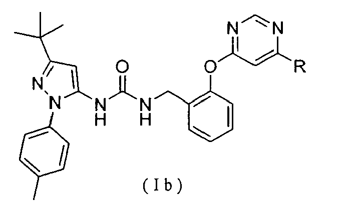 | | | | | | |

| Example No. | R | MS Found (M+H) | | Example No. | R | MS Found (M+H) |
|---|---|---|---|---|---|---|
| 319 | 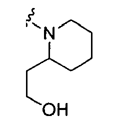 | 584 | | 320 | 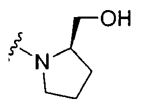 | 556 |
| 321 | 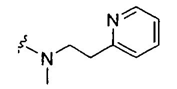 | 591 | | 322 | 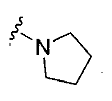 | 526 |
| 323 | 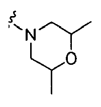 | 570 | | 324 | 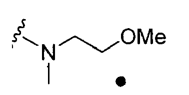 | 544 |
| 325 | 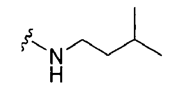 | 542 | | 326 | 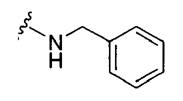 | 562 |
| 327 | 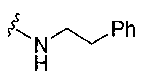 | 576 | | 328 | 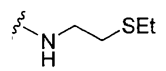 | 560 |
| 329 | 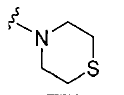 | 558 | | 330 | 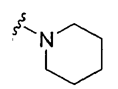 | 540 |
| 331 | 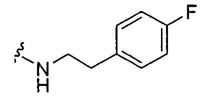 | 594 | | 332 | 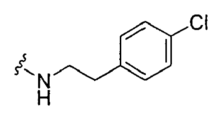 | 610 |
| 333 | 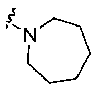 | 554 | | 334 | 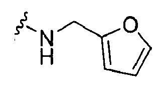 | 552 |
| 335 | 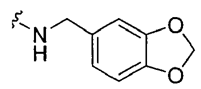 | 606 | | 336 | 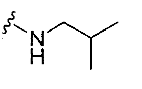 | 528 |
| 337 | 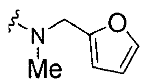 | 566 | | 338 | 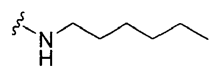 | 556 |

**[Table 2-5]**

| | | | | | | |
|---|---|---|---|---|---|---|
| 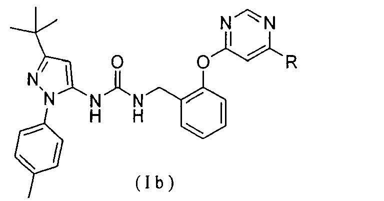 | | | | | | |

| Example No. | R | MS Found (M+H) | | Example | R | MS Found (M+H) |
|---|---|---|---|---|---|---|
| 339 | 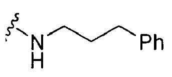 | 590 | | 340 | 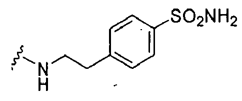 | 655 |
| 341 | 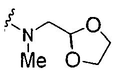 | 572 | | 342 | 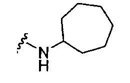 | 568 |
| 343 | 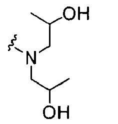 | 588 | | 344 | 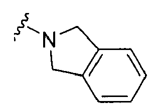 | 574 |
| 345 | 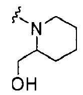 | 570 | | 346 | 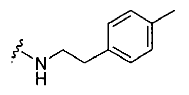 | 590 |
| 347 | 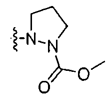 | 585 | | 348 | 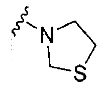 | 544 |
| 349 | 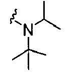 | 570 | | 350 | 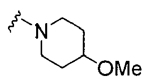 | 570 |
| 351 | 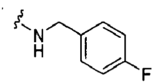 | 580 | | 352 | 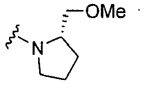 | 570 |
| 353 | 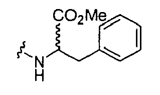 | 634 | | 354 | 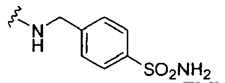 | 641 |
| 355 | 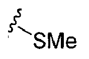 | 503 | | 356 | 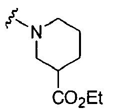 | 612 |
| 357 | 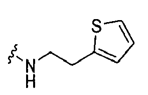 | 582 | | 358 | 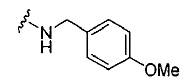 | 592 |
| 359 | 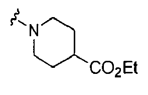 | 612 | | 360 | 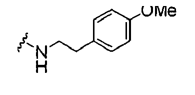 | 606 |

**[Table 2-6]**

| | | | | | | |
|---|---|---|---|---|---|---|
| 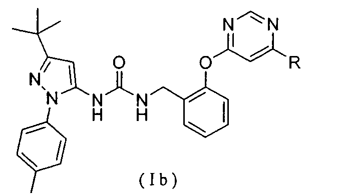 | | | | | | |

| Example No. | R | MS Found Example (M+H) | | Example No. | R | MS Found (M+H) |
|---|---|---|---|---|---|---|
| 361 | 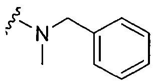 | 576 | | 362 | 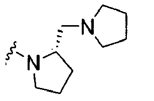 | 609 |
| 363 | 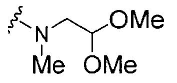 | 574 | | 364 | 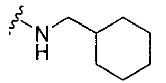 | 568 |
| 365 | 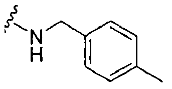 | 576 | | 366 | 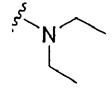 | 528 |
| 367 | 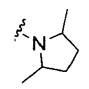 | 554 | | 368 | 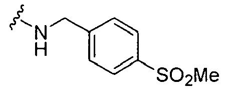 | 640 |
| 369 | 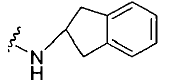 | 588 | | 370 | 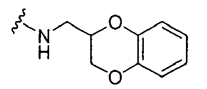 | 620 |
| 371 | 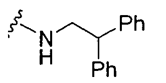 | 652 | | 372 | 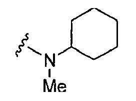 | 568 |
| 373 | 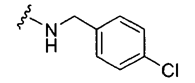 | 596 | | 374 | 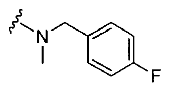 | 594 |
| 375 | 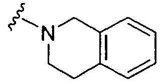 | 588 | | 376 | 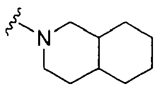 | 594 |
| 377 | 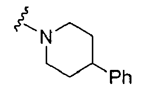 | 616 | | 378 | 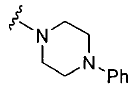 | 617 |
| 379 | 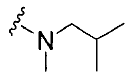 | 588 | | 380 | 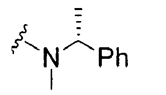 | 590 |

**[Table 2-7]**

| | | | | | | |
|---|---|---|---|---|---|---|
| 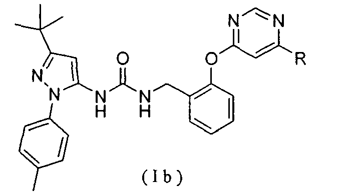 | | | | | | |

| Example No. | R | MS Found (M+H) | | Example No. | R | MS Found (M+H) |
|---|---|---|---|---|---|---|
| 381 | 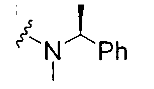 | 590 | | 382 | 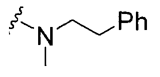 | 590 |
| 383 | 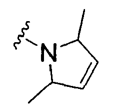 | 552 | | 384 | 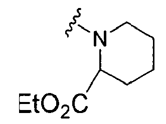 | 612 |
| 385 | 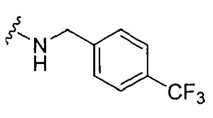 | 630 | | 386 | 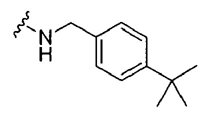 | 618 |
| 387 | 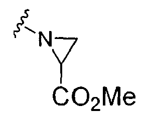 | 556 | | 388 | 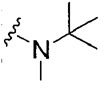 | 542 |
| 389 | 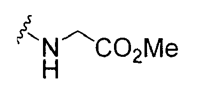 | 544 | | 390 | 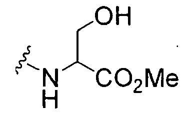 | 574 |
| 391 | 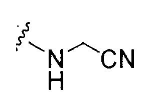 | 511 | | 392 | 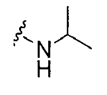 | 514 |

Examples 393-450

### 1-(2-(2-substituted pyrimidin-4-yloxy)-5-fluorobenzyl)-3-(3-t-butyl-1-p-tolyl-1H-pyrazol-5-yl)urea

Specific examples of the compound represented by Formula (Ic) described above were synthesized from 1-(2-(2-chloropyrimidin-4-yloxy)-5-fluorobenzyl)-3-(3-t-butyl-1-p-tolyl-1H-pyrazol-5-yl)urea by the same procedure as described in Example 9.

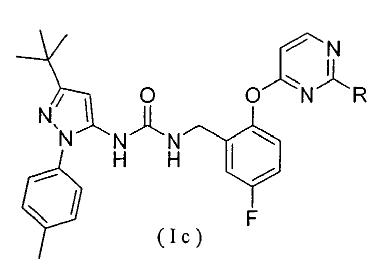

**[Table 3-1]**

| | | | | | | |
|---|---|---|---|---|---|---|
| 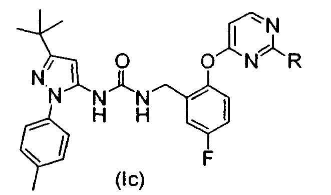 | | | | | | |

| Example No. | R | MS Found (M+H) | | Example No. | R | MS Found (M+H) |
|---|---|---|---|---|---|---|
| 393 | 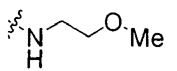 | 548 | | 394 | 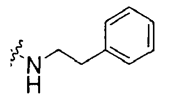 | 594 |
| 395 | 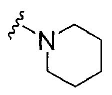 | 558 | | 396 | 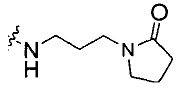 | 615 |
| 397 | 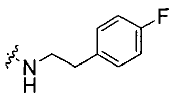 | 612 | | 398 | 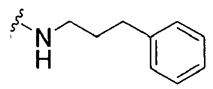 | 608 |
| 399 | 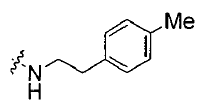 | 607 | | 400 | 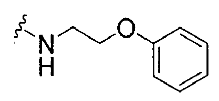 | 610 |
| 401 | 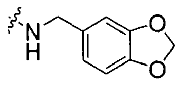 | 624 | | 402 | 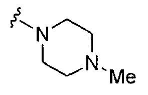 | 573 |
| 403 | 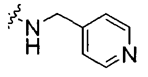 | 581 | | 404 | 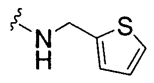 | 586 |
| 405 | 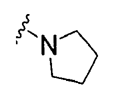 | 544 | | 406 | 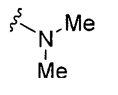 | 518 |
| 407 | 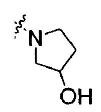 | 560 | | 408 | 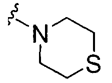 | 576 |
| 409 | 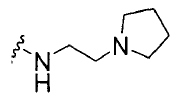 | 587 | | 410 | 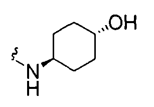 | 588 |
| 411 | 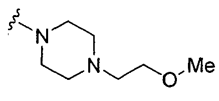 | 17 | | 412 | 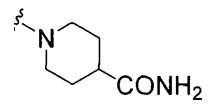 | 601 |

**[Table 3-2]**

| | | | | | | |
|---|---|---|---|---|---|---|
| 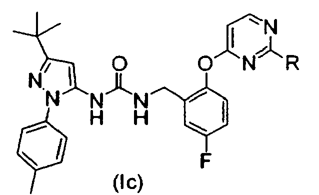 | | | | | | |

| Example No. | R | MS Found (M+H) | | Example No. | R | MS Found (M+H) |
|---|---|---|---|---|---|---|
| 413 | 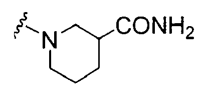 | 601 | | 414 | 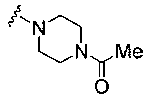 | 601 |
| 415 | 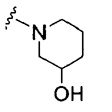 | 574 | | 416 | 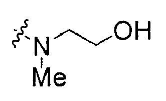 | 548 |
| 417 | 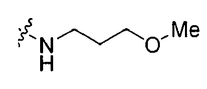 | 562 | | 418 | 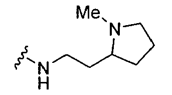 | 601 |
| 419 | 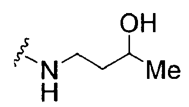 | 548 | | 420 | 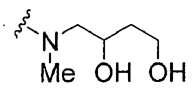 | 578 |
| 421 | 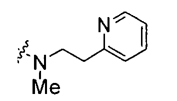 | 609 | | 422 | 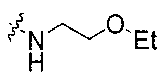 | 562 |
| 423 | 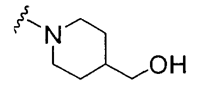 | 588 | | 424 | 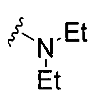 | 546 |
| 425 | 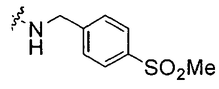 | 658 | | 426 | 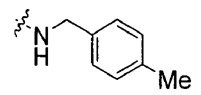 | 594 |
| 427 | 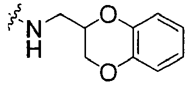 | 638 | | 428 | 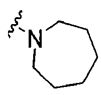 | 572 |
| 429 | 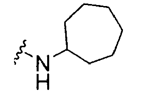 | 586 | | 430 | 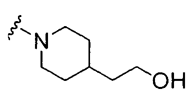 | 602 |
| 431 | 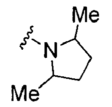 | 572 | | 432 | 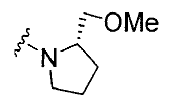 | 588 |

**[Table 3-3]**

| | | | | | | |
|---|---|---|---|---|---|---|
| 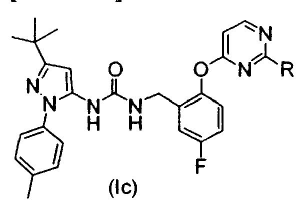 | | | | | | |

| Example No. | R | MS Found (M+H) | | Example No. | R | MS Found (M+H) |
|---|---|---|---|---|---|---|
| 433 | 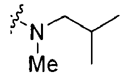 | 560 | | 434 | 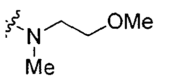 | 562 |
| 435 | 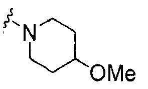 | 588 | | 436 | 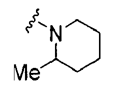 | 572 |
| 437 | 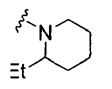 | 586 | | 438 | 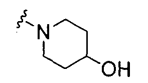 | 574 |
| 439 | 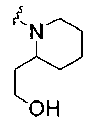 | 602 | | 440 | 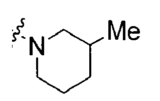 | 572 |
| 441 | 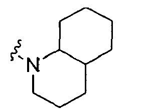 | 612 | | 442 | 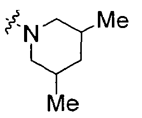 | 586 |
| 443 | 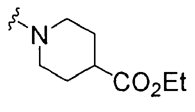 | 630 | | 444 | 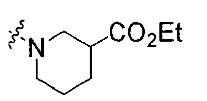 | 630 |
| 445 | 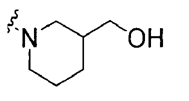 | 588 | | 446 | 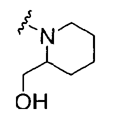 | 588 |
| 447 | 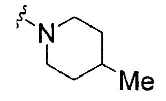 | 572 | | 448 | 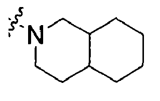 | 612 |
| 449 | 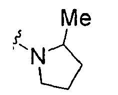 | 558 | | 450 | 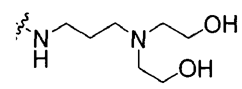 | 635 |

Example 451

### Inhibitory Activity on Cytokine-production in Human Whole Blood

Inhibitory activities of the arylmethylene urea derivatives according to the present invention to the production of TNFα induced by stimulation with a lipopolysaccharide (LPS) were investigated using human whole blood.

### Experimental Method

After mixing each of the compounds of the present invention with human blood in a 96-well flat-bottom plate, the lipopolysaccharide (LPS) was added and the mixture was allowed to react at 37°C for 5 hours. After the reaction, the resulting mixture was centrifuged at room temperature for 15 minutes, and supernatant of the resultant was recovered. For the quantification of TNFα in the supernatant, human cytokine kit (CIS biointernational) was used. The results are shown in Table 4.

**Table 4**

| Example Compound | TNFα-production Inhibitory Activity IC₅₀ (nM) |
|---|---|
| Example 2 | 720 |
| Example 5 | 843 |
| Example 6 | 119 |
| Example 7 | 296 |
| Example 9 | 231 |
| Example 10 | 1040 |
| Example 11 | 249 |
| Example 12 | 268 |
| Example 13 | 496 |
| Example 14 | 458 |
| Example 18 | 311 |
| Example 20 | 343 |
| Example 28 | 513 |
| Example 56 | 485 |
| Example 82 | 354 |
| Example 85 | 422 |
| Example 98 | 298 |
| Example 423 | 504 |

As is apparent from Table 4, the compounds of the present invention have an inhibitory activity on the production of TNFα which is a kind of inflammatory cytokine in human.

Example 452

### Inhibitory Effect of Compound on Dextran Sulfate Sodium (DSS)-induced Colitis Model of Mice (Part 1)

Female BALB/c mice (Japan SLC) of 7 to 9 weeks old were allowed to freely drink 3 w/v% dextran sulfate sodium (DSS) for 5 to 7 days, and then to freely drink sterilized distilled water for 5 days. This procedure was repeated 3 times to induce colitis. The uninduced (background) group was allowed to freely drink sterilized distilled water. The compound described in Example 2 was orally administered to the mice at a dose of 30 mg/kg every day from Day 27 to Day 31 counted from the start of the test, and rectal bleeding was scored (0: occult blood-negative, 2: occult blood-positive, 4: apparent bleeding) by using the method described by Murthy et al (see, for example, Dig Dis Sci., 38, 1722 (1993)).

The score on Day 32 (the last day of the test) of the colitis-uninduced group was 0, while the score of the colitis-induced group rose to 1.2 ± 0.2. On the other hand, the score of the group to which the compound of Example 2 was administered was 0.2 ± 0.1 that was statistically significantly (p < 0.01) lower, so that apparent amelioration of symptoms was observed.

### Comparative Example 1

On the other hand, the same test as in Example 452 was repeated except that 1-(4-(pyridin-4-yloxy)benzyl)-3-(3-t-butylisoxazol-5-yl)urea (the structural formula thereof is shown below) which is a compound included in WO 04/037789 was used in place of the compound of Example 2. The score on Day 32 (the last day of the test) of the colitis-uninduced group was 0, while the score of the colitis-induced group rose to 1.2 ± 0.2. On the other hand, the score of the group to which 1-(4-(pyridin-4-yloxy)benzyl)-3-(3-t-butylisoxazol-5-yl)urea was administered was 1.1 ± 0.2 which was not statistically significantly different, so that improvement in the score was not observed.

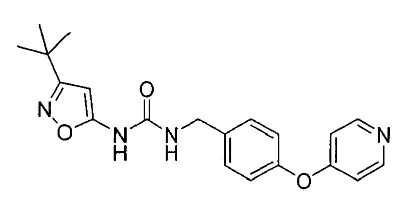

As is apparent from these results, the compound of the present invention has an excellent therapeutic effect for inflammatory bowel disease.

Example 453

### Inhibitory Effect of Compound on Dextran Sulfate Sodium (DSS)-induced Colitis Model of Mice (Part 2)

Female BALB/c mice (Japan SLC) of 7 to 9 weeks old were allowed to freely drink 3 w/v% dextran sulfate sodium (DSS) for 5 to 7 days, and then to freely drink sterilized distilled water for 5 days. This procedure was repeated 3 times to induce colitis. The uninduced (background) group was allowed to freely drink sterilized distilled water. The compound described in Example 10 was orally administered to the mice at a dose of 30 mg/kg every day from Day 27 to Day 31 counted from the start of the test, and rectal bleeding was scored (0: occult blood-negative, 2: occult blood-positive, 4: apparent bleeding) by using the method described by Murthy et al (see, for example, Dig Dis Sci., 38, 1722 (1993)).

The score on Day 32 (the last day of the test) of the colitis-uninduced group was 0, while the score of the colitis-induced group rose to 1.0 ± 0.3. On the other hand, the score of the group to which the compound of Example 10 was administered was 0.6 ± 0.3 that was apparently lower, so that marked amelioration of symptoms was observed.

### Comparative Example 2

On the other hand, the same test as in Example 453 was repeated except that 1-(4-(pyridin-4-yloxy)benzyl)-3-(5-t-butylisoxazol-5-yl)urea (the structural formula thereof is shown below) which is a compound included in WO 04/037789 was used in place of the compound of Example 10. The score on Day 32 (the last day of the test) of the colitis-uninduced group was 0, while the score of the colitis-induced group rose to 1.2 ± 0.2. On the other hand, the score of the group to which 1-(4-(pyridin-4-yloxy)benzyl)-3-(5-t-butylisoxazol-5-yl)urea was administered was 1.3 ± 0.3 which was not statistically significantly different, so that improvement in the score was not observed.

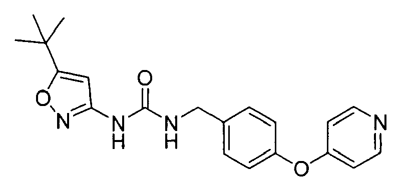

As is apparent from these results, the compound of the present invention has an excellent therapeutic effect for inflammatory bowel disease.

Example 454

### Inhibitory Effect on Rhythmic Bladder Contractions Model in Rats

Using anesthetized rat rhythmic bladder contractions model, the inhibitory effect of the arylmethylene urea derivatives of the present invention on the detrusor hyperreflexia was investigated.

### Experimental Method

Female SD rats were anesthetized with an intraperitoneal injection of urethane (1.0 g/kg). A polyethylene tube was inserted from external urethral orifice into the bladder and the tube was fixed by ligating. The other end of the tube was equipped with a three-necked cock, and one of the necks thereof was connected to a pressure transducer for the measurement of intravesical pressure and the other neck was connected to a syringe for the infusion of physiological saline into the bladder. Thirty minutes after the surgery was completed, physiological saline in appropriate volume was infused via the tube in order to induce rhythmic bladder contractions. After stable rhythmic bladder contractions were obtained, a solution of each test compound or the solvent thereof was administered from a caudal vein in a volume of 0.5 mL/kg, and the effect on the frequency of bladder contractions was investigated. As the test compounds, the compounds of Examples 2 and 9 were used.

### Results

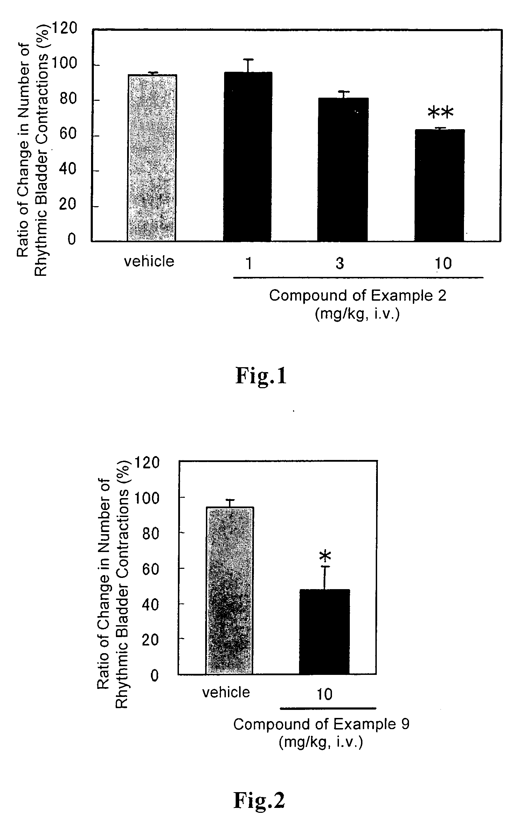

The results are shown in Figs. 1 and 2. In each of the figures, the ordinate indicates the number of rhythmic bladder contractions for 10 minutes before and after administration of the compound. In Fig. 1, "**" indicates that the result was statistically significant (P < 0.01) compared to the vehicle-treated group (multiple comparison test (parametric Williams test) or unpaired t-test). In Fig. 2, "*" indicates that the result was statistically significant (P < 0.05) compared to the vehicle-treated group (multiple comparison test (parametric Williams test) or unpaired t-test)). The compound of Example 2 inhibited the rhythmic bladder contractions dose-dependently from a dose of 3 mg/kg (i.v). The compound of Example 9 also inhibited the rhythmic bladder contractions statistically significantly at a dose of 10 mg/kg (i.v). These results indicate that the compounds of Examples 2 and 9 can inhibit the micturition reflex pathway, indicating that these compounds are effective for overactive bladder.

Example 455

### Effects on Cyclophosphamide-induced Overactive Bladder Model in Rats

Using cyclophosphamide-induced cystitis rats known as an overactive bladder (pollakiuria) model based on the hyperactivity of afferent sensory C-fibers, the effect of compounds on the cystometrograms was investigated.

### Experimental Method

Female SD rats were pre-treated with cyclophosphamide (150 mg/kg) intraperitoneally one day before (18 hours before) the assay in order to induce cystitis. On the next day, each of rats was anesthetized with an intrapertoneral injection of urethane (1.0 g/kg). Next, a small incision was formed in the hypogastrium, and both ureters were ligated, followed by forming small incision in the ureters at the side of the kidney. Then, the bladder apex was incised and a polyethylene tube filled with physiological saline was inserted and indwelled. The other end of the tube was equipped with a three-necked cock, and one of the necks thereof was connected to a pressure transducer for the measurement of intravesical pressure and the other neck was connected to an infusion pomp in order to infuse physiological saline.. Thirty minutes after the surgery was completed, physiological saline was continuously infused (3.6 mL/hr) in order to obtain a continuous cystometrograms. One minute before the infusion, a solution of each test compound or the solvent thereof was administered from a caudal vein in a volume of 0.5 mL/kg. The effects of compounds on the frequency of urination and detrusor contraction pressure in voiding during 20 minutes from the initiation of physiological saline infusion were investigated in comparison with those in the vehicle-treated group. As the test compounds, the compounds of Examples 2 and 81 were used.

### Results

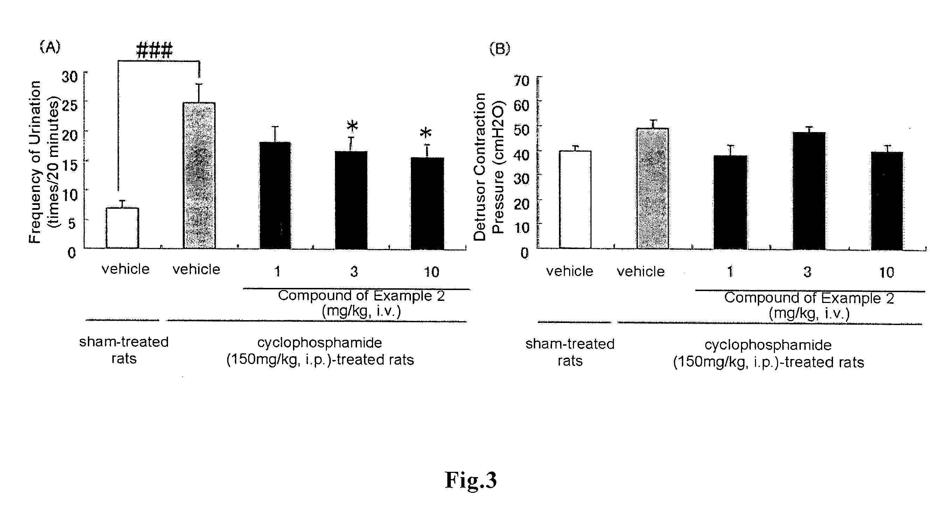

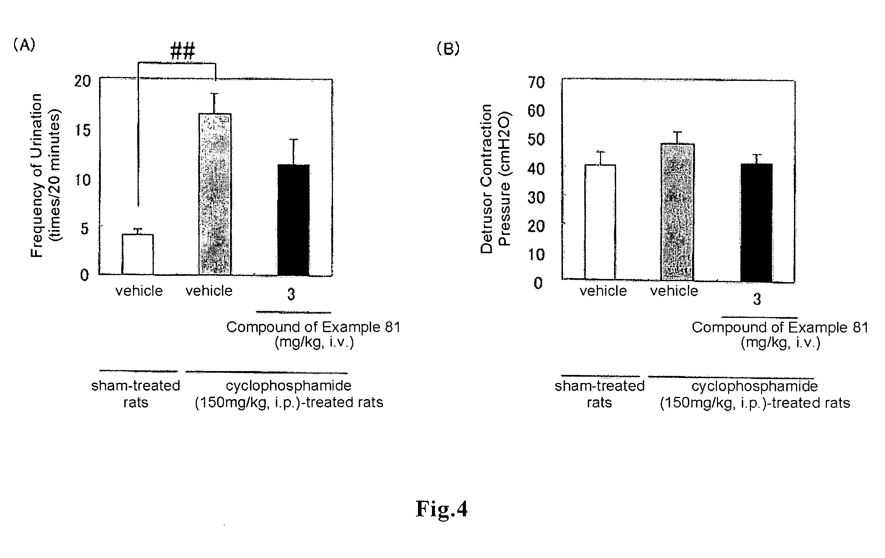

The results are shown in Figs. 3 and 4. In each figure, the ordinate in (A) indicates the number of voiding episodes (times) during 20 minutes, and the ordinate in (B) indicates the mean value of detrusor contraction pressure in voiding (cm H₂O) during 20 minutes. In Fig. 3, "###" indicates that the difference between the results of the sham-treated group and the cyclophosphamide-treated group was statistically significant (p < 0.001) (unpaired t-test), and "*"indicates that the result was statistically significant (P < 0.05) compared to the vehicle-treated group (multiple comparison test (parametric Williams test)). In Fig. 4, "##" indicates that the difference between the results of the sham-treated group and the cyclophosphamide-treated group was statistically significant (p < 0.01) (unpaired t-test). The compound of Example 2 dose-dependently reduced the increase in the frequency of urination observed in the cyclophosphamide-induced cystitis rats from a dose of 3 mg/kg (Fig. 3-A). The increase in the frequency of urination observed in the cyclophosphamide-induced cystitis rats was reduced by the administration of the compound of Example 81 (3 mg/kg) (Fig. 4-A). These results indicate that the compound of Example 2 suppresses the enhanced state of micturition reflex which is thought to be caused by the hyperactivity of afferent sensory C-fibers, and has a property of anti-pollakiuria indeed. On the other hand, these compounds do not affect on the detrusor contraction pressure in voiding, which suggests that they hardly occurs side effects such as increase in the amount of residual urine (Figs. 3-B and 4-B).

As is apparent from the results of Examples 454 and 455, the compounds of the present invention have excellent therapeutic effect for overactive bladder.

## Claims

1. An arylmethylene urea derivative of the Formula (I) or a pharmaceutically acceptable salt thereof: 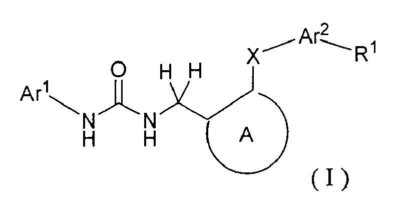 [wherein
A represents the Formula (II), (III), (IV) or (V): 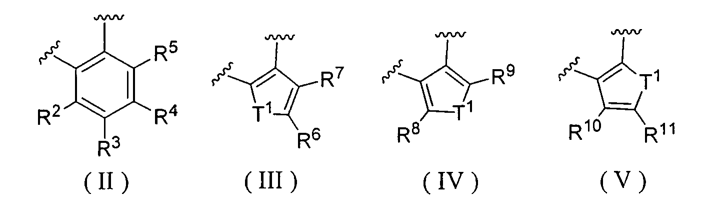 (wherein
R² to R¹¹ each independently represents hydrogen, cyano, hydroxy, amino, mercapto, nitro, fluoro, chloro, bromo, iodo, phenyl, benzyl, carboxy, carbamoyl, thiocarbamoyl, formyl, C₁-C₈ alkyl (which may be linear, branched, or partially or entirely cyclized, and which is optionally substituted with a fluorine(s), chlorine(s), bromine(s) and/or iodine(s), the term "C₁-C₈ alkyl" meaning, in all claims, C₁-C₈ alkyl which may be linear, branched, or partially or entirely cyclized, and which is optionally substituted with a fluorine(s), chlorine(s), bromine(s) and/or iodine(s) unless otherwise specified), C₁-C₈ hydroxyalkyl (which may be linear, branched, or partially cyclized, the term "C₁-C₈ hydroxyalkyl" meaning, in all claims, C₁-C₈ hydroxyalkyl which may be linear, branched, or partially cyclized unless otherwise specified), or C₁-C₈ alkoxy (which may be linear, branched, or partially cyclized, the term "C₁-C₈ alkoxy" meaning, in all claims, C₁-C₈ alkoxy which may be linear, branched, or partially cyclized unless otherwise specified);
T¹ represents -O-, -S- or -NR¹²-
(wherein R¹² represents hydrogen, C₁-C₈ alkyl, C₁-C₈ hydroxyalkyl, C₁-C₈ alkoxy or C₁-C₈ alkylsulfanyl (which may be linear, branched, or partially cyclized, the term "C₁-C₈ alkylsulfanyl" meaning, in all claims, C₁-C₈ alkylsulfanyl which may be linear, branched, or partially cyclized unless otherwise specified);
X represents -O-, -S-, -SO-, -SO₂ -, -NR¹³ - or -CH₂-
(wherein R¹³ represents hydrogen, C₁-C₈ alkyl, C₁-C₈ hydroxyalkyl or C₁-C₈ alkoxy);
Ar¹ represents pyrazole, thiophene, furan, pyrrole, imidazole, oxazole, thiazole, oxadiazole or thiadiazole
(wherein each of these optionally has one or more substituents independently selected from the group consisting of the following substituents:
cyano, hydroxy, amino, mercapto, nitro, fluoro, chloro, bromo, iodo, phenyl, benzyl, carboxy, carbamoyl, thiocarbamoyl, formyl, C₁-C₈ alkyl, C₂-C₈ alkenyl (which may be linear, branched, or partially cyclized, the term "C₂-C₈ alkenyl" meaning, in all claims, C₂-C₈ alkenyl which may be linear, branched, or partially cyclized unless otherwise specified), C₂-C₈ alkynyl (which may be linear, branched, or partially cyclized, the term "C₂-C₈ alkynyl" meaning, in all claims, C₂-C₈ alkynyl which may be linear, branched, or partially cyclized unless otherwise specified), C₁-C₈ hydroxyalkyl, C₁-C₈ alkoxy, C₁-C₁₆ alkylamino (which means an amino group substituted with one or two independently selected C₁-C₈ alkyls, which alkyl may be linear, branched, or partially or entirely cyclized, the term "C₁-C₁₆ alkylamino" meaning, in all claims, an amino group substituted with one or two independently selected C₁-C₈ alkyls, which alkyl may be linear, branched, or partially or entirely cyclized unless otherwise specified), C₁-C₈ alkylsulfanyl and 5- to 10-membered aromatic heterocycle (which means a 5- to 10-membered monocyclic or bicyclic heterocyclic aryl having 1 to 5 hetero atoms of 1 to 3 types selected from nitrogen atom, sulfur atom and oxygen atom in addition to carbon atoms, the term "5- to 10-membered aromatic heterocycle" meaning, in all claims, a 5- to 10-membered monocyclic or bicyclic heterocyclic aryl having 1 to 5 hetero atoms of 1 to 3 types selected from nitrogen atom, sulfur atom and oxygen atom in addition to carbon atoms unless otherwise specified),
(wherein each of these substituents optionally further has one or more substituents independently selected from the following substituents:
cyano, hydroxy, amino, mercapto, nitro, fluoro, chloro, bromo, iodo, phenyl, benzyl, carboxy, benzyloxy, carbamoyl, thiocarbamoyl, formyl, C₁-C₈ alkyl, C₂-C₈ alkenyl, C₂-C₈ alkynyl, C₁-C₈ hydroxyalkyl, C₁-C₈ alkoxy, C₁-C₈ alkoxycarbonyl, C₁-C₈ hydroxyalkyloxy (which may be linear, branched, or partially cyclized, the term "C₁-C₈ hydroxyalkyloxy" meaning, in all claims, a C₁-C₈ hydroxyalkyloxy which may be linear, branched, or partially cyclized unless otherwise specified), C₁-C₁₆ alkylamino, C₁-C₈ alkylsulfanyl, C₁-C₈ alkylsulfonyl (which may be linear, branched, or partially cyclized, the term "C₁-C₈ alkylsulfonyl" meaning, in all claims, a C₁-C₈ alkylsulfonyl which may be linear, branched, or partially cyclized unless otherwise specified), trifluoromethanesulfonyl, sulfonamide alkylated with C₁-C₈ alkyl and 5- to 10-membered aromatic heterocycle);
Ar² represents the Formula (VI) or Formula (VII): 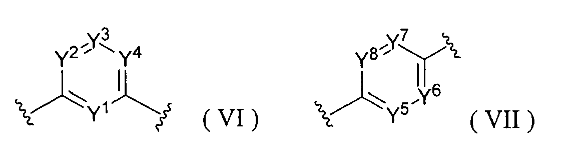
[wherein Y¹ to Y⁸ each independently represents N or CR¹⁴ with the proviso that at least two of Y¹ to Y⁴ are N and that at least two of Y⁵ to Y⁸ are N
(wherein R¹⁴ is independently selected from the following:
hydrogen, cyano, hydroxy, amino, mercapto, nitro, fluoro, chloro, bromo, iodo, phenyl, benzyl, carboxy, carbamoyl, thiocarbamoyl, formyl, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₈ alkylsulfanyl and 5- to 10-membered aromatic heterocycle
(wherein each of these substituents optionally further has one or more substituents independently selected from the following:
cyano, hydroxy, amino, mercapto, nitro, fluoro, chloro, bromo, iodo, phenyl, benzyl, carboxy, carbamoyl, thiocarbamoyl, formyl, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₈ alkylsulfanyl and 5- to 10-membered aromatic heterocycle)]
R¹ represents
hydrogen, cyano, hydroxy, amino, mercapto, nitro, fluoro, chloro, bromo, iodo, phenyl, benzyl, carboxy, carbamoyl, thiocarbamoyl, formyl, formylamino, aminosulfonyl, phenoxy, indan, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₁₆ hydroxyalkylamino (amino group substituted with one or two hydroxyalkyls selected independently), C₁-C₈ alkylsulfanyl, C₁-C₈ alkylsulfinyl (which may be linear, branched, or partially cyclized, the term "C₁-C₈ alkylsulfinyl" meaning, in all claims, a C₁-C₈ alkylsulfinyl which may be linear, branched, or partially cyclized unless otherwise specified), C₁-C₈ alkylsulfonyl, C₆-C₁₄ arylsulfanyl, C₆-C₁₄ arylsulfinyl, C₆-C₁₄ arylsulfonyl, amide alkylated with C₁-C₈ alkyl, sulfonamide alkylated with C₁-C₈ alkyl, carbamate alkylated with C₁-C₈ alkyl, urea alkylated with C₁-C₈ alkyl, carboxylated C₁-C₈ alkyl, C₁-C₈ alkoxycarbonyl, alkylcarbonyl containing C₁-C₈ alkyl, 5- to 10-membered aromatic heterocycle, 5- to 10-membered monocyclic heterocycle (which means a 5-to 10-membered monocyclic heterocyclic aryl having 1 to 5 hetero atoms of 1 to 3 types selected from nitrogen atom, sulfur atom and oxygen atom in addition to carbon atoms, or said 5- to 10-membered monocyclic heterocyclic aryl which is partially or entirely saturated and which optionally has a carbonyl group(s) in a part(s) of its ring, the term "5- to 10-membered monocyclic heterocycle" meaning, in all claims, a 5- to 10-membered monocyclic heterocyclic aryl having 1 to 5 hetero atoms of 1 to 3 types selected from nitrogen atom, sulfur atom and oxygen atom, or said 5- to 10-membered monocyclic heterocyclic aryl which is partially or entirely saturated and which optionally has a carbonyl group(s) in a part(s) of its ring unless otherwise specified), or 5- to 10-membered bicyclic heterocycle (which means a 5- to 10-membered bicyclic heterocyclic aryl having 1 to 5 hetero atoms of 1 to 3 types selected from nitrogen atom, sulfur atom and oxygen atom in addition to carbon atoms, or said 5- to 10-membered bicyclic heterocyclic aryl which is partially or entirely saturated, the term "5- to 10-membered bicyclic heterocycle" meaning, in all claims, a 5- to 10-membered bicyclic heterocyclic aryl having 1 to 5 hetero atoms of 1 to 3 types selected from nitrogen atom, sulfur atom and oxygen atom in addition to carbon atoms, or said 5- to 10-membered bicyclic heterocyclic aryl which is partially or entirely saturated unless otherwise specified),
(wherein each of these substituents optionally has one or more substituents independently selected from the following:
cyano, hydroxy, amino, mercapto, nitro, fluoro, chloro, bromo, iodo, phenyl, benzyl, carboxy, carbamoyl, thiocarbamoyl, formyl, formylamino, aminosulfonyl, phenoxy, indan, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₁₆ hydroxyalkylamino, C₁-C₈ alkylsulfanyl, C₁-C₈ alkylsulfinyl, C₁-C₈ alkylsulfonyl, C₆-C₁₄ arylsulfanyl, C₆-C₁₄ arylsulfinyl, C₆-C₁₄ arylsulfonyl, amide alkylated with C₁-C₈ alkyl, sulfonamide alkylated with C₁-C₈ alkyl, carbamate alkylated with C₁-C₈ alkyl, urea alkylated with C₁-C₈ alkyl, carboxylated C₁-C₈ alkyl, C₁-C₈ alkoxycarbonyl, C₁-C₈ alkylcarbonyl, 5- to 10-membered aromatic heterocycle, 5- to 10-membered monocyclic heterocycle, 5- to 10-membered bicyclic heterocycle, C₁-C₈ alkylene (which may be linear, branched, or partially cyclized, the term "C₁-C₈ alkylene" meaning, in all claims, a C₁-C₈ alkylene which may be linear, branched, or partially cyclized unless otherwise specified), C₂-C₈ alkenylene (which may be linear, branched, or partially cyclized, the term "C₁-C₈ alkenylene" meaning, in all claims, a C₁-C₈ alkenylene which may be linear, branched, or partially cyclized unless otherwise specified) and C₁-C₈ alkylenedioxy (which may be linear, branched, or partially cyclized, the term "C₁-C₈ alkylenedioxy" meaning, in all claims, a C₁-C₈ alkylenedioxy which may be linear, branched, or partially cyclized unless otherwise specified)
(wherein each of these substituents optionally further has one or more substituents independently selected from the following:
cyano, hydroxy, amino, mercapto, nitro, fluoro, chloro, bromo, iodo, phenyl, benzyl, carboxy, carbamoyl, thiocarbamoyl, formyl, formylamino, aminosulfonyl, indan, C₁-C₈ alkyl, C₁-C₈ hydroxyalkyl, C₁-C₈ alkoxy, C₁-C₆ alkylamino, C₁-C₁₆ hydroxyalkylamino, amide alkylated with C₁-C₈ alkyl, carbamate alkylated with C₁-C₈ alkyl, carboxylated C₁-C₈ alkyl, C₁-C₈ alkoxycarbonyl, C₁-C₈ alkylcarbonyl, 5- to 10-membered monocyclic heterocycle, 5- to 10-membered bicyclic heterocycle, 5- to 10-membered aromatic heterocycle and C₁-C₈ alkylenedioxy)].

2. The arylmethylene urea derivative or the pharmaceutically acceptable salt thereof according to claim 1, wherein in said Formula (I),
A represents the Formula (II) or (III)
(wherein T¹ represents -S-, and R² to R⁷ each independently represents hydrogen, fluoro, chloro, bromo, iodo, or C₁-C₈ alkyl);
X is -O-, -S-, -NR¹³-, or -CH₂-
(wherein R¹³ represents hydrogen or C₁-C₈ alkyl);
Ar¹ is pyrazole, thiophene, oxazole, thiazole, oxadiazole, or thiadiazole (wherein each of these optionally has one or more substituents independently selected from the following:
phenyl, C₁-C₈ alkyl, C₁-C₈ hydroxyalkyl, pyridyl and furyl
(wherein said phenyl, pyridyl, or furyl optionally further has one or more substituents independently selected from the following:
hydroxy, amino, nitro, fluoro, chloro, bromo, iodo, carboxy, benzyloxy, C₁-C₈ alkyl, C₁-C₈ alkoxy, C₁-C₈ alkoxycarbonyl, C₁-C₈ hydroxyalkyloxy, C₁-C₁₆ alkylamino, C₁-C₈ alkylsulfanyl, C₁-C₈ alkylsulfonyl, trifluoromethanesulfonyl, and sulfonamide alkylated with C₁-C₈ alkyl));
Ar² represents the Formula (VIII), (IX), (X), or (XI): 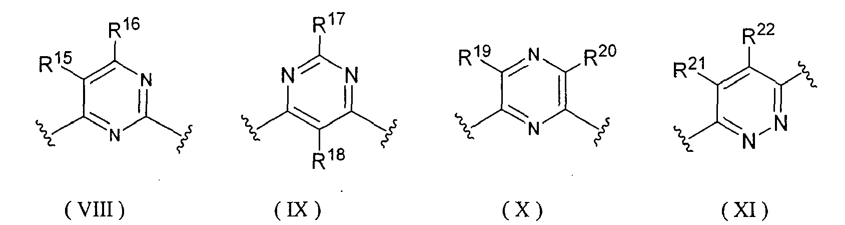 [wherein R¹⁵ to R²² each independently represents hydrogen or C₁-C₈ alkyl]; and
R¹ is
hydrogen, amino, mercapto, fluoro, chloro, bromo, iodo, C₁-C₁₆ alkylamino, C₁-C₁₆ hydroxyalkylamino, C₁-C₈ alkylsulfanyl, 5- to 10-membered aromatic heterocycle, 5-to 10-membered monocyclic heterocycle, or 5- to 10-membered bicyclic heterocycle (wherein each of these substituents optionally has one or more substituents independently selected from the following:
cyano, hydroxy, amino, mercapto, fluoro, chloro, bromo, iodo, phenyl, benzyl, carboxy, carbamoyl, C₁-C₈ alkyl, C₁-C₈ hydroxyalkyl, C₁-C₈ alkoxy, C₁-C₁₆ alkylamino, C₁-C₁₆ hydroxyalkylamino, amide alkylated with C₁-C₈ alkyl, carboxylated C₁-C₈ alkyl, C₁-C₈ alkoxycarbonyl, C₁-C₈ alkylcarbonyl, 5- to 10-membered aromatic heterocycle, 5- to 10-membered monocyclic heterocycle, 5- to 10-membered bicyclic heterocycle, and C₁-C₈ alkylenedioxy
(wherein each of these substituents optionally further has one or more substituents independently selected from the following:
cyano, hydroxy, amino, mercapto, fluoro, chloro, bromo, iodo, phenyl, benzyl, carboxy, carbamoyl, C₁-C₈ alkyl, C₁-C₈ hydroxyalkyl, C₁-C₈ alkoxy, C₁-C₁₆ alkylamino, C₁-C₁₆ hydroxyalkylamino, amide alkylated with C₁-C₈ alkyl, carboxylated C₁-C₈ alkyl, C₁-C₈ alkoxycarbonyl, C₁-C₈ alkylcarbonyl, 5- to 10-membered monocyclic heterocycle, 5- to 10-membered bicyclic heterocycle, 5- to 10-membered aromatic heterocycle, and C₁-C₈ alkylenedioxy)).

3. The arylmethylene urea derivative or the pharmaceutically acceptable salt thereof according to claim 1 or 2, wherein in said Formula (I),
Ar¹ is a pyrazole represented by the Formula (XII): 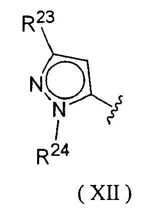 [wherein
R²³ is t-butyl, t-pentyl, neopentyl, cyclopropyl, cyclopentyl, furyl, or 2-methylfuryl;
R²⁴ is phenyl or pyridyl
(wherein each of these optionally has one or more substituents independently selected from the following:
hydroxy, amino, nitro, fluoro, chloro, bromo, iodo, carboxy, benzyloxy, C₁-C₈ alkyl, C₁-C₈ alkoxy, C₁-C₈ alkoxycarbonyl, C₁-C₈ hydroxyalkyloxy, C₁-C₁₆ alkylamino, C₁-C₈ alkylsulfanyl, C₁-C₈ alkylsulfonyl, trifluoromethanesulfonyl ,and sulfonamide alkylated with C₁-C₈ alkyl)]; and
X is -O- or -CH₂-.

4. The arylmethylene urea derivative or the pharmaceutically acceptable salt thereof according to claim 1, wherein in said Formula (I),
said urea alkylated with C₁-C₈ alkyl is (C₁-C₈ alkyl)-NHCONH-;
said one or more substituents which each of said one or more substituents optionally further has, the latter one or more substituents being those which said Ar¹ optionally has, are independently selected from:
cyano, hydroxy, amino, mercapto, nitro, fluoro, chloro, bromo, iodo, phenyl, benzyl, carboxy, carbamoyl, thiocarbamoyl, formyl, C₁-C₈ alkyl, C₂-C₈ alkenyl, C₂-C₈ alkynyl, C₁-C₈ hydroxyalkyl, C₁-C₈ alkoxy, C₁-C₁₆ alkylamino, C₁-C₈ alkylsulfanyl, and 5- to 10-membered aromatic heterocycle;
said one or more substituents which each of said one or more substituents optionally further has, the latter one or more substituents being those which said R¹ optionally has, are independently selected from:
cyano, hydroxy, amino, mercapto, nitro, fluoro, chloro, bromo, iodo, phenyl, benzyl, carboxy, carbamoyl, thiocarbamoyl, formyl, formylamino, aminosulfonyl, indan, C₁-C₈ alkyl, C₁-C₈ hydroxyalkyl, C₁-C₈ alkoxy, C₁-C₁₆ alkylamino, C₁-C₁₆ hydroxyalkylamino, carbamate alkylated with C₁-C₈ alkyl, 5- to 10-membered monocyclic heterocycle, 5- to 10-membered bicyclic heterocycle and 5- to 10-membered aromatic heterocycle.

5. The arylmethylene urea derivative or the pharmaceutically acceptable salt thereof according to claim 4, wherein in said Formula (I),
Ar¹ is a pyrazole represented by Formula (XII): 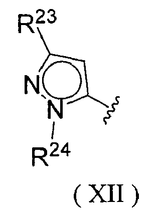 [wherein
R²³ is t-butyl, t-pentyl, neopentyl, cyclopropyl, cyclopentyl, furyl, or 2-methylfuryl;
R²⁴ is phenyl or pyridyl
(each of these optionally has one or more substituents independently selected from the following:
hydroxy, amino, nitro, fluoro, chloro, bromo, iodo, carboxy, C₁-C₈ alkyl, C₁-C₈
alkoxy, C₁-C₁₆ alkylamino, and C₁-C₈ alkylsulfanyl)]; and
X is -O- or -CH₂-.

6. The arylmethylene urea derivative or the pharmaceutically acceptable salt thereof according to claim 4 or 5, wherein in said Formula (I),
Ar¹ is a pyrazole represented by Formula (XII)
(wherein R²³ is t-butyl; and R²⁴ is 4-methylphenyl or 4-methyl-3-hydroxyphenyl); and
X is -O-.

7. The arylmethylene urea derivative or the pharmaceutically acceptable salt thereof according to any one of claims 1 to 6, wherein in said Formula (I),
Ar² is represented by Formula (VIII) or (IX): 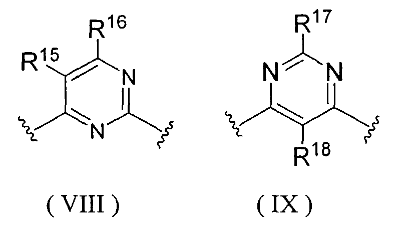 (wherein R¹⁵ to R¹⁸ each independently represents hydrogen or methyl).

8. The arylmethylene urea derivative or the pharmaceutically acceptable salt thereof according to any one of claims 1 to 7, wherein in said Formula (I),
A is represented by said Formula (II)
(wherein R² and R⁴ are hydrogen;
R³ and R⁵ are each independently selected from hydrogen, fluoro, chloro, methyl, and trifluoromethyl).

9. The arylmethylene urea derivative or the pharmaceutically acceptable salt thereof according to any one of claims 1 to 8, wherein in said Formula (I), R¹ is chloro, methylsulfanyl, morpholino, 3-carbamoylpiperidino, 4-carbamoylpiperidino, 4-acetylpiperazino, 3-(dimethylamino)propylamino, 3-(diethylamino)propylamino, 3-(2-oxo-pyrrolidin-1-yl)propylamino, 4-methylpiperazino, 4-(2-methoxyethyl)piperazino, or 2-(1-methylpyrrolidin-2-yl)ethylamino.

10. A pharmaceutical comprising said arylmethylene urea derivative or the pharmaceutically acceptable salt thereof according to any one of claims 1 to 9.

11. A therapeutic or prophylactic agent for inflammatory bowel disease, comprising said arylmethylene urea derivative or the pharmaceutically acceptable salt thereof according to any one of claims 1 to 9.

12. A therapeutic or prophylactic agent for overactive bladder, comprising said arylmethylene urea derivative or the pharmaceutically acceptable salt thereof according to any one of claims 1 to 9.

13. A method of therapy or prophylaxis for inflammatory bowel disease, said method comprising administering an effective amount of said arylmethylene urea derivative or the pharmaceutically acceptable salt thereof according to any one of claims 1 to 9 to a patient with inflammatory bowel disease or to a human in need thereof.

14. A method of therapy or prophylaxis for overactive bladder, said method comprising administering an effective amount of said arylmethylene urea derivative or the pharmaceutically acceptable salt thereof according to any one of claims 1 to 9 to a patient with overactive bladder or to a human in need thereof.

15. Use of said arylmethylene urea derivative or the pharmaceutically acceptable salt thereof according to any one of claims 1 to 9 for the production of a therapeutic or prophylactic agent for inflammatory bowel disease.

16. Use of said arylmethylene urea derivative or the pharmaceutically acceptable salt thereof according to any one of claims 1 to 9 for the production of a therapeutic or prophylactic agent for overactive bladder.
